(19) Europäisches Patentamt
European Patent Office
Office européen des brevets

(11) EP 1 606 282 B1

(12) EUROPEAN PATENT SPECIFICATION

(45) Date of publication and mention of the grant of the patent:
**12.11.2008 Bulletin 2008/46**

(21) Application number: **04713803.7**

(22) Date of filing: **23.02.2004**

(51) Int Cl.:
*C07D 401/14* (2006.01) *C07D 401/12* (2006.01)
*C07D 401/04* (2006.01) *C07D 417/14* (2006.01)
*C07D 211/62* (2006.01) *C07D 211/14* (2006.01)
*C07D 413/12* (2006.01) *C07D 413/04* (2006.01)
*C07D 401/10* (2006.01) *A61K 31/435* (2006.01)
*A61P 3/00* (2006.01)

(86) International application number:
**PCT/US2004/005555**

(87) International publication number:
**WO 2004/076413 (10.09.2004 Gazette 2004/37)**

(54) **PHENYL- AND PYRIDYLPIPEREIDINYE-DERIVATIVES AS MODULATORS OF GLUCOSE METABOLISM**

PHENYL- UND PYRIDYLPIPERIDINDERIVATE ALS MODULATOREN DES GLUCOSE-METABOLISMUS

DERIVES D'ARYLE ET HETEROARYLE SUSBTITUES TENANT LIEU DE MODULATEURS DU METABOLISME DU GLUCOSE ET PROPHYLAXIE ET TRAITEMENT DE TROUBLES ASSOCIES

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **24.02.2003 US 449788 P**

(43) Date of publication of application:
**21.12.2005 Bulletin 2005/51**

(73) Proprietor: **Arena Pharmaceuticals, Inc.**
**San Diego, CA 92121-3223 (US)**

(72) Inventors:
- **JONES, Robert, M.**
  **San Diego, CA 92130 (US)**
- **SEMPLE, Graeme**
  **San Diego, CA 92127 (US)**
- **CHOI, Jin Sun Karoline**
  **San Diego, CA 92131 (US)**
- **XIONG, Yifeng**
  **San Diego, CA 92130 (US)**
- **FIORAVANTI, Beatriz**
  **Tucson, Arizona 85704 (US)**

(74) Representative: **Wytenburg, Wilhelmus Johannes et al**
**Mewburn Ellis LLP**
**York House**
**23 Kingsway**
**London WC2B 6HP (GB)**

(56) References cited:
**EP-A- 0 604 800** **EP-A- 0 857 483**
**WO-A-00/35875** **US-B1- 6 239 126**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 606 282 B1

SEQUENCE LISTING

<110> Arena Pharmaceuticals, Inc.
Jones, Robert M.
Semple, Graeme
Choi, Jin Sun Karoline
Xiong, Yifeng
Fioravanti, Beatriz

<120> SUBSTITUTED ARYL AND HETEROARYL DERIVATIVES AS MODULATORS OF GLUCOSE METABOLISM AND THE PROPHYLAXIS AND TREATMENT OF DISORDERS THEREOF

<130> 53.w01

<150> 60/449,788
<151> 2003-02-24

<160> 7

<170> PatentIn version 3.2

<210> 1
<211> 1191
<212> DNA
<213> Homo sapien

<400> 1

atgtacccat acgacgtccc agactacgct ggaagcttgg aatcatcttt ctcatttgga 60
gtgatccttg ctgtcctggc ctccctcatc attgctacta acacactagt ggctgtggct 120
gtgctgctgt tgatccacaa gaatgatggt gtcagtctct gcttcacctt gaatctggct 180
gtggctgaca ccttgattgg tgtggccatc tctggcctac tcacagacca gctctccagc 240
ccttctcggc ccacacagaa gaccctgtgc agcctgcgga tggcatttgt cacttcctcc 300
gcagctgcct ctgtcctcac ggtcatgctg atcacctttg acaggtacct tgccatcaag 360
cagcccttcc gctacttgaa gatcatgagt gggttcgtgg ccggggcctg cattgccggg 420
ctgtggttag tgtcttacct cattggcttc ctcccactcg gaatccccat gttccagcag 480
actgcctaca aagggcagtg cagcttcttt gctgtatttc accctcactt cgtgctgacc 540
ctctcctgcg ttggcttctt cccagccatg ctcctctttg tcttcttcta ctgcgacatg 600
ctcaagattg cctccatgca cagccagcag attcgaaaga tggaacatgc aggagccatg 660
gctggaggtt atcgatcccc acggactccc agcgacttca aagctctccg tactgtgtct 720
gttctcattg ggagctttgc tctatcctgg accccttcc ttatcactgg cattgtgcag 780
gtggcctgcc aggagtgtca cctctaccta gtgctggaac ggtacctgtg gctgctcggc 840
gtgggcaact ccctgctcaa cccactcatc tatgcctatt ggcagaagga ggtgcgactg 900
cagctctacc acatggccct aggagtgaag aaggtgctca cctcattcct cctctttctc 960
tcggccagga attgtggccc agagaggccc agggaaagtt cctgtcacat cgtcactatc 1020
tccagctcag agtttgatgg cgaattcgga tccaagggca attctgcaga tatccagcac 1080
agtggcggcc gctcgagtct agagggcccg cggttcgaag gtaagcctat ccctaaccct 1140
ctcctcggtc tcgattctac gcgtaccggt catcatcacc atcaccattg a 1191

<210> 2
<211> 396
<212> PRT
<213> Homo sapien

<400> 2

Met Tyr Pro Tyr Asp Val Pro Asp Tyr Ala Gly Ser Leu Glu Ser Ser
1 5 10 15

Phe Ser Phe Gly Val Ile Leu Ala Val Leu Ala Ser Leu Ile Ile Ala
20 25 30

Thr Asn Thr Leu Val Ala Val Ala Val Leu Leu Leu Ile His Lys Asn
35 40 45

Asp Gly Val Ser Leu Cys Phe Thr Leu Asn Leu Ala Val Ala Asp Thr
50 55 60

Leu Ile Gly Val Ala Ile Ser Gly Leu Leu Thr Asp Gln Leu Ser Ser
65 70 75 80

Pro Ser Arg Pro Thr Gln Lys Thr Leu Cys Ser Leu Arg Met Ala Phe
85 90 95

Val Thr Ser Ser Ala Ala Ala Ser Val Leu Thr Val Met Leu Ile Thr
100 105 110

Phe Asp Arg Tyr Leu Ala Ile Lys Gln Pro Phe Arg Tyr Leu Lys Ile
115 120 125

Met Ser Gly Phe Val Ala Gly Ala Cys Ile Ala Gly Leu Trp Leu Val
130 135 140

Ser Tyr Leu Ile Gly Phe Leu Pro Leu Gly Ile Pro Met Phe Gln Gln
145 150 155 160

Thr Ala Tyr Lys Gly Gln Cys Ser Phe Phe Ala Val Phe His Pro His
165 170 175

Phe Val Leu Thr Leu Ser Cys Val Gly Phe Phe Pro Ala Met Leu Leu
180 185 190

Phe Val Phe Phe Tyr Cys Asp Met Leu Lys Ile Ala Ser Met His Ser
195 200 205

Gln Gln Ile Arg Lys Met Glu His Ala Gly Ala Met Ala Gly Gly Tyr
210 215 220

Arg Ser Pro Arg Thr Pro Ser Asp Phe Lys Ala Leu Arg Thr Val Ser
225 230 235 240

Val Leu Ile Gly Ser Phe Ala Leu Ser Trp Thr Pro Phe Leu Ile Thr
245 250 255

Gly Ile Val Gln Val Ala Cys Gln Glu Cys His Leu Tyr Leu Val Leu
260 265 270

Glu Arg Tyr Leu Trp Leu Leu Gly Val Gly Asn Ser Leu Leu Asn Pro
275 280 285

Leu Ile Tyr Ala Tyr Trp Gln Lys Glu Val Arg Leu Gln Leu Tyr His
290 295 300

Met Ala Leu Gly Val Lys Lys Val Leu Thr Ser Phe Leu Leu Phe Leu
305 310 315 320

Ser Ala Arg Asn Cys Gly Pro Glu Arg Pro Arg Glu Ser Ser Cys His
325 330 335

Ile Val Thr Ile Ser Ser Ser Glu Phe Asp Gly Glu Phe Gly Ser Lys
340 345 350

Gly Asn Ser Ala Asp Ile Gln His Ser Gly Gly Arg Ser Ser Leu Glu
355 360 365

Gly Pro Arg Phe Glu Gly Lys Pro Ile Pro Asn Pro Leu Leu Gly Leu
370 375 380

Asp Ser Thr Arg Thr Gly His His His His His His
385 390 395

<210> 3
<211> 24
<212> DNA
<213> Artificial

<220>
<223> Homo sapien Primer

<400> 3
cattgccggg ctgtggttag tgtc 24

<210> 4
<211> 24
<212> DNA
<213> Artificial

<220>
<223> Homo sapien Primer

<400> 4
ggcatagatg agtgggttga gcag 24

<210> 5
<211> 22
<212> DNA

<213> Artificial

<220>
<223> Rat Primer

<400> 5
catgggccct gcaccttctt tg 22

<210> 6
<211> 24
<212> DNA
<213> Artificial

<220>
<223> Rat Primer

<400> 6
gctccggatg gctgatgata gtga 24

<210> 7
<211> 23
<212> PRT
<213> Artificial

<220>
<223> Novel Sequence

<400> 7

```
Arg Gly Pro Glu Arg Thr Arg Glu Ser Ala Tyr His Ile Val Thr Ile
1               5                   10                  15

Ser His Pro Glu Leu Asp Gly
            20
```

## Description

## FIELD OF THE INVENTION

The present invention relates to certain substituted aryl and heteroaryl derivatives that are modulators of glucose metabolism. Accordingly, compounds of the present invention are useful in the prophylaxis or treatment of metabolic disorders and complications thereof, such as, diabetes and obesity.

## BACKGROUND OF THE INVENTION

Diabetes mellitus is a serious disease afflicting over 100 million people worldwide. In the United States, there are more than 12 million diabetics, with 600,000 new cases diagnosed each year.

Diabetes mellitus is a diagnostic term for a group of disorders characterized by abnormal glucose homeostasis resulting in elevated blood sugar. There are many types of diabetes, but the two most common are Type I (also referred to as insulin-dependent diabetes mellitus or IDDM) and Type II (also referred to as non-insulin-dependent diabetes mellitus or NIDDM).

The etiology of the different types of diabetes is not the same; however, everyone with diabetes has two things in common: overproduction of glucose by the liver and little or no ability to move glucose out of the blood into the cells where it becomes the body's primary fuel.

People who do not have diabetes rely on insulin, a hormone made in the pancreas, to move glucose from the blood into the cells of the body. However, people who have diabetes either don't produce insulin or can't efficiently use the insulin they produce; therefore, they can't move glucose into their cells. Glucose accumulates in the blood creating a condition called hyperglycemia, and over time, can cause serious health problems.

Diabetes is a syndrome with interrelated metabolic, vascular, and neuropathic components. The metabolic syndrome, generally characterized by hyperglycemia, comprises alterations in carbohydrate, fat and protein metabolism caused by absent or markedly reduced insulin secretion and/or ineffective insulin action. The vascular syndrome consists of abnormalities in the blood vessels leading to cardiovascular, retinal and renal complications. Abnormalities in the peripheral and autonomic nervous systems are also part of the diabetic syndrome.

People with IDDM, which accounts for about 5% to 10% of those who have diabetes, don't produce insulin and therefore must inject insulin to keep their blood glucose levels normal. IDDM is characterized by low or undetectable levels of endogenous insulin production caused by destruction of the insulin-producing β cells of the pancreas, the characteristic that most readily distinguishes IDDM from NIDDM. IDDM, once termed juvenile-onset diabetes, strikes young and older adults alike.

Approximately 90 to 95% of people with diabetes have Type II (or NIDDM). NIDDM subjects produce insulin, but the cells in their bodies are insulin resistant: the cells don't respond properly to the hormone, so glucose accumulates in their blood. NIDDM is characterized by a relative disparity between endogenous insulin production and insulin requirements, leading to elevated blood glucose levels. In contrast to IDDM, there is always some endogenous insulin production in NIDDM; many NIDDM patients have normal or even elevated blood insulin levels, while other NIDDM patients have inadequate insulin production (Rotwein, R. et al. N. Engl. J. Med. 308, 65-71 (1983)). Most people diagnosed with NIDDM are age 30 or older, and half of all new cases are age 55 and older. Compared with whites and Asians, NIDDM is more common among Native Americans, African-Americans, Latinos, and Hispanics. In addition, the onset can be insidious or even clinically inapparent, making diagnosis difficult

The primary pathogenic lesion on NIDDM has remained elusive. Many have suggested that primary insulin resistance of the peripheral tissues is the initial event Genetic epidemiological studies have supported this view. Similarly, insulin secretion abnormalities have been argued as the primary defect in NIDDM. It is likely that both phenomena are important contributors to the disease process (Rimoin, D. L., et al. Emery and Rimoin's Principles and Practice of Medical Genetics 3rd Ed. 1:1401-1402 (1996)).

Many people with NIDDM have sedentery lifestyles and are obese; they weigh approximately 20% more than the recommended weight for their height and build. Furthermore, obesity is characterized by hyperinsulinemia and insulin resistance, a feature shared with NIDDM, hypertension and atherosclerosis.

Obesity and diabetes are among the most common human health problems in industrialized societies. In industrialized countries a third of the population is at least 20% overweight. In the United States, the percentage of obese people has increased from 25% at the end of the 1970s, to 33% at the beginning the 1990s. Obesity is one of the most important risk factors for NIDDM. Definitions of obesity differ, but in general, a subject weighing at least 20% more than the recommended weight for his/her height and build is considered obese. The risk of developing NIDDM is tripled in subjects 30% overweight, and three-quarters with NIDDM are overweight.

Obesity, which is the result of an imbalance between caloric intake and energy expenditure, is highly correlated with insulin resistance and diabetes in experimental animals and human. However, the molecular mechanisms that are

inv olved in obesity-diabetes syndromes are not clear. During early development of obesity, increases insulin secretion balances insulin resistance and protects patients from hyperglycemia (Le Stunff, et al. Diabetes 43, 696-702 (1989)). However, after several decades, β cell function deteriorates and non-insulin-dependent diabetes develops in about 20% of the obese population (Pederson, P. Diab. Metab. Rev. 5, 505-509 (1989)) and (Brancati, F. L., et al., Arch. Intern. Med. 159, 957-963 (1999)). Given its high prevalence in modem societies, obesity has thus become the leading risk factor for NIDDM (Hill, J. O., et al., Science 280, 1371-1374 (1998)). However, the factors which predispose a fraction of patients to alteration of insulin secretion in response to fat accumulation remain unknown.

Whether someone is classified as overweight or obese is generally determined on the basis of their body mass index (BMI) which is calculated by dividing body weight (kg) by height squared ($m^2$). Thus, the units of BMI are $kg/m^2$ and it is possible to calculate the BMI range associated with minimum mortality in each decade of life. Overweight is defined as a BMI in the range 25-30 $kg/m^2$, and obesity as a BMI greater than 30 $kg/m^2$ (see TABLE below). There are problems with this definition in that it does not take into account the proportion of body mass that is muscle in relation to fat (adipose tissue). To account for this, obesity can also be defined on the basis ofbody fat content: greater than 25% and 30% in males and females, respectively.

**CLASSIFICATION OF WEIGHT BY BODY MASS INDEX (BMI)**

| BMI | CLASSIFICATION |
|---|---|
| < 18.5 | Underweight |
| 18.5-24.9 | Normal |
| 25.0-29.9 | Overweight |
| 30.0-34.9 | Obesity (Class I) |
| 35.0-39.9 | Obesity (Class II) |
| >40 | Extreme Obesity (Class III) |

As the BMI increases there is an increased risk of death from a variety of causes that is independent of other risk factors. The most common diseases with obesity are cardiovascular disease (particularly hypertension), diabetes (obesity aggravates the development of diabetes), gall bladder disease (particularly cancer) and diseases of reproduction. Research has shown that even a modest reduction in body weight can correspond to a significant reduction in the risk of developing coronary heart disease.

Compounds marketed as anti-obesity agents include Orlistat (XENICAL™) and Sibutramine. Orlistat (a lipase inhibitor) inhibits fat absorption directly and tends to produce a high incidence of unpleasant (though relatively harmless) side-effects such as diarrhea. Sibutramine (a mixed 5-HT/noradrenaline reuptake inhibitor) can increase blood pressure and heart rate in some patients. The serotonin releaser/reuptake inhibitors fenfluramine (Pondimin™) and dexfenfluramine (Redux™) have been reported to decrease food intake and body weight over a prolonged period (greater than 6 months). However, both products were withdrawn after reports of preliminary evidence of heart valve abnormalities associated with their use. Accordingly, there is a need for the development of a safer anti-obesity agent.

Obesity considerably increases the risk of developing cardiovascular diseases as well. Coronary insufficiency, atheromatous disease, and cardiac insufficiency are at the forefront of the cardiovascular complication induced by obesity. It is estimated that if the entire population had an ideal weight, the risk of coronary insufficiency would decrease by 25% and the risk of cardiac insufficiency and of cerebral vascular accidents by 35%. The incidence of coronary diseases is doubled in subjects less than 50 years of age who are 30% overweight. The diabetes patient faces a 30% reduced lifespan. After age 45, people with diabetes are about three times more likely than people without diabetes to have significant heart disease and up to five times more likely to have a stroke. These findings emphasize the inter-relations between risks factors for NIDDM and coronary heart disease and the potential value of an integrated approach to the prevention of these conditions based on the prevention of these conditions based on the prevention of obesity (Perry, I. J., et al., BMJ 310, 560-564 (1995)).

Diabetes has also been implicated in the development of kidney disease, eye diseases and nervous-system problems. Kidney disease, also called nephropathy, occurs when the kidney's "filter mechanism" is damaged and protein leaks into urine in excessive amounts and eventually the kidney fails. Diabetes is also a leading cause of damage to the retina at the back of the eye and increases risk of cataracts and glaucoma. Finally, diabetes is associated with nerve damage, especially in the legs and feet, which interferes with the ability to sense pain and contributes to serious infections. Taken together, diabetes complications are one of the nation's leading causes of death.

EP 0 857 483 describes certain pyrimidines of the following formula, wherein $R^1$, $R^2$, and $R^3$ are hydrogen, halogen, $C_{1-4}$alkoxy, $C_{1-4}$alkylthio, $C_{1-4}$alkyl, or $C_{3-5}$cylcoalkyl, and $R^4$ is hydrogen, formyl, acetyl, propionyl, or butyryl.

The compounds apparently inhibit glucose metabolism deterioration in mammals.

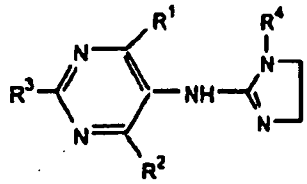

EP 0 604 800 describes certain carbonic acid compounds of the following formula. Apparently, the compounds are useful as fibrinogen receptor antagonists.

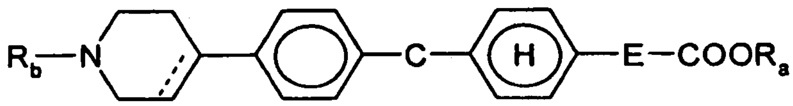

US Patent No 6,239,126 and WO 00/35875 describe certain arylpiperidine and aryl-1,2,5,6-tetrahydropyridine urea compounds of the following formula. Of the various substituents, only $R_0$, $R_1$, and $R_4$ may be aryl or heteroaryl. The compounds are apparently useful for the treatment of disorders of the central nervous system including anxiety, depression, panic, alcohol and drug addiction, sexual dysfunction, sleep disorder, migraine, obesity, cognitive disorders, and neurodegenerative diseases.

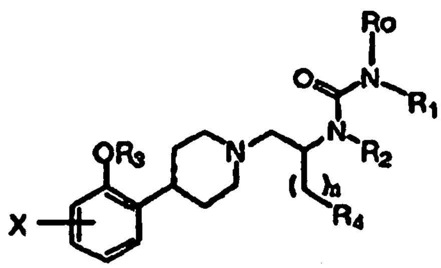

## SUMMARY OF THE INVENTION

The present invention is drawn to compounds, which bind to and modulate the activity of a GPCR referred to herein as RUP3, and uses thereof. The term RUP3, as used herein, includes the human sequences found in GeneBank accession number XM_066873, naturally-occurring allelic variants, mammalian orthologs, and recombinant mutants thereof. A preferred human **RUP3** for use in screening and testing of the compounds of the invention is provided in the nucleotide sequence of Seq. ID.No:1 and the corresponding amino acid sequence in Seq. ID.No:2.

One aspect of the present invention encompasses certain substituted aryl and heteroaryl derivatives as shown in Formula (**Ia**):

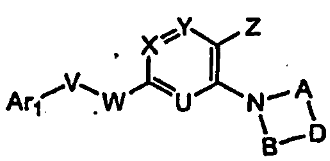

**(Ia)**

wherein:

A and B are both ethylene optionally substituted with 1 to 4 methyl groups;
U is N or $CR_1$;

D is $CR_2R_3$;
V is absent;
W is $-S(O)_2NR_4-$, $-NR_4-$, -O-, -S-, -S(O)-, $-S(O)_2-$; or W is absent;
X is $CR_5$;
Y is N or $CR_6$;
Z is nitro;
$Ar_1$ is aryl or heteroaryl optionally substituted with $R_9$, $R_{10}$, $R_{11}$, $R_{12}$ and $R_{13}$;
$R_1$ and $R_6$ are independently selected from the group consisting of H, $C_{1-5}$ acyloxy, $C_{2-6}$ alkenyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, amino, $C_{1-4}$ alkylamino, $C_{2-8}$ dialkylamino, carboxamide, cyano, $C_{3-6}$ cycloalkyl, $C_{2-6}$ dialkylcarboxamide, $C_{2-6}$ dialkylsulfonamide, halogen, $C_{1-4}$ haloalkoxy, $C_{1-4}$ haloalkyl, $C_{1-4}$ haloalkylsulfinyl, $C_{1-4}$ haloakylsulfonyl, $C_{1-4}$ haloalkylthio, hydroxyl and nitro;
$R_5$ is H or nitro;
$R_2$ is selected form the group consisting of H, $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfinyl, $C_{1-4}$ alkylsulfonyl, $C_{1-4}$ alkylthio, amino, carbo-$C_{1-6}$-alkoxy, carboxamide, carboxy, cyano, $C_{3-6}$-cycloalkyl, $C_{2-6}$ dialkylcarboxamide, $C_{1-4}$ haloalkoxy, $C_{1-4}$ haloalkyl, halogen, heteroaryl, hydroxyl and phenyl; and wherein $C_{1-8}$ alkyl, heteroaryl and phenyl are optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylamino, $C_{1-4}$ alkylcarboxamide, $C_{1-4}$ akylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl, $C_{3-6}$-cycloalkyl-$C_{1-3}$-heteroalkylene, $C_{2-8}$ dialkylamino, $C_{2-6}$ dialkylcarboxamide, $C_{1-4}$ dialkylthiocarboxamide, $C_{2-6}$ dialkylsulfonamide, $C_{1-4}$ alkyithioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, halogen, heterocyclic, hydroxyl, hydroxylamino and nitro; or
$R_2$ is $-Ar_2-Ar_3$ wherein $Ar_2$ and $Ar_3$ are independently aryl or heteroaryl optionally substituted with 1 to 5 substituents selected from the group consisting of H, $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfinyl, $C_{1-4}$ alkylsulfonyl, $C_{1-4}$ alkylthio, amino, carbo-$C_{1-6}$-alkoxy, carboxamide, carboxy, cyano, $C_{3-6}$-cycloalkyl, $C_{2-6}$ dialkylcarboxamide, $C_{1-4}$ haloalkoxy, $C_{1-4}$ haloalkyl, halogen, hydroxyl and nitro; or
$R_2$ is a group of Formula (B):

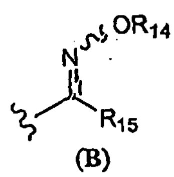

(B)

wherein:

$R_{14}$ is $C_{1-8}$ alkyl or $C_{3-6}$ cycloalkyl; and $R_{15}$ is F, Cl, Br or CN; or

$R_2$ is a group of Formula (C):

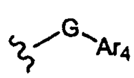

(C)

wherein:

G is C=O, $CR_{16}R_{17}$, O, S, S(O), $S(O)_2$; where $R_{16}$ and $R_{17}$ are independently H or $C_{1-8}$ alkyl; and
$Ar_4$ is phenyl or heteroaryl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$

haloalkylsulfonyl, $C_{1-4}$ haloalkylthio, halogen, heteroaryl, hydroxyl, hydroxylamino and nitro;

$R_3$ is H, $C_{1-8}$ alkyl, $C_{1-4}$ alkoxy or hydroxyl;
$R_4$ is H or $C_{1-8}$ alkyl;
$R_9$ is selected from the group consisting of $C_{1-5}$ acyl, $C_{1-5}$ acyloxy, $C_{2-6}$ alkenyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, amino, arylsulfonyl, carbo-$C_{1-6}$-alkoxy, carboxamide, carboxy, cyano, $C_{3-6}$ cycloalkyl, $C_{2-6}$ dialkylcarboxamide, halogen, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, heterocyclic, heterocyclicsulfonyl, heteroaryl, hydroxyl, nitro, $C_{4-7}$ oxo-cycloalkyl, phenoxy, phenyl, sulfonamide and sulfonic acid, and wherein $C_{1-5}$ acyl, $C_{1-4}$ alkoxy, $C_{1-8}$ alkyl, $C_{1-4}$ alkylsulfonamide, alkylsulfonyl, arylsulfonyl, heteroaryl, phenoxy and phenyl are optionally substituted with 1 to 5 substituents selected independently from the group consisting of $C_{1-5}$ acyl, $C_{1-5}$ acyloxy, $C_{2-6}$ alkenyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, carbo-$C_{1-6}$-alkoxy, carboxamide, carboxy, cyano, $C_{3-6}$ cycloalkyl, $C_{2-6}$ dialkylcarboxamide, halogen, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, heteroaryl, heterocyclic, hydroxyl, nitro and phenyl; or
$R_9$ is a group of Formula (D):

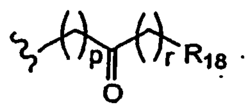

**(D)**

wherein:

"p" and "r" are independently 0, 1, 2 or 3; and
$R_{18}$ is H, $C_{1-6}$ acyl, $C_{2-6}$ alkenyl, $C_{1-8}$ alkyl, $C_{1-4}$ alkylcarboxamide, $C_{2-6}$ alkynyl, $C_{1-4}$ alkylsulfonamide, carbo-$C_{1-6}$-alkoxy, carboxamide, carboxy, cyano, $C_{3-6}$ cycloalkyl, $C_{2-6}$ dialkylcarboxamide, halogen, heteroaryl or phenyl, and wherein the heteroaryl or phenyl optionally substituted with 1 to 5 substituents selected independently from the group consisting of $C_{1-4}$ alkoxy, $C_{1-8}$ alkyl, amino, $C_{1-4}$ alkylamino, $C_{2-6}$ alkynyl, $C_{2-8}$ dialkylamino, halogen, $C_{1-4}$ haloalkoxy, $C_{1-4}$ haloalkyl and hydroxyl; and

$R_{10}$-$R_{13}$ are independently selected form the group consisting of $C_{1-5}$ acyl, $C_{1-5}$ acyloxy, $C_{2-6}$ alkenyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, amino, carbo-$C_{1-6}$-alkoxy, carboxamide, carboxy, cyano, $C_{3-6}$ cycloalkyl, $C_{2-6}$ dialkylcarboxamide, halogen, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, hydroxyl and nitro; or two adjacent $R_{10}$-$R_{11}$ groups form a 5, 6 or 7 membered cycloalkyl, cycloalkenyl or heterocyclic group with $Ar_1$ wherein the 5, 6 or 7 membered group is optionally substituted with halogen; or

a pharmaceutically acceptable salt, hydrate or solvate thereof.
One aspect of the present invention encompasses N-oxides of substituted aryl and heteroaryl derivatives of Formula (**Ia**).
Some embodiments of the present invention include a pharmaceutical composition comprising at least one compound of the present invention in combination with a pharmaceutically acceptable carrier.
Described herein are methods for prophylaxis or treatment of a metabolic disorder and/or complications thereof comprising administering to an individual in need of such prophylaxis or treatment a therapeutically effective amount of a compound of the present invention or a pharmaceutical composition thereof.
Described herein are methods for controlling or decreasing weight gain comprising administering to an individual in need of such controlling or decreaseing weight gain a therapeutically effective amount of a compound of the present invention or pharmaceutical composition thereof.
Described herein are methods of modulating a **RUP3** receptor comprising contacting the receptor with an effective amount of a compound of the ' present invention.
Described herein are methods of modulating a **RUP3** receptor in an individual comprising contacting the receptor with an effective amount of a compound of the present invention.
In some embodiments the compound is an agonist.
In some embodiments the compound is an inverse agonist.
Described herein are methods of modulating a RUP3 receptor in an individual comprising contacting the receptor

with a compound of the present invention wherein the modulation of the RUP3 receptor is prophylaxis or treatment of a metabolic disorder and/or complications thereof.

Described herein are methods of modulating a RUP3 receptor in an individual comprising contacting the receptor with a compound of the present invention wherein the modulation of the RUP3 receptor controls or reduces weight gain of the individual.

Some embodiments of the present invention include the uses of a compound of the present invention for production of a medicament for use in prophylaxis or treatment of a metabolic disorder.

Some embodiments of the present invention include the uses of a compound of the present invention for production of a medicament for use in controlling or decreasing weight gain in an individual.

Some embodiments of the present invention include compounds, as described herein, or a pharmaceutical composition thereof for use in a method of treatment of the human or animal body by therapy.

Some embodiments of the present invention include compounds, as described herein, or a pharmaceutical composition thereof for use in a method of prophylaxis or treatment of a metabolic disorder of the human or animal body by therapy.

Some embodiments of the present invention include methods of producing a pharmaceutical composition comprising admixing at least one compound of the present invention and a pharmaceutically acceptable carrier.

In some embodiments the metabolic disorder or complications thereof is type I, type II diabetes, inadequate glucose tolerance, insulin resistance, hyperglycemia, hyperlipidemia, hypertriglyceridemia, hypercholesterolemia, dyslipidemia or syndrome X. In some embodiments the metabolic disorder is type II diabetes. In some embodiments the metabolic disorder is hyperglycemia. In some embodiments the metabolic disorder is hyperlipidemia. In some embodiments the metabolic disorder is hypertriglyceridemia. In some embodiments the metabolic disorder is type I diabetes. In some embodiments the metabolic disorder is dyslipidemia. In some embodiments the metabolic disorder is syndrome X.

In some embodiments the individual is a mammal.

In some embodiments the mammal is a human.

## BRIEF DESCRIPTION OF THE DRAWINGS

**Figure 1A** shows RT-PCR analysis of **RUP3** expression in human tissues. A total of twenty-two (22) human tissues were analyzed.

**Figure 1B** shows the cDNA Dot-Blot analysis of **RUP 3** expression in human tissues.

**Figure 1C** shows analysis of **RUP3** by RT-PCR with isolated huyman pancreatic islets of Langerhans.

**Figure 1D** shows analysis af **RUP3** expression with cDNAs of rat origin by RT-PCR.

**Figure 2A** shows a polyclonal anti-**RUP3** antibody prepared in Rabbits.

**Figure 2B** shows the expression of **RUP3** in insulin-producing β cells of pancreatic islets.

**Figure 3** shows functional activities of **RUP3** *In vitro*.

**Figure 4** shows a RUP3 RNA blot.

**Figure 5** shows a representative scheme for the syntheses of compounds of the present invention.

## DEFINITIONS

The scientific literature that has evolved around receptors has adopted a number of terms to refer to ligands having various effects on receptors. For clarity and consistency, the following definitions will be used throughout this patent document

**AGONISTS** shall mean moieties that activate the intracellular response when they bind to the receptor, or enhance GTP binding to membranes.

**AMINO ACID ABBREVIATIONS** used herein are set out in Table 1:

| TABLE 1 | | |
|---|---|---|
| ALANINE | ALA | A |
| ARGININE | ARG | R |
| ASPARAGINE | ASN | N |
| ASPARTIC ACID | ASP | D |
| CYSTEINE | CYS | C |

(continued)

| TABLE 1 | | |
|---|---|---|
| GLUTAMIC ACID | GLU | E |
| GLUTAMINE | GLN | Q |
| GLYCINE | GLY | G |
| HISTIDINE | HIS | H |
| ISOLEUCINE | ILE | I |
| LEUCINE | LEU | L |
| LYSINE | LYS | K |
| METHIONINE | MET | . M |
| PHENYLALANINE | PHE | F |
| PROLINE | PRO | P |
| SERINE | SER | S |
| THREONINE | THR | T |
| TRYPTOPHAN | TRP | W |
| TYROSINE | TYR | Y |
| VALINE | VAL | V |

**CHEMICAL GROUP, MOIETY OR RADICAL:**

The term **"$C_{1-5}$ acyl"** denotes an alkyl radical attached to a carbonyl wherein the definition of alkyl has the same definition as described herein; some examples include formyl, acetyl, propionyl, butanoyl, *iso*-butanoyl, pentanoyl, hexanoyl, heptanoyl, and the like.

The term **"$C_{1-5}$ acyloxy"** denotes an acyl radical attached to an oxygen atom wherein acyl has the same definition has described herein; some examples include acetyloxy, propionyloxy, butanoyloxy, iso-butanoyloxy and the like.

The term **"$C_{2-6}$ alkenyl"** denotes a radical containing 2 to 6 carbons wherein at least one carbon-carbon double bond is present, some embodiments are 2 to 4 carbons, some embodiments are 2 to 3 carbons, and some embodiments have 2 carbons. Both E and Z isomers are embraced by the term **"alkenyl."** Furthermore, the term "alkenyl" includes di- and tri-alkenyls. Accordingly, if more than one double bond is present then the bonds may be all E or Z or a mixtures of *E* and *Z*. Examples of an alkenyl include vinyl, allyl, 2-butenyl, 3-butenyl, 2-pentenyl, 3-pentenyl, 4-pentenyl, 2-hexenyl, 3-hexenyl, 4-hexenyl, 5-hexanyl, 2,4-hexadienyl and the like.

The term **"$C_{1-4}$ alkoxy"** as used herein denotes a radical alkyl, as defined herein, attached directly to an oxygen atom. Example include for example methoxy, ethoxy, *n*-propoxy, *iso*-propoxy, n-butoxy, t-butoxy, iso-butoxy and the like.

The term **"alkyl"** denotes a straight or branched carbon radical, in some embodiments the term $C_{1-8}$ alkyl denotes an alkyl group consisting of 1 to 8 carbons, in some embodiments there are 1 to 6 carbons, in some embodiments there are 1 to 3 carbons, and in some embodiments there are 1 or 2 carbons. Examples of an alkyl include methyl, ethyl, n-propyl, iso-propyl, n-butyl, sec-butyl, t-butyl, amyl, t-amyl, n-pentyl and the like.

The term **"$C_{1-4}$ alkylcarboxamido"** denotes a single alkyl group attached to an amide, wherein alkyl has the same definition as found herein. The $C_{1-5}$ alkylcarboxamido may be represented by the following:

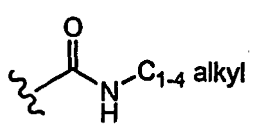

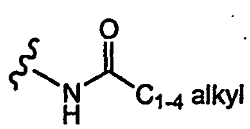

Examples include *N*-methylcarboxamide, *N*-ethylcarboxamide, *N*-(*iso*-propyl)carboxamide and the like.

The term **"$C_1$-$C_3$ alkylene"** refers to a divalent straight carbon group, such as, $-CH_2-$, $-CH_2CH_2-$, $-CH_2CH_2CH_2-$.

The term **"$C_{1-4}$ alkylsufinyl"** denotes an alkyl radical attached to a sulfoxide radical of the formula: -S(O)- wherein the alkyl radical has the same definition as described herein. Examples include methylsulfinyl, ethylsulfinyl and the like.

The term **"$C_{1-4}$ alkylsulfonamide"** refers to the groups

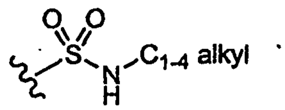

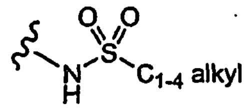

The term **"$C_{1-4}$ alkylsulfonyl"** denotes an alkyl radical attached to a sulfone , radical of the formula: $-S(O)_2-$ wherein the alkyl radical has the same definition as described herein. Examples include methylsulfonyl, ethylsulfonyl and the like.

The term **"$C_{1-4}$ alkylthio"** denotes an alkyl radical attached to a sulfide of the formula: -S- wherein the alkyl radical has the same definition as described herein. Examples include methylsulfanyl (i.e., $CH_3S$-), ethylsulfanyl, iso-propylsulfanyl and the like.

The term **"$C_{1-4}$ alkylthiocarboxamide"** denotes a thioamide of the following formulae:

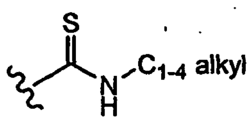

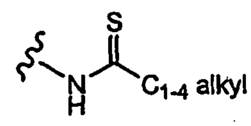

The term **"$C_{1-4}$ alkylthioureyl"** denotes the group of the formula: NC(S)N- wherein one are both of the nitrogens are substituted with the same or different alkyl group and alkyl has the same definition as described herein. Examples of an alkylthioureyl include, $CH_3NHC(O)NH$-, $NH_2C(O)NCH_3$-, $(CH_3)_2N(O)NH$-, $(CH_3)_2N(O)NH$-, $(CH_3)_2N(O)NCH_3$-, $CH_3CH_2NHC(O)NH$-, $CH_3CH_2NHC(O)NCH_3$-, and the like.

The term **"$C_{1-4}$ alkylureyl"** denotes the group of the formula: -NC(O)N- wherein one are both of the nitrogens are substituted with the same or different alkyl group wherein alkyl has the same definition as described herein. Examples of an alkylureyl include, $CH_3NHC(O)NH$-, $NH_2C(O)NCH_3$-, $(CH_3)_2N(O)NH$-, $(CH_3)_2N(O)NH$-, $(CH_3)_2N(O)NCH_3$-, $CH_3CH_2NHC(O)NH$-, $CH_3CH_2NHC(O)NCH_3$-, and the like.

The term **"$C_{2-6}$ alkynyl"** denotes a radical containing 2 to 6 carbons and at least one carbon-carbon triple bond, some embodiments are 2 to 4 carbons, some embodiments are 2 to 3 carbons, and some embodiments have 2 carbons. Examples of an alkynyl include ethynyl, ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like. The term **"alkynyl"** includes di- and triynes.

The term "amino" denotes the group $-NH_2$.

The term **"$C_{1-4}$ alkylamino"** denotes one alkyl radical attached to an amino radical wherein the alkyl radical has the same meaning as described herein. Some examples include methylamino, ethylamino, propylamino and the like.

The term **"aryl"** denotes an aromatic ring radical containing 6 to 10 ring carbons. Examples include phenyl and naphthyl.

The term **"arylalkyl"** defines a $C_1$-$C_4$ alkylene, such as $-CH_2-$, $-CH_2CH_2-$ and the like, which is further substituted with an aryl group. Examples of an "arylalkyl" include benzyl, phenethylene and the like.

The term **"arylcarboxamido"** denotes a single aryl group attached to the amine of an amide, wherein aryl has the same definition as found herein. The example is *N*-phenylcarboxamide.

The term **"arylureyl"** denotes the group -NC(O)N- where one of the nitrogens are substituted with an aryl.

The term **"benzyl"** denotes the group $-CH_2C_6H_5$.

The term **"carbo-$C_{1-6}$-alkoxy"** refers to an alkyl ester of a carboxylic acid, wherein the alkyl group is $C_{1-6}$. Examples include carbomethoxy, carboethoxy, carboisopropoxy and the like.

The term **"carboxamide"** refers to the group $-CONH_2$.

The term **"carboxy"** or **"carboxyl"** denotes the group $-CO_2H$; also referred to as a carboxylic acid.

The term "cyano" denotes the group -CN.

The term **"$C_{3-6}$ cycloalkenyl"** denotes a non-aromatic ring radical containing 3 to 6 ring carbons and at least one double bond; some embodiments contain 3 to 5 carbons; some embodiments contain 3 to 4 carbons. Examples include cyclopropenyl, cyclobutenyl, cyclopentenyl, cyclopentenyl, cyclohexenyl, and the like.

The term **"$C_{3-6}$ cycloalkyl"** denotes a saturated ring radical containing 3 to 6 carbons; some embodiments

contain 3 to 5 carbons; some embodiments contain 3 to 4 carbons. Examples include cyclopropyl, cyclobutyl, cyclopentyl, cyclopenyl, cyclohexyl, cycloheptyl and the like.

The term **"$C_{4-8}$ diacylamino"** denotes an amino group bonded with two acyl groups defined herein wherein the acyl groups may be the same or different, such as:

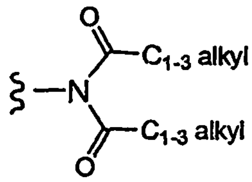

Represented dialkylamino groups include diacetylamino, dipropionylamino, acetylpropionylamino and the like.

The term **"$C_{2-6}$ dialkylamino"** denotes an amino substituted with two of the same or different alkyl radicals wherein alkyl radical has the same definition as described herein. Some examples include dimethylamino, methylethylamino, diethylamino and the like.

The term **"$C_{1-4}$ dialkylcarboxamido"** denotes two alkyl radicals, that are the same or different, attached to an amide group, wherein alkyl has the same definition as described herein. A $C_{1-4}$ dialkylcarboxamido may be represented by the following groups:

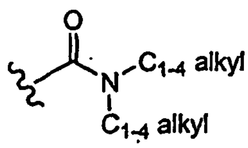

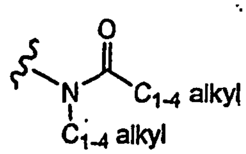

Examples of a dialkylcarboxamide include *N,N*-dimethylcarboxamide, *N*-methyl-*N*-ethylcarboxamide and the like.

The term **"$C_{2-6}$ dialkylsulfonamide"** refers to one of the following groups shown below:

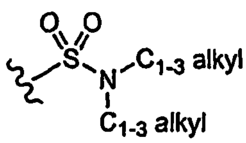

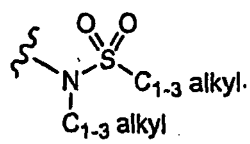

The term **"$C_{1-4}$ dialkylthiocarboxamido"** denotes two alkyl radicals, that are the same or different, attached to a thioamide group, wherein alkyl has the same definition as described herein. A $C_{1-4}$ dialkylthiocarboxamido may be represented by the following groups:

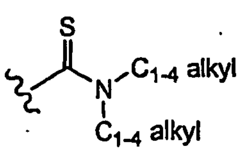

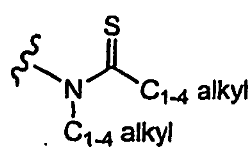

Examples of a dialkylthiocarboxamide include *N,N*-dimethylthiocarboxamide, *N*-methyl-*N*-ethylthiocarboxamide and the like.

The term **"$C_{1-4}$ dialkylsulfonylamino"** refers to an amino group bonded with two $C_{1-4}$ alkylsulfonyl groups as defined herein.

The term **"ethynylene"** refers to the carbon-carbon triple bond group as represented below:

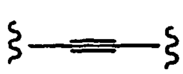

The term **"formyl"** refers to the group -CHO.

The term **"$C_{1-4}$ haloalkoxy"** denotes a haloalkyl, as defined herein, that is directly attached to an oxygen to form a difluoromethoxy, trifluoromethoxy, 2,2,2-trifluoroethoxy, pentafluoroethoxy and the like.

The term **"$C_{1-4}$ haloalkyl"** denotes an alkyl group, defined herein, wherein the alkyl is substituted with one halogen up to fully substituted represented by the formula $C_nF_{2n+1}$; when more than one halogen is present they may be the same or different and selected from F, Cl, Br or I. Examples include fluoromethyl, difluoromethyl, trifluoromethyl, chlorodifluoromethyl, 2,2,2-trifluoroethyl, pentafluoroethyl and the like.

The term **"$C_{1-4}$ haloalkylcarboxamide"** denotes an alkylcarboxamide group, defined herein, wherein the alkyl is substituted with one halogen up to fully substituted represented by the formula $C_nF_{2n+1}$ and "n" is 1, 2, 3 or 4. When more than one halogen is present they may be the same or different and selected from F, Cl, Br or I. Examples include 2-fluoroacetyl, 2,2-difluoroacetyl, 2,2,2-trifluoroacetyl, 2-chloro-2,2-difluoroacetyl, 3,3,3-trifluoropropionyl, 2,2,3,3,3-pentafluoropropionyl and the like.

The term **"$C_{1-4}$ haloalkylsulfinyl"** denotes a haloalkyl radical attached to a sulfoxide of the formula: -S(O)- wherein the alkyl radical has the same definition as described herein. Examples include trifluoromethylsulfinyl, 2,2,2-trifluoroethylsulfinyl, 2,2-difluoroethylsulfinyl and the like.

The term **"$C_{1-4}$ haloalkylsulfonyl"** denotes a haloalkyl attached to a sulfone of the formula: $-S(O)_2-$ wherein haloalkyl has the same definition as described herein. Examples include trifluoromethylsulfonyl, 2,2,2-trifluoroethylsulfonyl, 2,2-difluoroethylsulfonyl and the like.

The term **"$C_{1-4}$ haloalkylthio"** denotes an alkylthio radical substituted with one or more halogens. Examples include trifluoromethylthio, 1,1-difluoroethylthio, 2,2,2-trifluoroethylthio and the like.

The term **"halogen"** or **"halo"** denotes to a fluoro, chloro, bromo or iodo group.

The term **"$C_{1-2}$ heteroalkylene"** refers to a $C_{1-2}$ alkylene bonded to a heteroatom selected from O, S, S(O), $S(O)_2$ and NH. Some represented examples include the groups of the following formulae:

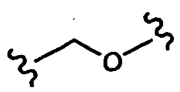 , 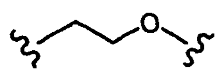 , 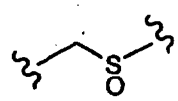 , 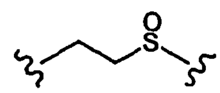 ,
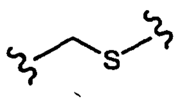 , 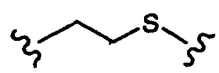 , 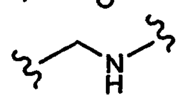 , 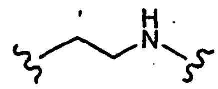 .

The term **"heteroaryl"** denotes an aromatic ring system that may be a single ring, two fused rings or three fused rings containing carbons and at least one ring heteroatom selected from O, S and N. Examples ofheteroaryl groups include, but not limited to, pyridyl, pyrazinyl, pyridazinyl, pyrimidinyl, triazinyl, quinoline, benzoxazole, benzothiazole, 1*H*-benzimidazole, isoquinoline, quinazoline, quinoxaline, pyridinone and the like.

The term **"heterocyclic"** denotes a non-aromatic carbon ring (i.e., cycloalkyl or cycloalkenyl as defined herein) wherein one, two or three ring carbons are replaced by one, two or three heteroatoms, such as, piperidinyl, morpholinyl, piperzinyl, pyrrolidinyl, and the like. Additional examples of heterocyclic groups are shown in Tables 2B, 2C, 2D, 2E, 2F and 2G, *infra*.

The term **"heterocycliccarboxamido"** denotes a heterocyclic group with a ring nitrogen where the ring nitrogen is bonded directly to the carbonyl forming an amide. Examples include:

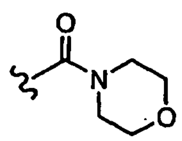 , 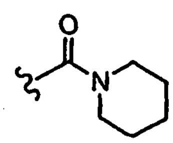 , 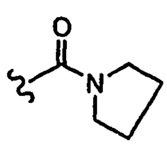

and the like.

The term **"heterocyclicsulfonyl"** denotes a heterocyclic group with a ring nitrogen where the ring nitrogen is bonded directly to an $SO_2$ group forming an sulfonamide. Examples include:

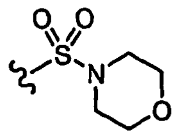, 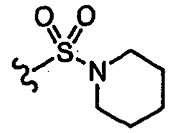, 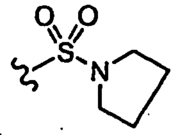

and the like.

The term **"hydroxyl"** refers to the group -OH.

The term **"hydroxylamino"** refers to the group -NHOH.

The term "nitro" refers to the group $-NO_2$.

The term **"$C_{4-7}$ oxo-cycloalkyl"** refers to a $C_{4-7}$ cycloalkyl, as defined herein, wherein one of the ring carbons is replaced with a carbonyl. Examples of $C_{4-7}$ oxo-cycloalkyl include but are not limited to: 2-oxo-cyclobutyl, 3-oxo-cyclobutyl, 3-oxo-cyclopentyl, 4-oxo-cyclohexyl, and the like and represented the following structures respectively:

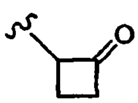, 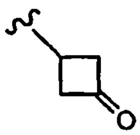, 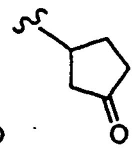, 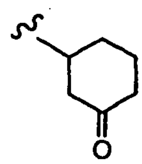, 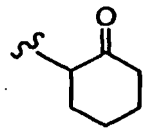 and 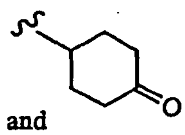

The term **"perfluoroalkyl"** denotes the group of the formula $-C_nF_{2n+1}$; stated differently, a perfluoroalkyl is an alkyl as defined herein herein wherein the alkyl is fully substituted with fluorine atoms and is therefore considered a subset of haloalkyl.
Examples of perfluoroalkyls include $CF_3$, $CF_2CF_3$, $CF_2CF_2CF_3$, $CF(CF_3)_2$, $CF_2CF_2CF_2CF_3$, $CF_2CF(CF_3)_2$, $CF(CF_3)CF_2CF_3$ and the like.

The term "phenoxy" refers to the group $C_6H_5O$-.

The term **"phenyl"** refers to the group $C_6H_5$-.

The term"**sulfonic acid"** refers to the group $-SO_3H$.

The term **"tetrazolyl"** refers to the five membered heteroaryl of the following formulae:

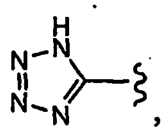, 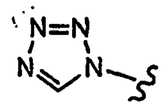.

In some embodiments, the tetrazolyl group is further substituted at either the 1 or 5 position resepectively.

The term **"thiol"** denotes the group -SH.

CODON shall mean a grouping of three nucleotides (or equivalents to nucleotides) which generally comprise a nucleoside (adenosine (A), guanosine (G), cytidine (C), uridine (U) and thymidine (T)) coupled to a phosphate group and which, when translated, encodes an amino acid.

**COMPOSITION** shall mean a material comprising at least two compounds or two components; for example, and not limitation, a Pharmaceutical Composition is a Composition.

**CONTACT** or **CONTACTING** shall mean bringing at least two moieties together, whether in an in vitro system or an in vivo system.

**IN NEED OF PROPHYLAXIS OR TREATMENT** as used herein refers to a judgment made by a caregiver (e.g. physician, nurse, nurse practitioner, etc. in the case of humans; veterinarian in the case of animals, including non-human mammals) that an individual or animal requires or will benefit from prophylaxis or treatment. This judgment is made based on a variety of factors that are in the realm of a caregiver's expertise, but that includes the knowledge that the individual or animal is ill, or will be ill, as the result of a disease, condition or disorder that is treatable by the compounds of the invention. In general, "in need of prophylaxis" refers to the judgment made by the caregiver that the individual will become ill. In this context, the compounds of the invention are used in a protective or preventive manner. However, "in need of treatment" refers to the judgment of the caregiver that the individual is already ill, therefore, the compounds of the present invention are used to alleviate, inhibit or ameliorate the disease, condition or disorder.

**INDIVIDUAL** as used herein refers to any animal, including mammals, preferably mice, rats, other rodents,

rabbits, dogs, cats, swine, cattle, sheep, horses, or primates, and most preferably humans.

**INHIBIT** or **INHIBITING,** in relationship to the term "response" shall mean that a response is decreased or prevented in the presence of a compound as opposed to in the absence of the compound.

**INVERSE AGONISTS** shall mean moieties that bind the endogenous form of the receptor or to the constitutively activated form of the receptor, and which inhibit the baseline intracellular response initiated by the active form of the receptor below the normal base level of activity which is observed in the absence of agonists or partial agonists, or decrease GTP binding to membranes. Preferably, the baseline intracellular response is inhibited in the presence of the inverse agonist by at least 30%, more preferably by at least 50%, and most preferably by at least 75%, as compared with the baseline response in the absence of the inverse agonist.

**LIGAND** shall mean an endogenous, naturally occurring molecule specific for an endogenous, naturally occurring receptor.

As used herein, the terms **MODULATE** or **MODULATING** shall mean to refer to an increase or decrease in the amount, quality, response or effect of a particular activity, function or molecule.

**PHARMACEUTICAL COMPOSITION** shall mean a composition comprising at least one active ingredient, whereby the composition is amenable to investigation for a specified, efficacious outcome in a mammal (for example, and not limitation, a human). Those of ordinary skill in the art will understand and appreciate the techniques appropriate for determining whether an active ingredient has a desired efficacious outcome based upon the needs of the artisan.

## DETAILED DESCRIPTION

One aspect of the present invention encompasses certain substituted aryl and heteroaryl derivatives as shown in Formula (**Ia**):

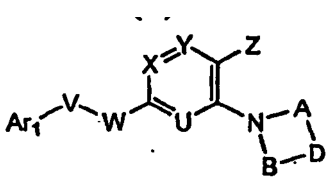

(Ia)

or a pharmaceutically acceptable salt, hydrate or solvate thereof, wherein A, B, D, V, W, X, Y, Z, U, and $Ar_1$ are as described herein, *supra* and *infra*.

One aspect of the present invention encompasses N-oxides of certain substituted aryl and heteroaryl derivatives of Formula (**Ia**).

One aspect of the present invention encompasses certain substituted aryl and heteroaryl derivatives as shown in Formula (**Ia**) wherein:

A and B are both ethylene optionally substituted with 1 to 4 methyl groups;
U is N or $CR_1$;
D is $CR_2R_3$;
V is absent;
W is $-S(O)_2NR_4-$, $-NR_4-$, -O- or absent;
X is $CR_5$;
Y is $CR_6$;
Z is nitro;
$Ar_1$ is aryl or heteroaryl optionally substituted with $R_9$, $R_{10}$, $R_{11}$, $R_{12}$ and $R_{13}$;
$R_1$ and $R_6$ are each independently selected from the group consisting ofH, halogen, and nitro;
$R_5$ is H or nitro;
$R_2$ is selected from the group consisting of $C_{1-5}$ acyl, $C_{1-8}$ alkyl, and heteroaryl; and wherein $C_{1-8}$ alkyl, and heteroaryl are optionally substituted with 1 to 5 substituents selected from the group consisting of $C_{1-4}$ alkoxy, $C_{1-8}$ alkyl, and halogen;
or
$R_2$ is a group of Formula (C), wherein G is S, S(O), $S(O)_2$; and $Ar_4$ is phenyl or heteroaryl optionally substituted with 1 to 5 substituents selected from the group consisting of $C_{1-4}$ alkoxy, $C_{1-8}$ alkyl, cyano, $C_{1-4}$ haloalkoxy, $C_{1-4}$ haloalkyl, and halogen;
$R_3$ is H;
$R_4$ is H or $C_{1-8}$ alkyl;

$R_9$ is selected from the group consisting of $C_{1-5}$ acyl, $C_{2-6}$ alkenyl, $C_{1-4}$ alkoxy, $C_{1-8}$ alkyl, $C_{1-4}$ alkylsulfonyl, amino, arylsulfonyl, carbo-$C_{1-6}$-alkoxy, carboxamide, carboxy, cyano, $C_{3-6}$ cycloalkyl, halogen, $C_{1-4}$ haloalkoxy, $C_{1-4}$ haloalkyl, heterocyclic, heteroaryl, hydroxyl, $C_{4-7}$ oxo-cycloalkyl, phenyl, and sulfonic acid, and wherein $C_{1-5}$ acyl, $C_{1-8}$ alkyl, arylsulfonyl, heteroaryl, and phenyl are optionally substituted with 1 to 5 substituents selected independently from the group consisting of $C_{1-4}$ alkoxy, $C_{1-8}$ alkyl, $C_{1-4}$ alkylsulfonyl, cyano, halogen, $C_{1-4}$ haloalkoxy, $C_{1-4}$ haloalkyl, heteroaryl, and hydroxyl; or

$R_9$ is a group of Formula (D) wherein "p" and "r" are independently 0, 1, 2 or 3; and $R_{18}$ is H, carbo-$C_{1-6}$-alkoxy, carboxy, heteroaryl or phenyl, and wherein the heteroaryl and phenyl are optionally substituted with 1 to 5 substituents selected independently from the group consisting of $C_{1-4}$ alkoxy, $C_{1-8}$ alkyl, halogen, $C_{1-4}$ haloalkoxy, and $C_{1-4}$ haloalkyl; and

$R_{10}$-$R_{13}$ are independently selected form the group consisting of $C_{1-4}$ alkoxy, $C_{1-8}$ alkyl, amino, cyano, halogen, $C_{1-4}$ haloalkoxy, $C_{1-4}$ haloalkyl, and hydroxyl; or

a pharmaceutically acceptable salt, hydrate or solvate thereof.

It is appreciated that certain features of the invention, which are, for clarity, described in the context of separate embodiments, may also be provided in combination in a single embodiment Conversely, various features of the invention which are, for brevity, described in the context of a single embodiment, may also be provided separately or in any suitable subcombination.

As used herein, "substituted" indicates that at least one hydrogen atom of the chemical group is replaced by a non-hydrogen substituents or group. When a chemical group herein is "substituted" it may have up to the full valance of substitution; for example, a methyl group can be substituted by 1, 2, or 3 substituents, a methylene group can be substituted by 1 or 2 substituents, a phenyl group can be substituted by 1, 2, 3, 4, or 5 substituents, and the like.

It is understood that certain groups used to describe compounds of the present invention contain a prefix designating the number of carbons in the particular group; for example, $C_{1-8}$ alkyl is understood to encompass a one (1) carbon alkyl (i.e., methyl) to eight (8) carbon alkyl groups.

One aspect of the present invention encompasses certain substituted aryl and heteroaryl derivatives as shown in Formula **(Ia)** wherein W is -$S(O)_2NR_4$- or -$NR_4$-. In some embodiments W is -$S(O)_2NR_4$- and compounds may be represented by Formula **(Ib)** as shown below:

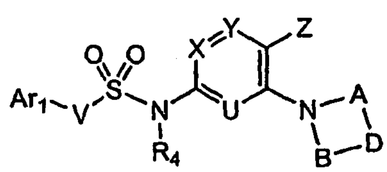

**(Ib)**

wherein each variable in Formula **(Ib)** has the same meaning as described herein. In some embodiments $R_4$ is H.

One aspect of the present invention encompasses certain substituted aryl and heteroaryl derivatives as shown in Formula **(Ia)** wherein W is -$NR_4$- and compounds may be represented by Formula **(Ic)** as shown below:

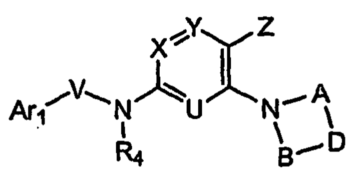

**(Ic)**

wherein each variable in Formula **(Ic)** has the same meaning as described herein. In some embodiments $R_4$ is H.

One aspect of the present invention encompasses certain substituted aryl and heteroaryl derivatives as shown in Formula **(Ia)** wherein W is -O-, an oxygen atom, and compounds may be represented by Formula **(Id)** as shown below:

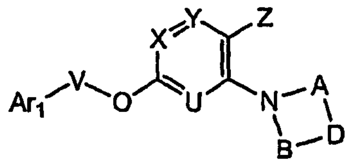

**(Id)**

wherein each variable in Formula **(Id)** has the same meaning as described herein.

One aspect of the present invention encompasses certain substituted aryl and heteroaryl derivatives as shown in Formula **(Ia)** wherein W is S, S(O) or $S(O)_2$ and compounds may be represented by Formulae **(Ie), (If)** and **(Ig)** respectively as shown below:

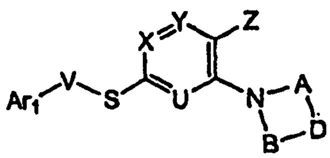

**(Ie)**

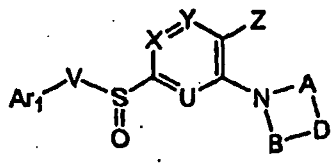

**(If)**

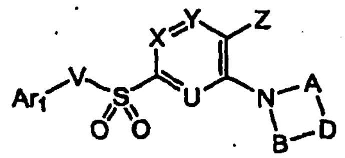

**(Ig)**

wherein each variable in Formula **(Ie), (If)** and **(Ig)** has the same meaning as described herein. In some embodiments W is -S-. In some embodiments W is -S(O)-. In some embodiments W is $-S(O)_2-$.

One aspect of the present invention encompasses certain substituted aryl and heteroaryl derivatives as shown in Formula **(Ia)** wherein W is absent In some embodiments, compounds may be represented by Formula **(Ih)** as shown below:

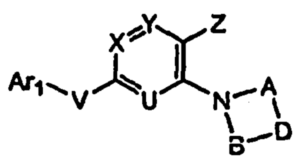

**(Ih)**

wherein each variable in Formula **(Ih)** has the same meaning as described herein.

In all embodiments, V is absent. In some embodiments compounds of the present invention are of Formula **(Ih)** wherein both V and W are absent and accordingly these compounds may be represented by Formula **(Ii)** as shown below:

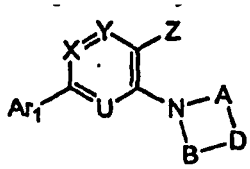

**(Ii)**

wherein each variable in Formula **(Ii)** has the same meaning as described herein.

In some embodiments A and B are both ethylene (i.e., $-CH_2CH_2-$) wherein A and B are optionally substituted with 1 to 4 methyl groups. In some embodiments A and B are both ethylene. Compounds in these embodiments may be represented by Formula **(Ip)** as shown below:

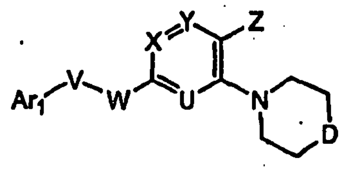

**(Ip)**

wherein each variable in Formula **(Ip)** has the same meaning as described herein. In some embodiments, compounds of the present invention are of Formula **(Ip)** wherein D is $CR_2R_3$. In some embodiments D is $-CHR_2-$.

In some embodiments D is $CR_2R_3$ wherein $R_2$ is selected from the group consisting of $C_{1-5}$ acyl, $C_{1-8}$ alkyl, and heteroaryl; and wherein $C_{1-8}$ alkyl, and heteroaryl are optionally substituted with 1 to 5 substituents selected from the group consisting of $C_{1-4}$ alkoxy, $C_{1-8}$ alkyl, and halogen.

In all embodiments D is $CR_2R_3$.

In some embodiments $R_2$ is selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfinyl, $C_{1-4}$ alkylsulfonyl, $C_{1-4}$ alkylthio, amino, carbo-$C_{1-6}$-alkoxy, carboxamide, carboxyl, $C_{3-6}$ cycloalkyl, $C_{1-4}$ haloalkoxy, $C_{1-4}$ haloalkyl, halogen and hydroxyl.

In some embodiments $R_2$ is selected from the group consisting of $C(O)CH_3$, $C(O)CH_2CH_3$, $C(O)CH_2CH_2CH_3$, $C(O)CH(CH_3)_2$, $C(O)CH_2CH_2CH_2CH_3$, $OC(O)CH_3$, $OC(O)CH_1CH_3$, $OC(O)CH_2CH_2CH_3$, $OCH_3$, $OCH_2CH_3$, $OCH_2CH_2CH_3$, $OCH(CH_3)_2$, $OCH_2(CH_2)_2CH_3$, $CH_3$, $CH_2CH_3$, $CH_2CH_2CH_3$, $CH(CH_3)_2$, $CH(CH_3)(CH_2CH_3)$, $CH_2(CH_2)_2CH_3$, $CH_2(CH_2)_3CH_3$, $C(O)NHCH_3$, $C(O)NHCH_2CH_3$, $C(O)NHCH_2CH_2CH_3$, $C(O)NHCH(CH_3)_2$, $C(O)NHCH_2(CH_2)_2CH$, $CO_2CH_3$, $CO_2CH_2CH_3CO_2CH_2CH_2CH_3$, $CO_2CH(CH_3)_2$ and $CO_2CH_2(CH_2)_2CH_3$.

In some embodiments $R_2$ is selected from the group consisting of $C(O)CH_3$, $C(O)CH_2CH_3$, $C(O)CH_2CH_2CH_3$, $C(O)CH(CH_3)_2$, $C(O)CH_2CH_2CH_2CH_3$, $CH_3$, $CH_2CH_3$, $CH_2CH_2CH_3$, $CH(CH_3)_2$, $CH(CH_3)(CH_2CH_3)$, $CH_2(CH_2)_2CH_3$, and $CH_2(CH_2)_3CH_3$.

In some embodiments $R_2$ is selected from the group consisting of $CO_2CH_3$, $CO_2CH_2CH_3$, $CO_2CH_2CH_2CH_3$, $CO_2CH(CH_3)_2$ and $CO_2CH_2(CH_2)_2CH_3$.

In some embodiments $R_2$ is selected from the group consisting of $SCH_3$, $SCH_2CH_3$, $SCH_2CH_2CH_3$, $SCH(CH_3)_2$, $SCH_2(CH_2)_2CH_3$, $S(O)CH_3$, $S(O)CH_2CH_3$, $S(O)CH_2CH_2CH_3$, $S(O)CH(CH_3)_2$, $S(O)CH_2(CH_2)_2CH_3$, $S(O)_2CH_3$, $S(O)_2CH_2CH_3$, $S(O)_2CH_2CH_2CH_3$, $S(O)_2CH(CH_3)_2$, $S(O)_2CH_2(CH_2)_2CH_3$, cyclopropyl, cyclobutyl, cyclopentyl, cyclohexyl, $OCF_3$, $OCHF_2$, $CF_3$, $CHF_2$ and F.

In some embodiments $R_2$ is $C_{1-8}$ alkyl optionally substituted with 1 to 5 substituents selected from the group consisting of $C_{1-4}$ alkoxy, $C_{1-8}$ alkyl, and halogen.

In some embodiments $R_2$ is selected from the group consisting of $CH_2OCH_3$, $CH_2CH_2OCH_3$, $CH_2OCH_2CH_3$, $CH_2OCH_2CH_2CH_3$, $CH_2CH_2OCH_2CH_3$, $CH_2CH_2OCH_2CH_2CH_3$, $CH_2OCH(CH_3)_2$, and $CH_2CH_2CH(CH_3)_2$.

In some embodiments $R_2$ is $C_{1-8}$ alkyl, heteroaryl or phenyl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylamino, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl-$C_{1-3}$-heteroalkylene, $C_{2-8}$ dialkylamino, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, halogen, heterocyclic, hydroxyl, hydroxylamino and nitro.

In some embodiments $R_2$ is selected from the group consisting of $CH_2OCH_3$, $CH_2CH_2OCH_3$, $CH_2OCH_2CH_3$, $CH_2OCH_2CH_2CH_3$, $CH_2CH_2OCH_2CH_3$, $CH_2CH_2OCH_2CH_2CH_3$, $CH_2OCH(CH_3)_2$, $CH_2OCH_2CH(CH_3)_2$, $CH_2CO_2H$, $CH_2CH_2CO_2H$, $CH_2OH$, $CH_2CH_2OH$ and $CH_2CH_2CH_2OH$.

In some embodiments $R_2$ is selected from the group consisting of $CH_2SCH_3$, $CH_2SCH_2CH_3$, $CH_2SCH_2CH_2CH_3$, $CH_2SCH(CH_3)_2$, $CH_2SCH_2(CH_2)_2CH_3$, $CH_2CH_2SCH_3$, $CH_2CH_2SCH_2CH_3$, $CH_2CH_2SCH_2CH_2CH_3$, $CH_2CH_2SCH(CH_3)_2$, $CH_2CH_2SCH_2(CH_2)_2CH_3$, $CH_2S(O)CH_3$, $CH_2S(O)CH_2CH_3$, $CH_2S(O)CH_2CH_2CH_3$, $CH_2S(O)CH(CH_3)_2$, $CH_2S(O)CH_2(CH_2)_2CH_3$, $CH_2CH_2S(O)CH_3$, $CH_2CH_2S(O)CH_2CH_3$, $CH_2CH_2S(O)CH_2CH_2CH_3$, $CH_2CH_2S(O)CH(CH_3)_2$, $CH_2CH_2S(O)CH_2(CH_2)_2CH_3$, $CH_2S(O)_2CH_3$, $CH_2S(O)_2CH_2CH_3$, $CH_2S(O)_2CH_2CH_2CH_3$, $CH_2S(O)_2CH(CH_3)_2$, $CH_2S(O)_2CH_2(CH_2)_2CH_3$, $CH_2CH_2S(O)_2CH_3$, $CH_2CH_2S(O)_2CH_2CH_3$, $CH_2CH_2S(O)_2CH_2CH_2CH_3$, $CH_2CH_2S(O)_2CH(CH_3)_2$ and $CH_2CH_2S(O)_2CH_2(CH_2)_2CH_3$.

In some embodiments $R_2$ is selected from the group consisting of $CH_2OCH_2$-cyclopropyl, $CH_2OCH_2$-cyclobutyl, $CH_2OCH_2$-cyclopentyl, $CH_2OCH_2$-cyclohexyl, $CH_2OCH_2CH_2$-cyclopropyl, $CH_2OCH_2CH_2$-cyclobutyl, $CH_2OCH_2CH_2$-cyclopentyl, $CH_2OCH_2CH_2$-cyclohexyl, $CH_2CH_2OCH_2$-cyclopropyl, $CH_2CH_2OCH_2$-cyclobutyl, $CH_2CH_2OCH_2$-cyclopentyl, $CH_2CH_2OCH_2$-cyclohexyl, $CH_2CH_2OCH_2CH_2$-cyclopropyl, $CH_2CH_2OCH_2CH_2$-cyclobutyl, $CH_2CH_2OCH_2CH_2$-cy-

clopentyl and $CH_2CH_2OCH_2CH_2$-cyclohexyl.

In some embodiments $R_2$ is selected from the group consisting of 1,2,4-oxadiazol-3-yl, 1,2,4-oxadiazol-5-yl, 1,3,4-xadiazol-2-yl, 1,2,4-triazol-5-yl and 1,2,4-triazol-1-yl, 3-methyl-1,2,4-oxadiazol-5-yl, 3-methyl-1,2,4-oxadiazol-5-yl, 3-ethyl-1,2,4-oxadiazol-5-yl, 3-ethyl-1,2,4-oxadiazol-5-yl, 5-methyl-1,3,4-oxadiazol-2-yl, 5-ethyl-l,3,4-oxadiazol-2-yl, 3-methyl-1,2,4-triazol-5-yl, 3-ethyl-1,2,4-triazol-5-yl, 3-methyl-1,2,4-triazol-1-yl, 3-ethyl-1,2,4-triazol-1-yl, 5-methyl-1,2,4-triazol-1-yl and 5-ethyl-1,2,4-triazol-1-yl.

In some embodiments $R_2$ is a 1,2,4-oxadiazolyl optionally substituted with $C_{1-8}$ alkyl.

In some embodiments $R_2$ is 3-methyl-1,2,4-oxadiazol-5-yl, 3-ethyl-1,2,4-oxadiazol-5-yl, 3-propyl-1,2,4-oxadiazol-5-yl, 3-isopropyl-1,2,4-oxadiazol-5-yl, 3-butyl-1,2,4-xadiazol-5-yl, and 3-isobutyl-1,2,4-oxadiazol-5-yl.

In some embodiments $R_2$ is a heteroaryl comprising 5-atoms in the aromatic ring and are represented by the following formulae as illustrated in TABLE 2A:

**TABLE 2A**

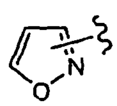, 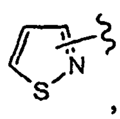, 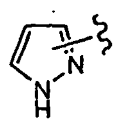, 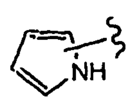, 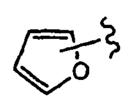,

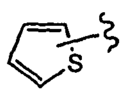, 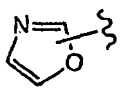, 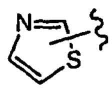, 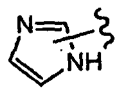, 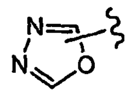,

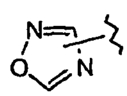, 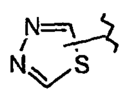, 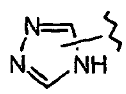, 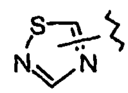,

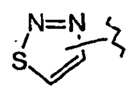 and 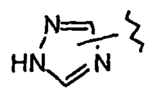

wherein the 5-membered heteroaryl is bonded at any available position of the ring, for example, a imidazolyl ring can be bonded at one of the ring nitrogens (i.e., imidazol-1-yl group) or at one of the ring carbons (i.e., imidazol-2-yl, imidazol-4-yl or imiadazol-5-yl group).

In some embodiments $R_2$ is a 5-membered heteroaryl optionally substituted with 1 to 4 substituents selected from the group consisting of $C_{1-5}$ acyl, $C_{1-5}$ acyloxy, $C_{1-4}$ alkoxy, $C_{1-8}$ alkyl, $C_{1-4}$ alkylamino, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloakl-$C_{1-3}$-heteroalkylene, $C_{2-8}$ dialkylamino, $C_{2-6}$ diakylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, halogen, heterocyclic, hydroxyl, hydroxylamino and nitro.

In some embodiments $R_2$ is a heterocyclic represented, for example, by the formulae in TABLE 2B.

**TABLE 2B**

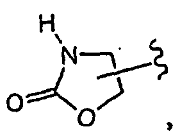, 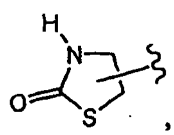, 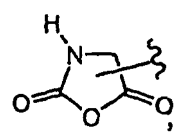, 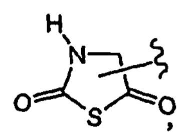,

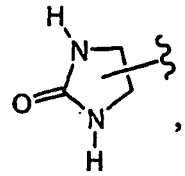, 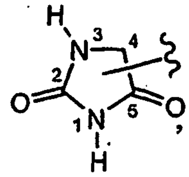,

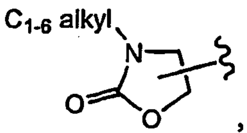, 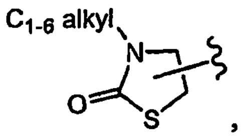, 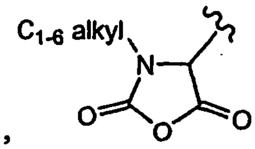, 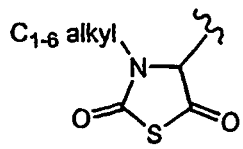,

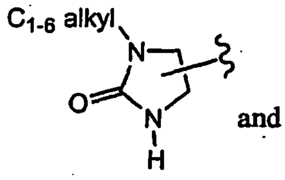 and 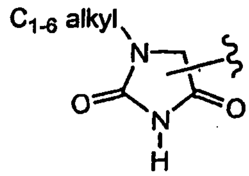.

It is understood that any one of the heterocyclic groups shown in TABLES 2B to 2E may be bonded at any available ring carbon or ring nitrogen as allowed by the respective formula. For example, a 2,5-dioxo-imidazolidinyl group may be bonded at the ring carbon or at either of the two ring nitrogens to give the following formulae respectively:

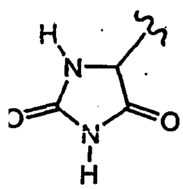, 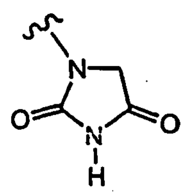, 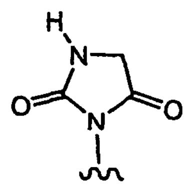.

In some embodiments $R_2$ is a heterocyclic represented, for example, by the formulae in TABLE 2C.

**TABLE 2C**

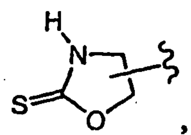, 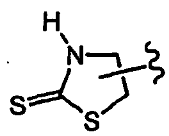, 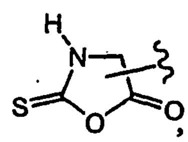, 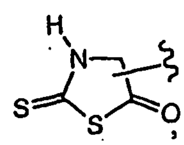,

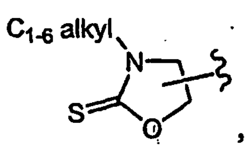, 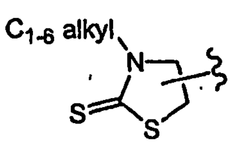, 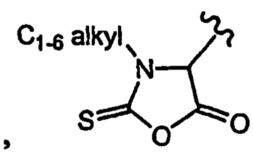 and 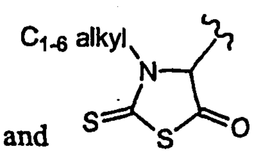.

In some embodiments $R_2$ is a heterocyclic represented, for example, by the formulae in TABLE 2D.

**TABLE 2D**

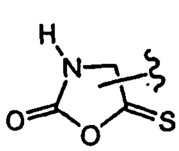, 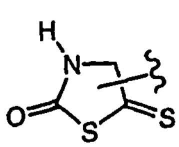, 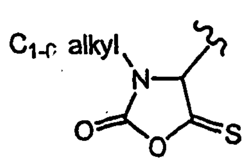 and 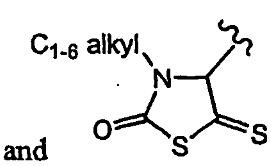.

In some embodiments $R_2$ is a heterocyclic represented, for example, by the formulae in TABLE 2E.

**TABLE 2E**

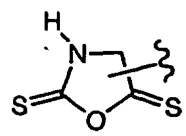
,
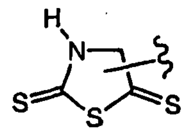
,
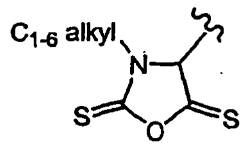
and
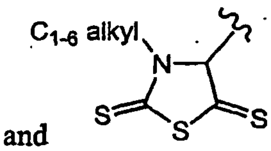
.

In some embodiments $R_2$ is a heterocyclic represented, for example, by the formulae in TABLE 2F wherein the $C_{1-6}$ alkyl group on the respective ring nitrogen atoms may be the same or different.

**TABLE 2F**

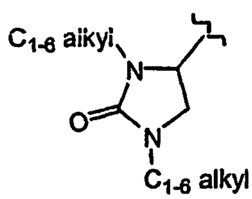
and
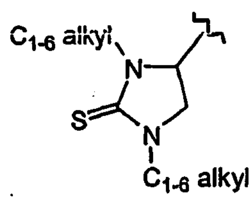
.

In some embodiments $R_2$ is a heterocyclic represented, for example, by the formulae in TABLE 2G wherein the $C_{1-6}$ alkyl group on the respective ring nitrogen atoms may be the same or different.

**TABLE 2G**

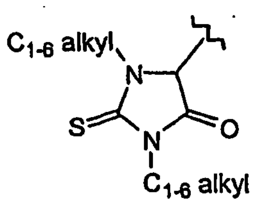
,
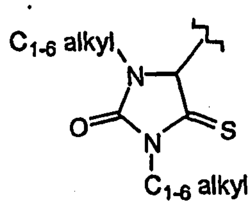
and
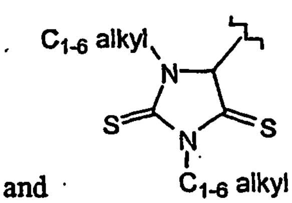
.

In some embodiments D is $CR_2R_3$ and $R_2$ is $-Ar_2-Ar_3$ wherein $Ar_2$ and $Ar_3$ are independently aryl or heteroaryl optionally substituted with 1 to 5 substituents selected from the group consisting of H, $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfinyl, $C_{1-4}$ alkylsulfonyl, $C_{1-4}$ alkylthio, amino, carbo-$C_{1-6}$-alkoxy, carboxamide, carboxy, cyano, $C_{3-6}$-cycloalkyl, $C_{2-6}$, dialkylcarboxamide, $C_{1-4}$ haloalkoxy, $C_{1-4}$ haloalkyl, halogen, hydroxyl and nitro.

In some embodiments compounds of the present invention are represented by Formula (Is) as shown below:

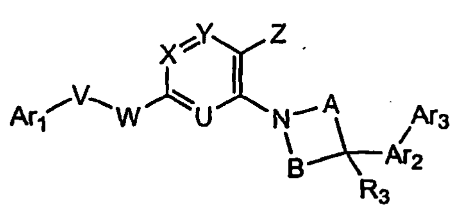

**(Is)**

wherein each variable in Formula **(Is)** has the same meaning as described herein.

In some embodiments $Ar_2$ is a heteroaryl comprising 5-atoms in the aromatic ring and are represented by the following formulae:

**TABLE 3**

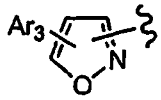, 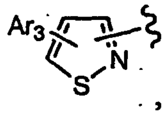, 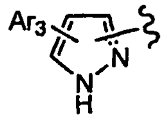, 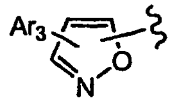, 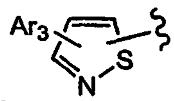,

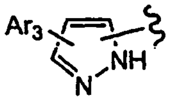, 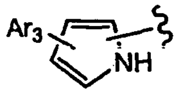, 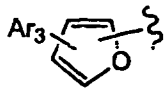, 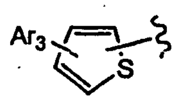, 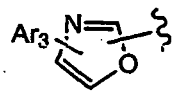,

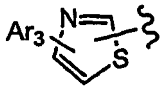, 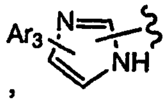, 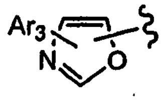, 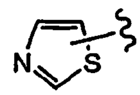, 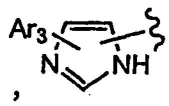,

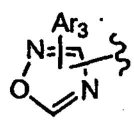, 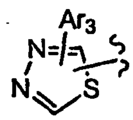, 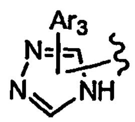, 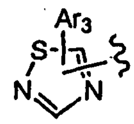,

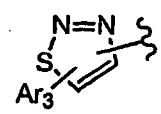 and 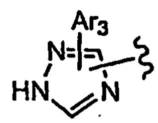

wherein the 5-membered heteroaryl is bonded at any available position of the ring, for example, a imidazolyl ring can be bonded at one of the ring nitrogens (i.e., imidazol-1-yl group) or at one of the ring carbons (i.e., imidazol-2-yl, imidazol-4-yl or imiadazol-5-yl group) and $Ar_3$ is bonded to any remaining available ring atom.

In some embodiments $Ar_2$ is a heteroaryl and $Ar_3$ is phenyl.

In some embodiments the heteroaryl and phenyl are optionally substituted with 1 to 5 substituents selected from the group consisting of H, $C_{1-4}$ alkoxy, $C_{1-8}$ alkyl, $C_{1-4}$ alkylcarboxamide, $C_{1-4}$ alkylsulfinyl, $C_{1-4}$ alkylsulfonyl, $C_{1-4}$ alkylthio, $C_{1-4}$ haloalkoxy, $C_{1-4}$ haloalkyl, halogen, hydroxyl and nitro.

In some embodiments D is $CR_2R_3$ and $R_2$ is Formula (B):

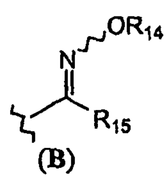

**(B)**

wherein:

$R_{14}$ is $C_{1-8}$ alkyl or $C_{3-6}$ cycloalkyl; and $R_{15}$ is F, Cl, Br or CN.

In some embodiments $R_{14}$ is $C_{1-8}$ alkyl and $R_{15}$ is F, Cl or CN.

In some embodiments D is $CR_2R_3$ and $R_2$ is Formula (C):

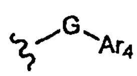

**(C)**

wherein:

G is C=O, $CR_{16}R_{17}$, O, S, S(O), $S(O)_2$; where $R_{16}$ and $R_{17}$ are independently H or $C_{1-8}$ alkyl; and $Ar_4$ is phenyl or heteroaryl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl,

$C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl-$C_{1-3}$-heteroalkylene, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloakylsulfinyl, $C_{1-4}$ haloalkylsulfonyl, $C_{1-4}$ haloalkylthio, halogen, heteroaryl, hydroxyl, hydroxylamino and nitro.

In some embodiments, compounds of the present invention are represented by Formula (It) as shown below:

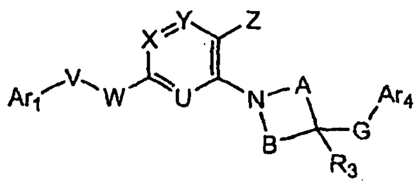

**(It)**

wherein each variable in Formula **(It)** has the same meaning as described herein.

In some embodiments D is $CR_2R_3$, $R_2$ is Formula (C) and G is C=O, $CR_{16}R_{17}$ or O.

In some embodiments $Ar_4$ is phenyl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl-$C_{1-3}$-heteroalkylene, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, halogen, heteroaryl, hydroxyl, hydroxylamino and nitro.

In some embodiments $Ar_4$ is phenyl optionally substituted with 1 to 5 substituents selected from the group consisting of $C_{1-5}$ acyl, $C_{1-4}$ alkoxy, $C_{1-8}$ alkyl, $C_{1-4}$ alkylcarboxamide, $C_{1-4}$ alkylsulfonamide, $C_{1-4}$ alkylsulfinyl, $C_{1-4}$ alkylsulfonyl, $C_{1-4}$ alkylthio, carboxamide, $C_{1-4}$ haloalkoxy, $C_{1-4}$ haloalkyl, $C_{1-4}$ haloalkylsulfinyl, $C_{1-4}$ haloalkylsulfonyl, halogen and hydroxyl.

In some embodiments $Ar_4$ is phenyl optionally substituted with 1 to 5 substituents selected from the group consisting of $C_{1-5}$ acyl, $C_{1-4}$ alkoxy, $C_{1-8}$ alkyl, $C_{1-4}$ alkylsulfinyl, $C_{1-4}$ alkylsulfonyl, $C_{1-4}$ alkylthio, $C_{1-4}$ haloalkoxy, $C_{1-4}$ haloalkyl, halogen and hydroxyl.

In some embodiments $Ar_4$ is heteroaryl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl-$C_{1-3}$-heteroalkylene, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, halogen, heteroaryl, hydroxyl, hydroxylamino and nitro.

In some embodiments $Ar_4$ is heteroaryl optionally substituted with 1 to 5 substituents selected from the group consisting of $C_{1-5}$ acyl, $C_{1-4}$ alkoxy, $C_{1-8}$ alkyl, $C_{1-4}$ alkylcarboxamide, $C_{1-4}$ alkylsulfonamide, $C_{1-4}$ alkylsulfinyl, $C_{1-4}$ alkylsulfonyl, $C_{1-4}$ alkylthio, carboxamide, $C_{1-4}$ haloalkoxy, $C_{1-4}$ haloalkyl, $C_{1-4}$ haloalkylsulfinyl, $C_{1-4}$ haloalkylsulfonyl, halogen and hydroxyl.

In some embodiments $Ar_4$ is heteroaryl optionally substituted with 1 to 5 substituents selected from the group consisting of $C_{1-5}$ acyl, $C_{1-4}$ alkoxy, $C_{1-8}$ alkyl, $C_{1-4}$ alkylsulfinyl, $C_{1-4}$ alkylsulfonyl, $C_{1-4}$ alkylthio, $C_{1-4}$ haloalkoxy, $C_{1-4}$ haloalkyl, halogen and hydroxyl.

In some embodiments $Ar_4$ is a 5-membered heteroaryl, for example, as shown in TABLE 2A *supra*.

In some embodiments $Ar_4$ is a 6-membered heteroaryl, for example, the 6-membered heteroaryls as shown in TABLE 4:

TABLE 4

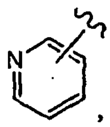, 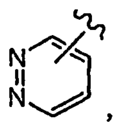, 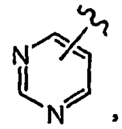, 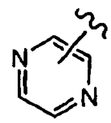, 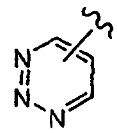,

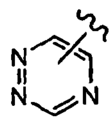, 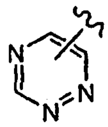, 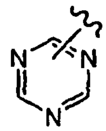 and 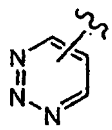

wherein the heteroaryl group is bonded at any ring carbon.

In some embodiments $Ar_4$ is selected from the group consisting of pyridinyl, pyridazinyl, pyrimidinyl and pyrazinyl. In some embodiments $Ar_4$ is 2-pyridyl.

In some embodiments $R_2$ is Formula (C), G is $CR_{16}R_{17}$ and $R_{16}$ and $R_{17}$ are independently H or $C_{1-2}$ alkyl.

In some embodiments $R_2$ is Formula (C) and G is S, S(O) or $S(O)_2$.

In some embodiments $Ar_4$ is phenyl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl-$C_{1-3}$-heteroalkylene, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, halogen, heteroaryl, hydroxyl, hydroxylamino and nitro.

In some embodiments $Ar_4$ is phenyl optionally substituted with 1 to 5 substituents selected from the group consisting of $C_{1-5}$ acyl, $C_{1-4}$ alkoxy, $C_{1-8}$ alkyl, $C_{1-4}$ alkylcarboxamide, $C_{1-4}$ alkylsulfonamide, $C_{1-4}$ alkylsulfinyl, $C_{1-4}$ alkylsulfonyl, $C_{1-4}$ alkylthio, carboxamide, $C_{1-4}$ haloalkoxy, $C_{1-4}$ haloalkyl, $C_{1-4}$ haloalkylsulfinyl, $C_{1-4}$ haloalkylsulfonyl, halogen and hydroxyl.

In some embodiments $Ar_4$ is phenyl optionally substituted with 1 to 5 substituents selected from the group consisting of $C_{1-5}$ acyl, $C_{1-4}$ alkoxy, $C_{1-8}$ alkyl, $C_{1-4}$ alkylsulfinyl, $C_{1-4}$ alkylsulfonyl, $C_{1-4}$ alkylthio, $C_{1-4}$ haloalkoxy, $C_{1-4}$ haloalkyl, halogen and hydroxyl.

In some embodiments $Ar_4$ is heteroaryl optionally substituted with 1 to 5 substituents selected from the group consisting of $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{1-6}$-cycloalkyl-$C_{1-3}$-heteroalkylene, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, halogen, heteroaryl, hydroxyl, hydroxylamino and nitro.

In some embodiments $Ar_4$ is heteroaryl optionally substituted with 1 to 5 substituents selected from the group consisting of $C_{1-5}$ acyl, $C_{1-4}$ alkoxy, $C_{1-8}$ alkyl, $C_{1-4}$ alkylcarboxamide, $C_{1-4}$ alkylsulfonamide, $C_{1-4}$ alkylsulfmyl, $C_{1-4}$ alkylsulfonyl, $C_{1-4}$ alkylthio, carboxamide, $C_{1-4}$ haloalkoxy, $C_{1-4}$ haloalkyl, $C_{1-4}$ haloalkylsulfinyl, $C_{1-4}$ haloalkylsulfonyl, halogen and hydroxyl.

In some embodiments $Ar_4$ is heteroaryl optionally substituted with 1 to 5 substituents selected from the group consisting of $C_{1-5}$ acyl, $C_{1-4}$ alkoxy, $C_{1-8}$ alkyl, $C_{1-4}$ alkylsulfinyl, $C_{1-4}$ alkylsulfonyl, $C_{1-4}$ alkylthio, $C_{1-4}$ haloalkoxy, $C_{1-4}$ haloalkyl, halogen and hydroxyl.

In some embodiments $Ar_4$ is heteroaryl optionally substituted with 1 to 5 substituents selected from the group consisting of $C_{1-4}$ alkoxy, $C_{1-8}$ alkyl, cyano, $C_{1-4}$ haloalkoxy, $C_{1-4}$ haloalkyl, and halogen.

In some embodiments $Ar_4$ is a 5-membered heteroaryl, for example, as shown in TABLE 2A, *supra*.

In some embodiments $Ar_4$ is a 6-membered heteroaryl, for example, as shown in TABLE 4, *supra*.

In some embodiments $Ar_4$ is selected from the group consisting of pyridinyl, pyridazinyl, pyrimidinyl and pyrazinyl.

In some embodiments D is $CR_2R_3$, $R_2$ is the group of Formula (C) wherein G is S, S(O), $S(O)_2$; and $Ar_4$ is phenyl or heteroaryl optionally substituted with 1 to 5 substituents selected from the group consisting of $C_{1-4}$ alkoxy, $C_{1-8}$ alkyl, cyano, $C_{1-4}$ haloalkoxy, $C_{1-4}$ haloalkyl, and halogen.

In some embodiments G is -S-.

In some embodiments $Ar_4$ is a pyridyl group.

In some embodiments $Ar_4$ is 2-pyridyl.

In some embodiments $R_3$ is H.

In all embodiments Z is nitro.

In some embodiments $R_1$ is selected from the group consisting of H, $C_{1-4}$ alkoxy, $C_{1-8}$ alkyl, $C_{2-6}$ alkynyl, amino, $C_{3-6}$ cycloalkyl and $C_{1-4}$ haloalkyl.

In some embodiments $R_1$ is H or amino.

In some embodiments $R_1$ is H.

In some embodiments $Ar_1$ is phenyl.

In some embodiments $R_9$ is selected from the group consisting of $C_{1-5}$ acyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, carboxamide, halogen and sulfonamide.

In some embodiments $R_9$ is selected from the group consisting of $C(O)CH_3$, $C(O)CH_2CH_3$, $C(O)CH_2CH_2CH_3$, $C(O)CH(CH_3)_2$, $C(O)CH_2CH_2CH_2CH_3$, $OCH_3$, $OCH_2CH_3$, $OCH_2CH_2CH_3$, $OCH(CH_3)_2$, $OCH_2CH_2CH_2CH_3$, $CH_3$, $CH_2CH_3$, $CH_2CH_2CH_3$, $CH(CH_3)_2$, $CH(CH_3)(CH_2CH_3)$, $CH_2(CH_2)_2CH_3$, $CH_2(CH_2)_3CH_3$, $CH_2(CH_2)_4CH_3$, $CH_2(CH_2)_5CH_3$, $C(O)NHCH_3$, $C(O)NHCH_2CH_3$, $C(O)NHCH_2CH_2CH_3$, $C(O)NHCH(CH_3)_2$, $C(O)NHCH_2(CH_2)_2CH_3$, CCH, $S(O)_2NHCH_3$, $S(O)_2NHCH_2CH_3$, $S(O)_2NHCH_2CH_2CH_3$, $S(O)_2NHCH(CH_3)_2$, $S(O)_2NHCH_2(CH_2)_2CH_3$, $S(O)_2NHCH(CH_3)CH_2CH_3$, $S(O)CH_3$, $S(O)CH_2CH_3$, $S(O)CH_2CH_2CH_3$, $S(O)CH(CH_3)_2$, $S(O)CH_2(CH_2)_2CH_3$, $S(O)CH(CH_3)CH_2CH_3$, $S(O)_2CH_3$, $S(O)_2CH_2CH_3$, $S(O)_2CH_2CH_2CH_3$, $S(O)_2CH(CH_3)_2$, $S(O)_2CH_2(CH_2)_2CH_3$, $S(O)_2CH(CH_3)CH_2CH_3$, $SCH_3$, $SCH_2CH_3$, $SCR_2CH_2CH_3$, $SCH(CH_3)_2$ and $SCH_2(CH_2)_2CH_3$.

In some embodiments $R_9$ is selected from the group consisting of amino, arylsulfonyl, carboxy, cyano, $C_{3-6}$ cycloalkyl, halogen, $C_{1-4}$ haloalkoxy, $C_{1-4}$ haloalkyl and $C_{1-4}$ haloalkylthio.

In some embodiments $R_9$ is selected from the group consisting of phenylsulfonyl, cyclopropyl, cyclobutyl, cyclopentyl, cyclohexyl, Cl, F, Br, $OCF_3$, $OCHF_2$, $OCH_2CF_3$, $CF_3$, $CHF_2$, $CH_2CF_3$, $SCF_3$, $SCHF_2$ and $SCH_2CF_3$.

In some embodiments $R_9$ is selected from the group consisting of heterocyclic, heteroaryl, $C_{4-7}$ oxo-cycloalkyl, phenoxy and phenyl.

In some embodiments $R_9$ is selected from the group consisting of morpholin-4-yl, thiomorpholin-4-yl, 1-oxo-1$\lambda^4$-thiomorpholin-4-yl, 1,1-Dioxo-1$\lambda^6$-thiomorpholin-4-yl, piperazin-1-yl, 4-methyl-piperazin-1-yl, 4-ethyl-piperazin-1-yl, 4-propyl-piperazin-1-yl, piperidin-1-yl, pyrrolidin-1-yl, 2,5-dioxo-imidazolidin-4-yl, 2,4-dioxo-thiazolidin-5-yl, 4-oxo-2-thioxo-thiazolidin-5-yl, 3-methyl-2,5-dioxo-imidazolidin-4-yl, 3-methyl-2,4-dioxo-thiazolidin-5-yl, 3-methyl-4-oxo-2-thioxo-thiazolidin-5-yl, 3-ethyl-2,5-dioxo-imidazolidin-4-yl, 3-ethyl-2,4-dioxo-thiazolidin-5-yl, and 3-ethyl-4-oxo-2-thioxo-thiazolidin-5-yl.

In some embodiments $R_9$ is selected from the group consisting of 1H-imidazol-4-yl, [1,2,4]triazol-1-yl, [1,2,3]triazol-1-yl, [1,2,4]triazol-4-yl, pyrrol-1-yl, pyrazol-1-yl, 1H-pyrazol-3-yl, imidazol-1-yl, oxazol-5-yl, oxazol-2-yl, [1,3,4]oxadiazol-2-yl, [1,3,4]thiadiazol-2-yl, [1,2,4]oxadiazol-3-yl, [1,2,4]thiadiazol-3-yl, tetrazol-1-yl, pyrimidin-5-yl, pyrimidin-2-yl, pyrimidin-4-yl, pyridazin-3-yl, pyridazin-4-yl, pyrazin-2-yl, 1,3-dioxo-1,3-dihydroisoindol-2-yl and [1,2,3]thiadiazol-4-yl.

In some embodiments $R_9$ is $C_{1-8}$ alkyl or $C_{1-4}$ alkoxy optionally substituted with 1 to 5 substituents selected independently from the group consisting of $C_{1-4}$ alkoxy, $C_{1-4}$ alkylcarboxamide, $C_{1-4}$ alkylsulfonamide, $C_{1-4}$ alkylsulfinyl, $C_{1-4}$ alkylsulfonyl, $C_{1-4}$ alkylthio, carbo-$C_{1-6}$-alkoxy, carboxamide, carboxy, cyano, heterocyclic, hydroxyl and phenyl.

In some embodiments $R_9$ is $C_{1-4}$ alkylsulfonyl optionally substituted with 1 to 5 substituents selected independently from the group consisting of $C_{1-4}$ alkoxy, carboxamide, heteroaryl, heterocyclic and phenyl.

In some embodiments $R_9$ is $C_{1-4}$ alkylsulfonyl substituted with the heteroaryl group.

In some embodiments the heteroaryl is selected from the group consisting of 1H-imidazol-4-yl, [1,2,4]triazol-1-yl, [1,2,3]triazol-1-yl, [1,2,4]triazol-4-yl, pyrrol-1-yl, pyrazol-1-yl, 1H-pyrazol-3-yl, imidazol-1-yl, oxazol-5-yl, oxazol-2-yl, [1,3,4]oxadiazol-2-yl, [1,3,4]thiadiazol-2-yl, [1,2,4]oxadiazol-3-yl, [1,2,4]thiadiazol-3-yl, tetrazol-1-yl, pyrimidin-5-yl, pyrimidin-2-yl, pyrimidin-4-yl, pyridazin-3-yl, pyridazin-4-yl, pyrazin-2-yl, 1,3-dioxo-1,3-dihydro-isoindol-2-yl and [1,2,3]thiadiazol-4-yl.

In some embodiments $R_9$ is arylsulfonyl, heteroaryl, phenoxy or phenyl optionally substituted with 1 to 5 substituents selected independently from the group consisting of $C_{1-5}$ acyl, $C_{1-4}$ alkoxy, $C_{1-8}$ alkyl, $C_{1-4}$ alkylsulfonamide, $C_{1-4}$ alkylsulfinyl, $C_{1-4}$ alkylsulfonyl, $C_{1-4}$ alkylthio, carboxamide, carboxy, cyano, halogen, $C_{1-4}$ haloalkoxy, $C_{1-4}$ haloalkyl, $C_{1-4}$ haloalkylthio and hydroxyl.

In some embodiments $R_9$ is arylsulfonyl, heteroaryl, phenoxy or phenyl optionally substituted with 1 to 5 substituents selected independently from the group consisting of $C_{1-4}$ alkoxy, $C_{1-8}$ alkyl, cyano, halogen, $C_{1-4}$ haloalkoxy, $C_{1-4}$ haloalkyl and hydroxyl.

In some embodiments $Ar_1$ is phenyl and $R_9$ is of Formula (**D**):

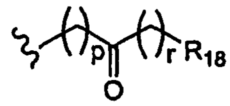

(D)

wherein:

"p" and "r" are independently 0,1, 2 or 3; and $R_{18}$ is H, $C_{1-5}$ acyl, $C_{2-6}$ alkenyl, $C_{1-8}$ alkyl, $C_{1-4}$ alkylcarboxamide, $C_{2-6}$ alkynyl, $C_{1-4}$ alkylsulfonamide, carbo-$C_{1-6}$-alkoxy, carboxamide, carboxy, cyano, $C_{3-6}$ cycloalkyl, $C_{2-6}$ dialkylcarboxamide, halogen, heteroaryl or phenyl, and wherein the heteroaryl or phenyl may be optionally substituted with 1 to 5 substituents selected independently from the group consisting of $C_{1-4}$ alkoxy, amino, $C_{1-4}$ alkylamino, $C_{2-6}$ alkynyl, $C_{2-8}$ dialkylamino, halogen, $C_{1-4}$ haloalkoxy, $C_{1-4}$ haloalkyl and hydroxyl.

In some embodiments p = 0 and r = 0.

In some embodiments $R_{18}$ is heteroaryl or phenyl optionally substituted with 1 to 5 substituents selected independently from the group consisting of $C_{1-4}$ alkoxy, amino, $C_{1-4}$ alkylamino, $C_{2-6}$ alkynyl, $C_{2-8}$ dialkylamino, halogen, $C_{1-4}$ haloalkoxy, $C_{1-4}$ haloalkyl and hydroxyl.

In some embodiments the heteroaryl is selected from the group consisting of 1H-imidazol-4-yl, [1,2,4]triazol-1-yl, [1,2,3]triazol-1-yl, [1,2,4]triazol-4-yl, pyrrol-1-yl, pyrazol-1-yl, 1H-pyrazol-3-yl, imidazol-1-yl, oxazol-5-yl, oxazol-2-yl, [1,3,4]oxadiazol-2-yl, [1,3,4]thiadiazol-2-yl, [1,2,4]oxadiazol-3-yl, [1,2,4]thiadiazol-3-yl, tetrazol-1-yl, pyrimidin-5-yl, pyrimidin-2-yl, pyrimidin-4-yl, pyridazin-3-yl, pyridazin-4-yl, pyrazin-2-yl, 1,3-dioxo-1,3-dihydro-isoindol-2-yl and [1,2,3]thiadiazol-4-yl.

In some embodiments p = 0 and r =1.

In some embodiments $R_{18}$ is carbo-$C_{1-6}$-alkoxy or carboxy.

In some embodiments p = 2 and r = 1.

In some embodiments $R_{18}$ is H, $C_{1-5}$ acyl or $C_{1-8}$ alkyl.

In some embodiments $R_{10}$-$R_{13}$ are independently H, $C_{1-5}$ acyl, $C_{1-4}$ alkoxy, $C_{1-8}$ alkyl, $C_{1-4}$ alkylcarboxamide, $C_{1-4}$ alkylureyl, carbo-$C_{1-6}$-alkoxy, carboxamide, carboxy, cyano, $C_{3-6}$ cycloalkyl, halogen, $C_{1-4}$ haloalkoxy and $C_{1-4}$ haloalkyl.

In some embodiments $R_9$ is substituted at the para position on the phenyl and may be represented by Formula (**Iw**) as shown below:

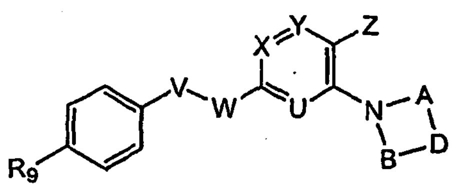

(Iw)

wherein each variable in Formula (**Iw**) has the same meaning as described herein.

In some embodiments, in addition to $R_9$ in Formula (**Iw**), the phenyl ring can be optionally substituted with $R_{10}$ to $R_{13}$ wherein each $R_{10}$ to $R_{13}$ is selected independently from the group consisting ofH, $C_{1-5}$ acyl, $C_{1-4}$ alkoxy, $C_{1-8}$ alkyl, halogen, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, hydroxyl and nitro.

In some embodiments, $R_{10}$ to $R_{13}$ is selected independently from the group consisting of H and halogen.

In some embodiments $Ar_1$ is phenyl and two adjacent $R_{10}$-$R_{11}$ groups form a 5, 6 or 7 membered cycloalkyl, cycloalkenyl or heterocyclic group with the phenyl group wherein the 5, 6 or 7 membered group is optionally substituted with halogen.

In some embodiments $Ar_1$ is phenyl and two adjacent $R_{10}$-$R_{11}$ groups form a 5, 6 or 7 membered cycloalkyl group with the phenyl group and is of the formulae shown below:

**TABLE 5**

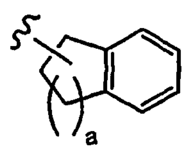 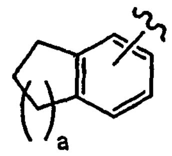

wherein "a" is 1, 2 or 3 to give a 5, 6 or 7 membered cycloalkyl fused together with the phenyl group where two of the ring carbons are shared between the cycloalkyl and phenyl group.

In some embodiments the cycloalkyl is optionally substituted with halogen.

In some embodiments the halogen is fluorine.

In some embodiments $Ar_1$ is phenyl and two adjacent $R_{10}$-$R_{11}$ groups form a 5, 6 or 7 membered cycloalkenyl group with the phenyl group and is of the formulae shown in TABLE 5 and has at least one carbon-carbon ring double bond present that is not part of the phenyl group (i.e., cycloalkenyl), for example, 1*H*-Indenyl and dihydro-naphthyl.

In some embodiments the heterocyclic group is optionally substituted with halogen.

In some embodiments the halogen is fluorine.

In some embodiments $Ar_1$ is phenyl and two adjacent $R_{10}$-$R_{11}$ groups form a 5, 6 or 7 heterocyclic group with the phenyl group and is of the formulae in TABLE 5 wherein one or more ring cycloalkyl carbons are replaced by a O, S, S(O), $S(O)_2$, NH or N-alkyl group.

In some embodiments the heterocyclic group is optionally substituted with halogen. In some embodiments the halogen is fluorine.

In some embodiments $Ar_1$ is phenyl and two adjacent $R_{10}$-$R_{11}$ groups form a 5 membered heterocyclic group with the phenyl group.

In some embodiments the 5 membered heterocyclic group with the phenyl group together form a 2,3-dihydro-benzofuran-5-yl or benzo[1,3]dioxol-5-yl group.

In some embodiments the two adjacent groups form a 6 membered heterocyclic group with the phenyl group. In some embodiments the 6 membered heterocyclic group with the phenyl group together form a 2,3-dihydro-benzo[1,4]dioxin-6-yl or 2,3-dihydro-benzo[1,4]dioxin-2-yl group.

In some embodiments the two adjacent groups form a 7 membered heterocyclic group with the phenyl group.

In some embodiments the 7 membered heterocyclic group with the phenyl group together form a 3,4-dihydro-2H-benzo[b][1,4]dioxepin-7-yl group.

In some embodiments $Ar_1$ is heteroaryl.

In some embodiments $Ar_1$ is a heteroaryl selected from TABLE 2A.

In some embodiments $Ar_1$ is a heteroaryl selected from TABLE 4.

In some embodiments $Ar_1$ is heteroaryl and two adjacent $R_{10}$-$R_{11}$ groups form a 5, 6 or 7 membered cycloalkyl, cycloalkenyl or heterocyclic group with the heteroaryl group wherein the 5, 6 or 7 membered group is optionally substituted with halogen.

In some embodiments the two adjacent groups form a 5 membered heterocyclic group with the heteroaryl group. In some embodiments the two adjacent groups form a 6 membered heterocyclic group with the heteroaryl group. In some embodiments the two adjacent groups form a 7 membered heterocyclic group with the heteroaryl group.

In some embodiments $R_9$ is a heterocyclic group as described herein.

In some embodiments $R_9$ is a heterocyclic group represented by the formulae shown in Table 2B, *supra.*

In some embodiments $R_9$ is a heterocyclic group represented by the formulae shown in Table 2C, *supra.*

In some embodiments $R_9$ is a heterocyclic group represented by the formulae shown in Table 2D, *supra.*

In some embodiments $R_9$ is a heterocyclic group represented by the formulae shown in Table 2E, *supra.*

In some embodiments $R_9$ is a heterocyclic group represented by the formulae shown in Table 2F, *supra.*

In some embodiments $R_9$ is a heterocyclic group represented by the formulae shown in Table 2G, *supra.*

In some embodiments $Ar_1$ is phenyl, pyridyl, or pyridinone optionally substituted with $R_9$ and $R_{10}$.

In some embodiments $R_9$ is selected from the group consisting of $C_{1-5}$ acyl, vinyl, $C_{1-8}$ alkyl, $C_{1-4}$ alkylsulfonyl, amino, benzenesulfonyl, carboxamide, cyclopentyl, halogen, $C_{1-4}$ haloalkyl, 2,5-dioxo-imidazolidinyl, imidazolyl, pyrrolyl, triazol-1-yl, thiadiazolyl, 1,3-dioxo-1,3-dihydro-isoindolyl, pyrazolyl, [1,3,4]oxadiazolyl, [1,2,4]oxadiazolyl, hydroxyl, oxocyclohexyl, phenyl, and sulfonic acid, and wherein $C_{1-5}$ acyl, $C_{1-8}$ alkyl, benzenesulfonyl, and phenyl are optionally substituted with 1 to 5 substituents selected independently from the group consisting of $C_{1-4}$ alkoxy, $C_{1-8}$ alkyl, cyano, heteroaryl, and hydroxyl.

In some embodiments $R_9$ is selected from the group consisting of is selected from the group consisting of acetyl, 4-hydroxy-benzenesulfonyl, 2-methoxy-ethyl, vinyl, methyl, methylsulfonyl, ethanesulfonyl, amino, 4-hydroxy-benzenesulfonyl, 4-cyanophenyl, 4-methoxyphenyl, carboxamide, cyclopentyl, fluoro, chloro, bromo, trifluoromethyl,

2,5-dioxoimidazolidinyl, imidazol-1-yl, pyrrolyl, triazol-1-yl, thiadiazol-4-yl, 1,3-dioxo-1,3-dihydroisoindolyl, pyrazolyl, 5-methyl-[1,3,4]oxadiazol-2-yl, 3-methyl-[1,2,4]oxadiazol-5-yl, hydroxyl, 4-oxo-cyclohexyl, phenyl, and sulfonic acid.

In some embodiments $R_9$ is the group of Formula (D), wherein "p" and "r" are independently 0, 1, 2 or 3; and $R_{18}$ is H, carbo-$C_{1-6}$-alkoxy, carboxy, heteroaryl or phenyl, and wherein the heteroaryl and phenyl are optionally substituted with 1 to 5 substituents selected independently from the group consisting of $C_{1-4}$ alkoxy, $C_{1-8}$ alkyl, halogen, $C_{1-4}$ haloalkoxy, and $C_{1-4}$ haloalkyl.

In some embodiments $R_9$ is 2-methoxycarbonyl-acetyl, benzoyl, 3-oxo-butyl, 2-carboxy-ethyl, 2-carboxy-2-oxo-ethyl, $CH_3(CH_2)_2C(O)$, $CH_3(CH_2)_3C(O)$, and $CH_3(CH_2)_4C(O)$.

In some embodiments $R_{10}$ is selected form the group consisting of $C_{1-4}$ alkoxy, $C_{1-8}$ alkyl, amino, cyano, halogen, $C_{1-4}$ haloalkoxy, $C_{1-4}$ haloalkyl, and hydroxyl.

In some embodiments $R_{10}$ is selected from the group consisting of amino, methoxy, methyl, cyano, fluoro, chloro, bromo, trifluoromethoxy, trifluoromethyl, and hydroxyl.

In some embodiments U is N, X is $CR_5$, and Y is $CR_6$, compounds in this embodiment may be represented Formula (IIa) as shown below:

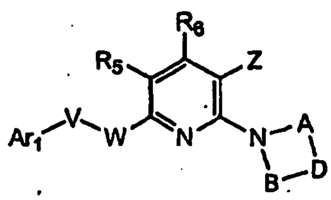

(IIa)

wherein each variable in Formula (IIa) has the same meaning as described herein. In some embodiments $R_5$ and $R_6$ is H.

In some embodiments U is N, X is $CR_5$, and Y is N, compounds in this embodiment may be represented Formula (IIc) as shown below:

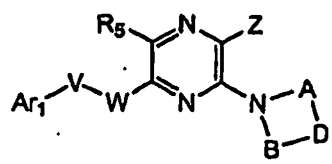

(IIc)

wherein each variable in Formula (IIc) has the same meaning as described herein. In some embodiments $R_5$ is H.

In some embodiments U is $CR_1$, X is $CR_5$ and Y is $CR_6$, compounds in this embodiment may be represented Formula (IIe) as shown below:

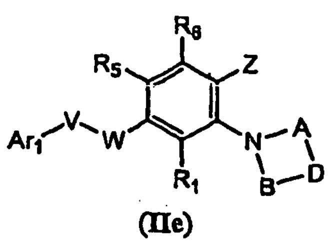

(IIe)

wherein each variable in Formula (IIe) has the same meaning as described herein. In some embodiments $R_1$, $R_5$ and $R_6$ are H.

In some embodiments U is $CR_1$, X is $CR_5$ and Y is N, compounds in this embodiment may be represented Formula (IIg) as shown below:

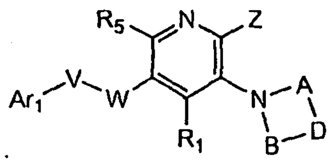

(IIg)

wherein each variable in Formula **(IIg)** has the same meaning as described herein.

In some embodiments $R_1$ and $R_5$ are H.

In some embodiments X is $CR_5$.

In some embodiments Y is $CR_6$.

In some embodiments $R_6$ is H.

In some embodiments U is N.

In some embodiments U is is $CR_1$.

In some embodiments $R_1$ is H.

In some embodiments U is N, X and Y are both CH.

In some embodiments U is CH, X is CH or C-$NO_2$, and Y is CH.

In some embodiments, the present invention include compounds wherein A and B are both -$CH_2$-$CH_2$-; D is $CR_2R_3$, wherein $R_2$ is selected from the group consisting of $CO_2CH_2CH_3$, $CH_2CH_2CH_3$, pyridin-2-ylsulfanyl, $CH_2OCH_3$, and 3-methyl-1,2,4-oxadiazol-5-yl; and $R_3$ is H; V is absent, W is -O-; Z is nitro; and $Ar_1$ is phenyl optionally substituted by $R_9$ and $R_{10}$, wherein $R_9$ is acetyl, vinyl, ethanesulfonyl, triazol-1-yl, 2-(3-methyl-[1,2,4]oxadiazol-5-yl)-acetyl, 5-hydroxy-1-methyl-1H-pyrazol-3-yl, 5-trifluoromethyl-pyridin-2-yl, 5-Bromo-pyridin-2-yl, 2-methoxycarbonyl-acetyl, benzoyl, 3-oxo-butyl, 2-carboxy-ethyl, 2-carboxy-2-oxo-ethyl, $CH_3(CH_2)_2C(O)$, $CH_3(CH_2)_3C(O)$, and $CH_3(CH_2)_4C(O)$; and $R_{10}$ is amino; or a pharmaceutically acceptable salt, solvate or hydrate thereof.

In some embodiments, the present invention include compounds wherein D is $CR_2R_3$, wherein $R_2$ is selected from the group consisting of $C(O)CH_3$, $CO_2CH_2CH_3$, $CH_2CH_2CH_3$, and pyridin-2-ylsulfanyl; and $R_3$ is H; V is absent, W is -O-; Z is nitro; and $Ar_1$ is phenyl optionally substituted by $R_9$ and $R_{10}$, wherein $R_9$ is acetyl, 2-methoxy-ethyl, ethanesulfonyl, 4-hydroxy-benzenesulfonyl, 4-cyanophenyl, 4-methoxyphenyl, carboxamide, cyclopentyl, 2,5-dioxo-imidazolidinyl, imidazol-1-yl, pyrrolyl, triazol-1-yl, thiadiazol-4-yl, 1,3-dioxo-1,3-dihydro-isoindolyl, 4-oxo-cyclohexyl, sulfonic acid, 2-methoxycarbonyl-acetyl, benzoyl, or 3-oxo-butyl; and $R_{10}$ is amino; or a pharmaceutically acceptable salt, solvate or hydrate thereof.

Compounds disclosed herein were named accordingly to AutoNom Version 2.2 contained within Chem Draw Ultra Version 7.0.

Some embodiments of the present invention and other reference compounds include compounds illustrated in TABLES A and B; these TABLES are shown below.

**TABLE A**

| Cmpd# | Structure | Chemical Name |
|---|---|---|
| A1 | 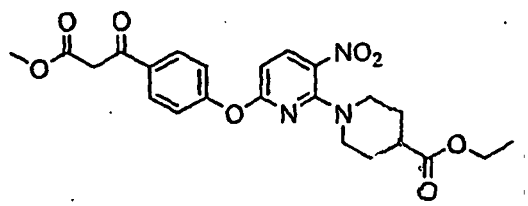 | 6'-[4-(2-Methoxycarbonyl-acetyl)-phenoxy]-3'-nitro-3,4,5,6-tetrahydro-2H-[1,2']bipyridinyl-4-carboxylic acid ethyl ester |
| A2 | 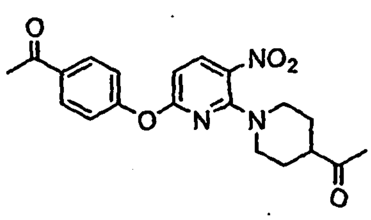 | 1-[4-(4-Acetyl-3'-nitro-3,4,5,6-tetrahydro-2H-[1,2']bipyridinyl-6'-yloxy)-phenyl]-ethanone |

(continued)

| Cmpd# | Structure | Chemical Name |
|---|---|---|
| A3 | 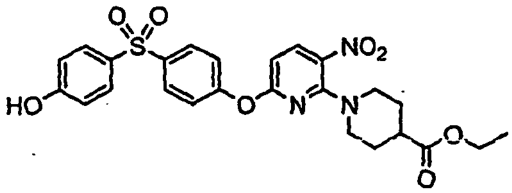 | 6'-[4-(4-Hydroxy-benzenesulfonyl)-phenoxy]-3'-nitro-3,4,5,6-tetrahydro-2H-[1,2']bipyridinyl-4-carboxylic acid ethyl ester |
| A4 | 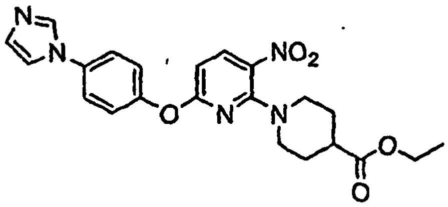 | 6'-(4-Imidazol-1-yl-phenoxy)-3'-nitro-3,4,5,6-tetrahydro-2H-[1,2']bipyridinyl-4-carboxylic acid ethyl ester |
| A5 | 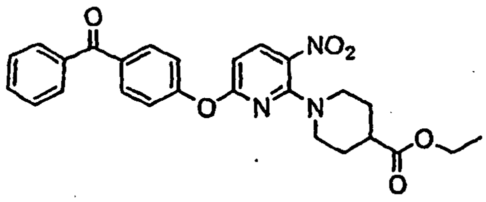 | 6'-(4-Benzoyl-phenoxy)-3'-nitro-3,4,5,6-tetrahydro-2H-[1,2']bipyridinyl-4-carboxylic acid ethyl ester |
| A6 | 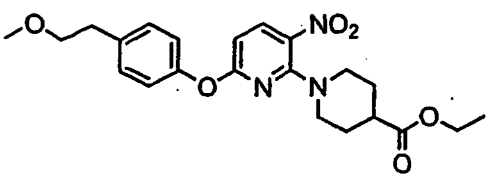 | 6'-[4-(2-Methoxy-ethyl)-phenoxy]-3'-nitro-3,4,5,6-tetrahydro-2H-[1,2']bipyridinyl-4-carboxylic acid ethyl ester |
| A7 | 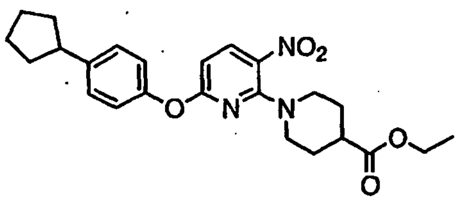 | 6'-(4-Cyclopentyl-phenoxy)-3'-nitro-3,4,5,6-tetrahydro-2H-[1,2']bipyridinyl-4-carboxylic acid ethyl ester |
| A8 | 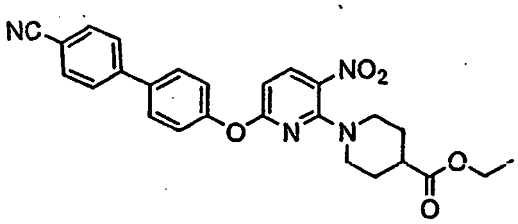 | 6'-(4'-Cyano-biphenyl-4-yloxy)-3'-nitro-3,4,5,6-tetrahydro-2H-[1,2']bipyridinyl-4-carboxylic acid ethyl ester |

(continued)

| Cmpd# | Structure | Chemical Name |
|---|---|---|
| A9 | 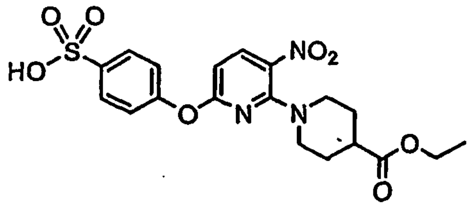 | 3'-Nitro-6'-(4-sulfo-phenoxy)-3,4,5,6-tetrahydro-2H-[1,2']bipyridinyl-4-carboxylic acid ethyl ester |
| A10 | 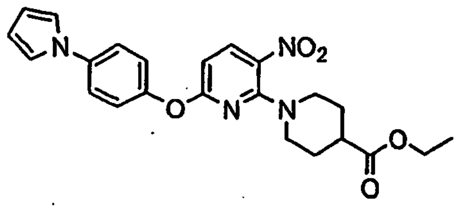 | 3'-Nitro-6'-(4-pyrrol-yl-phenoxy)-3,4,5,6-tetrahydro-2H-[1,2']bipyridinyl-4-carboxylic acid ethyl ester |
| A11 | 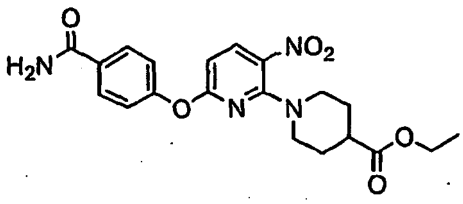 | 6'-(4-Carbamoyl-phenoxy)-3'-nitro-3,4,5,6-tetrahydro-2H-[1,2']bipyridinyl-4-carboxylic acid ethyl ester |
| A12 | 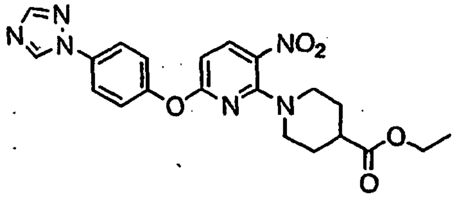 | 3'-Nitro-6'-(4-[1,2,4]triazol-1-yl-phenoxy)-3,4,5,6-tetrahydro-2H-[1,2']bipyridinyl-4-carboxylic acid ethyl ester |
| A13 | 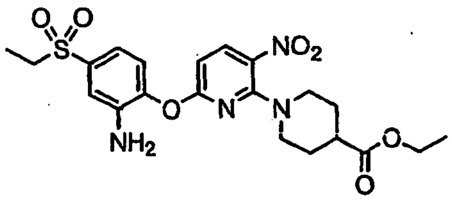 | 6'-(2-Amino-4-ethanesulfonyl-phenoxy)-3'-nitro-3,4,5,6-tetrahydro-2H-[1,2']bipyndinyl-4-carboxylic acid ethyl ester |
| A14 | 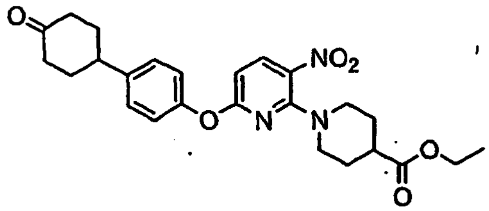 | 3'-Nitro-6'-[4-(4-oxo-cyclohexyl)-phenoxy]-3,4,5,6-tetrahydro-2H-[1,2']bipyridinyl-4-carboxylic acid ethyl ester |

(continued)

| Cmpd# | Structure | Chemical Name |
|---|---|---|
| A15 | 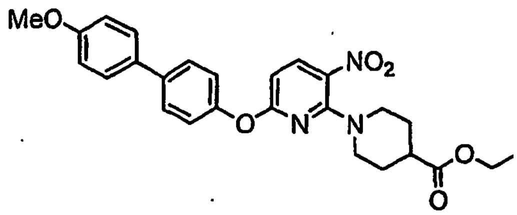 | 6'-(4'-Methoxy-biphenyl-4-yloxy)-3'-nitro-3,4,5,6-tetrahydro-2H-[1,2']bipyridinyl-4-carboxylic acid ethyl ester |
| A16 | 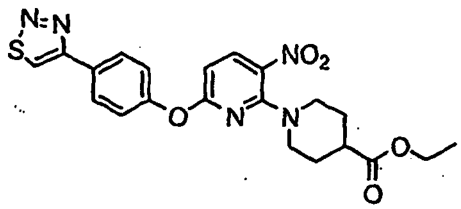 | 3'-Nitro-6'-(4-[1,2,3]thiadiazol-4-yl-phenoxy)-3,4,5,6-tetrahydro-2H-[1,2']bipyridinyl-4-carboxylic acid ethyl ester |
| A17 | 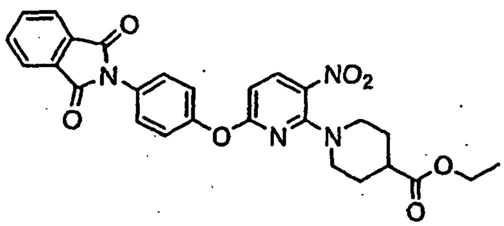 | 6'-[4-(1,3-Dioxo-1,3-dihydro-isoindol-2-yl)-phenoxy]-3'-nitro-3,4,5,6-tetrahydro-2H-[1,2'] bipyridiny1-4-carboxylic acid ethyl ester |
| A18 | 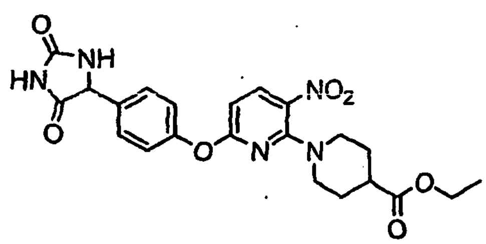 | 6'-[4-(2,5-Dioxo-imidazolidin-4-yl)-phenoxy]-3'-nitro-3,4,5,6-tetrahydro-2H-[1,2']bipyridinyl-4-carboxylic acid ethyl ester |
| A19 | 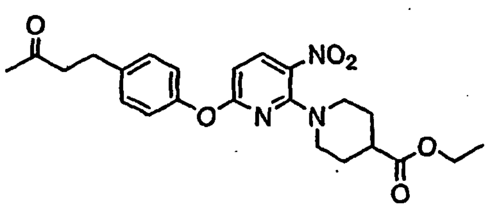 | 3'-Nitro-6'-[4-(3-oxo-butyl)-phenoxy]-3,4,5,6-tetrahydro-2H-[1,2']bipyridinyl-4-carboxylic acid ethyl ester |
| A20 | 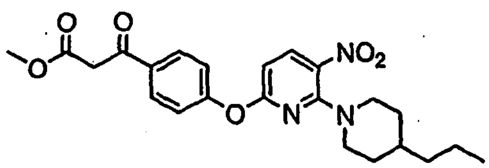 | 3-[4-(3'-Nitro-4-propyl-3,4,5,6-tetrahydro-2H-[1,2']bipyridinyl-6'-yloxy)-phenyl]-3-oxo-propionic acid methyl ester |

(continued)

| Cmpd# | Structure | Chemical Name |
|---|---|---|
| A21 | 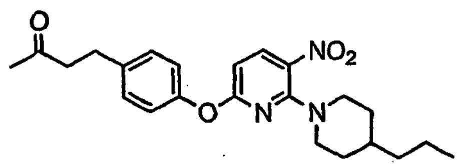 | 4-[4-(3'-Nitro-4-propyl-3,4,5,6-tetrahydro-2H-[1,2']bipyridinyl-6'-yloxy)-phenyl]-butan-2-one |
| A22 | 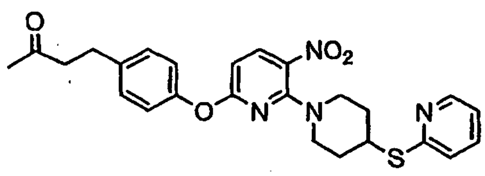 | 4-{4-[3'-Nitro-4-(pyridin-2-ylsulfanyl)-3,4,5,6-tetrahydro-2H-[1,2']bipyridinyl-6'-yloxy]-phenyl}-butan-2-one |
| A23 | 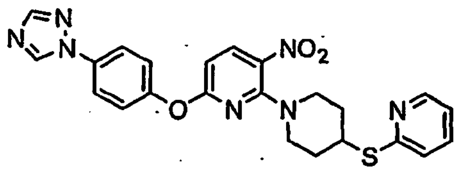 | 3'-Nitro-4-(pyridin-2-ylsulfanyl)-6'-(4-[1,2,4] triazol-1-yl-phenoxy)-3,4,5,6-tetrahydro-2H-[1,2'] bipyridinyl |
| A24 | 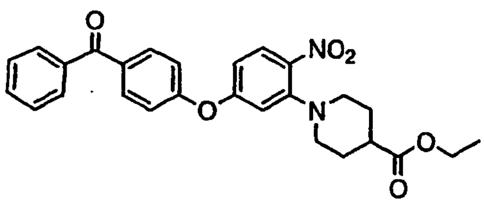 | 1-[5-(4-Benzoyl-phenoxy)-2-nitro-phenyl]-piperidine-4-carboxylic acid ethyl ester |
| A25 | 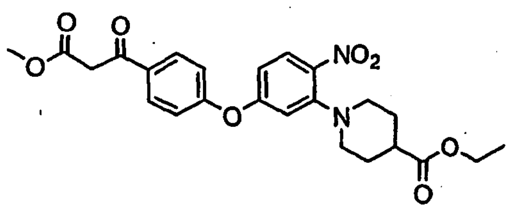 | 1-{5-[4-(2-Methoxycarbonyl-acetyl)-phenoxy]-2-nitro-phenyl}-piperidine-4-carboxylic acid ethyl ester |
| A26 | 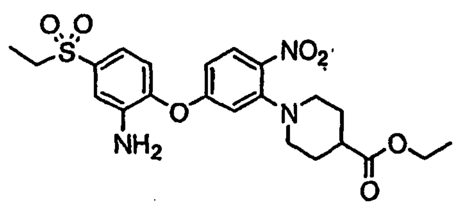 | 1-[5-(2-Amino-4-ethanesulfonyl-phenoxy)-2-nitro-phenyl]-piperidine-4-carboxylic acid ethyl ester |

(continued)

| Cmpd# | Structure | Chemical Name |
|---|---|---|
| A27 | 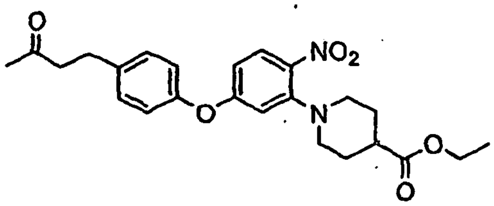 | 1-{2-Nitro-5-[4-(3-oxo-butyl)-phenoxy]-phenyl}-piperidine-4-carboxylic acid ethyl ester |
| A28 | 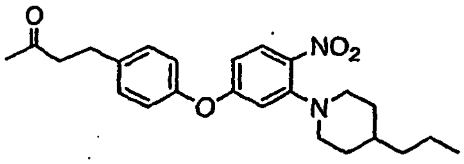 | 4-{4-[4-Nitro-3-(4-propyl-piperidin-1-yl)-phenoxy]-phenyl}-butan-2-one |
| A29 | 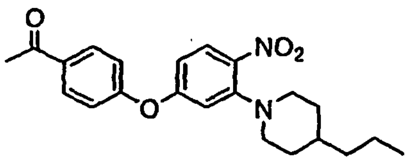 | 1-{4-[4-Nitro-3-(4-propyl-piperidin-1-yl)-phenoxy]-phenyl}-ethanone |
| A30 | 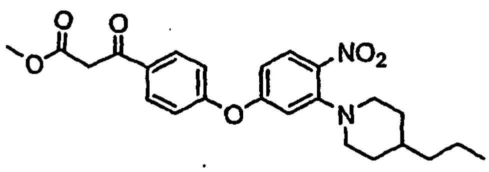 | 3-{4-[4-Nitro-3-(4-propyl-pipendin-1-yl)-phenoxy]-phenyl}-3-oxo-propionic acid methyl ester |
| A31 | 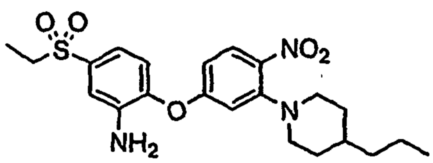 | 5-Ethanesulfonyl-2-[4-nitro-3-(4-propyl-piperidin-1-yl)-phenoxy]-phenylamine |
| A32 | 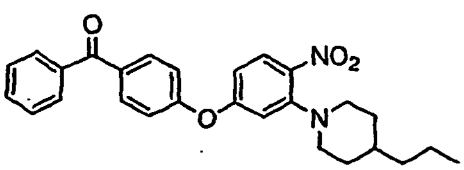 | {4-[4-Nitro-3-(4-propyl-piperidin-1-yl)-phenoxy]-phenyl}-phenyl-methanone |
| A33 (Ref) | 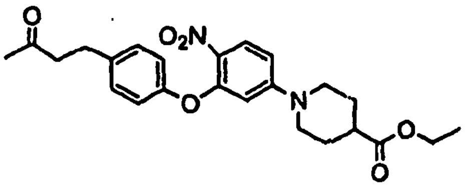 | 1-{4-Nitro-3-[4-(3-oxo-butyl)-phenoxy]-phenyl}-piperidine-4-carboxylic acid ethyl eater |

(continued)

| Cmpd# | Structure | Chemical Name |
|---|---|---|
| A34 (Ref) | 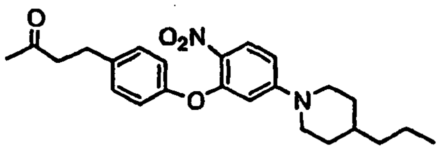 | 4-{4-[2-Nitro-5-(4-propyl-piperidin-1-yl)-phenoxy]-phenyl}-butan-2-one |
| A35 (Ref) | 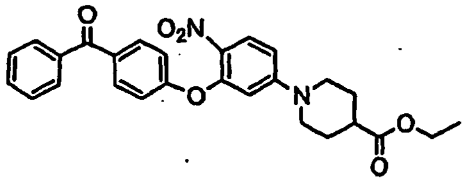 | 1-[3-(4-Benzoyl-phenoxy)-4-nitro-phenyl]-piperidine-4-carboxylic acid ethyl ester |
| A36 (Ref) | 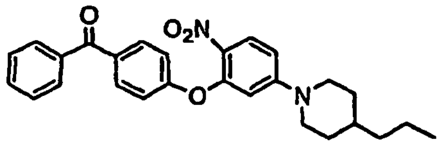 | {4-[2-Nitro-5-(4-propyl-piperidin-1-yl)-phenoxy]-phenyl}-phenyl-methanone |
| A37 | 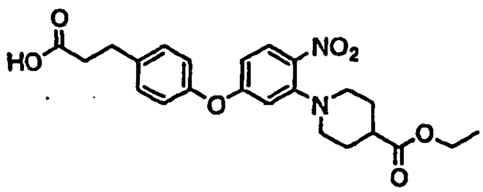 | 1-{5-[4-(2-Carboxy-ethyl)-phenoxy]-2-nitro-phenyl}-piperidine-4-carboxylic acid ethyl ester |
| A38 | 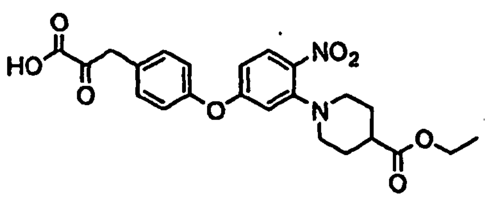 | 1-{5-[4-(2-Carboxy-2-oxo-ethyl)-phenoxy]-2-nitro-phenyl}-piperidine-4-carboxylic acid ethyl ester |
| A39 | 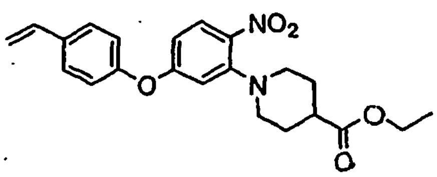 | 1-[2-Nitro-5-(4-vinyl-phenoxy)-phenyl]-piperidine-4-carboxylic acid ethyl ester |
| A40 | 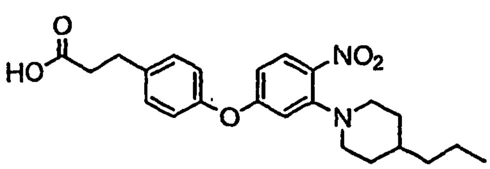 | 3-{4-[4-Nitro-3-(4-propyl-pipendin-1-yl)-phenoxy]-phenyl}-propionic acid |

(continued)

| Cmpd# | Structure | Chemical Name |
|---|---|---|
| A41 | 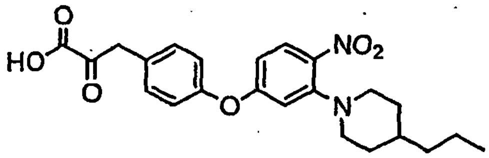 | 3-{4-[4-Nitro-3-(4-propyl-piperidin-1-yl)-phenoxy]-phenyl}-2-oxo-propionic acid |
| A42 | 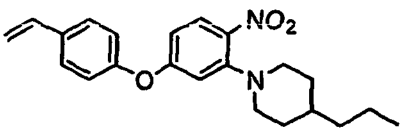 | 1-[2-Nitro-5-(4-vinyl-phenoxy)-phenyl]-4-propyl-piperidine |
| A43 | 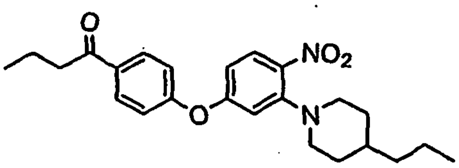 | 1-{4-[4-Nitro-3-(4-propyl-piperidin-1-yl)-phenoxy]-phenyl}-butan-1-one |
| A44 | 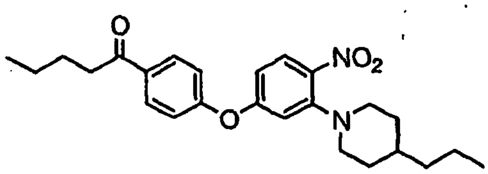 | 1-{4-[4-Nitro-3-(4-propyl-piperidin-1-yl)-phenoxy]-phenyl}-pentan-1-one |
| A45 | 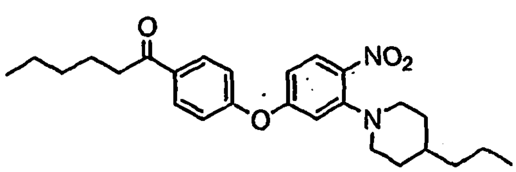 | 1-{4-[4-Nitro-3-(4-propyl-piperidin-1-yl)-phenoxy]-phenyl}-hexan-1-one |
| A46 | 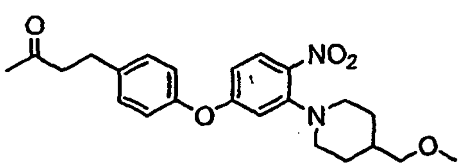 | 4- {4-[3-(4-Methoxymethyl-piperidin-1-yl)-4-nitro-phenoxy]-phenyl}-butan-2-one |
| A47 | 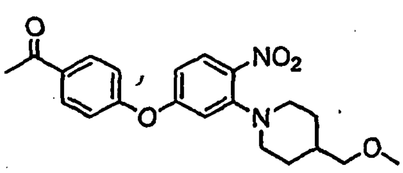 | 1-{4-[3-(4-Methoxymethyl-piperidin-1-yl)-4-nitro-phenoxy]-phenyl}-ethanone |

(continued)

| Cmpd# | Structure | Chemical Name |
|---|---|---|
| A48 | 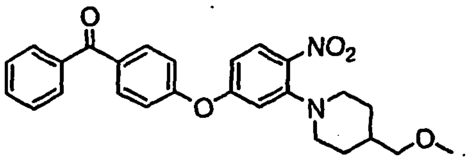 | {4-[3-(4-Methoxymethyl-piperidin-1-yl)-4-nitro-phenoxy]-phenyl}-phenyl-methanone |
| A49 | 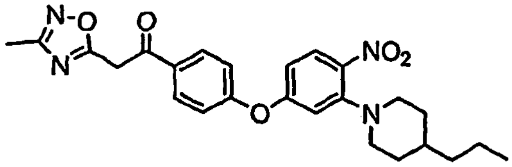 | 2-(3-Methyl-[1,2,4]oxadiazol-5-yl)-1-{4-[4-nitro-3-(4-propyl-piperidin-1-yl)-phenoxy]-phenyl}-ethanone |
| A50 | 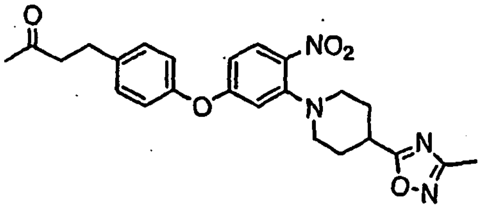 | 4-(4-{3-[4-(3-Methyl-[1,2,4]oxadiazol-5-yl)-piperidin-1-yl]-4-nitro-phenoxy}-phenyl)-butan-2-one |
| A51 | 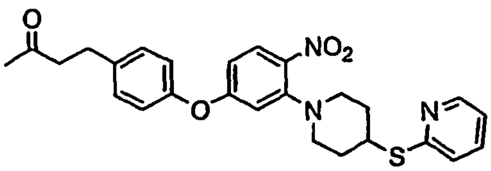 | 4-(4-{4-Nitro-3-[4-(pyridin-2-ylsulfanyl)-piperidin-1-yl]-phenoxy}-phenyl)-butan-2-one |
| A52 | 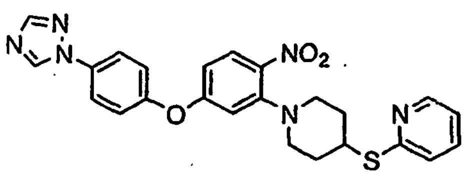 | 2-{1-[2-Nitro-5-(4-[1,2,4]triazol-1-yl-phenoxy)-phenyl]-piperidin-4-ylsulfanyl}-pyridine |
| A53 | 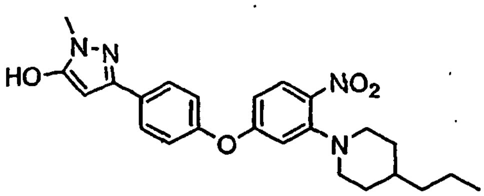 | 2-Methyl-5-{4-[4-nitro-3-(4-propyl-piperidin-1-yl)-phenoxy]-phenyl}-2H-pyrazol-3-ol |
| A54 | 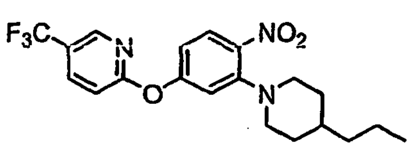 | 2-[4-Nitro-3-(4-propyl-piperidin-1-yl)-phenoxy]-5-trifluoromethyl-pyridine |

(continued)

| Cmpd# | Structure | Chemical Name |
|---|---|---|
| A55 | 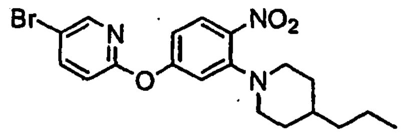 | 5-Bromo-2-[4-nitro-3-(4-propyl-piperidin-1-yl)-phenoxy]-pyridine |
| A56 | 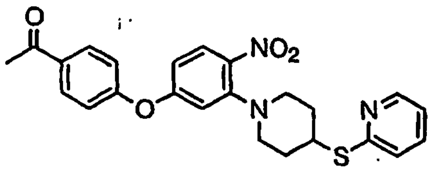 | 1-(4-{4-Nitro-3-[4-(pyridin-2-ylsulfanyl)-piperidin-1-yl]-phenoxy}-phenyl)-ethanone |
| A57 | 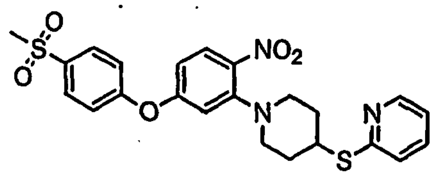 | 2-{1-[5-(4-Methanesulfonyl-phenoxy)-2-nitro-phenyl]-piperidin-4-ylsulfanyl}-pyridine |
| A58 | 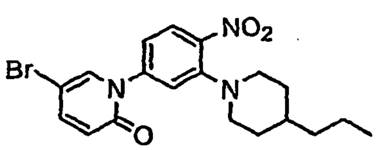 | 5-Bromo-1-[4-nitro-3-(4-propyl-piperidin-1-yl)-phenyl]-11H-pyridin-2-one |
| A59 | 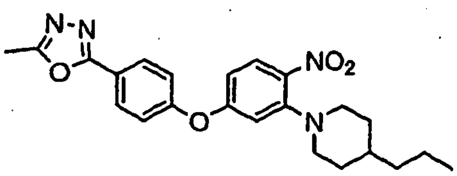 | 1-{5-[4-(5-Methyl-[1,3,4]oxadiazol-2-yl)-phenoxy]-2-nitro-phenyl}-4-propyl-piperidine |
| A60 | 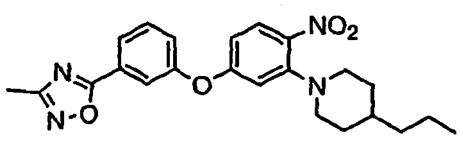 | 1-{5-[3-(3-Methyl-[1,2,4]oxadiazol-5-yl)-phenoxy]-2-nitro-phenyl}-4-propyl-piperidine |

**TABLE B**

| Cmpd# | Structure | Chemical Name |
|---|---|---|
| B1 | 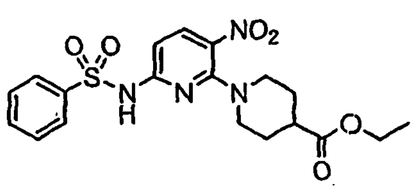 | 6'-Benzenesulfonylamino-3'-nitro-3,4,5,6-tetrahydro-2H-[1,2']bipyridinyl-4-carboxylic acid ethyl ester |

(continued)

| Cmpd# | Structure | Chemical Name |
| --- | --- | --- |
| B2 | 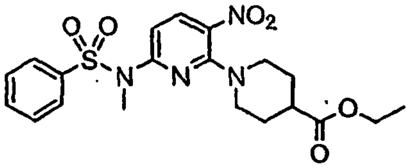 | 6'-(Benzenesulfonyl-methyl-amino)-3'-nitro-3,4,5,6-tetrahydro-2H-[1,2']bipyridinyl-4-carboxylic acid ethyl ester |
| B3 | 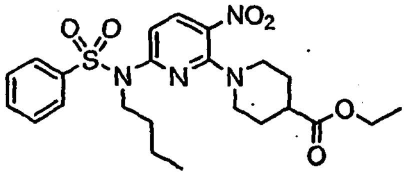 | 6'-(Benzenesulfonyl-butyl-amino)-3'-nitro-3,4,5,6-tetrahydro-2H-[1,2']bipyridinyl-4-carboxylic acid ethyl ester |
| B4 | 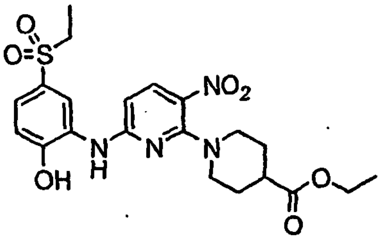 | 6'-(5-Ethanesulfonyl-2-hydroxy-phenylamino)-3'-nitro-3,4,5,6-tetrahydro-2H-[1,2']bipyridinyl-4-carboxylic acid ethyl ester |
| B5 | 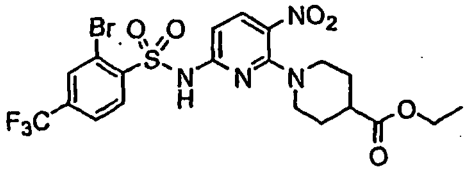 | 6'-(2-Bromo-4-trifluoromethyl-benzenesulfonylamino)-3'-nitro-3,4,5,6-tetrahydro-2H-[1,2']bipyridinyl-4-carboxylic acid ethyl ester |
| B6 | 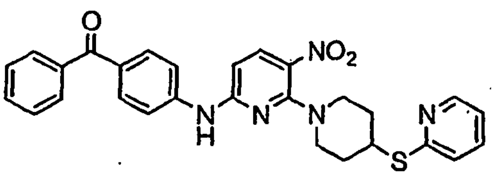 | {4-[3'-Nitro-4-(pyridin-2-ylsulfanyl)-3,4,5,6-tetrahydro-2H-[1,2']bipyridinyl-6'-ylamino]-phenyl}-phenyl-methanone |
| B7 | 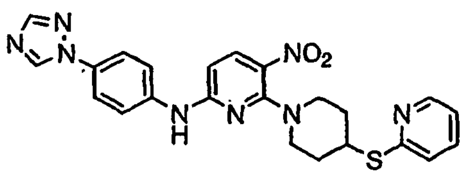 | [3'-Nitro-4-(pyridin-2-ylsulfanyl)-3,4,5,6-tetrahydro-2H-[1,2']bipyridinyl-6'-yl]-(4-[1,2,4]triazol-1-yl-phenyl)-amine |
| B8 | 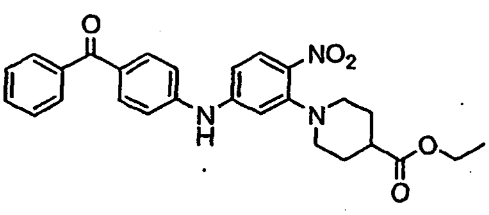 | 1-[5-(4-Benzoyl-phenylamino)-2-nitro-phenyl]-piperidine-4-carboxylic acid ethyl ester |

(continued)

| Cmpd# | Structure | Chemical Name |
|---|---|---|
| B9 | 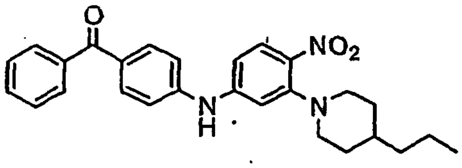 | {4-[4-Nitro-3-(4-propyl-piperidin-1-yl)-phenylamino]-phenyl}-phenyl-methanone |

Some embodiments of the present invention include a pharmaceutical composition comprising at least one compound according to any of the compound embodiments disclosed herein and a pharmaceutically acceptable carrier.

Additionally, compounds of Formula **(Ia)** encompass all pharmaceutically acceptable solvates, particularly hydrates, thereof. The present invention also encompasses diastereomers as well as optical isomers, e.g. mixtures of enantiomers including racemic mixtures, as well as individual enantiomers and diastereomers, which arise as a consequence of structural asymmetry in certain compounds of Formula **(Ia).** Separation of the individual isomers or selective synthesis of the individual isomers is accomplished by application of various methods which are well known to practitioners in the art.

The phrase "pharmaceutically acceptable" is employed herein to refer to those compounds, materials, compositions, and/or dosage forms which are, within the scope of sound medical judgement, suitable for use in contact with the tissues of human beings and animals without excessive toxicity, irritation, allergic response, or other problem or complication, commensurate with a reasonable benefit/risk ratio.

The present invention also includes pharmaceutically acceptable salts of the compounds described herein. As used herein, "pharmaceutically acceptable salts" refers to derivatives of the disclosed compounds wherein the parent compound is modified by converting an existing acid or base moiety to its salt form. Examples of pharmaceutically acceptable salts include, but are not limited to, mineral or organic acid salts of basic residues such as amines; alkali or organic salts of acidic residues such as carboxylic acids; and the like. The pharmaceutically acceptable salts of the present invention include the conventional non-toxic salts or the quaternary ammonium salts of the parent compound formed, for example, from non-toxic inorganic or organic acids. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, Pa.,1985, p. 1418, and the most recent edition thereof; and Journal of Pharmaceutical Science, 66, 2 (1977).

**INDICATIONS**

In addition to the foregoing beneficial uses for compounds of the present invention disclosed herein, compounds of the invention are useful in the prophylaxis or treatment of additional diseases. Without limitation, these include the following.

The most significant pathologies in Type II diabetes are impaired insulin signaling at its target tissues ("insulin resistance") and failure of the insulin-producing cells of the pancreas to secrete an appropriate degree of insulin in response to a hyperglycemic signal. Current therapies to treat the latter include inhibitors of the β-cell ATP-sensitive potassium channel to trigger the release of endogenous insulin stores, or administration of exogenous insulin. Neither of these achieves accurate normalization of blood glucose levels and both carry the risk of inducing hypoglycemia. For these reasons, there has been intense interest in the development of pharmaceuticals that function in a glucose-dependent action, i.e. potentiators of glucose signaling. Physiological signaling systems which function in this manner are well-characterized and include the gut peptides GLP1, GIP and PACAP. These hormones act via their cognate G-protein coupled receptor to stimulate the production of cAMP in pancreatic β-cells. The increased cAMP does not appear to result in stimulation of insulin release during the fasting or preprandial state. However, a series of biochemical targets of cAMP signaling, including the ATP-sensitive potassium channel, voltage-sensitive potassium channels and the exocytotic machinery, are modified in such a way that the insulin secretory response to a postprandial glucose stimulus is markedly enhanced. Accordingly, agonists of novel, similarly functioning, β-cell GPCRs, including **RUP3,** would also stimulate the release of endogenous insulin and consequently promote normoglycemia in Type II diabetes.

It is also established that increased cAMP, for example as a result of GLP1 stimulation, promotes β-cell proliferation, inhibits β-cell death and thus improves islet mass. This positive effect on β-cell mass is expected to be beneficial

in both Type II diabetes, where insufficient insulin is produced, and Type I diabetes, where β-cells are destroyed by an inappropriate autoimmune response.

Some β-cell GPCRs, including RUP3, are also present in the hypothalamus where they modulate hunger, satiety, decrease food intake, controlling or decreasing weight and energy expenditure. Hence, given their function within the hypothalamic circuitry, agonists or inverse agonists of these receptors mitigate hunger, promote satiety and therefore modulate weight.

It is also well-established that metabolic diseases exert a negative influence on other physiological systems. Thus, there is often the codevelopment of multiple disease states (e.g. type I diabetes, type II diabetes, inadequate glucose tolerance, insulin resistance, hyperglycemia, hyperlipidemia, hypertriglyceridemia, hypercholesterolemia, dyslipidemia, obesity or cardiovascular disease in "syndrome X") or secondary diseases which clearly occur secondary to diabetes (e.g. kidney disease, peripheral neuropathy). Thus, it is expected that effective treatment of the diabetic condition will in turn be of benefit to such interconnected disease states.

Described herein is a method for prophylaxis or treatment of a metabolic disorder or complications thereof comprising administering to an individual in need of such prophylaxis or treatment a therapeutically effective amount of a compound of the present invention or a pharmaceutical composition thereof

Described herein is a method of decreasing food intake comprising administering to an individual in need of decreasing food intake a therapeutically effective amount of a compound of the present invention or pharmaceutical composition thereof.

Described herein is a method of inducing satiety comprising administering to an individual in need of inducing satiety a therapeutically effective amount of a compound of the present invention or pharmaceutical composition thereof. In some embodiments the individual is a mammal. In some embodiments the mammal is a human.

Described herein is a method of controlling or decreasing weight gain comprising administering to an individual in need of such controlling or decreasing weight gain a therapeutically effective amount of a compound of the present invention or pharmaceutical composition thereof.

Described herein is a method of modulating a **RUP3** receptor in an individual comprising contacting the receptor with a compound of the present invention. In some embodiments the compound is an agonist. In some embodiments the compound is an inverse agonist.

Described herein is a method of modulating a RUP3 receptor in an individual comprising contacting the receptor with a compound of the present invention wherein the modulation of the RUP3 receptor is prophylaxis or treatment of a metabolic disorder and complications thereof.

Described herein is a method of modulating a RUP3 receptor in an individual comprising contacting the receptor with a compound of the present invention wherein the modulation of the RUP3 receptor reduces food intake of the individual.

Described herein is a method of modulating a RUP3 receptor in an individual comprising contacting the receptor with a compound of the present invention wherein the modulation of the RUP3 receptor induces satiety in the individual.

Described herein is a method of modulating a **RUP3** receptor in an individual comprising contacting the receptor with a compound of the present invention wherein the modulation of the RUP3 receptor controls or reduces weight gain of the individual.

Some embodiments of the present invention include the use of a compound of the present invention for production of a medicament for use in prophylaxis or treatment of a metabolic disorder.

Some embodiments of the present invention include the use of a compound of the present invention for production of a medicament for use in decreasing food intake of an individual.

Some embodiments of the present invention include the use of a compound of the present invention for production of a medicament for use of inducing satiety in an individual.

Some embodiments of the present invention include the use of a compound of the present invention for production of a medicament for use in controlling or decreasing weight gain in an individual.

In some embodiments the metabolic disorder is type I, type II diabetes, inadequate glucose tolerance, insulin resistance, hyperglycemia; hyperlipidemia, hypertriglyceridemia, hypercholesterolemia, dyslipidemia or syndrome X.

In some embodiments of the present invention the metabolic disorder is type II diabetes.

In some embodiments of the present invention the metabolic disorder is hyperglycemia.

In some embodiments of the present invention the metabolic disorder is hyperlipidemia.

In some embodiments of the present invention the metabolic disorder is hypertriglyceridemia.

In some embodiments of the present invention the metabolic disorder is type I diabetes.

In some embodiments of the present invention the metabolic disorder is dyslipidemia.

In some embodiments of the present invention the metabolic disorder is syndrome X.

In some embodiments of the present invention the individual is a mammal.

In some embodiments of the present invention the mammal is a human.

In some embodiments of the present invention the human has a body mass index of about 18.5 to about 45.

In some embodiments of the present invention the human has a body mass index of about 25 to about 45.

In some embodiments of the present invention the human has a body mass index of about 30 to about 45.

In some embodiments of the present invention the human has a body mass index of about 35 to about 45.

Compounds of the present invention are identified as an agonist or an inverse agonist using methods known to those skilled in art, such as an assay as described in Example 1.

**Pharmaceutical compositions**

Some embodiments of the present invention include a method of producing a pharmaceutical composition comprising admixing at least one compound according to any of the compound embodiments disclosed herein and a pharmaceutically acceptable carrier.

A compound of the present invention can be formulated into pharmaceutical compositions using techniques well known to those in the art. Suitable pharmaceutically-acceptable carriers, outside those mentioned herein, are available to those in the art; for example, see Remington's Pharmaceutical Sciences, 16th Edition, 1980, Mack Publishing Co., (Oslo et al., eds.) and the most recent edition thereof.

While it is possible that, for use in the prophylaxis or treatment, a compound of the invention may in an alternative use be administered as a raw or pure chemical, it is preferable however to present the compound or active ingredient as a pharmaceutical formulation or composition further comprising a pharmaceutically acceptable carrier.

The invention thus further provides pharmaceutical formulations comprising a compound of the invention or a pharmaceutically acceptable salt or derivative thereof together with one or more pharmaceutically acceptable carriers therefor and/or prophylactic ingredients. The carrier(s) must be "acceptable" in the sense of being compatible with the other ingredients of the formulation and not overly deleterious to the recipient thereof.

Pharmaceutical formulations include these suitable for oral, rectal, nasal, topical (including buccal and sub-lingual), vaginal or parenteral (including intramuscular, subcutaneous and intravenous) administration or in a form suitable for administration by inhalation or insufflation.

The compounds of the invention, together with a conventional adjuvant, carrier, or diluent, may thus be placed into the form of pharmaceutical formulations and unit dosages thereof, and in such form may be employed as solids, such as tablets or filled capsules, or liquids such as solutions, suspensions, emulsions, elixirs, gels or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are capsules, tablets, powders, granules or a suspension, with conventional additives such as lactose, mannitol, corn starch or potato starch; with binders such as crystalline cellulose, cellulose derivatives, acacia, corn starch or gelatins; with disintegrators such as corn starch, potato starch or sodium carboxymethyl-cellulose; and with lubricants such as talc or magnesium stearate. The active ingredient may also be administered by injection as a composition wherein, for example, saline, dextrose or water may be used as a suitable pharmaceutically acceptable carrier.

The dose when using the compounds of Formula **(Ia)** can vary within wide limits, and as is customary and is known to the physician, it is to be tailored to the individual conditions in each individual case. It depends, for example, on the nature and severity of the illness to be treated, on the condition of the patient, on the compound employed or on whether an acute or chronic disease state is treated or prophylaxis is conducted or on whether further active compounds are administered in addition to the compounds of the Formula **(Ia).** Representative doses of the present invention include, about 0.01 mg to about 1000 mg, about 0.01 to about 750 mg, about 0.01 to about 500 mg, 0.01 to about 250 mg, 0.01 mg to about 200 mg, about 0.01 mg to 150 mg, about 0.01 mg to about 100 mg, and about 0.01 mg to about 75 mg. Multiple doses may be administered during the day, especially when relatively large amounts are deemed to be needed, for example 2, 3 or 4, doses. If appropriate, depending on individual behavior and as appropriate from the patients physician or care-giver it may be necessary to deviate upward or downward from the daily dose.

The amount of active ingredient, or an active salt or derivative thereof, required for use in treatment will vary not only with the particular salt selected but also with the route of administration, the nature of the condition being treated and the age and condition of the patient and will ultimately be at the discretion of the attendant physician or clinician. In general, one skilled in the art understands how to extrapolate in vivo data obtained in a model system, typically an animal model, to another, such as a human. Typically, animal models include, but are not limited to, the rodents diabetes models as described in Example 6, *infra* (other animal models have been reported by Reed and Scribner in Diabetes, Obesity and Metabolism,1, 1999, 75-86) or the inhibition of food intake model as described in Example 7, *infra.* In some circumstances, these extrapolations may merely be based on the weight of the animal model in comparison to another, such as a mammal, preferably a human, however, more often, these extrapolations are not simply based on weights, but rather incorporate a variety of factors. Representative factors include the type, age, weight, sex, diet and medical condition of the patient, the severity of the disease, the route of administration, pharmacological considerations such as the activity, efficacy, pharmacokinetic and toxicology profiles of the particular compound employed, whether a drug delivery system is utilized, on whether an acute or chronic disease state is being treated or prophylaxis is conducted or on whether further active compounds are administered in addition to the compounds of the Formula (Ia) and as part of a drug combination. The dosage regimen for treating a disease condition with the compounds and/or compositions of this invention is selected in accordance with a variety factors as cited above. Thus, the actual dosage regimen employed may vary widely and therefore may deviate from a preferred dosage regimen and one skilled in the art will recognize that dosage and dosage regimen outside these typical ranges can be tested and, where appropriate, may be used in the methods of this invention.

The desired dose may conveniently be presented in a single dose or as divided doses administered at appropriate intervals, for example, as two, three, four or more sub-doses per day. The sub-dose itself may be further divided, e.g., into a number of discrete loosely spaced administrations. The daily dose can be divided, especially when relatively large amounts are administered as deemed appropriate, into several, for example 2, 3 or 4, part administrations. If appropriate, depending on individual behavior, it maybe necessary to deviate upward or downward from the daily dose indicated.

The compounds of the present invention can be administrated in a wide variety of oral and parenteral dosage forms. It will be obvious to those skilled in the art that the following dosage forms may comprise, as the active component, either a compound of the invention or a pharmaceutically acceptable salt of a compound of the invention.

For preparing pharmaceutical compositions from the compounds of the present invention, the selection of a suitable pharmaceutically acceptable carrier can be either solid, liquid or a mixture of both. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid which is in a mixture with the finely divided active component.

In tablets, the active component is mixed with the carrier having the necessary binding capacity in suitable proportions and compacted to the desire shape and size.

The powders and tablets may contain varying percentage amounts of the active compound. A representative amount in a powder or tablet may contain from 0.5 to about 90 percent of the active compound; however, an artisan would know when amounts outside of this range are necessary. Suitable carriers for powders and tablets are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as an admixture of fatty acid glycerides or cocoa butter, is first melted and the active component is dispersed homogeneously therein, as by stirring. The molten homogenous mixture is then poured into convenient sized molds, allowed to cool, and thereby to solidify.

Formulations suitable for vaginal administration may be presented as pessaries, tampons, creams, gels, pastes, foams or sprays containing in addition to the active ingredient such carriers as are known in the art to be appropriate.

Liquid form preparations include solutions, suspensions, and emulsions, for example, water or water-propylene glycol solutions. For example, parenteral injection liquid preparations can be formulated as solutions in aqueous polyethylene glycol solution. Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The pharmaceutical compositions may take such forms as suspensions, solutions, or emulsions in oily or aqueous vehicles, and may contain formulatory agents such as suspending, stabilizing and/or dispersing agents. Alternatively, the active ingredient may be in powder form, obtained by aseptic isolation of sterile solid or by lyophilization from solution, for constitution with a

suitable vehicle, e.g. sterile, pyrogen-free water, before use.

Aqueous solutions suitable for oral use can be prepared by dissolving the active component in water and adding suitable colorants, flavours, stabilizing and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

For topical administration to the epidermis the compounds according to the invention may be formulated as ointments, creams or lotions, or as a transdermal patch.

Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents.

Formulations suitable for topical administration in the mouth include lozenges comprising active agent in a flavored base, usually sucrose and acacia or tragacanth; pastilles comprising the active ingredient in an inert base such as gelatin and glycerin or sucrose and acacia; and mouthwashes comprising the active ingredient in a suitable liquid carrier.

Solutions or suspensions are applied directly to the nasal cavity by conventional means, for example with a dropper, pipette or spray. The formulations may be provided in single or multi-dose form. In the latter case of a dropper or pipette, this may be achieved by the patient administering an appropriate, predetermined volume of the solution or suspension. In the case of a spray, this may be achieved for example by means of a metering atomizing spray pump.

Administration to the respiratory tract may also be achieved by means of an aerosol formulation in which the active ingredient is provided in a pressurized pack with a suitable propellant. If the compounds of the Formula **(Ia)** or pharmaceutical compositions comprising them are administered as aerosols, for example as nasal aerosols or by inhalation, this can be carried out, for example, using a spray, a nebulizer, a pump nebulizer, an inhalation apparatus, a metered inhaler or a dry powder inhaler. Pharmaceutical forms for administration of the compounds of the Formula (Ia) as an aerosol can be prepared by processes well-known to the person skilled in the art. For their preparation, for example, solutions or dispersions of the compounds of the Formula **(Ia)** in water, water/alcohol mixtures or suitable saline solutions can be employed using customary additives, for example benzyl alcohol or other suitable preservatives, absorption enhancers for increasing the bioavailability, solubilizers, dispersants and others, and, if appropriate, customary propellants, for example include carbon dioxide, CFC's, such as, dichlorodifluoromethane, trichlorofluoromethane, or dichlorotetrafluoroethane; and the like. The aerosol may conveniently also contain a surfactant such as lecithin. The dose of drug may be controlled by provision of a metered valve.

In formulations intended for administration to the respiratory tract, including intranasal formulations, the compound will generally have a small particle size for example of the order of 10 microns or less. Such a particle size may be obtained by means known in the art, for example by micronization. When desired, formulations adapted to give sustained release of the active ingredient may be employed.

Alternatively the active ingredients may be provided in the form of a dry powder, for example, a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

The pharmaceutical preparations arc preferably in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration are preferred compositions.

The term "prodrug" refers to compounds that are rapidly transformed in vivo to yield the parent compound of the above formulae, for example, by hydrolysis in blood. A thorough discussion is provided in T. Higuchi and V. Stella, "Pro-drugs as Novel Delivery Systems," VoL 14 of the A.C.S. Symposium Series, and in Bioreversible Carriers in Drug Design, ed. Edward B. Roche, American Pharmaceutical Association and Pergamon Press, 1987.

**Combination Therapy/Prophylaxis**

While the compounds of the invention can be administered as the sole active pharmaceutical agent as described herein above, they can also be used in combination with one or more agents belonging to the class of drugs known as α-glucosidase inhibitors, aldose reductase inhibitors, biguanides, HMG-CoA reductase inhibitors, squalene synthesis inhibitors, fibrate compounds, LDL catabolism enhancers and angiotensin converting enzyme (ACE) inhibitors.

α-Glucosidase inhibitors belong to the class of drugs which competitively inhibit digestive enzymes such as α-amylase, maltase, α-dextrinase, sucrase, etc. in the pancreas and or small intesting. The reversible inhibition by α-glucosidase inhibitors retard, diminish or otherwise reduce blood glucose levels by delaying the digestion of starch and sugars. Some representative examples of α-glucosidase inhibitors include acarbose, N-(1,3-dihydroxy-2-propyl)valiolamine (generic name; voglibose), miglitol, and α-glucosidase inhibitors known in the art.

The class of aldose reductase inhibitors are drugs which inhibit the first-stage rate-limiting enzyme in the polyol pathway that prevent or arrest diabetic complications. In the hyperglycemic state of diabetes, the utilization of glucose in the polyol pathway is increased and the excess sorbitol accumulated intracellularly as a consequence acts as a tissue toxin and hence evokes the onset of complications such as diabetic neuropathy, retinopathy, and nephropathy. Examples of the aldose reductase inhibitors include tolurestat; epalrestat; 3,4-dihydro-2,8-diisopropyl-3 -thioxo-2*H*-1,4-benzoxazine-4-acetic acid; 2,7-difluorospiro(9*H*-fluorene-9,4'-imidazolidine)-2',5'-dione (generic name: imirestat); 3-[(4-bromo-2-flurophenyl)methy]-7-chloro-3,4-dihydro-2,4-dioxo-1(2*H*)-quinazoline acetic acid (generic name: zenarestat); 6-fluoro-2,3-dihydro-2',5'-dioxo-spiro[4*H*-1-benzopyran-4,4'-imidazolidine]-2-carboxamide (SNK-860); zopolrestat; sorbinil; and 1-[(3-bromo-2-benzofuranyl)sulfonyl]-2,4-imidazolidinedione (M-16209), and aldose reductase inhibitors known in the art.

The biguanides are a class of drugs that stimulate anaerobic glycolysis, increase the sensitivity to insulin in the peripheral tissues, inhibit glucose absorption from the intestine, suppress of hepatic gluconeogenesis, and inhibit fatty acid oxidation. Examples of biguanides include phenformin, metformin, buformin, and biguanides known in the art.

Statin compounds belong to a class of drugs that lower blood cholesterol levels by inhibiting hydroxymethylglutalyl CoA (HMG-CoA) reductase. HMG-CoA reductase is the rate-limiting enzyme in cholesterol biosynthesis. A statin that inhibits this reductase lowers serum LDL concentrations by upregulating the activity of LDL receptors and responsible for clearing LDL from the blood. Examples of the tion compounds include rosuvastatin, pravastatin and its sodium salt, simvastatin, lovastatin, atorvastatin, fluvastatin, cerivastatin, and HMG-CoA reductase inhibitors known in the art.

Squalene synthesis inhibitors belong to a class of drugs that lower blood cholesterol levels by inhibiting synthesis of squalene. Examples of the squalene synthesis inhibitors include (S)-α-[Bis[2,2-dimethyl-1-oxopropoxy)methoxy] phosphinyl]-3-phenoxybenzenebutanesulfonic acid, mono potassium salt (BMS-188494) and squalene synthesis inhibitors known in the art.

Fibrate compounds belong to a class of drugs that lower blood cholesterol levels by inhibiting synthesis and secretion of triglycerides in the liver and activating a lipoprotein lipase. Fibrates have been known to activate peroxisome proliferators-activated receptors and induce lipoprotein lipase expression. Examples of fibrate compounds include bezafibrate, beclobrate, binifibrate, ciplofibrate, clinofibrate, clofibrate, clofibric acid, etofibrate, fenofibrate, gemfibrozil, nicofibrate, pirifibrate, ronifibrate, simfibrate, theofibrate, and fibrates known in the art.

LDL (low-density lipoprotein) catabolism enhancers belong to a class of drugs that lower blood cholesterol levels by increasing the number of LDL (low-density lipoprotein) receptors, examples include LDL catabolism enhancers known in the art.

Angiotensin converting enzyme (ACE) inhibitors belong to the class of drugs that partially lower blood glucose levels as well as lowering blood pressure by inhibiting angiotensin converting enzymes. Examples of the angiotensin converting enzyme inhibitors include captopril, enalapril, alacepril, delapril; ramipril, lisinopril, imidapril, benazepril, ceronapril, cilazapril, enalaprilat, fosinopril, moveltopril, perindopril, quinapril, spirapril, temocapril, trandolapril, and angiotensin converting enzyme inhibitors known in the art.

Insulin secretion enhancers belong to the class of drugs having the property to promote secretion of insulin from pancreatic β cells. Examples of the insulin secretion enhancers include sulfonylureas (SU). The sulfonylureas (SU) are drugs which promote secretion of insulin from pancreatic β cells by transmitting signals of insulin secretion via SU receptors in the cell membranes. Examples of the sulfonylureas include tolbutamide; chlorpropamide; tolazamide; acetohexamide; 4-chloro-N-[(1-pyrolidinylamino) carbonyl]-benzenesulfonamide (generic name: glycopyramide) or its ammonium salt; glibenclamide (glyburide); gliclazide; 1-butyl-3-metanilylurea; carbutamide; glibonuride; glipizide; gliquidone; glisoxepid; glybuthiazole; glibuzole; glyhexamide; glymidine; glypinamide; phenbutamide; tolcyclamide, glimepiride, and other insulin secretion enhancers known in the art. Other insulin secretion enhancers include N-[[4-(1-methylethyl)cyclohexyl)carbonyl]-D-phenylalanine (Nateglinide); calcium (2S)-2-benzyl 3-(cis-hexahydro-2-isoindolinylcarbonyl)propionate dihydrate (Mitiglinide, KAD-1229); and other insulin secretion enhancers known in the art

Thiazolidinediones belong to the class of drugs more commoningly known as TZDs. Examples of thiazolidinediones include rosiglitazone, pioglitazone, and thiazolidinediones known in the art.

Some embodiments of the invention include, a pharmaceutical composition comprising a compound of Formula **(Ia)** or a pharmaceutically acceptable salt thereof in combination with at least one member selected from the group consisting of an α-glucosidase inhibitor, an aldose reductase inhibitor, a biguanide, a HMG-CoA reductase inhibitor, a squalene synthesis inhibitor, a fibrate compound, a LDL catabolism enhancer and an angiotensin converting enzyme inhibitor. In another embodiment, the pharmaceutical composition is a compound of Formula **(Ia)** or a pharmaceutically acceptable salt thereof in combination with a HMG-CoA reductase inhibitor. In still another embodiment, the HMG-CoA reductase inhibitor is selected from the group consisting of prevastatin, simvastatin, lovastatin, atorvastatin, fluvastatin and lipitor.

In accordance with the present invention, the combination can be used by mixing the respective active components either all together or independently with a physiologically acceptable carrier, excipient, binder, diluent, etc., as described herein above, and administering the mixture or mixtures either orally or non-orally as a pharmaceutical composition. When a compound or a mixture of compounds of Formula **(Ia)** are administered as a combination therapy or prophylaxis with another active compound the therapeutic agents can be formulated as a separate pharmaceutical compositions given at the same time or at different times, or the therapeutic agents can be given as a single composition.

***Other Utility***

Described herein are radiolabelled compounds of Formula **(Ia)** that would be useful not only in radio-imaging but also in assays, both in vitro and in vivo, for localizing and quantitating **RUP3** in tissue samples, including human, and for identifying **RUP3** ligands by inhibition binding of a radiolabelled compound.

Described herein are novel **RUP3** assays which comprise such radiolabelled compounds.

Suitable radionuclides that may be incorporated in compounds of the present invention include but are not limited to $^{3}H$ (also written as T), $^{11}C$, $^{14}C$, $^{18}F$, $^{125}I$, $^{82}Br$, $^{123}I$, $^{124}I$, $^{125}I$, $^{131}I$, $^{75}Br$, $^{76}Br$, $^{15}O$, $^{13}N$, $^{35}S$ and $^{77}Br$. The radionuclide that is incorporated in the instant radiolabelled compounds will depend on the specific application of that radiolabelled compound. Thus, for in vitro **RUP3** labeling and competition assays, compounds that incorporate $^{3}H$, $^{14}C$, $^{125}I$, $^{131}I$, $^{35}S$ or $^{82}Br$ will generally be most useful. For radio-imaging applications $^{11}C$, $^{18}F$, $^{125}I$, $^{123}I$, $^{124}I$, $^{131}I$, $^{75}Br$, $^{76}Br$ or $^{77}Br$ will generally be most useful.

It is understood that a "radio-labelled " or "labelled compound" is a compound of Formula **(Ia)** that has incorporated at least one radionuclide; in some embodiments the radionuclide is elected from the group consisting of $^{3}H$, $^{14}C$, $^{125}I$, $^{35}S$ and $^{82}Br$; in some embodiments the radionuclide $^{3}H$ or $^{14}C$. Moreover, it should be understood that all of the atoms represented in the compounds of the invention can be either the most commonly occurring isotope of such atoms or the more scarce radio-isotope or nonradio-active isotope.

Synthetic methods for incorporating radio-isotopes into organic compounds including those applicable to those compounds of the invention are well known in the art and include incorporating activity levels of tritium into target molecules include: **A.** Catalytic Reduction with Tritium Gas - This procedure normally yields high specific activity products and requires halogenated or unsaturated precursors. **B.** Reduction with Sodium Borohydride [$^{3}H$] - This procedure is rather inexpensive and requires precursors containing reducible functional groups such as aldehydes, ketones, lactones, esters, and the like, **C.** Reduction with Lithium Aluminum Hydride [$^{3}H$] - This procedure offers products at almost theoretical specific activities. It also requires precursors containing reducible functional groups such as aldehydes, ketones, lactones, esters, and the like. **D.** Tritium Gas Exposure Labeling - This procedure involves exposing precursors containing exchangeable protons to tritium gas in the presence of a suitable catalyst. **E.** N-Methylation using Methyl Iodide [$^{3}H$] - This procedure is usually employed to prepare O-methyl or N-methyl ($^{3}H$) products by treating appropriate precursors with high specific activity methyl iodide ($^{3}H$). This method in general allows for high specific activity, such as about 80-87 Ci/mmol.

Synthetic methods for incorporating activity levels of $^{125}I$ into target molecules include: **A.** Sandmeyer and like reactions - This procedure transforms an aryl or heteroaryl amine into a diazonium salt, such as a tetrafluoroborate salt, and subsequently to $^{125}I$ labelled compound using $Na^{125}I$. A represented procedure was reported by Zhu, D.-G.,and co-workers in J. Org. Chem. 2002, 67, 943-948. **B.** Ortho $^{125}$Iodination of phenols - This procedure allows for the incorporation of $^{125}I$ at the ortho position of a phenol as reported by Collier, T. L. and co-workers in J. Labelled Compd Radiopharm. 1999, 42, S264-S266. **C.** Aryl and heteroaryl bromide exchange with $^{125}I$ - This method is generally a two step process. The first step is the conversion of the aryl or heteroaryl bromide to the corresponding tri-alkyltin intermediate using for example, a Pd catalyzed reaction [i.e. $Pd(Ph_3P)_4$] or through an aryl or heteroaryl lithium, in the presence of a tri-alkyltinhalide or hexaalkylditin [e.g., $(CH_3)_3SnSn(CH_3)_3$]. A represented procedure was reported by Bas, M.-D. and co-workers in J. Labelled Compd Radiopharm. 2001, 44, S280-S282.

A radiolabelled **RUP3** compound of Formula **(I)** can be used in a screening assay to identify/evaluate compounds. In general terms, a newly synthesized or identified compound (i.e., test compound) can be evaluated for its ability to reduce binding of the "radiolabelled compound of Formula **(Ia)"** to the **RUP3** receptor. Accordingly, the ability of a test compound to compete with the "radio-labelled compound of Formula **(Ia)"** for the binding to the **RUP3** receptor

directly correlates to its binding affinity.

The labelled compounds of the present invention bind to the **RUP3** receptor. In one embodiment the labelled compound has an $IC_{50}$ less than about 500 $\mu$M, in another embodiment the labelled compound has an $IC_{50}$ less than about 100 $\mu$M, in yet another embodiment the labelled compound has an $IC_{50}$, less than about 10 $\mu$M, in yet another embodiment the labelled compound has an $IC_{50}$ less than about 1 $\mu$M, and in still yet another embodiment the labelled inhibitor has an $IC_{50}$ less than about 0.1 $\mu$M.

Other uses of the disclosed receptors and methods will become apparent to those in the art based upon, inter alia, a review of this patent document.

The following examples are given to illustrate the invention and are not intended to be inclusive in any manner:

## EXAMPLES

The compounds of the present invention and their syntheses are further illustrated by the following examples. The examples are provided to further define the invention without, however, limiting the invention to the specifics of these examples.

### Example 1

### 96- well Cyclic AMP membrane assay for RUP3

Materials:

1) Adenlyl cyclase Activation Flashplate Assay kit from Perkin Elmer - 96 wells (SMP004B) and $^{125}$I tracer (NEX130) which comes with the kit. Keep in refrigerator, in a box, and do not expose the Flashplates to light.
2) Phosphocreatine - Sigma P-7936
3) Creatine Phosphokinase --- Sigma C-3755
4) GTP - Sigma G-8877
5) ATP- Sigma A-2383
6) IBMX - Sigma I-7018
7) Hepes - 1M solution in distilled water- Gibco #15630080
8) MgC12 - Sigma M-1028- 1M Solution
9) NaCl - Sigma - S6546 - 5M Solution
10) Bradford Protein Assay Kit - Biorad # 5000001
11) Proclin 300- Sigma #4-8126

Binding Buffer - filter through 45- micron Nalgene filter and keep in refrigerator. All buffers and membranes should be kept cold (in ice bucket) while performing assay.
20 mM Hepes, pH7.4
1 mM MgCl2
100 mM NaCl
2X Regeneration Buffer (make in binding buffer):

20 mM Phosphocreatine (1.02 gm/200 ml binding buffer)
20 units Creatine phosphokinase (4 mg/200 ml)
20 uM GTP (make up 10.46 mg/ml in binding buffer and add 200 ul /200 ml)
0.2 mM ATP (22.04 mg/200 ml)
100 mM IBMX (44.4 mg IBMX dissolved in 1 ml 100% DMSO first and then add the entire amount to 200 ml of buffer).

Regeneration buffer can be aliquotted into 40-45 ml portions (in 50 ml sterile tubes) and kept frozen for up to 2 months. Simply put the tube in a beaker with room temperature water to thaw out the regeneration buffer on the day of the assay.

### A. Assay procedure

1) Pipet 50 ul regeneration buffer in all 96 wells using Matrix 1250 8-channel pipettor.

2) Pipet 5 ul DMSO in columns 1 and columns 11 and 12.

3) Pipet 50 ul cAMP standards in columns 11 and 12 in this format: 50 pmole/well for row A, 25 pmole/well for row B, 12.5 pmol/well for row C, 5 picomol/well for row D, 2.5 pmole/well for row E, 1.25 pmole/well for row F, 0.5 pmole/well for row G, and 0 pmole/well (buffer only) for row H.

4) Pipet 5 ul compounds from each well of a compound dilution plate, for IC50s, using the following dilution scheme:

Well H: 400 uM compound (final concentration of compound in reaction mix = 5/100 x 400 uM = 20 uM
Well G: 1:10 dilution of Well H (i.e. 5ul compound from well H + 45 ul 100% DMSO) (final concentration = 2 uM)
Well F: 1:10 dilution of well G (final concentration = 0.2 uM)
Well E: 1:10 dilution of well F (final concentration = 0.02 uM)
Well D: 1:10 dilution of well E (final concentration = 0.002 uM)
Well C: 1:10 dilution of well D (final concentration = 0.0002 uM
Well B: 1:10 dilution of well C (final concentration = 0.00002 uM)
Well A: 1:10 dilution of well B (final concentration = 0.000002 uM)

$IC_{50}$s or $EC_{50}$s are done in triplicate. One Flashplate can therefore be set up to handle 3 compounds. (i.e., columns 2, 3, and 4 are for compound #1, columns 5, 6, and 7 are for compound #2, and columns 8, 9, and 10 are for compound #3.)

5) Add 50 ul **of RUP3** membranes to all wells in Columns 2 to 10. (Prior to the start of the assay, the frozen membrane pellets for both **RUP3** and CMV (cells transfected with an expression plasmid containing no **RUP3** sequences), are suspended in binding buffer, usually 1 ml binding buffer for 1 plate of membranes. The membranes are kept in ice all the time, and a polytron (Brinkmann polytron, model # PT-3100) is used (setting 6-7, for 15-20 seconds) to obtain a homogeneous membrane suspension.) Protein concentration is determined by Bradford protein assay kit using instructions given in the kit, using the standard supplied with the kit as a reference. The protein concentration of the membranes is adjusted with binding buffer, so that 50 ul membranes = 15 ug protein (i.e. 0.3 mg/ml protein).

6) In column 1, Wells A, B, C, and D, add 50 ul **RUP3** membranes. To wells E, F, G, and H, add 50 ul CMV membranes, (CMV membranes being of the same protein concentration as the **RUP3** membranes).

7) Incubate 1 hour at room temperature with agitation on a rotating platform shaker. Cover with foil while shaking.

8) After 1 hour, add (to all 96 wells), 100 ul of the $^{125}I$ tracer in detection buffer supplied with the Flashplate kit plus proclin, made up in the following manner:
Pipet per 10 ml per Flashplate: 100 ml of detection buffer + 1 ml $^{125}I$ + 0.2 ml of Proclin (the proclin helps to stop the production of cAMP). Make a smaller quantity of detection buffer mix if you have fewer plates.

9) Shake the plates on a rotating platform shaker for 2 hours, covering the plates with lead sheeting.

10) Seal the plates with the plastic film sealers provided with the Flashplate kit.

11) Count the plates using a TRILUX 1450 Microbeta Counter. See the door of the counter to determine which counting protocol to use.

12) Data is analyzed on the Arena Database according to the RUP3 non-fusion, $IC_{50}$ $EC_{50}$ for 96-well cAMP membrane assay, and the compound numbers and the concentrations of compounds must be entered by the user.

**B. Membrane Cyclase Criteria**

1) Signal to Noise:

An acceptable signal-to-noise ratio for **RUP3** can vary from 4 to 6. The raw cpms are approximately 1800 to 2500 for RUP3 and 3500-4500 for CMV. The cpm (or ultimately pmoles of cAMP/well) cannot be outside the standard curve, and should not approach well A of the standard curve (50 pmole/well) and well H (no cAMP).

Generally, the pmoles of cAMP produced by **RUP3** receptor are around 11 to 13 pmole/well (for 15 ug/well protein), and for CMV are between 2 to 3 pmole/well (for 15 ug protein /well).

2) Standard curve:

The slope should be linear and the error bars for duplicates should be very small. The receptor and CMV controls cannot be off scale of the standard curve, as described above. If the receptor controls are off the high end of the standard curve,i.e. 50 pmole/well or higher, one must repeat the experiment using less protein. However, such a case has not been observed with transiently transfected **RUP3** membranes (10 ug DNA/15 cm plate, using 60 ul Lipofectamine, and preparing membranes after 24 hour of transfection.)

3) The $IC_{50}$ or $EC_{50}$ curve should be at 100% (+ or- 20%) of control **RUP3** membranes at the top, and should go down to 0 (or up to 20%) at the bottom. The standard error of the triplicate determinations should be + or - 10%.

The compounds in the Examples, infra, were screened in the Membrane Cyclase Assay. Representative compounds are shown in the table below:

| Compound | RUP3 ($IC_{50}$) Membrane Cyclase ($\mu$M) |
|---|---|
| A30 | 0.185 |
| A51 | 0.346 |
| A52 | 0.230 |

The other compounds in the Examples were tested and they showed $IC_{50}$ activities in the membrane cyclase assay less than about 500 $\mu$M.

**C. Stimulation of cAMP in HIT-T15 cells**

HIT-T15 (ATCC CRL#1777) is an immortalized hamster insulin-producing cell line. These cells express **RUP3** and therefore can be used to assess the ability of **RUP3** ligands to stimulate or inhibit cAMP accumulation via its endogenously expressed receptor. In this assay, cells are grown to 80% confluence and then distributed into a 96-well Flashplate (50,000 cells/ well) for detection of cAMP via a "cAMP Flashplate Assay" (NEN, Cat # SMP004). Briefly, cells are placed into anti-cAMP antibody-coated wells that contain either vehicle, the test ligand(s) at a concentration of interest, or 1 uM forskolin. The latter is a direct activator of adenylyl cyclase and serves as a positive control for stimulation of cAMP in HIT-T15 cells. All conditions are tested in triplicate. After a 1 hour incubation to allow for stimulation of cAMP, a Detection Mix containing $^{125}$I-cAMP is added to each well and the plate is allowed to incubate for another 1 hour. The wells are then aspirated to remove unbound $^{125}$I-cAMP. Bound $^{125}$I-cAMP is detected using a Wallac Microbeta Counter. The amount of cAMP in each sample is determined by comparison to a standard curve, obtained by placing known concentrations of cAMP in some wells on the plate.

**D. Stimulation of insulin secretion in HIT-T15 cells**

It is known that stimulation of cAMP in HIT-T15 cells causes an increase in insulin secretion when the glucose concentration in the culture media is changed from 3mM to 15 mM. Thus, **RUP3** ligands can also be tested for their ability to stimulate glucose-dependent insulin secretion (GSIS) in HIT-T15 cells. In this assay, 30,000 cells/well in a 12-well plate are incubated in culture media containing 3 mM glucose and no serum for 2 hours. The media is then changed; wells receive media containing either 3 mM or 15 mM glucose, and in both cases the media contains either vehicle (DMSO) or **RUP3** ligand at a concentration of interest. Some wells receive media containing 1 uM forskolin as a positive control. All conditions are tested in triplicate. Cells are incubated for 30 minutes, and the amount of insulin secreted into the media is determined by ELISA, using a kit from either Peninsula Laboratories (Cat # ELIS-7536) or Crystal Chem Inc. (Cat # 90060).

**E. Stimulation of insulin secretion in isolated rat islets**

As with HIT-T15 cells, it is known that stimulation of cAMP in isolated rat islets causes an increase in insulin

secretion when the glucose concentration in the culture media is changed from 60 mg/dl to 300 mg/dl. **RUP3** is an endogenously expressed **GPCR** in the insulin-producing cells of rat islets. Thus, **RUP3** ligands can also be tested for their ability to stimulate GSIS in rat islet cultures. This assay is performed as follows:

**A.** Select 75-150 islet equivalents (IEQ) for each assay condition using a dissecting microscope. Incubate overnight in low-glucose culture medium. (Optional.)

**B.** Divide the islets evenly into triplicate samples of 25-40 islet equivalents per sample. Transfer to 40 μm mesh sterile cell strainers in wells of a 6-well plate with 5 ml of low (60 mg/dl) glucose Krebs-Ringers Buffer (KRB) assay medium.

**C.** Incubate 30 minutes (1 hour if overnight step skipped) at 37˚C and 5% $CO_2$. Save the supernatants if a positive control for the RIA is desired.

**D.** Move strainers with islets to new wells with 5 ml/well low glucose KRB. This is the second pre-incubation and serves to remove residual or carryover insulin from the culture medium. Incubate 30 minutes.

**E.** Move strainers to next wells (Low 1) with 4 or 5 ml low glucose KRB. Incubate @ 37˚ C for 30 minutes. Collect supernatants into low-binding polypropylene tubes pre-labelled for identification and keep cold.

**F.** Move strainers to high glucose wells (300mg/dl, which is equivalent to 16.7mM). Incubate and collect supernatants as before. Rinse islets in their strainers in low-glucose to remove residual insulin. If the rinse if to be collected for analysis, use one rinse well for each condition (i.e. set of triplicates.)

**G.** Move strainers to final wells with low-glucose assay medium (Low 2). Incubate and collect supernatants as before.

**H.** Keeping cold, centrifuge supernatants at 1800rpm for 5 minutes @ 4-8˚C to remove small islets/islet pieces that escape the 40mm mesh. Remove all but lower 0.5 - 1 ml and distribute in duplicate to pre-labelled low-binding tubes. Freeze and store at <-20˚ C until insulin concentrations can be determined.

**I.** Insulin determinations are done as above, or by Linco Labs as a custom service, using their rat insulin RIA (Cat. # RI-13K).

**Example 2**

**A. RT-PCR analysis of RUP3 expression in human tissues (Figure 1A).**

RT-PCR was applied to determine the tissue distribution of **RUP3.** Oligonucleotides used for PCR had the following sequences:

ZC47: 5'-CATTGCCGGGCTGTGGTTAGTGTC-3' (forward primer), (SEQ ID NO:3);
ZC48: 5'-GGCATAGATGAGTGGGTTGAGCAG-3' (reverse primer), (SEQ ID NO:4);

and the human multiple tissue cDNA panels (MTC, Clontech) were used as templates (1 ng cDNA per PCR amplification). Twenty-two (22) human tissues were analyzed. **PCR** was performed using Platinum PCR SuperMix (Life Technologies, Inc.; manufacture instructions were followed) in a 50 ul reaction by the following sequences: step 1,95˚C for 4 min; step 2, 95˚C for 1 min; step 3, 60˚C for 30 sec; step 4, 72˚C for 1 min; and step 5, 72˚C for 7 min. Steps 2 through 4 were repeated 35 times.

The resulting PCR reactions (15 μl) were loaded on a 1.5% agarose gel to analyze the RT-PCR products, and a specific 466 base-pair DNA fragment representing RUP3 was specifically amplified from cDNA of pancreas origin. Low expression was also evident in subregions of brain.

**B. cDNA Dot-Blot analysis of RUP3 expression in human tissues (Figure 1B).**

Results from RT-PCR analysis were further confirmed in cDNA dot-blot analysis. In this assay, a dot-blot membrane containing cDNA from 50 human tissues (Clontech) was hybridized with a $^{32}P$-radiolabelled DNA probe having sequences derived from human **RUP3.** Hybridyzation signals were seen in pancreas and fetal liver, suggesting these tissues express **RUP3.** No significant expression was detected in other tissues analyzed.

**C. Analysis of RUP3 by RT-PCR with isolated human pancreatic islets of Langerhans (Figure 1C).**

Further analysis of **RUP3** by RT-PCR with isolated human pancreatic islets of Langerhans showed robust expression of **RUP3** in islet cells but not in control samples.

**D. Analysis of RUP3 expression with cDNAs of rat origin by RT-PCR (Figure 1D).**

**RUP3** expression was further analyzed with cDNAs of rat origin by RT-PCR technique. Tissue cDNAs used for this assay were obtained from Clontech except those for hypothalamus and islets, which were prepared in house. Concentrations of each cDNA sample were normalized via a control RT-PCR analysis of the house-keeping gene GAPDH before assaying for **RUP3** expression. Oligonucleotides used for PCR had the following sequences:

rat **RUP3 ("rRUP3")** forward: 5'-CATGGGCCCTGCACCTTCTTTG-3' (SEQ ID NO:5);
**rRUP3** reverse: 5'-GCTCCGGATGGCTGATGATAGTGA-3' (SEQ ID NO:6).

PCR was performed using Platinum PCR SuperMix (Life Technologies, Inc.; manufacture instructions were followed) in a 50 μl reaction by the following sequences: step 1,95˚C for 4 min; step 2, 95˚C for 1 min; step 3,60˚C for 30 sec; step 4, 72˚C for 1 min; and step 5, 72˚C for 7 min. Steps 2 through 4 were repeated 35 times.

The resulting PCR reactions (15 μl) were loaded on a 1.5% agarose gel to analyze the RT-PCR products, and a specific 547 base-pair DNA fragment representing rat **RUP3** was specifically amplified from cDNA of pancreas origin, revealing a similar expression profile with human. Of particular note, robust expression was seen in isolated islets and hypothalamus.

**Example 3**

**RUP3 protein expression is restricted to β cell lineage of pancreatic islets (Figure 2).** ›

**A. A polyclonal anti-RUP3 antibody was prepared in rabbits (Figure 2A).**

Rabbits were immunized with an antigenic peptide with sequence derived from rat RUP3 (**"rRUP3"**). The peptide sequence was RGPERTRESAYHIVTISHPELDG (SEQ ID NO: 7) and shared 100% identity with mouse **RUP3** in the corresponding region. A cysteine residue was incorporated at the N-terminal end of this antigenic peptide to facilitate KLH crosslinking before injecting into rabbits. The resulting antisera ("anti-**rRUP3**") and the corresponding preimmune sera ("pre-r**RUP3**") were tested for immune reactivity to mouse **RUP3** in immunobloting assays (lanes 1 thought 4). In this assay, the GST-**RUP3** fusion protein was readily recognized by the anti-**rRUP3** antisera (lane 4), but not by the preimmune sera (lane 2). The immunoreactive signal could be efficiently eliminated when the immunobloting assay was performed in the presence of excess antigenic peptide (lane 6).

**B. RUP3 expression in insulin-producing β cells of pancreatic islets (Figure 2B).**

Rat pancreas was perfused with 4% paraformaldehyde (PFA) in PBS and embedded in OCT embedding medium. Ten micron sections were prepared, fixed on glass slides, and immunostained with either pre-**rRUP3** (Figure **2B,** panel *a*) or with anti-**rRUP3** antisera (Figure **2B,** panels c and e) followed by secondary staining with donkey anti-rabbit IgG conjugated to the fluorochrome Cy-3. Each section was also co-immunostained with a monoclonal anti-insulin antibody (Santa Cruz, Figure **2B,** panels b and d) in primary staining followed by a secondary staining with donkey anti-mouse IgG conjugated with FITC, or with a goat anti-glucagon antibody (Santa Cruz, Figure **2B,** panel f) and donkey anti-goat IgG coupled to FITC. Immunofluorescent signals were examined under a fluorescent microscope. **RUP3** was found expressed in insulin producing cells (panels c and d), but not in glucagons producing cells (panels e and f). These data demonstrated that **RUP3** is expressed in β cells but not in β cells of the rat pancreatic islets. Analogous results were obtained when mouse pancreatic sections were investigated for **RUP3** expression.

**Example 4**

**Functional Activities of RUP3 *In Vitro* (Figure 3).**

It was established that **RUP3** stimulates the production of cAMP by cotransfection of 293 cells with: (1) a CRE-Luciferase reporter, wherein the ability to stimulate the production of firefly luciferase depends on increased cAMP in cells, and (2) an expression plasmid encoding the human form of **RUP3** (Figure **3A**). Note that cells co-transfected with

an expression plasmid containing no **RUP3** sequences ("CMV" in Figure **3A**) produce very little luciferase activity, whereas cells transfected with an expression plasmid encoding **RUP3** (Figure **3A**) have at least a 10-fold increase in luciferase activity. This indicates that **RUP3** stimulates the production of cAMP when introduced into 293 cells. This property of **RUP3** is conserved across species, because hamster **RUP3** stimulates luciferase activity when introduced into 293 cells in a manner analogous to that described for human **RUP3** (Figure **3B**).

It is established that, when cAMP is increased in insulin-producing cells of the pancreas, these cells exhibit an enhanced ability to secrete insulin when glucose concentrations rise. To test whether **RUP3** might impart enhanced glucose-dependent insulin release, retrovirus containing human **RUP3** was used to generate Tu6 cells that express high levels of **RUP3.** Tu6 cells produce insulin, but do not express appreciable levels of **RUP3** and do not normally exhibit an increase in insulin release when increased glucose is present in the culture media. As shown in Figure **3C**, Tu6 cells transduced with a control virus that contains no receptor are still able to produce insulin, but do not show an increase in insulin secretion when the concentration of glucose in the culture media is shifted from 1 mM to 16 mM. By contrast, Tu6 cells transduced with **RUP3**-containing retrovirus display significant glucose-dependent insulin secretion (Figure 3C).

**Example 5**

**Functional Activities of RUP3 Agonists *In Vitro.***

To demonstrate that **RUP3** agonists stimulate endogenously expressed **RUP3** in insulin-producing cells, two *in vitro* models can be used. In the first of these, **RUP3** agonists are used to stimulate HIT-T15 cells, which express **RUP3** at significant levels, as indicated in the Northern blot shown in Figure **4.** Moreover, these cells are known to exhibit enhanced glucose-dependent insulin release when intracellular cAMP concentrations are elevated. In this example a **RUP3** agonist can be evaluated for its ability to stimulate cAMP production in HIT cells in comparison to the level seen with the adenyl cyclase activator forskolin. In this assay Compound A30 has been shown to be a robust stimulator of cAMP in HIT-T15 cells. Furthermore, Compound A30 has also been shown to stimulate insulin secretion in HIT cells exposed to 15 mM glucose (at a level comparable to that seen with the adenyl cyclase activator forskolin). This indicates that Compound A30 is a very robust potentiator of insulin secretion in HIT-T15 cells.

Isolated rat islets are the other *in vitro* model used to demonstrate the efficacy of **RUP3** agonists. In this model, agents that induce cAMP are not expected to stimulate insulin secretion when glucose concentrations are low (e.g. 60 mg/dl). However, when glucose concentrations are increased (e.g. to 300 mg/dl), these agents are expected to enhance insulin secretion to levels above those seen with glucose alone. In this model Compound A30 (10 μM) was shown to enhance glucose-dependent insulin release. Moreover, the level of enhancement can be compared to that seen with 25 nM GLP-1, a gut hormone known to act on islets in this manner.

**Example 6**

***In vivo* effects of RUP3 agonists on glucose homeostasis in mice.**

**A. Oral Glucose tolerance test (oGTT).**

Male C57bl/6N mice at age of 8 weeks are fasted for 18 hours and randomly grouped (n=11) to receive **a RUP3** agonist doses, or with control extendin-4 (ex-4, 1 μg/kg), a GLP-1 peptide analog known to stimulate glucose-dependent insulin secretion. A test compound is delivered, such as for example, orally via a gavage needle (p.o. volume at 100 μl). Control Ex-4 is delivered intraperitoneally. Thirty minutes after administration of test compound and control ex-4, mice are administered orally with dextrose at 5 g/kg dose. Levels of blood glucose are determined at the indicated time points using Glucometer Elite XL (Bayer).

**B. Acute response of db mice to RUP3 agonist.**

Male db mice (C57BL/KsOlahsd-Leprdb, diabetic, Harlan) at age of 10 weeks are randomly grouped (n=6) to receive vehicle (oral gavage), test compound, (such as for example 60 mg/kg, oral gavage), or Ex-4 (1 μg/kg, intraperitoneally). After compound administration, food is removed and blood glucose levels are determined at selected times. Reduction in blood glucose at each time point is expressed as percentage of original glucose levels, averaged from six animals for each group. These animals had blood glucose levels (fed state) of 300-400 mg/dl, significantly higher than non-diabetic wild type animals. Treatment with Ex-4 significantly reduced glucose levels compared to vehicle control.

### Example 7

### Inhibition Of Food Intake In Normal Fed Male Sprague-Dawley Rats

The *in vivo* activity of a test compound is assayed for its ability to regulate feeding behavior by measuring food consumption in normal fed rats during their dark cycle. Food intake is monitored over the dark phase since animals consume most of their food intake during the nocturnal period.

The test compound is assessed following acute administration. The study is based on a between-subject design (n=8 per group) and the effects of various doses of the test compound is compared to those of vehicle and a positive control. Rats [male Sprague-Dawley rats (220-300g)] naïve to drug treatment (i.e. never been exposed to drug prior to study). The anorectic drug d-fenfluramine (or, alternatively, exendin-4) serves as a positive control.

Prior to the study, the animals are weighed and separated into treatment groups in order to balance groups according to body weight. On the test day, animals are placed into individual cages for an hour. After this habituation period, animals are administered the test compound at doses, such as, 6.67, 20 and 60 mg/kg dissolved in 80% PEG400, 10% Tween 80 and 10% EtOH. The compound is administered intraperitoneally (volume of 1cc/kg) 30 min prior to the beginning of the dark phase. Animals are subsequently presented with a preweighed food cup with standard laboratory chow. Food consumption is determined by weighing the food cup at 2, 4, 6 and 22hr after the beginning of the dark cycle (i.e. lights off). Care is taken to collect all spillage. The food intake of animals in the different groups is monitored concurrently.

Food intake data is subjected to two-way repeated analysis of variance (ANOVA) with drug treatment as a between-subject factor and time as a repeated factor. Newman-Keuls tests are performed to assess whether differences between the vehicle mean and the drug-treated mean at each time point were significant. All statistical analyses are performed using Sigma Stat (version 2.0)

### Example 8

### CRE-Luciferase Assay in 293 Cells

293 cells were plated in 96-well tissue culture plates at a concentration of 20,000 cells per well. The following day, the cells are transfected with a mixture of pCRE-Luc (Stratagene, Cat. # 219076), the indicated expression plasmid, and pEGFP-N1 (Clontech, Cat. # 6085-1) at a ratio of 5:1:0.25 using Lipofectamine Reagent (Invitrogen, Cat. #18324-020) according to the manufacturer's directions. pEGFP-N1 en odes a "green fluorescent protein" and was used as a control to determine that most cells were successfully transfected. After 24-48 hr, the cells were lysed *in situ* with 100 ul/ well reconstituted Luclite buffer (Luclite Reporter Gene Assay Kit, Packard, Cat. # 6016911), according to the manufacturer's directions. After a 10 minute incubation in the dark, luminescence was measured using a TRILUX 1450 Microbeta Counter (Wallac).

### Example 9

### Generation of Tu6/RUP3 Stable Lines

To produce Tu6 cells that express **RUP3** at high levels, a retrovirus bearing an expression cassette for **RUP3** was generated. Briefly, **RUP3** coding sequence was cloned into the retroviral vector pLNCX2 (Clontech, Cat # 6102-1). The amphotropic packaging cell line PT-67 (Clontech , K1060-D) was then transfected with either the parental vector pLNCX2 or pLNCX2/RUP3 using Lipofectamine and stable lines were established using guidelines provided by the PT-67 vendor. Retrovirus-containing supernatant was obtained by collecting media from the resultant stables according to the manufacturer's directions. Tu6 cells, in a 10 cm dish, were then infected with retrovirus by incubating in a solution of 1 ml viral supernatant/ 9 ml culture media containing 40 ug/ml polybrene for 24 hours. The medium was then changed to culture media containing 300 ug/ml G418. G418-resistant clones were ultimately created by virtue of the neomycin-resistance gene cassette present in the pLNCX2 vector, thus indicating the successful integration of retrovirus into the Tu6 genome. The expression of **RUP3** in the Tu6/**RUP3** G418-resistant colonies was confirmed by Northern blot.

### Example 10

### Insulin secretion, Tu6 Stables

To measure insulin secretion from rodent insulin-producing cell lines, cells were first cultured overnight in serum-free, glucose-deficient media. The following morning, the cells were then placed in the same media supplemented

with either 1 mM or 16 mM glucose. After an incubation of 4 hours, the media was collected and analyzed for insulin content using a Rat Insulin Enzyme-Immunoassay (ELA) System (Amersham Pharmacia Biotech, Cat. # RPN 2567). Typically, the assay was performed using multiple dilutions of sample media in order to ensure that the sample measurements fell within the boundaries of the standard curve (generated using known amounts of insulin), as recommended by the manufacturer.

**Example 11**

**RUP3 RNA Blot**

To determine the expression of **RUP3** in insulin-producing or non islet cells, the following cell lines were obtained and cultured according to guidelines provided by American Type Culture Collection or the indicated provider.

| Cell Line | Provider | Cat. # |
|---|---|---|
| HIT-T15 | American Type Culture Collection | CRL-1777 |
| NIT-1 | American Type Culture Collection | CRL-2055 |
| RIN-5F | American Type Culture Collection | CRL-2058 |
| Tu-6 | Ole Madsen, Hagedom Res. Lab. | N/A |
| $\alpha$TC-9 | American Type Culture Collection | CRL-2350 |
| RIN-14B | American Type Culture Collection | CRL-2059 |
| ARIP | American Type Culture Collection | CRL-1674 |
| AR42J | American Type Culture Collection | CRL-1492 |
| Panc-1 | American Type Culture Collection | CRL-1469 |
| BxPc-3 | American Type Culture Collection | CRL-1687 |
| 293 | Q-Biogene | AES0503 |
| NIH-3T3 | American Type Culture Collection | CRL-1658 |

Total RNA was isolated from each of these cell lines using TRIZOL (Invitrogen, Cat # 15596-018), subjected to electrophoresis through an agarose/formaldehyde gel and an RNA blot was prepared using standard molecular biological techniques. A radiolabelled **RUP3** probe, corresponding to the full-length coding sequence of **RUP3,** was prepared using a Prime-It II Random Primer Labeling Kit (Stratagene, Cat # 300385). The denatured probe, 10 ml ExpressHyb solution (Clontech, Cat # 8015-2) and the RNA blot were incubated in a hybridization oven, washed and exposed to film using standard molecular biology practices.

**Example 12**

**Receptor Binding Assay**

In addition to the methods described herein, another means for evaluating a test compound is by determining binding affinities to the **RUP3** receptor. This type of assay generally requires a radiolabelled ligand to the **RUP3** receptor. Absent the use of known ligands for the **RUP3** receptor and radiolabels thereof, compounds of Formula **(Ia)** can be labelled with a radioisotope and used in an assay for evaluating the affinity of a test compound to the **RUP3** receptor.

A radiolabelled **RUP3** compound of Formula **(I)** can be used in a screening assay to identify/evaluate compounds. In general terms, a newly synthesized or identified compound (i.e., test compound) can be evaluated for its ability to reduce binding of the "radiolabelled compound of Formula **(Ia)"** to the **RUP3** receptor. Accordingly, the ability to compete with the "radio-labelled compound of Formula **(Ia)"** or **Radiolabelled RUP3 Ligand** for the binding to the RUP3 receptor directly correlates to its binding affinity of the test compound to the RUP3 receptor.

**ASSAY PROTOCOL FOR DETERMINING RECEPTOR BINDING FOR RUP3:**

**A. RUP3 RECEPTOR PREPARATION**

293 cells (human kidney, ATCC), transiently transfected with 10 ug human RUP3 receptor and 60 ul Lipofectamine (per 15-cm dish), were grown in the dish for 24 hours (75% confluency) with a media change and removed with 10 ml/dish of Hepes-EDTA buffer (20mM Hepes + 10 mM EDTA, pH 7.4). The cells were then centrifuged in a, Beckman Coulter centrifuge for 20 minutes, 17,000 rpm (JA-25.50 rotor). Subsequently, the pellet was resuspended in 20 mM Hepes + 1 mM EDTA, pH 7.4 and homogenized with a 50- ml Dounce homogenizer and again centrifuged. After removing the supernatant, the pellets were stored at -80˚C, until used in binding assay. When used in the assay, membranes were thawed on ice for 20 minutes and then 10 mL of incubation buffer (20 mM Hepes, 1 mM $MgCl_2$, 100 mM NaCl, pH 7.4) added. The membranes were then vortexed to resuspend the crude membrane pellet and homogenized with a Brinkmann PT-3100 Polytron homogenizer for 15 seconds at setting 6. The concentration of membrane protein was determined using the BRL Bradford protein assay.

**B. BINDING ASSAY**

For total binding, a total volume of 50ul of appropriately diluted membranes (diluted in assay buffer containing 50mM Tris HCl (pH 7.4), 10mM $MgCl_2$, and 1mM EDTA; 5-50ug protein) is added to 96-well polyproylene microtiter plates followed by addition of 100ul of assay buffer and 50ul of **Radiolabelled RUP3 Ligand.** For nonspecific binding, 50 ul of assay buffer is added instead of 100ul and an additional 50ul of 10uM cold **RUP3** is added before 50ul of **Radiolabelled RUP3 Ligand** is added. Plates are then incubated at room temperature for 60-120 minutes. The binding reaction is terminated by filtering assay plates through a Microplate Devices GF/C Unifilter filtration plate with a **Brandell** 96-well plate harvestor followed by washing with cold 50 mM Tris HCl, pH 7.4 containing 0.9% NaCl. Then, the bottom of the filtration plate are sealed, 50ul of Optiphase Supermix is added to each well, the top of the plates are sealed, and plates are counted in a Trilux MicroBeta scintillation counter. For compound competition studies, instead of adding 100ul of assay buffer, 100ul of appropriately diluted test compound is added to appropriate wells followed by addition of 50ul of **Radiolabelled RUP3 Ligand.**

**C. CALCULATIONS**

The test compounds are initially assayed at 1 and 0.1 μM and then at a range of concentrations chosen such that the middle dose would cause about 50% inhibition of a **Radio-RUP3 Ligand** binding (i.e., $IC_{50}$). Specific binding in the absence of test compound ($B_O$) is the difference of total binding ($B_T$) minus non-specific binding (NSB) and similarly specific binding (in the presence of test compound) (B) is the difference of displacement binding ($B_D$) minus non-specific binding (NSB). $IC_{50}$ is determined from an inhibition response curve, logit-log plot of % $BB_O$ vs concentration of test compound.

$K_i$ is calculated by the Cheng and Prustoff transformation:

$$K_i = IC_{50} / (1 + [L]/K_D)$$

where [L] is the concentration of a **Radio-RUP3 Ligand** used is the assay and $K_D$ is the dissociation constant of a **Radio-RUP3 Ligand** determined independently under the same binding conditions.

**CHEMISTRY**

**SYNTHESES OF COMPOUNDS OF THE PRESENT INVENTION**

**EXAMPLE 13**

Illustrated syntheses for compounds of Formula **(Ia)** are shown in Figure 5 where the symbols have the same definitions as used throughout this disclosure.

**Chemistry:**

Proton nuclear magnetic resonance ($^1H$ NMR) spectra were recorded on a Varian Mercury Vx-400 equipped

with a 4 nucleus auto switchable probe and z-gradient or a Bruker Avance-400 equipped with a QNP (Quad Nucleus Probe) or a BBI (Broad Band Inverse) and z-gradient. Chemical shifts are given in parts per million (ppm) with the residual solvent signal used as reference. NMR abbreviations are used as follows: s = singlet, d = doublet, t = triplet, q = quartet, m = multiplet, br = broad. Microwave irradiations were carried out using the Smith Synthesizer (Personal Chemistry). Thin-layer chromatography (TLC) was performed on silica gel 60 $F_{254}$ (Merck), preparatory thin-layer chromatography (prep TLC) was preformed on PK6F silica gel 60 A 1 mm plates (Whatman), and column chromatography was carried out on a silica gel column using Kieselgel 60, 0.063-0.200 mm (Merck). Evaporation was done in vacuo on a Buchi rotary evaporator. Celite 545 ® was used during palladium filtrations.

LCMS specs: 1) PC: HPLC-pumps: LC-10AD *VP*, Shimadzu Inc.; HPLC system controller: SCL-10A *VP*, Shimadzu Inc; UV-Detector: SPD-10A *VP,* Shimadzu Inc; Autosampler: CTC HTS, PAL, Leap Scientific; Mass spectrometer: API 150BX with Turbo Ion Spray source, AB/MDS Sciex; Software: Analyst 1.2. 2) Mac: HPLC-pumps: LC-8A *VP,* Shimadzu Inc; HPLC system controller: SCL-10A *VP*, Shimadzu Inc.

UV-Detector: SPD-10A *VP,* Shimadzu Inc; Autosampler: 215 Liquid Handler, Gilson Inc; Mass spectrometer: API 150EX with Turbo Ion Spray source, AB/MDS Sciex Software: Masschrom 1.5.2.

**EXAMPLE 13.1**

**SYNTHESES OF COMPOUNDS OF THE PRESENT INVENTION AND OTHER REFERENCE COMPOUNDS**

**General Method 1a.**

**Representative Example**

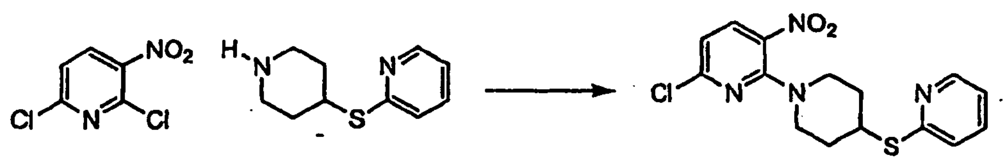

Compound 2,6-dichloro-3-nitropyridine (2 g, 0.01 mol) was dissolved in dichloromethane (17 ml) and cooled to 0 ˚C. To this was added diisopropylethyl amine (1.5 eqv, 2 g, 0.015 mol) followed by a solution of 2-(piperidin-4-ylsulfanyl)pyridine (1 eqv., 1.63 g, 0.01 mol) in dichloromethane (2 ml) drop wise. The mixture was stirred at 0˚C for 30minutes and then concentrated in vacuo. Flash column chromatography [Hexane: Ethyl Acetate=2:1] provided 6'-chloro-3'-nitro-4-(pyridin-2-ylsulfanyl)-3,4,5,6-tetrahydro-2H-[1,2']bipyridinyl as yellow oil (2.59 g, 83 %). $^1$H NMR 400MHz $CDCl_3$, δ (ppm): 8.09 (d, 1H); 6.68 (d, 1H): 4.17 (q, 2H); 3.81 (dt, 2H); 3.18 (td, 2H); 2.61 (m, 1H); 2.08 (dd, 2H); 1.86 (td, 2H); 1.28 (t, 3H). Exact mass calculated for $C_{13}H_{16}ClN_3O_4$ 313.08, LCMS (ESI) m/z 314.2 ($M+H^+$, 100 %)

**General Method 1b.**

**Representative Example**

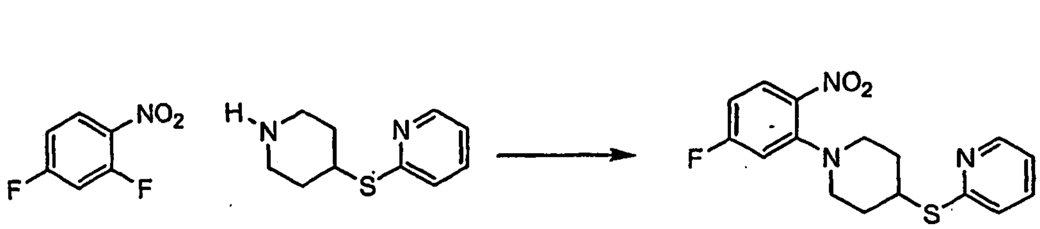

To a solution of 2,4-difluoronitrobenzene (175 mg, 1.1 mmol) and dichloromethane (17 ml) was added diisopropylethyl amine (2 eqv., 284 mg, 2.2 mmol) followed by a solution of 2-(piperidin-4-ylsulfanyl)pyridine 2HCl (1.1 eqv, 318 mg, 1.2 mmol) and diisopropylethyl amine (2 eqv, 310 mg, 2.4 mmol) in dichloromethane (2 ml) drop wise. The mixture was stirred at room temperature for 24 hours and then concentrated in vacuo. Flash column chromatography [Hexane: Ethyl Acetate=9:1] provided 2-[1-(5-fluoro-2-nitro-phenyl)-piperidin-4-ylsulfanyl]-pyridine as yellow oil (288 mg,

78 % yield). $^1$H NMR 400MHz $CDCl_3$ δ (ppm): 8.28 (s, 1H); 7.74 (dd, 1H); 7.33 (td, 1H); 7.03 (d, 1H); 6.84 (dd, 1H); 6.62 (dd, 1H); 6.50 (td, 1H); 3.94 (m, 1H); 3.16 (dt, 2H); 2.89 (td, 2H); 2.08 (dt, 2H); 1.78 (td, 2H). Exact mass calculated for $C_{16}H_{16}FN_3O_2S$ 333.09, LCMS (ESI) m/z 334.4 (M+H$^+$, 100 %).

**Compound A1:**

**6'-[4-(2-Methoxycarbonyl-acetyl)-phenoxy]-3'-nitro-3,4,5,6-tetrahydro-2H-[1,2']bipyridinyl4-carboxylic acid ethyl ester.**

**General Method 2**

A mixture of 6'-chloro-3'-nitro-3,4,5,6-tetrahydro-2H-[1,2']bipyridinyl-4-carboxylic acid ethyl ester (106 mg, 0.34 mmol), 4-(2-Methoxycarbonyl-acetyl)-phenol (1.5 eqv, 98 mg, 0.51 mmol) and potassium carbonate(1.5 eqv, 72 mg, 0.51 mmol) in DMF (5 ml) was stirred at room temperature overnight. The crude product was quenched with water (5 ml) and extracted with Ethyl Acetate (5 ml x 3). The mixture was concentrated in vacuo. Flash chromatography (hexane: ethyl acetate = 3:1) provided Compound **A1** as yellow solid (73 mg, 46 % yield). $^1$H NMR 400MHz $CDCl_3$ δ (ppm): 8.32 (d, 1H); 8.03 (d, 2H); 7.87 (d, 1H); 7.23 (d, 1H); 6.35 (d, 1H); 4.14 (q, 2H); 3.81 (s, 3H); 3.61 (d, 2H); 3.02 (t, 2H); 2.53 (m,1H); 1.91 (t, 2H); 1.77 (d, 2H); 1.28 (t, 3H). Exact mass calculated for $C_{23}H_{25}N_3O_8$ 471.16, LCMS (ESI) m/z 472.0 (M+H$^+$, 100 %).

**COMPOUND A2**

**1-[4-(4-Acetyl-3'-nitro-3,4,5,6-tetrahydro-2H-[1,2']bipyridinyl-6'-yloxy)-phenyl]-ethanone**

**General Method 3**

A mixture of 6'-chloro-3'-nitro-3,4,5,6-tetrahydro-2H-[1,2']bipyridinyl-4-carboxylic acid ethyl ester (63 mg, 0.2 mmol), 4-acetylphenol (1.3 eqy, 35 mg, 0.26 mmol) and potassium carbonate (1.3 eqv, 36 mg, 0.26 mmol) in DMF(1 ml) was stirred at 100 ˚C for 3 minutes under microwave conditions on a Smith Synthesizer. HPLC provided Compound **A2** as yellow oil (57 mg, 69 % yield). $^1$H NMR 400MHz $CDCl_3$ δ (ppm): 8.31 (d, 1H); 8.02 (d, 2H); 7.23 (t, 2H); 6.31 (d, 1H); 4.18 (q, 2H); 3.58 (dt, 2H); 3.01 (td, 2H); 2.63 (s, 3H); 2.57 (m, 1H); 1.91 (dt, 2H); 1.74 (td, 2H); 1.26(t, 3H). Exact mass calculated for $C_{21}H_{23}N_3O_6$ 413.16, LCMS (ESI) m/z 414.1 (M+H$^+$, 100 %).

**COMPOUND A3**

**6'-[4-(4-Hydroxy-benzenesulfonyn-phenoxy]-3'-nitro-3,4,5,6-tetrahydro-2H-[1,2']bipyridinyl-4-carboxylic acid ethyl ester**

Method 3. HPLC provided Compound **A3** as yellow oil (59 mg, 56 % yield). $^1$H NMR 400MHz $CDCl_3$ δ (ppm): 8.31 (d, 1H); 7.92 (d, 2H); 7.81 (d, 1H); 7.23 (d, 2H); 6.92 (d, 2H); 6.31 (d,1H); 4.18 (q, 2H); 3.48 (dt,2H); 2.81 (td, 2H); 2.51 (m, 1H); 1.81 (dt,2H); 1.64 (td, 2H); 1.31 (t, 3H). Exact mass calculated for $C_{25}H_{25}N_3O_8S$ 527.14, LCMS (ESI) m/z 528.1 (M+H$^+$, 100 %).

**COMPOUND A4**

**6'-(4-Imidazol-1-yl-phenoxy)-3'-nitro-3,4,5,6-tetrahydro-2H-[1,2']bipyridinyl-4-carboxylic acid ethyl ester**

Method 3. HPLC provided Compound **A4** as yellow oil (77 mg, 88 % yield). $^1$H NMR 400MHz $CDCl_3$ δ (ppm): 8.97(s, 1H); 8.31(d, 1H); 7.57(d, 2H);7.55(d, 1H); 7.5 (d, 1H);7.41(d, 2H); 6.38 (d,1H); 4.18 (q, 2H); 3.58 (dt,2H); 3.01 (td, 2H); 2.51(m, 1H); 1.81(dt,2H); 1.74(td, 2H); 1.28(t, 3H). Exact mass calculated for $C_{22}H_{23}N_5O_5$ 437.17, LCMS (ESI) m/z 438.1(M+H$^+$, 100 %).

**COMPOUND A5**

**6'-(4-Benzoyl-phenoxy)-3'-nitro-3,4,5,6-tetrahydro-2H-[1,2']bipyridinyl-4-carboxylic acid ethyl ester**

Method 3. HPLC provided Compound **A5** as yellow oil (23 mg, 24 % yield). $^1$H NMR 400MHz $CDCl_3$ δ (ppm): 8.31(d, 1H); 7.90(d,2H);7.81(d, 2H);7.61(t, 1H); 7.52(t, 2H); 7.26(d, 2H); 6.38 (d,1H); 4.18 (q, 2H);3.61(dt,2H); 3.01 (td,

2H); 2.53(m; 1H); 1.81(dt,2H); 1.74(td, 2H); 1.28(t, 3H). Exact mass calculated for $C_{26}H_{25}N_3O_6$ 475.17, LCMS (ESI) m/z 476.2($M+H^+$, 100%).

**COMPOUND A6**

**6'-[4-(2-Methoxy-ethyl)-phenoxy]-3'-nitro-3,4,5,6-tetrahydro-2H-[1,2']bipyridinyl-4-carboxylic acid ethyl ester**

Method 3. HPLC provided Compound **A6** as yellow oil (23 mg, 24 % yield). $^1$H NMR-400MHz $CDCl_3$ δ (ppm): 8.22 (d, 1H); 7.23 (d,2H); 7.04 (d, 2H); 6.11 (d,1H); 4.18 (q, 2H); 3.61 (dt,2H); 3.59 (t,2H); 3.39 (s,3H); 3.01 (td, 2H); 2.92 (t,2H); 2.53 (m, 1H); 1.91 (dt,2H); 1.74 (td, 2H); 1.28(t, 3H). Exact mass calculated for $C_{22}H_{27}N_3O_6$ 429.19, LCMS (ESI) m/z 430.3 ($M+H^+$, 100 %).

**COMPOUND A7**

**6'-(4-Cyclopentyl-phenoxy)-3'-nitro-3,4,5,6-tetrahydro-2H-[1,2']bipyridinyl-4-carboxylic acid ethyl ester**

Method 3. HPLC provided Compound **A7** as yellow oil (56 mg, 64 % yield). $^1$H NMR 400MHz $CDCl_3$ δ(ppm): 8.22 (d, 1H); 7.23 (d,2H); 7.04 (d, 2H); 6.20 (d,1H); 4.18 (q, 2H); 3.61 (dt,2H); 3.02 (td,2H); 3.01 (q, 1H); 2.53 (m, 1H); 2.10 (dt, 2H); 1.84 (td, 2H); 1.75 (m, 4H); 1.61 (m, 4H); 1.28 (t, 3H). Exact mass calculated for $C_{24}H_{29}N_3O_5$ 439.2, LCMS (ESI) m/z 440.4 ($M+H^+$, 100 %).

**COMPOUND A8**

**6'-(4'-Cyano-biphenyl-4-yloxy)-3'-nitro-3,4,5,6-tetrahydro-2H-[1,2']bipyridinyl-4-carboxylic acid ethyl ester**

Method 3. HPLC provided Compound **27** as yellow oil (78mg, 83% yield). $^1$H NMR 400MHz $CDCl_3$ δ (ppm): 8.29 (d, 1H); 7.77 (d,2H); 7.75 (d, 2H); 7.63 (d, 2H); 7.23 (d, 2H); 6.31 (d, 1H); 4.18 (q, 2H); 3.61 (dt, 2H); 3.02 (td, 2H); 2.53 (m, 1H); 1.98 (dt, 2H); 1.72 (td, 2H); 1.28 (t, 3H). Exact mass calculated for $C_{24}H_{29}N_3O_5$ 439.2, LCMS (ESI) m/z 440.4 ($M+H^+$, 100%).

**COMPOUND A9**

**3'-Nitro-6'-(4-sulfo-phenoxy)-3,4,5,6-tetrahydro-2H-[1,2']bipyridinyl-4-carboxylic acid ethyl ester**

Method 3. HPLC provided Compound A9 as yellow powder (21 mg, 23 % yield). $^1$H NMR 400 MHz $CDCl_3$ 8 (ppm): 8.3 5 (d, 1H); 7.90 (d, 2H); 7.16 (d, 2H); 6.43 (d, 1H); 4.18 (q, 2H); 3.61 (dt, 2H); 3.08 (td, 2H); 2.53 (m, 1H);1.98 (dt,2H); 1.76 (td, 2H); 1.21 (t, 3H). Exact mass calculated for $C_{19}H_{20}N_3NaO_8S$ 450.5, LCMS (ESI) m/z 451.9 ($M+H^+$, 100 %).

**COMPOUND A10**

**3'-Nitro-6'-(4-pyrrol-1-yl-phenoxy)-3,4,5,6-tetrahydro-2H-[1,2']bipyridinyl-4-carboxylic acid ethyl ester**

Method 3. HPLC provided Compound **A10** as yellow powder (66 mg, 76 % yield). $^1$H NMR 400MHz $CDCl_3$ δ (ppm): 8.30 (d, 1H); 7.43 (d,2H); 7.21 (d, 2H); 7.11 (d, 2H); 6.41 (d, 2H); 6.30 (d, 2H); 4.18 (q, 2H); 3.61 (dt, 2H); 3.08 (td, 2H); 2.53 (m, 1H); 1.81 (dt, 2H); 1.76 (td, 2H); 1.24 (t, 3H). Exact mass calculated for $C_{23}H_{24}N_4O_5$ 436.17, LCMS (ESI) m/z 437.2 ($M+H^+$, 100%).

**COMPOUND A11**

**6'-(4-Carbamoyl-phenoxy)-3'-nitro-3,4,5,6-tetrahydro-2H-[1,2']bipyridinyl-4-carboxylic acid ethyl ester**

Method 3. HPLC provided Compound A11 as yellow powder (59 mg, 71 % yield). $^1$H NMR 400MHz $CDCl_3$ δ (ppm): 8.20(d, 1H);7.80(d,2H); 7.19(d, 2H);6.21(d,1H); 4.08 (q, 2H);3.51(dt,2H);2.92(td,2H); 2.43(m, 1H);1.81 (dt,2H); 1.62(td, 2H); 1.18(t, 3H). Exact mass calculated for $C_{20}H_{22}N_4O_6$ 414.15, LCMS (ESI) m/z 415.4($M+H^+$, 100%).

**COMPOUND A12**

**3'-Nitro-6'-(4-[1,2,4]triazol-1-yl-phenoxy)-3,4,5,6-tetrahydro-2H-[1,2']bipyridinyl-4-carboxylic acid ethyl ester**

Method 3. HPLC provided Compound **A12** as yellow powder (61 mg, 69 % yield). $^1H$ NMR 400MHz $CDCl_3$ $\delta$ (ppm): 8.59 (s, 1H); 8.30 (d, 1H); 8.15 (s, 1H); 7.76 (d, 2H); 7.31 (d, 2H); 6.35 (d, 1H); 4.10 (q, 2H); 3.60 (dt, 2H); 3.01 (td, 2H); 2.50 (m, 1H); 1.83 (dt, 2H); 1.74 (td, 2H); 1.13 (t, 3H). Exact mass calculated for $C_{21}H_{22}N_6O_5$ 438.17, LCMS (ESI) m/z 439.6(M+H$^+$, 100%)

**COMPOUND A13**

**3'-Nitro-6'-(4-[1,2,4]triazol-1-yl-phenoxy)-3,4,5,6-tetrahydro-2H-[1,2']bipyridinyl-4-carboxylic acid ethyl ester**

Method 3. HPLC provided mixture of Compound **A13** and the N linked isomer. Flash column chromatography [Hexane:Ethyl Acetate=1:2] provided Compound **A13** (15mg) $^1H$ NMR 400MHz $CDCl_3$ $\delta$ (ppm): **A13** 8.30(d, 1H);7.35 (d,1H);7.26(d,1H); 7.19(d, 1H);6.31(d,1H); 4.18 (q, 2H);3.57(dt,2H); 3.15(q,2H);3.00(td,2H); 2.53(m, 1H);1.84 (dt,2H); 1.72(td, 2H); 1.34(t, 3H).1.30(t,3H). Exact mass calculated for $C_{21}H_{26}N_4O_7S$ 478.15, LCMS (ESI) m/z 479.1(M+H$^+$, 100%).

**COMPOUND A14**

**3'-Nitro-6'-[4-(4-oxo-cyclohexyl)-phenoxy]-3,4,5,6-tetrahydro-2H-[1,2']bipyridinyl-4-carboxylic acid ethyl ester**

Method 2. Flash column chromatography [Hexane:Ethyl Acetate, 2:1] provided Compound **A14** as yellow solid (47 mg, 50 %). $^1H$ NMR 400MHz $CDCl_3$ $\delta$ (ppm): 8.14 (d, 1H); 7.31 (d, 2H); 7.08 (d, 2H); 6.21 (d,1H); 4.15 (q, 2H); 3.60 (dt,2H); 3.10(td,2H); 3.01 (m, 1H); 2.54 (m, 1H); 2.50 (dt,2H); 2.13 (dt, 2H); 1.95 (td,2H); 1.85 (dt,2H); 1.74 (td, 2H); 1.23 (t, 3H). Exact mass calculated for $C_{25}H_{29}N_3O_6$ 467.21, LCMS (ESI) m/z 468.5 (M+H$^+$, 100 %)

**COMPOUND A15**

**6'-(4'-Methoxy-biphenyl-4-yloxy)-3'-nitro-3,4,5,6-tetrahydro-2H-[1,2']bipyridinyl-4-carboxylic acid ethyl ester**

Method 2. HPLC provided Compound **A15** as yellow powder (46 mg, 48 % yield). $^1H$ NMR 400MHz $CDCl_3$ $\delta$ (ppm): 8.28(d, 1H); 7.58(d, 2H);7.54(d, 2H);7.20(d,H); 7.01(d, 2H); 6.26(d,1H); 4.18 (q, 2H);3.84(s,3H);3.63(dt,2H);3.03 (td,2H);2.50(m, 1H);1.91(dt, 2H); 1.75(td,2H); 1.23(t, 3H). Exact mass calculated for $C_{26}H_{27}N_3O_6$ 477.19, LCMS (ESI) m/z 478.1(M+H$^+$, 100%)

**COMPOUND A16**

**3'-Nitro-6'-(4-[1,2,3]thiadiazol-4-yl-phenoxy)-3,4,5,6-tetrahydro-2H-[1,2']bipyridinyl-4-carboxylic acid ethyl ester**

Method 2. HPLC provided Compound A16 as yellow powder (39 mg, 43 % yield). $^1H$ NMR 400MHz $CDCl_3$ $\delta$ (ppm): 8.65(s, 1H); 8.30 (dd, 1H); 8.10 (dd, 2H); 7.25 (dd, 2H); 6.30 (dd, 1H); 4.18 (q, 2H); 3.60 (dt, 2H); 3.00 (td, 2H); 2.50 (m, 1H); 1.81(dt, 2H); 1.75 td, 2H); 1.23 (t, 3H). Exact mass calculated for $C_{21}H_{21}N_5O_5S$ 455.13, LCMS (ESI) m/z 456.3(M+H$^+$, 100%)

**COMPOUND A17**

**6'-[4-(1,3-Dioxo-1,3-dihydro-isoindol-2-yl)-phenoxyl-3'-nitro-3,4,5,6-tetrahydro-2H-[1,2']bipyridinyl-4-carboxylic acid ethyl ester**

Method 2. Flash column chromatography [Hexane:Ethyl Acetate=2:1] provided Compound **A17** as yellow solid (53 mg, 51 % yield). $^1H$ NMR 400MHz $CDCl_3$ $\delta$ (ppm): 8.28 (d, 1H); 7.98 (dd, 2H); 7.81 (dd, 2H); 7.50 (dd, 2H); 7.30 (dd, 2H); 6.30 (d, 1H); 4.18 (q, 2H); 3.63 (dt, 2H); 3.01 (td, 2H); 2.50 (m, 1H); 1.81 (dt, 2H); 1.78 (td, 2H); 1.23(t, 3H). Exact mass calculated for $C_{27}H_{24}N_4O_7$ 516.16, LCMS (ESI) m/z 517.3 (M+H$^+$, 100 %).

**COMPOUND A18**

**6'-[4-(2,5-Dioxo-imidazolidin-4-yl)-phenoxy]-3'-nitro-3,4,5,6-tetrahydro-2H-[1,2']bipyridinyl-4-carboxylic acid ethyl ester**

Method 2. HPLC provided Compound **A18** with its isomer as white solid (13 mg, 14 % yield).Exact mass calculated for $C_{22}H_{23}N_5O_7$ 469.16, LCMS (ESI) m/z 470.3 ($M+H^+$, 100 %).

**COMPOUND A19**

**3'-Nitro-6'-[4-(3-oxo-butyl)-phenoxy]-3,4,5,6-tetrahydro-2H-[1,2']bipyridinyl-4-carboxylic acid ethyl ester**

Method 3. HPLC provided Compound **A19** as yellow solid (39 mg, 44 %). $^1H$ NMR 400MHz $CDCl_3$ $\delta$ (ppm): 8.16 (d, 1H); 7.11 (d, 2H); 6.96 (d, 2H); 6.13 (d,1H); 4.06 (q, 2H); 3.52 (dt, 2H); 2.91 (td, 2H); 2.83 (t, 2H); 2.69 (t, 2H); 2.50 (m, 1H); 2.07 (s,3H); 1.79 (dt, 2H); 1.65 (td, 2H); 1.18 (t,3H). Exact mass calculated for $C_{23}H_{27}N_3O_6$441.19 LCMS (ESI) m/z 442.2($M+H^+$, 100%).

**COMPOUND A20**

**3-[4-(3'-Nitro-4-propyl-3,4,5,6-tetrahydro-2H-[1,2']bipyridinyl-6'-yloxy)-phenyl]-3-oxo-propionic acid methyl ester**

Method 3. HPLC provided Compound **A20** as yellow oil (36 mg, 41 %). $^1H$ NMR 400MHz $CDCl_3$ $\delta$ (ppm): 8.07 (d, 1H); 7.80 (d, 2H); 7.07 (d, 2H); 6.06 (d, 1H); 3.83 (s, 2H); 3.57 (s, 3H); 3.37 (d, 2H); 2.68 (td, 2H); 1.44 (d, 2H); 1.28-1.21 (m, 1H); 1.11 (q, 2H); 1.07 (m, 2H); 0.95 (m, 2H); 0.67 (t, 3H). Exact mass calculated for $C_{23}H_{27}N_3O_6$441.19 LCMS (ESI) m/z 442.4($M+H^+$, 100%).

**COMPOUND A21**

**4-[4-(3'-Nitro-4-propyl-3,4,5,6-tetrahydro-2H-[1,2']bipyridinyl-6'-yloxy)-phenyl]-butan-2-one**

Method 3. HPLC provided Compound **A21** as yellow oil (21 mg, 26 %). $^1H$ NMR 400MHz $CDCl_3$ $\delta$ (ppm): 8.45 (d,1H); 7.40 (d, 2H); 7.24 (d, 2H); 6.35 (d,1H); 3.83 (d, 2H); 3.16 (t, 2H); 3.07 (t,2H); 2.98 (t, 2H); 2.36 (s, 3H); 1.78 (d, 2H); 1.69-1.62 (m, 1H); 1.56-1.38 (m,6H); 1.34 (t,3H). Exact mass calculated for $C_{23}H_{29}N_3O_4$ 411.22 LCMS (ESI) m/z 412.2 ($M+H^+$, 100%).

**COMPOUND A22**

**4-{4-[3'-Nitro-4-(pyridin-2-ylsulfanyl)-3,4,5,6-tetrahydro-2H-[1,2']bipyridinyl-6'-yloxy]-phenyl}-butan-2-one**

Method 3. HPLC provided Compound **A22** as yellow solid (28 mg, 29 % yield). $^1H$ NMR 400MHz $CDCl_3$ $\delta$ (ppm): 8.39 (d, 1H); 8.08 (d, 1H); 7.47(t, 1H); 7.12(d, 1H); 7.01(d, 2H); 6.98 (t, 1H); 6.86(d, 2H); 6.06(d, 1H); 3.86(m, 1H); 3.42(dt, 2H); 2.96 (td, 2H); 2.72(t,2H); 2.58(t, 2H); 1.95( s,3H); 1.88(dt,2H); 1.55(td, 2H). Exact mass calculated for $C_{25}H_{26}N_4O_4S$ 478.17, LCMS (ESI) m/z 478.8($M+H^+$, 100%).

**COMPOUND A23**

**3'-Nitro-4-(pyridin-2-ylsulfanyl)-6'-(4-[1,2,4]triazol-1-yl-phenoxy)-3,4,5,6-tetrahydro-2H-[1,2']bipyridinyl**

Method 3. HPLC provided Compound **A23** as yellow solid (42 mg, 44 % yield). $^1H$ NMR 400MHz $CDCl_3$ $\delta$ (ppm): 8.52(s, 1H); 8.35 (d, 1H); 8.23(d, 1H); 8.05(s, 1H); 7.65(d, 2H); 7.42 (t, 1H); 7.24(d, 2H); 7.09(d, 1H); 6.93(t, 1H); 6.26(d, 1H); 3.99(m, 1H); 3.51 (dt,2H); 3.09 (td, 2H); 2.01 (dt,2H); 1.64 (td,2H). Exact mass calculated for $C_{23}H_{21}N_7O_3$ S 475.14, LCMS (ESI] m/z 476.2 ($M+H^+$, 100 %).

**COMPOUND A24**

**1-[5-(4-Benzoyl-phenoxy)-2-nitro-phenyl]-piperidine-4-carboxylic acid ethyl ester**

Method 2. The mixture was purified by HPLC to give Compound **A24** as an oil (20 mg, 21%). $^1$HNMR ($CDCl_3$, 400 MHz) δ 1.28 (t, 3H), 1.90-2.05 (m, 4H), 2.50-2.57 (m, 1H), 2.90 (t, 2H), 3.30-3.38 (m, 2H), 4.16 (q, 2H), 6.67 (d, 1H), 6.78 (d, 1H), 7.14 (d, 2H), 7.50 (t, 2H), 7.60 (t, 1H), 7.78 (d, 2H), 7.88 (d, 2H), 7.94 (d, 1H). Exact mass calculated for $C_{27}H_{26}N_2O_6$ 474.18, found 475.1 ($MH^+$).

**COMPOUND A25**

**1-{5-[4-(2-Methoxycarbonyl-acetyl)-phenoxyl-2-nitro-phenyl}-piperidine-4-carboxylic acid ethyl ester**

Method 3.Following the general procedure, Compound **A25** was obtained as an oil (5 %). $^1$HNMR ($CDCl_3$, 400 MHz) δ 1.28 (t, 3H), 1.90-2.05 (m, 4H), 2.40-2.47 (m, 1H), 2.62 (s, 3H), 2.85 (t, 2H), 3.22-3.26 (m, 2H), 4.16 (q, 2H), 6.55 (d, 1H), 6.71 (s, 1H), 7.05 (d, 2H), 7.85 (d, 1H), 8.00 (d, 2H). Exact mass calculated for $C_{24}H_{26}N_2O_8$ 470.17, found 471.0 ($MH^+$).

**COMPOUND A26**

**1-[5-(2-Amino-4-ethanesulfonyl-phenoxy)-2-nitro-phenyl]-piperidine-4-carboxylic acid ethyl ester**

Method 3. Following the general procedure, Compound **A26** was obtained as a yellow solid (48 %). $^1$HNMR ($CDCl_3$, 400 MHz) δ 1.27-1.33 (m, 6H), 1.90-2.05 (m, 4H), 2.50-2.57 (m, 1H), 2.88 (t, 2H), 3.13 (q, 2H), 3.22-3.25 (m, 2H), 4.16 (q, 2H), 6.52 (d, 1H), 6.70 (d, 1H), 7.00 (d, 1H), 7.24 (d, 1H), 7.36 (d, 1H), 7.90 (d, 1H). Exact mass calculated for $C_{22}H_{27}N_3O_7S$ 477.16, found 478.1 ($MH^+$).

**COMPOUND A27**

**1-{2-Nitro-5-[4-(3-oxo-butyl)-phenoxy]-phenyl}-piperidine-4-carboxylic acid ethyl ester**

Method 3. Following the general procedure, Compound **A27** was obtained as an oil (23 %). $^1$HNMR ($CDCl_3$, 400 MHz) δ 1.28 (t, 3H), 1.90-2.05 (m, 4H), 2.18 (s, 3H), 2.50-2.57 (m, 1H), 2.78-2.96 (m, 4H), 3.20-3.24 (m, 2H), 4.16 (q, 2H), 6.40 (d, 1H), 6.60 (s, 1H), 6.98 (d, 2H), 7.20 (d, 2H), 7.82 (d, 1H). Exact mass calculated for $C_{24}H_{28}N_2O_6$ 440.19, found 441.4 ($MH^+$).

**COMPOUND A28**

**4-{4-[4-Nitro-3-(4-propyl-piperidin-1-yl)-phenoxy]-phenyl}-butan-2-one**

Method 3. Following the general procedure, Compound **A28** was obtained as an oil (90 %). $^1$HNMR ($CDCl_3$, 400 MHz) δ 0.98 (t, 3H), 1.20-1.45 (m, 7H), 1.76 (d, 2H), 2.10 (s, 3H), 2.70-2.72 (m, 4H), 2.80-2.83 (m, 2H), 3.22-3.24 (m, 2H), 6.32 (dd, 1H), 6.52 (d, 1H), 6.90 (d, 2H), 7.16 (d, 2H), 7.80 (d, 1H). Exact mass calculated for $C_{24}H_{30}N_2O_4$ 410.22, found 411.2 ($MH^+$).

**COMPOUND A29**

**1-{4-[4-Nitro-3-(4-propyl-plperidin-1-yl)-phenoxy]-phenyl}-ethanone**

Method 3. Following the general procedure, Compound **A29** was obtained as a yellow solid (34 %). $^1$HNMR ($CDCl_3$, 400 MHz) δ 0.98 (t, 3H), 1.20-1.45 (m, 7H), 1.76 (d, 2H), 2.52 (s, 3H), 2.85-2.92 (m, 2H), 3.22-3.24 (m, 2H), 6.42 (d, 1H), 6.70 (d, 1H), 7.02 (d, 2H), 7.80 (d, 1H), 7.95 (d, 2H). Exact mass calculated for $C_{22}H_{26}N_2O_4$ 382.19, found 383.3 ($MH^+$).

**COMPOUND A30**

**3-{4-[4-Nitro-3-(4-propyl-piperidin-1-yl)-phenoxy]-phenyl}-3-oxo-propionic acid methyl ester**

Method 3. Following the general procedure, Compound **A30** was obtained as an oil (6 %). $^1$HNMR ($CDCl_3$, 400 MHz) δ 0.98 (t, 3H), 1.20-1.45 (m, 7H), 1.76 (d, 2H), 2.85-2.92 (m, 2H), 3.22-3.24 (m, 2H), 3.78 (s, 3H), 4.12 (s, 2H), 6.52 (d, 1H), 6.80 (d, 1H), 7.18 (d, 2H), 7.95 (d, 1H), 8.06 (d, 2H). Exact mass calculated for $C_{34}H_{28}N_2O_6$ 440.19, found 441.2

**COMPOUND A31**

**5-Ethanesulfonyl-2-(4-nitro-3-(4-propyl-piperidin-1-yl)-phenoxy]-phenylamine**

Following the general procedure, Compound **A31** was obtained as a yellow solid (56 %). $^1$HNMR ($CDCl_3$, 400 MHz) δ 0.98 (t, 3H), 1.20-1.45 (m, 10H), 1.76 (d, 2H), 2.76 (t, 2H), 3.05 (q, 2H), 3.22 (d, 2H), 6.42 (d, 1H), 6.67 (d, 1H), 6.92(d, 1H), 7.20 (dd, 1H), 7.29 (d, 1H), 7.82 (d, 1H). Exact mass calculated for $C_{22}H_{29}N_3O_5S$ 447.18, found 448.2 ($MH^+$).

**COMPOUND A32**

**{4-[4-Nitro-3-(4-propyl-piperidin-1-yl)-phenoxy]-phenyl}-phenyl-methanone**

Method 3. Following the general procedure, Compound **A32** was obtained as a yellow solid (83%). $^1$HNMR ($CDCl_3$, 400 MHz) δ 0.98 (t, 3H), 1.20-1.45 (m, 7H), 1.76 (d, 2H), 2.76 (t, 2H), 3.22 (d, 2H), 6.66 (dd, 1H), 6.76 (d, 1H), 7.15 (d, 2H), 7.52 (t, 2H), 7.62 (t, 1H), 7.83 (d, 2H), 7.89-7.93 (m, 3H). Exact mass calculated for $C_{27}H_{28}N_2O_4$ 444.20, found 445.2 ($MH^+$).

**Reference COMPOUND A33**

**1-{4-Nitro-3-[4-(3-oxo-butyl)-phenoxy]-phenyl}-piperidine-4-carboxylic acid ethyl ester**

Method 3. Following the general procedure, Compound **A33** was obtained as a yellow oil (19%). $^1$HNMR ($CDCl_3$, 400 MHz) δ 0.98 (t, 3H), 1.54-1.57 (m, 2H), 1.74-1.78 (m, 2H), 1.93 (s, 3H), 2.31-2.40 (m, 1H), 2.51-2.55 (m, 2H), 2.64-2.68 (m, 2H), 2.78 (t, 2H), 3.49 (d, 2H), 3.95 (q, 2H), 6.10 (d, 1H), 6.35 (d, 1H), 6.70 (d, 2H), 6.93 (d, 2H), 7.84 (d, 1H). Exact mass calculated for $C_{24}H_{28}N_2O_6$ 440.19, found 441.5 ($MH^+$).

**Reference COMPOUND A34**

**4-{4-[2-Nitro-5-(4-propyl-piperidin-1-yl)-phenoxy]-pbenyl}-butan-2-one**

Method 3. Following the general procedure, Compound **A34** was obtained as a yellow oil (35 %). $^1$HNMR ($CDCl_3$, 400 MHz) δ 0.98 (t, 3H), 1.23-1.34 (m, 7H), 1.76 (d, 2H), 2.16 (s, 3H), 2.77-2.93 (m, 6H), 3.78 (d, 2H), 6.33 (d, 1H), 6.60 (dd, 1H), 6.93 (d, 2H), 7.16 (d, 2H), 8.07 (d, 1H). Exact mass calculated for $C_{34}H_{30}N_2O_4$ 410.22, found 411.4 ($MH^+$).

**Reference COMPOUND A35**

**1-[3-(4-Benzoyl-phenory)-4-nitro-phenyl]-piperidine-4-carboxylic acid ethyl ester**

Method 3. Following the general procedure, Compound **A35** was obtained as a yellow solid (14 %). $^1$HNMR ($CDCl_3$, 400 MHz) δ 1.20 (t, 3H), 1.73-1.80 (m, 2H), 1.92-2.00 (m, 2H), 2.55-2.60 (m, 1H), 3.02 (t, 2H), 3.78 (d, 2H), 4.08 (2H), 6.41 (d, 1H), 6.62 (dd, 1H), 6.93 (d, 2H), 7.42 (t, 2H0, 7.50 (t, 1H), 7.69 (d, 2H), 7.74 (d, 2H), 8.05 (d, 1H). Exact mass calculated for $C_{27}H_{26}N_2O_6$ 474.18, found 475.1 ($MH^+$).

**Reference COMPOUND A36**

**{4-[2-Nitro-5-(4-propyl-piperidin-1-yl)-phenoxy]-phenyl}-phenyl-methanone**

Method 3. Following the general procedure, Compound **A36** was obtained as a yellow oil (24 %). $^1$HNMR

($CDCl_3$, 400 MHz) δ 0.88 (t, 3H), 1.13-1.24 (m, 6H), 1.30-1.33 (m, 1H), 1.76 (d, 2H), 2.85 (t, 2H), 3.78 (d, 2H), 6.44 (d, 1H), 6.66 (d, 1H), 6.92 (d, 2H), 7.36 (t, 2H), 7.48 (t, 1H), 7.66 (d, 2H), 7.72 (d, 2H), 8.02 (d, 2H). Exact mass calculated for $C_{27}H_{28}N_2O_4$ 444.20, found 445.2 ($MH^+$).

**COMPOUND A37**

**1-{5-[4-(2-Carboxy-ethyl)-phenoxy]-2-nitro-phenyl}-piperidine-4-carboxylic acid ethyl ester**

Following the general procedure, Compound **A37** was obtained as a yellow solid (51%). $^1$HNMR ($CDCl_3$, 400 MHz) δ 1.26 (t, 3H), 1.90-2.05 (m, 4H), 2.50-2.57 (m, 1H), 2.62 (t, 2H), 2.80 (t, 2H), 2.95 (t, 2H), 3.22 (d, 2H), 4.14 (q, 2H), 6.42 (d, 1H), 6.68 (d, 1H), 7.02 (d, 2H), 7.30 (d, 2H), 7.82 (d, 1H). Exact mass calculated for $C_{23}H_{26}N_2O_7$ 442.17, found 443.3 ($MH^+$).

**COMPOUND A38**

**1-{5-[4-(2-Carboxy-2-oxo-ethyn-phenoxy]-2-nitro-phenyl}-piperidine-4-carboxylic acid ethyl ester**

Following the general procedure, Compound **A38** was obtained as a yellow solid (14 %). $^1$HNMR ($CDCl_3$, 400 MHz) δ 1.26 (t, 3H), 1.90-2.05 (m, 4H), 2.50-2.57 (m, 1H), 2.80 (t, 2H), 3.22 (d, 2H), 3.32 (s, 2H), 4.14 (q, 2H), 6.42 (d, 1H), 6.60 (d, 1H), 6.78 (d, 1H), 7.02 (d, 2H), 7.82 (dd, 2H). Exact mass calculated for $C_{23}H_{24}N_2O_8$ 456.15, found 457.2 ($MH^+$).

**COMPOUND A39**

**1-[2-Nitro-5-(4-vinyl-phenoxy)-phenyl]-piperidine-4-carboxylic acid ethyl ester**

Following the general procedure, Compound **A39** was obtained as a yellow solid (59%). $^1$HNMR ($CDCl_3$, 400 MHz) δ 1.18 (t, 3H), 1.80-1.90 (m, 4H), 2.50-2.57 (m, 1H), 2.80 (t, 2H), 3.22 (d, 2H), 4.08 (q, 2H), 5.20 (d, 1H), 5.66 (d, 1H), 6.38 (dd, 1H), 6.56 (d, 1H), 6.62 (dd, 1H), 6,92 (d, 2H), 7.36 (d, 2H), 7.82 (d, 1H). Exact mass calculated for $C_{22}H_{24}N_2O_4$ 396.17, found 396.9 ($MH^+$).

**COMPOUND A40**

**3-{4-[4-Nitro-3-(4-propyl-piperidin-1-yl)-phenoxy]-phenyl}-propionic acid**

Following the general procedure, Compound **A40** was obtained as a yellow solid (39 %). $^1$HNMR ($CDCl_3$, 400 MHz) δ 0.93 (t, 3H), 1.23-1.34 (m, 7H), 1.76 (d, 2H), 2.60 (t, 2H), 2.78 (t, 2H), 2.95 (t, 2H), 3.22 (d, 2H), 6.42 (dd, 1H), 6.67 (d, 1H), 6.98 (d, 2H), 7.28 (d, 2H), 7.82 (d, 1H). Exact mass calculated for $C_{23}H_{280}N_2O_5$ 412.20, found 413.3 ($MH^+$).

**COMPOUND A41**

**3-{4-[4-Nitro-3-(4-propyl-piperidin-1-yl)-phenoxy]-phenyl}-2-oxo-propionic acid**

Following the general procedure, Compound **A41** was obtained as a brown solid (4 %). $^1$HNMR ($CDCl_3$, 400 MHz) δ 0.93 (t, 3H), 1.23-1.34 (m, 7H), 1.76 (d, 2H), 2.78 (t, 2H), 3.22 (d, 2H), 3.31 (s, 2H), 6.42 (d, 1H), 6.60 (dd, 1H), 6.80 (d, 1H), 7.05 (d, 2H), 7.84 (dd, 2H). Exact mass calculated for $C_{23}H_{26}N_2O_6$ 426.18, found 427.3 ($MH^+$).

**COMPOUND A42**

**1-[2-Nitro-5-(4-vinyl-phenoxy)-phenyl]-4-propyl-piperidine**

Following the general procedure, Compound **A42** was obtained as a yellow solid (45 %). $^1$HNMR ($CDCl_3$, 400 MHz) δ 0.83 (t, 3H), 1.23-1.34 (m, 7H), 1.76 (d, 2H), 2.78 (t, 2H), 3.22 (d, 2H), 5.19 (d, 1H), 5.62 (d, 1H), 6.38 (dd, 1H), 6.56 (d, 1H), 6.64 (dd, 1H), 6.94 (d, 2H), 7.36 (d, 2H), 7.78 (d, 1H). Exact mass calculated for $C_{22}H_{26}N_2O_3$ 366.19, found 367.1 ($MH^+$).

**COMPOUND A43**

**1-[2-Nitro-5-(4-vinyl-phenoxy)-phenyl]-4-propyl-piperidine**

Method 2. HPLC provided Compound **A43** as orange solid (7 1 mg, 87% yield). $^1H$ NMR 400MHz $CDCl_3$ δ (ppm): 7.94(d, 2H); 7.83 (d, 1H); 7.02(d, 2H); 6.66 (s, 1H); 6.47 (d, 2H); 3.22 ( d, 2H); 2.87 (t, 2H); 2.76 (t, 2H); 1.75-1.66 (m, 4H); 1.37-1.17 (m, 7H); 0.94 (t, 3H); 0.82( t, 3H). Exact mass calculated for $C_{24}H_{30}N_2O_4$ 410.22, LCMS (ESI) m/z 411.3($M+H^+$, 100%).

**COMPOUND A44**

**1-{4-[4-Nitro-3-(4-propyl-piperidin-1-yl)-phenoxyl-phenyl}-pentan-1-one**

Method 3. HPLC provided Compound **A44** as orange solid (62 mg, 73 % yield). $^1H$ NMR 400MHz $CDCl_3$ δ (ppm): 7.98 (d, 2H); 7.91 (d, 1H); 7.07(d, 2H); 6.79 (s, 1H); 6.57 (d, 1H); 3.33 ( d, 2H); 2.93 (t, 2H); 2.88 (d, 2H); 1.77 (d, 2H); 1.70(qu, 2H); 1.45-1.24 (m, 9H); 0.93( t, 3H); 0.87(t, 3H). Exact mass calculated for $C_{25}H_{32}N_2O_4$ 424.24, LCMS (ESI) m/z 425.2($M+H^+$, 100%).

**COMPOUND A45**

**1-{4-[4-Nitro-3-(4-propyl-piperidin-1-yl)-phenoxy]-phenyl}-hexan-1-one**

Method 3. HPLC provided Compound **A45** as orange solid (59 mg, 67 % yield). $^1H$ NMR 400MHz $CDCl_3$ δ (ppm): 7.92(d, 2H); 7.80 (d, 1H); 7.00(d, 2H); 6.62 (s, 1H); 6.42(d, 1H); 3.17 ( d, 2H); 2.86 (t, 2H); 2.71(t, 2H); 1.65(m, 4H); 1.34-1.17(m, 11H); 0.83 (t, 3H); 0.81 ( t, 3H). Exact mass calculated for $C_{26}H_{34}N_2O_4$ 438.25, LCMS (ESI) m/z 439.4 ($M+H^+$, 100%).

**COMPOUND A46**

**4-{4-[3-(4-Methoxymethyl-piperidin-1-yl)-4-nitro-phenoxy]-phenyl}-butan-2-one**

Method 2. Following the general procedure, Compound **A46** was obtained as a yellow oil (70%). $^1H$NMR ($CDCl_3$, 400 MHz) δ 1.20-1.26 (m, 2H), 1.50-1.60 (m, 3H), 1.96 (s, 3H), 2.58-2.60 (m, 4H), 2.70-2.72 (m, 2H), 3.03-3.10 (m, 4H), 3.15 (s, 3H), 6.20 (dd, 1H), 6.42 (d, 1H), 6.75 (d, 2H), 7.02 (d, 2H), 7.64 (d, 1H). Exact mass calculated for $C_{23}H_{28}N_2O_5$ 412.20, found 413.2 ($MH^+$).

**COMPOUND A47**

**1-{4-[3-(4-Methoxymethyl-piperidin-1-yl)-4-nitro-phenoxy]-phenyl}-ethanone**

Method 3. Following the general procedure, Compound **A47** was obtained as a yellow oil (4 %). $^1H$NMR ($CDCl_3$, 400 MHz) δ 1.20-1.26 (m, 2H), 1.50-1.60 (m, 3H), 2.54 (s, 3H), 2.72 (t, 2H), 3.20-3.25 (m, 4H), 3.30 (s, 3H), 6.40 (dd, 1H), 6.64 (d, 1H), 7.02 (d, 2H), 7.82 (d, 1H), 7.94 (d, 2H). Exact mass calculated for $C_{21}H_{24}N_2O_5$ 384.17, found 385.2 ($MH^+$).

**COMPOUND A48**

**{4-[3-(4-Methoxymethyl-piperidin-1-yl)-4-nitro-phenoxy]-phenyl}-phenyl-methanone**

Method 2. Following the general procedure, Compound **A48** was obtained as a yellow oil (81%). $^1H$NMR ($CDCl_3$, 400 MHz) δ 1.50-1.56 (m, 2H), 1.90-2.00 (m, 3H), 3.00 (t, 2H), 3.40-3.45 (m, 4H), 3.52 (s, 3H), 6.72 (dd, 1H), 6.90 (d, 1H), 7.28 (d, 2H), 7.62 (t, 2H), 7.78 (t, 1H), 7.92 (d, 2H), 8.00 (d, 2H), 8.06 (d, 1H). Exact mass calculated for $Q_{26}H_{26}N_2O_5$ 446.18, found 447.0 ($MH^+$).

**COMPOUND A49**

**2-(3-Methyl-[1,2,4]oxadiazol-5-yl)-1-{4-[4-nitro-3-(4-propyl-piperidin-1-yl)-phenoxy]-phenyl}-ethanone**

Method 2. Following the general procedure, Compound **A49** was obtained as a yellow solid (41%). $^1$HNMR ($CDCl_3$, 400 MHz) δ 0.81 (t, 3H), 1.15-1.35 (m, 7H), 1.65 (d, 2H), 2.62-2.80 (m, 2H), 3.14 (d, 2H), 3.20 (s, 3H), 4.78 (s, 2H), 6.62 (dt, 1H), 6.82 (dd, 1H), 7.10 (t, 2H), 7.80 (d, 2H), 8.03 (d, 1H). Exact mass calculated for $C_{25}H_{28}N_4O_5$ 464.21, found 465.2 ($MH^+$).

**COMPOUND A50**

**4-(4-{3-[4-(3-Methyl-[1,2,4]oxadiazol-5-yl)-piperidin-1-yl]-4-nitro-phenoxy}-phenyl)-butan-2-one**

Method 3. Following the general procedure, Compound **A50** was obtained as a yellow solid (61%). $^1$HNMR ($CDCl_3$, 400 MHz) δ 2.05-2.15 (m, 7H), 2.32 (s, 3H), 2.70-2.75 (m, 2H), 2.83-2.86 (m, 4H), 3.00-3.05 (m, 1H), 3.24-3.28 (m, 2H), 6.38 (dd, 1H), 6.57 (d, 1H), 6.90 (d, 2H), 7.15 (d, 2H), 7.82 (d, 1H). Exact mass calculated for $C_{24}H_{26}N_4O_5$ 450.1, found 451.0 ($MH^+$).

**COMPOUND A51**

**4-(4-{4-Nitro-3-[4-(pyridin-2-ylsulfanyl)-piperidin-1-yl]-phenoxy}-phenyl)-butan-2-one**

Method 3 provided Compound **A51** as yellow solid (89 mg, 94 % yield). $^1$H NMR 400MHz $CDCl_3$ δ (ppm): 8.67 (d, 1H); 7.84(d, 1H); 7.83(t, 1H); 7.45(d, 1H); 7.31 (t, 1H); 7.15( d, 2H); 6.91 (d, 2H); 6.55 (s, 1H); 6.43(d, 1H); 4.00-3.93 (m, 1H); 3.21(dt, 2H); 2.90(d, 2H); 2.85(t,2H); 2.73(t, 2H);2.14(d,2H); 2.10(s,3H);1.94-1.85(m,2H). Exact mass calculated for $C_{26}H_{27}N_3O_4S$477.17, LCMS (ESI) m/z 477.9($M+H^+$, 100%).

**COMPOUND A52**

**2-{1-[2-Nitro-5-(4-[1,2,4]triazol-1-yl-phenoxyrphenyl]-piperidin-4-ylsulfanyl}-pyridine**

Method 3 provided Compound **A52** as yellow solid (94 mg, 98 % yield). $^1$H NMR 400MHz $CDCl_3$ δ (ppm): 8.53 (s, 1H); 8.40(d, 1H); 8.06(s, 1H); 7.84(d, 1H); 7.65 (d, 2H); 7.48( t, 1H); 7.15 (d, 1H); 7.14 (d, 1H); 6.98(t, 1H); 6.61(s, 1H); 6.47(d, 1H); 3.97(m, 1H); 3.20(dt,2H); 2.92(td, 2H);2.13(d,2H); 1.90-1.81(m,2H). Exact mass calculated for $C_{24}H_{22}N_6O_3S$ 474.15, LCMS (ESI) m/z 475.2($M+H^+$, 100%).

**COMPOUND A53**

**2-Methyl-5-{4-[4-nitro-3-(4-propyl-piperidia-1-yl)-phenoxy]-phenyl}-2H-pyrazol-3-ol**

Method 3. Following the general procedure, Compound **A53** was obtained as a yellow solid (18%). $^1$HNMR ($CDCl_3$, 400 MHz) δ 0.82 (t, 3H), 1.17-1.30 (m, 7H), 1.70 (d, 2H), 2.81 (t, 2H), 3.21 (d, 2H), 3.86 (s, 3H), 5.87 (s, 1H), 6.62 (dd, 1H), 6.75 (d, 2H), 6.82 (d, 1H), 7.43 (d, 2H), 7.85 (d, 1H), 8.92 (s, 1H). Exact mass calculated for $C_{24}H_{28}N_4O_4$ 436.2, found 437.1 ($MH^+$).

**COMPOUND A54**

**2-[4-Nitro-3-(4-propyl-piperidin-1-yl)-phenoxy]-5-trifluoromethyl-pyridine**

Following the general procedure, Compound **A54** was obtained as an oil (70 %). $^1$HNMR ($CDCl_3$, 400 MHz) δ 0.92 (t, 3H), 1.17-1.30 (m, 7H), 1.70 (d, 2H), 2.92 (t, 2H), 3.35 (d, 2H), 6.92-6.96 (m, 2H), 7.14 (d, 1H), 7.70 (dd, 1H), 7.80 (d, 1H), 7.94 (d, 1H). Exact mass calculated for $C_{20}H_{22}F_3N_3O_3$ 409.16, found 410.4 ($MH^+$)

**COMPOUND A55**

**5-Bromo-2-[4-nitro-3-(4-propyl-piperidin-1-yl)-phenoxy]-pyridine**

Following the general procedure, Compound **A56** was obtained as an oil (60 %). $^1$HNMR ($CDCl_3$, 400 MHz)

δ 0.92 (t, 3H), 1.17-1.30 (m, 7H), 1.70 (d, 2H), 2.92 (t, 2H), 3.35 (d, 2H), 6.80 (d, 1H), 6.92 (dd, 1H), 7.10 (d, 1H), 7.55 (d, 1H), 7.63 (dd, 1H), 7.90 (d, 1H). Exact mass calculated for $C_{19}H_{22}BrN_3O_3$ 419.08, found 422.3 ($MH^+$).

**COMPOUND A56**

**1-(4-{4-Nitro-3-[4-(pyridin-2-ylsulfanyl)-piperidin-1-yl]-phenoxy}-phenyl)-ethanone**

Method 3 provided Compound **A56** as yellow solid (73 mg, 81 % yield). $^1H$ NMR 400MHz $CDCl_3$ δ (ppm): 8.70 (s, 1H); 7.94(d, 2H); 7.93(d, 1H); 7.87(d, 1H); 7.52 (d, 1H); 7.41( t, 1H); 7.04 (d, 2H); 6.63(d, 1H); 6.54(d, 1H); 3.97(m, 1H); 3.22(t, 2H); 2.89(t, 2H); 2.54(s,3H); 2.13(t, 2H);1.94-1.85(m,2H). Exact mass calculated for $C_{24}H_{23}N_3O_4S$ 449.14, LCMS (ESI) m/z 450.5($M+H^+$, 100%).

**COMPOUND A57**

**2-{1-[5-(4-Methanesulfonyl-phenoxy)-2-nitro-phenyl]-piperidin-4-ylsulfanyl}-pyridine**

Method 3 provided Compound **A57** as yellow solid (99 mg, 98 % yield). $^1H$ NMR 400MHz $CDCl_3$ δ (ppm): 9.40 (s, 1H); 8.65 (s, 1H); 7.90 (d, 2H); 7.87 (d, 1H); 7.81 (t, 1H); 7.43 (d, 1H); 7.30 (t, 1H); 7.12 (d, 2H); 6.67 (d, 1H); 6.55 (d, 1H); 4.01-3.96 (m, 1H); 3.23 (dt,2H); 3.01 (s, 3H); 2.92 (td,2H); 2.13 (dd,2H); 1.93-1.85 (m, 2H). Exact mass calculated for $C_{23}H_{23}N_3O_5S_2$ 485.11, LCMS (ESI) m/z 486.0($M+H^+$, 100%).

**COMPOUND A58**

**5-Bromo-1-[4-nitro-3-(4-propyl-piperidin-1-yl)-phenyl]-1H-pyridin-2-one**

Following the general procedure, Compound **A58** was obtained as a yellow solid (20 %). $^1HNMR$ ($CDCl_3$, 400 MHz) δ 0.92 (t, 3H), 1.17-1.30 (m, 7H), 1.70 (d, 2H), 2.92 (t, 2H), 3.25 (d, 2H), 6.58 (dd, 1H), 6.78 (d, 1H), 6.82 (d, 1H), 7.78 (dd, 1H), 7.84 (d, 1H), 8.20 (d, 1H). Exact mass calculated for $C_{19}H_{22}BrN_3O_3$ 419.08, found 422.3 ($MH^+$).

**COMPOUND A59**

**1-{5-[4-(5-Methyl-[1,3,4]oxadiazol-2-yl)-phenoxy]-2-nitro-phenyl}-4-propyl-piperidine**

Following the general procedure, Compound **A59** was obtained as a yellow solid. $^1HNMR$ ($CDCl_3$, 400 MHz) δ 0.82 (t, 3H), 1.27-1.46 (m, 7H), 1.83 (d, 2H), 3.09 (t, 2H), 3.35 (d, 2H), 3.81 (s, 3H), 6.90 (d, 2H), 7.30 (d, 1H), 7.83 (d, 2H), 7.91-7.95 (m, 2H).

**COMPOUND A60**

**1-{5-[3-(3-Methyl-[1,2,4]oxadiazol-5-yl)-phenoxy]-2-nitro-phenyl}-4-propyl-piperidine**

Following the general procedure, Compound **A60** was obtained as a yellow solid (83 %). $^1HNMR$ ($CDCl_3$, 400 MHz) δ 0.82 (t, 3H), 1.27-1.46 (m, 7H), 1.67 (d, 2H), 2.40 (s, 3H), 2.74 (t, 2H), 3.22 (d, 2H), 6.42 (dd, 1H), 6.65 (d, 1H), 7.22 (dd, 1H), 7.50 (t, 1H), 7.73 (t, 1H), 7.82 (d, 1H), 7.88 (d, 1H). Exact mass calculated for $C_{23}H_{26}N_4O_4$ 422.2, found 423.2 ($MH^+$).

**EXAMPLE 13.2**

**SYNTHESES OF COMPOUNDS OF THE PRESENT INVENTION**

**COMPOUND B1**

**6'-Benzenesulfonylamino-3'-nitro-3,4,5,6-tetrahydro-2H-[1,2']bipyridinyl4-carboxylic acid ethyl ester. General Method 5.**

A mixture of the 6'-chloro-3'-nitro-3,4,5,6-tetrahydro-2H-[1,2']bipyridinyl-4-carboxylic acid ethyl ester (150 mg, 0.48 mM), Benzenesulfonamide (1.4 eqv, 105 mg, 0.67 mM) and sodium hydride (2 eqv, 22 mg, 0.95 mM) in DMF (5 ml) was stirred at room temperature for 2 days. The crude product was quenched with hydrochloric acid (5 ml) and

extracted with Ethyl Acetate (5 ml×3). The mixture was dried over sodium sulfate and concentrated in vacuo. Flash chromatography (hexane: ethyl acetate = 3:1 and Methanol:Dichloromethane=1:9) provided Compound **B1** as yellow oil (47 mg, 23 % yield). $^1$H NMR 400MHz $CDCl_3$ δ (ppm): 8.16(d, 1H); 7.94(d, 2H); 7.62(t, 1H); 7.53(t,2H); 6.52 (d, 1H); 4.18 (q, 2H); 3.65 (dt, 2H); 3.08 (td, 2H); 2.57(m, 1H); 1.92(dt,2H); 1.78(td, 2H); 1.3(t, 3H). Exact mass calculated for $C_{19}H_{22}N_4O_6S$ 434.13, LCMS (ESI) m/z 435.1(M+H$^+$, 100%).

**COMPOUND B2**

**6'-(Benzenesulfonyl-methyl-amino)-3'-nitro-3,4,5,6-tetrahydro-2H-[1,2']bipyridinyl-4-carboxylic acid ethyl ester**

Method 4. Flash chromatography (hexane: ethyl acetate = 2: 1) provided Compound **B2** as yellow oil (95 mg, 44 % yield).$^1$H NMR 400MHz $CDCl_3$ δ (ppm): 8.18(d, 1H); 7.75(d, 2H); 7.59(t, 1H); 7.53(t,2H); 7.08 (d, 1H); 4.18 (d, 2H); 3.63 (dt, 2H); 3.08 (td, 2H); 2.57(m, 1H); 1.82(dt,2H); 1.74(td, 2H); 1.3(t, 3H). Exact mass calculated for $C_{20}H_{24}N_4O_6S$ 448.14, LCMS (ESI) m/z 449.1(M+H$^+$, 100%).

**COMPOUND B3**

**6'-(Benzenesulfonyl-butyl-amino)-3'-nitro-3,4,5,6-tetrahydro-2H-[1,2']bipyridinyl-4-carboxylic acid ethyl ester**

Method 4. Flash chromatography (hexane: ethyl acetate = 2:1) provided Compound B3 as yellow oil (25mg, 10% yield).$^1$H NMR 400MHz $CDCl_3$ δ (ppm): 8.18(d, 1H); 7.75(d, 2H); 7.59(t, 1H); 7.53(t,2H); 6.94 (d, 1H); 4.18 (q, 2H); 3.94(t,2H); 3.63 (dt, 2H); 3.08 (td, 2H); 2.57(m, 1H); 1.86(dt,2H); 1.74(td, 2H); 1.46(m,2H);1.40(m, 2H); 1.3(t, 3H); 0.94 (t,3H). Exact mass calculated for $C_{23}H_{30}N_4O_6S$ 490.19, LCMS (ESI) m/z 491.2(M+H$^+$, 100%).

**COMPOUND B4**

**6'-(5-Ethanesulfonyl-2-hydroxy-phenylamino)-3'-nitro-3,4,5,6-tetrahydro-2H-[1,2']bipyridinyl-4-carboxylic acid ethyl ester**

Method 3. provided Compound **B4** (20 mg) as yellow solid (total 35 mg, 36 %).$^1$H NMR 400MHz $CDCl_3$ δ (ppm): 8.25(d, 1H);8.10(s,1H);7.58(dd,1H); 7.35(s, 1H); 7.12(d,1H); 6.19(d,1H); 4.18 (q, 2H);3.75(dt,2H);3.20(td,2H); 3.10(q,2H);3.00(td,2H); 2.60(m, 1H);2.05(dt,2H); 1.91(td, 2H); 1.34(t, 3H).1.30(t,3H)Exact mass calculated for $C_{21}H_{26}N_4O_7S$ 478.15, LCMS (ESI) m/z 479.1(M+H$^+$, 100%).

**COMPOUND B5**

**6'-(2-Bromo-4-trifluoromethyl-benzenesulfonylamino)-3'-nitro-3,4,5,6-tetrahydro-2H-[1,2']bipyridinyl-4-carboxylic acid ethyl ester**

Method 4. Flash chromatography (hexane: ethyl acetate = 3:1) and HPLC provided Compound **B5** as yellow oil (38 mg, 14 % yield). $^1$H NMR 400MHz $CDCl_3$ δ (ppm): 8.39(d, 1H); 8.14(d, 2H); 7.98 (s, 1H); 7.78 (d,1H); 6.43(d, 1H); 4.18 (q, 2H);3.61(dt,2H); 3.08 (td, 2H); 2.57(m, 1H); 1.86(dt,2H); 1.74(td, 2H); 1.3(t, 3H). Exact mass calculated for $C_{20}H_{20}BrF_3N_4O_6S$ 580.36, LCMS (ESI) m/z 583.5(M+H$^+$, 100%).

**COMPOUND B6**

**{4-[3'-Nitro-4-(pyridin-2-ylsulfanyl)-3,4,5,6-tetrahydro-2H-[1,2']bipyridinyl-6'-ylamino]-phenyl}-phenyl-methanone. General Method 4.**

A mixture of pyrimidine (70 mg, 0.22 mmol), aniline (1.0 eqv, 39 mg, 0.2 mmol) and potassium carbonate (1.1 eqv, 31 mg, 22 mmol) in DMF(1 ml) was stirred at 100 ˚C for 2 min,150 ˚C for 5 min, and 180 ˚C for 10 min in microwave. Flash column chromatography [Hexane: Ethyl Acetate=1:2 and 2:1] provided Compound **B6** as yellow solid (5 mg, 5 % yield).$^1$H NMR 400MHz $CDCl_3$ δ (ppm): 8.37(d, 1H); 8.18 (d, 1H); 7.78(d, 2H); 7.71(d, 2H); 7.55(d, 2H); 7.51 (t, 1H); 7.43(t, 2H); 7.11(d, 1H); 6.97(s, 1H); 6.93(t, 1H); 6.13 (d, 1H); 4.09(m,1H); 3.76 (dt, 2H); 3.29 (td, 2H); 2.17(dt,2H); 1.87-1.77(m,2H). Exact mass calculated for $C_{28}H_{25}N_5O_3$ S 511.17, LCMS (ESI) m/z 512.3(M+H$^+$, 100%).

**COMPOUND B7**

**[3'-Nitro-4-(pyridin-2-ylsulfanyl)-3,4,5,6-tetrahydro-2H-[1,2']bipyridinyl-6'-yl]-(4-[1,2,4]triazol-1-yl-phenyl)-amine**

General Procedure provided Compound **B7** as yellow solid (13mg, 16% yield). $^1$H NMR 400MHz $CDCl_3$ δ (ppm): 8.69(s, 1H); 8.50 (d, 2H); 8.27(d, 2H); 8.22(s, 1H); 7.89(d, 2H); 7.57 (t, 2H); 7.47(d, 2H); 7.24(d, 2H); 7.07(t, 2H); 6.63(d, 2H); 4.20-4.15 (m, 2H); 3.69(dt,4H); 3.27(td, 4H); 2.19( dt,4H); 1.82(td,4H). Exact mass calculated for $C_{38}H_{36}N_{12}O_4$ $S_2$ 788.24, LCMS (ESI) m/z 789.1($M+H^+$, 100%).

**COMPOUND B8**

**1-[5-(4-Benzoyl-phenylamino)-2-nitro-phenyl]-piperidine-4-carboxylic acid ethyl ester**

Following the general procedure, Compound **B8** was obtained as a brown solid (22 %). $^1$HNMR ($CDCl_3$, 400 MHz) δ 1.23 (t, 3H), 2.05-2.20 (m, 4H), 2.50-2.57 (m, 1H), 3.32-3.50 (m, 4H), 4.12 (q, 2H), 6.88 (d, 1H), 7.10-7.20 (m, 2H), 7.42 (t, 2H), 7.50 (t, 1H), 7.70-7.80 (m, 5H), 8.02 (d, 1H). Exact mass calculated for $C_{27}H_{27}N_3O_5$ 473.20, found 474.4 ($MH^+$).

**COMPOUND B9**

**{4-[4-Nitro-3-(4-propyl-piperidin-1-yl)-phenylamino]-phenyl}-phenyl-methanone**

Following the general procedure, Compound **B9** was obtained as a yellow solid (3 %). $^1$HNMR ($CDCl_3$, 400 MHz) δ 0.95 (t, 3H), 1.23-1.34 (m, 5H), 1.55-1.60 (m, 2H), 1.70-1.80 (m, 2H), 2.02-2.08 (m, 2H), 3.44-3.60 (m, 2H), 7.02 (d, 1H), 7.30 (d, 2H), 7.42-7.55 (m, 3H), 7.60-7.62 (m, 1H), 7.78 (d, 2H), 7.83 (d, 2H), 8.08 (d, 1H). Exact mass calculated for $C_{27}H_{29}N_3O3$ 443.22, found 444.4 ($M+H^+$).

## Claims

1. A compound selected from compounds of Formula (I**a**) and pharmaceutically acceptable salts, hydrates, and solvates thereof:

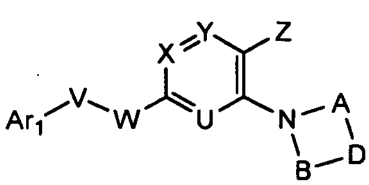

**(Ia)**

wherein:

A and B are both ethylene optionally substituted with 1 to 4 methyl groups;
U is N or $CR_1$;
D is $CR_2R_3$;
V is absent;

W is $-S(O)_2NR_4-$, $-NR_4-$, -O-, -S-, -S(O)-, or $-S(O)_2-$;
or W is absent;
X is $CR_5$;
Y is N or $CR_6$;
Z is nitro;
$Ar_1$ is aryl or heteroaryl optionally substituted with $R_9$, $R_{10}$, $R_{11}$, $R_{12}$ and $R_{13}$;
$R_1$ and $R_6$ are independently selected from H, $C_{1-5}$ acyloxy, $C_{2-6}$ alkenyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, amino, $C_{1-4}$ alkylamino, $C_{2-8}$ dialkylamino, carboxamide, cyano, $C_{3-6}$ cycloalkyl, $C_{2-6}$ dialkylcarboxamide, $C_{2-6}$ dialkylsulfonamide, halogen, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, hydroxyl and nitro;
$R_5$ is H or nitro;
$R_2$ is selected from $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfinyl, $C_{1-4}$ alkylsulfonyl, $C_{1-4}$ alkylthio, amino, carbo-$C_{1-6}$-alkoxy, carboxamide, carboxy, cyano, $C_{3-6}$-cycloalkyl, $C_{2-6}$ dialkylcarboxamide, $C_{1-4}$ haloalkoxy, $C_{1-4}$ haloalkyl, halogen, heteroaryl, hydroxyl and phenyl; and wherein $C_{1-8}$ alkyl, heteroaryl and phenyl are optionally substituted with 1 to 5 substituents selected from $C_{1-5}$ acyl, $C_{1-5}$ acyloxy, $C_{1-4}$ alkoxy, $C_{1-8}$ alkyl, $C_{1-4}$ alkylamino, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl, $C_{3-6}$-cycloalkyl-$C_{1-3}$-heteroalkylene, $C_{2-8}$ dialkylamino, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, halogen, heterocyclic, hydroxyl, hydroxylamino and nitro; or
$R_2$ is $-Ar_2-Ar_3$ wherein $Ar_2$ and $Ar_3$ are independently aryl or heteroaryl optionally substituted with 1 to 5 substituents selected from H, $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfinyl, $C_{1-4}$ alkylsulfonyl, $C_{1-4}$ alkylthio, amino, carbo-$C_{1-6}$-alkoxy, carboxamide, carboxy, cyano, $C_{3-6}$-cycloalkyl, $C_{2-6}$dialkylcarboxamide, $C_{1-4}$ haloalkoxy, $C_{1-4}$ haloalkyl, halogen, hydroxyl and nitro; or
$R_2$ is a group of Formula (B):

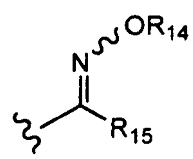

**(B)**

wherein:

$R_{14}$ is $C_{1-8}$ alkyl or $C_{3-6}$ cycloalkyl; and $R_{15}$ is F, Cl, Br or CN; or
$R_2$ is a group of Formula (C):

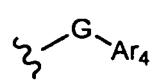

**(C)**

wherein:

G is C=O, $CR_{16}R_{17}$, O, S, S(O), $S(O)_2$; where $R_{16}$ and $R_{17}$ are independently H or $C_{1-8}$ alkyl; and
$Ar_4$ is phenyl or heteroaryl optionally substituted with 1 to 5 substituents selected from $C_{1-5}$ acyl, $C_{1-5}$ acyloxy, $C_{1-4}$ alkoxy, $C_{1-8}$ alkyl, $C_{1-4}$ alkylcarboxamide, $C_{1-4}$ alkylthiocarboxamide, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylthioureyl, $C_{1-4}$ alkylureyl, amino, carbo-$C_{1-6}$-alkoxy, carboxamide, carboxy, cyano, $C_{3-6}$-cycloalkyl, $C_{2-6}$ dialkylcarboxamide, $C_{1-4}$ dialkylthiocarboxamide, $C_{2-6}$ dialkylsulfonamide, $C_{1-4}$ alkylthioureyl, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, halogen, heteroaryl, hydroxyl, hydroxylamino and nitro;

$R_3$ is H, $C_{1-8}$ alkyl, $C_{1-4}$ alkoxy or hydroxyl;
$R_4$ is H or $C_{1-8}$ alkyl;
$R_9$ is selected from $C_{1-5}$ acyl, $C_{1-5}$ acyloxy, $C_{2-6}$ alkenyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, amino, arylsulfonyl, carbo-$C_{1-6}$-alkoxy, carboxamide, carboxy, cyano, $C_{3-6}$ cycloalkyl, $C_{2-6}$ dialkylcarboxamide, halogen, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, heterocyclic, heterocyclicsulfonyl, heteroaryl, hydroxyl, nitro, $C_{4-7}$ oxo-cycloalkyl, phenoxy, phenyl, sulfonamide and sulfonic acid; wherein $C_{1-5}$ acyl, $C_{1-4}$ alkoxy, $C_{1-8}$ alkyl, $C_{1-4}$ alkylsulfonamide, alkylsulfonyl, arylsulfonyl, heteroaryl, phenoxy and phenyl are optionally substituted with 1 to 5 substituents selected independently from $C_{1-5}$ acyl, $C_{1-5}$ acyloxy, $C_{2-6}$ alkenyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, carbo-$C_{1-6}$-alkoxy, carboxamide, carboxy, cyano, $C_{3-6}$ cycloalkyl, $C_{2-6}$ dialkylcarboxamide, halogen, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, heteroaryl, heterocyclic, hydroxyl, nitro and phenyl; or
$R_9$ is a group of Formula (D):

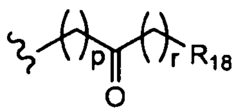

**(D)**

wherein:

"p" and "r" are independently 0, 1, 2 or 3; and
$R_{18}$ is H, $C_{1-5}$ acyl, $C_{2-6}$ alkenyl, $C_{1-8}$ alkyl, $C_{1-4}$ alkylcarboxamide, $C_{2-6}$ alkynyl, $C_{1-4}$ alkylsulfonamide, carbo-$C_{1-6}$-alkoxy, carboxamide, carboxy, cyano, $C_{3-6}$ cycloalkyl, $C_{2-6}$ dialkylcarboxamide, halogen, heteroaryl or phenyl, wherein heteroaryl and phenyl are optionally substituted with 1 to 5 substituents selected independently from $C_{1-4}$ alkoxy, $C_{1-8}$ alkyl, amino, $C_{1-4}$ alkylamino, $C_{2-6}$ alkynyl, $C_{2-8}$ dialkylamino, halogen, $C_{1-4}$ haloalkoxy, $C_{1-4}$ haloalkyl and hydroxyl; and

$R_{10}$-$R_{13}$ are independently selected from $C_{1-5}$ acyl, $C_{1-5}$ acyloxy, $C_{2-6}$ alkenyl, $C_{1-4}$ alkoxy, $C_{1-8}$ alkyl, $C_{1-4}$ alkylcarboxamide, $C_{2-6}$ alkynyl, $C_{1-4}$ alkylsulfonamide, $C_{1-4}$ alkylsulfinyl, $C_{1-4}$ alkylsulfonyl, $C_{1-4}$ alkylthio, $C_{1-4}$ alkylureyl, amino, carbo-$C_{1-6}$-alkoxy, carboxamide, carboxy, cyano, $C_{3-6}$ cycloalkyl, $C_{2-6}$ dialkylcarboxamide, halogen, $C_{1-4}$ haloalkoxy, $C_{1-4}$ haloalkyl, $C_{1-4}$ haloalkylsulfinyl, $C_{1-4}$ haloalkylsulfonyl, $C_{1-4}$ haloalkylthio, hydroxyl and nitro; or
two adjacent $R_{10}$-$R_{11}$ groups form a 5, 6 or 7 membered cycloalkyl, cycloalkenyl or heterocyclic group with $Ar_1$ wherein the 5, 6 or 7 membered group is optionally substituted with halogen.

**2.** A compound according to claim 1 wherein:

A and B are both ethylene optionally substituted with 1 to 4 methyl groups;
U is N or $CR_1$;
D is $CR_2R_3$;
V is absent;
W is $-S(O)_2NR_4-$, $-NR_4-$, or -O-;
or W is absent;
X is $CR_5$;
Y is $CR_6$;
Z is nitro;
$Ar_1$ is aryl or heteroaryl optionally substituted with $R_9$, $R_{10}$, $R_{11}$, $R_{12}$ and $R_{13}$;
$R_1$ and $R_6$ are each independently selected from H, halogen, and nitro;
$R_5$ is H or nitro;
$R_2$ is selected from $C_{1-5}$ acyl, $C_{1-8}$ alkyl, and heteroaryl; and wherein $C_{1-8}$ alkyl, and heteroaryl are optionally substituted with 1 to 5 substituents selected from $C_{1-4}$ alkoxy, $C_{1-8}$ alkyl, and halogen;
or
$R_2$ is a group of Formula (C), wherein G is S, S(O), or $S(O)_2$; and $Ar_4$ is phenyl or heteroaryl optionally substituted with 1 to 5 substituents selected from $C_{1-4}$ alkoxy, $C_{1-8}$ alkyl, cyano, $C_{1-4}$ haloalkoxy, $C_{1-4}$ haloalkyl, and halogen;

$R_3$ is H;
$R_4$ is H or $C_{1-8}$ alkyl;
$R_9$ is selected from $C_{1-5}$ acyl, $C_{2-6}$ alkenyl, $C_{1-4}$ alkoxy, $C_{1-8}$ alkyl, $C_{1-4}$ alkylsulfonyl, amino, arylsulfonyl, carbo-$C_{1-6}$-alkoxy, carboxamide, carboxy, cyano, $C_{3-6}$ cycloalkyl, halogen, $C_{1-4}$ haloalkoxy, $C_{1-4}$ haloalkyl, heterocyclic, heteroaryl, hydroxyl, $C_{4-7}$ oxo-cycloalkyl, phenyl, and sulfonic acid; wherein $C_{1-5}$ acyl, $C_{1-8}$ alkyl, arylsulfonyl, heteroaryl, and phenyl are optionally substituted with 1 to 5 substituents selected independently from $C_{1-4}$ alkoxy, $C_{1-8}$ alkyl, $C_{1-4}$ alkylsulfonyl, cyano, halogen, $C_{1-4}$ haloalkoxy, $C_{1-4}$ haloalkyl, heteroaryl, and hydroxyl; or
$R_9$ is a group of Formula (D) wherein "p" and "r" are independently 0, 1, 2 or 3; and $R_{18}$ is H, carbo-$C_{1-6}$-alkoxy, carboxy, heteroaryl or phenyl; wherein heteroaryl and phenyl are optionally substituted with 1 to 5 substituents selected independently from $C_{1-4}$ alkoxy, $C_{1-8}$ alkyl, halogen, $C_{1-4}$ haloalkoxy, and $C_{1-4}$ haloalkyl; and
$R_{10}$-$R_{13}$ are independently selected from $C_{1-4}$ alkoxy, $C_{1-8}$ alkyl, amino, cyano, halogen, $C_{1-4}$ haloalkoxy, $C_{1-4}$ haloalkyl, and hydroxyl.

**3.** A compound according to claim 1 or 2, wherein W is $-S(O)_2NR_4-$.

**4.** A compound according to claim 1 or 2, wherein W is $-NR_4-$.

**5.** A compound according to claim 3 or 4, wherein $R_4$ is H.

**6.** A compound according to claim 1 or 2, wherein W is -O-.

**7.** A compound according to claim 1 or 2, wherein W is absent.

**8.** A compound according to any one of claims 1 to 7, wherein A and B are both ethylene.

**9.** A compound according to any one of claims 1 to 8, wherein D is $CR_2R_3$, wherein $R_2$ is selected from $C_{1-5}$ acyl, $C_{1-8}$ alkyl, and heteroaryl; and wherein $C_{1-8}$ alkyl, and heteroaryl are optionally substituted with 1 to 5 substituents selected from $C_{1-4}$ alkoxy, $C_{1-8}$ alkyl, and halogen.

**10.** A compound according to any one of claims 1 to 9, wherein $R_2$ is selected from $C(O)CH_3$, $C(O)CH_2CH_3$, $C(O)CH_2CH_2CH_3$, $C(O)CH(CH_3)_2$, $C(O)CH_2CH_2CH_2CH_3$, $CH_3$, $CH_2CH_3$, $CH_2CH_2CH_3$, $CH(CH_3)_2$, $CH(CH_3)(CH_2CH_3)$, $CH_2(CH_2)_2CH_3$, and $CH_2(CH_2)_3CH_3$.

**11.** A compound according to any one of claims 1 to 9, wherein $R_2$ is selected from $CO_2CH_3$, $CO_2CH_2CH_3$, $CO_2CH_2CH_2CH_3$, $CO_2CH(CH_3)_2$ and $CO_2CH_2(CH_2)_2CH_3$.

**12.** A compound according to any one of claims 1 to 9, wherein $R_2$ is $C_{1-8}$ alkyl optionally substituted with 1 to 5 substituents selected from $C_{1-4}$ alkoxy, $C_{1-8}$ alkyl, and halogen.

**13.** A compound according to any one of claims 1 to 9, wherein $R_2$ is selected from $CH_2OCH_3$, $CH_2CH_2OCH_3$, $CH_2OCH_2CH_3$, $CH_2OCH_2CH_2CH_3$, $CH_2CH_2OCH_2CH_3$, $CH_2CH_2OCH_2CH_2CH_3$, $CH_2OCH(CH_3)_2$, and $CH_2OCH_2CH(CH_3)_2$.

**14.** A compound according to any one of claims 1 to 9, wherein $R_2$ is a 1,2,4-oxadiazolyl optionally substituted with $C_{1-8}$ alkyl.

**15.** A compound according to any one of claims 1 to 9, wherein $R_2$ is 3-methyl-1,2,4-oxadiazol-5-yl, 3-ethyl-1,2,4-oxadiazol-5-yl, 3-propyl-1,2,4-oxadiazol-5-yl, 3-isopropyl-1,2,4-oxadiazol-5-yl, 3-butyl-1,2,4-oxadiazol-5-yl, or 3-isobutyl-1,2,4-oxadiazol-5-yl.

**16.** A compound according to any one of claims 1 to 8, wherein D is $CR_2R_3$, wherein $R_2$ is the group of Formula (C), wherein:

G is S, S(O), or $S(O)_2$; and
$Ar_4$ is phenyl or heteroaryl optionally substituted with 1 to 5 substituents selected from $C_{1-4}$ alkoxy, $C_{1-8}$ alkyl, cyano, $C_{1-4}$ haloalkoxy, $C_{1-4}$ haloalkyl, and halogen.

**17.** A compound according to any one of claims 1 to 8 and 16, wherein $Ar_4$ is heteroaryl optionally substituted with 1 to 5 substituents selected from $C_{1-4}$ alkoxy, $C_{1-8}$ alkyl, cyano, $C_{1-4}$ haloalkoxy, $C_{1-4}$ haloalkyl, and halogen.

**18.** A compound according to any one of claims 1 to 8, 16 and 17, wherein $Ar_4$ is a pyridyl group.

**19.** A compound according to any one of claims 1 to 8 and 16 to 18, wherein $Ar_4$ is 2-pyridyl.

**20.** A compound according to any one of claims 1 to 8 and 16 to 19, wherein G is -S-.

**21.** A compound according to any one of claims 1 to 20, wherein $R_3$ is H.

**22.** A compound according to any one of claims 1 to 21, wherein $R_1$ is H.

**23.** A compound according to any one of claims 1 to 22, wherein $Ar_1$ is phenyl, pyridyl, or pyridinone optionally substituted with $R_9$ and $R_{10}$.

**24.** A compound according to any one of claims 1 to 23, wherein $R_9$ is selected from $C_{1-5}$ acyl, vinyl, $C_{1-8}$ alkyl, $C_{1-4}$ alkylsulfonyl, amino, benzenesulfonyl, carboxamide, cyclopentyl, halogen, $C_{1-4}$ haloalkyl, 2,5-dioxo-imidazolidinyl, imidazolyl, pyrrolyl, triazol-1-yl, thiadiazolyl, 1,3-dioxo-1,3-dihydro-isoindolyl, pyrazolyl, [1,3,4]oxadiazolyl, [1,2,4] oxadiazolyl, hydroxyl, oxo-cyclohexyl, phenyl, and sulfonic acid, and wherein $C_{1-5}$ acyl, $C_{1-8}$ alkyl, benzenesulfonyl, and phenyl are optionally substituted with 1 to 5 substituents selected independently from $C_{1-4}$ alkoxy, $C_{1-8}$ alkyl, cyano, heteroaryl, and hydroxyl.

**25.** A compound according to any one of claims 1 to 23, wherein $R_9$ is selected from acetyl, 4-hydroxy-benzenesulfonyl, 2-methoxy-ethyl, vinyl, methyl, methylsulfonyl, ethansulfonyl, amino, 4-hydroxybenzenesulfonyl, 4-cyanophenyl, 4-methoxyphenyl, carboxamide, cyclopentyl, fluoro, chloro, bromo, trifluoromethyl, 2,5-dioxo-imidazolidinyl, imidazol-1-yl, pyrrolyl, triazol-1-yl, thiadiazol-4-yl, 1,3-dioxo-1,3-dihydro-isoindolyl, pyrazolyl, 5-methyl-[1,3,4]oxadiazol-2-yl, 3-methyl- [1,2,4]oxadiazol-5-yl, hydroxyl, 4-oxo-cyclohexyl, phenyl, and sulfonic acid.

**26.** A compound according to any one of claims 1 to 23, wherein $R_9$ is the group of Formula (D), wherein:

"p" and "r" are independently 0, 1, 2 or 3; and
$R_{18}$ is H, carbo-$C_{1-6}$-alkoxy, carboxy, heteroaryl or phenyl, and wherein the heteroaryl and phenyl are optionally substituted with 1 to 5 substituents selected independently from $C_{1-4}$ alkoxy, $C_{1-8}$ alkyl, halogen, $C_{1-4}$ haloalkoxy, and $C_{1-4}$ haloalkyl.

**27.** A compound according to any one of claims 1 to 23, wherein $R_9$ is 2-methoxycarbonyl-acetyl, benzoyl, 3-oxo-butyl, 2-carboxy-ethyl, 2-carboxy-2-oxo-ethyl, $CH_3(CH_2)_2C(O)$, $CH_3(CH_2)_3C(O)$, or $CH_3(CH_2)_4C(O)$.

**28.** A compound according to any one of claims 1 to 27, wherein $R_{10}$ is selected from $C_{1-4}$ alkoxy, $C_{1-8}$ alkyl, amino, cyano, halogen, $C_{1-4}$ haloalkoxy, $C_{1-4}$ haloalkyl, and hydroxyl.

**29.** A compound according to any one of claims 1 to 27, wherein $R_{10}$ is selected from amino, methoxy, methyl, cyano, fluoro, chloro, bromo, trifluoromethoxy, trifluoromethyl, and hydroxyl.

**30.** A compound according to any one of claims 1 to 29, wherein Y is $CR_6$.

**31.** A compound according to claim 30, wherein $R_6$ is H.

**32.** A compound according to any one of claims 1 to 31, wherein U is N.

**33.** A compound according to any one of claims 1 to 31, wherein U is $CR_1$.

**34.** A compound according to claim 33, wherein $R_1$ is H.

**35.** A compound according to claim 1 or 2, wherein:

U is N, and

X and Y are both CH.

**36.** A compound according to claim 35, wherein:

A and B are both $-CH_2CH_2-$;
D is $CR_2R_3$, wherein $R_2$ is selected from $C(O)CH_3$, $CO_2CH_2CH_3$, $CH_2CH_2CH_3$, and pyridin-2-ylsulfanyl; and $R_3$ is H;
V is absent,
W is -O-;
Z is nitro; and
$Ar_1$ is phenyl optionally substituted by $R_9$ and $R_{10}$, wherein $R_9$ is acetyl, 2-methoxy-ethyl, ethansulfonyl, 4-hydroxy-benzenesulfonyl, 4-cyanophenyl, 4-methoxyphenyl, carboxamide, cyclopentyl, 2,5-dioxo-imidazolidinyl, imidazol-1-yl, pyrrolyl, triazol-1-yl, thiadiazol-4-yl, 1,3-dioxo-1,3-dihydro-isoindolyl, 4-oxo-cyclohexyl, sulfonic acid, 2-methoxycarbonyl-acetyl, benzoyl, or 3-oxo-butyl; and $R_{10}$ is amino.

**37.** A compound according to claim 1 or 2, wherein:

U is CH,
X is CH or $C\text{-}NO_2$, and
Y is CH.

**38.** A compound according to claim 37, wherein:

A and B are both $-CH_2CH_2-$;
D is $CR_2R_3$, wherein $R_2$ is selected from $CO_2CH_2CH_3$, $CH_2CH_2CH_3$, pyridin-2-ylsulfanyl, $CH_2OCH_3$, and 3-methyl-1,2,4-oxadiazol-5-yl; and $R_3$ is H;
V is absent,
W is -O-;
Z is nitro; and
$Ar_1$ is phenyl optionally substituted by $R_9$ and $R_{10}$, wherein $R_9$ is acetyl, vinyl, ethansulfonyl, triazol-1-yl, 2- (3-methyl- [1,2,4]oxadiazol-5-yl)-acetyl, 5-hydroxy-1-methyl-1H-pyrazol-3-yl, 5-trifluoromethyl-pyridin-2-yl, 5-bromo-pyridin-2-yl, 2-methoxycarbonyl-acetyl, benzoyl, 3-oxo-butyl, 2-carboxy-ethyl, 2-carboxy-2-oxo-ethyl, $CH_3(CH_2)_2C(O)$, $CH_3(CH_2)_3C(O)$, or $CH_3(CH_2)_4C(O)$; and $R_{10}$ is amino.

**39.** A compound according to claim 1, selected from the following compounds and pharmaceutically acceptable salts, hydrates, and solvates thereof:

(A1) 6'-[4-(2-Methoxycarbonyl-acetyl)-phenoxy]-3'-nitro-3,4,5,6-tetrahydro-2H-[1,2']bipyridinyl-4-carboxylic acid ethyl ester;
(A2) 1-[4-(4-Acetyl-3'-nitro-3,4,5,6-tetrahydro-2H-[1,2']bipyridinyl-6'-yloxy)-phenyl]-ethanone;
(A3) 6'-[4-(4-Hydroxy-benzenesulfonyl)-phenoxy]-3'-nitro-3,4,5,6-tetrahydro-2H-[1,2']bipyridinyl-4-carboxylic acid ethyl ester;
(A4) 6'-(4-Imidazol-1-yl-phenoxy)-3'-nitro-3,4,5,6-tetrahydro-2H-[1,2']bipyridinyl-4-carboxylic acid ethyl ester;
(A5) 6'-(4-Benzoyl-phenoxy)-3'-nitro-3,4,5,6-tetrahydro-2H-[1,2']bipyridinyl-4-carboxylic acid ethyl ester;
(A6) 6'-[4-(2-Methoxy-ethyl)-phenoxy]-3'-nitro-3,4,5,6-tetrahydro-2H-[1,2']bipyridinyl-4-carboxylic acid ethyl ester;
(A7) 6'-(4-Cyclopentyl-phenoxy)-3'-nitro-3,4,5,6-tetrahydro-2H-[1,2']bipyridinyl-4-carboxylic acid ethyl ester;
(A8) 6'-(4'-Cyano-biphenyl-4-yloxy)-3'-nitro-3,4,5,6-tetrahydro-2H-[1,2']bipyridinyl-4-carboxylic acid ethyl ester;
(A9) 3'-Nitro-6'-(4-sulfo-phenoxy)-3,4,5,6-tetrahydro-2H-[1,2']bipyridinyl-4-carboxylic acid ethyl ester;
(A10) 3'-Nitro-6'-(4-pyrrol-1-yl-phenoxy)-3,4,5,6-tetrahydro-2H-[1,2']bipyridinyl-4-carboxylic acid ethyl ester;
(A11) 6'-(4-Carbamoyl-phenoxy)-3'-nitro-3,4,5,6-tetrahydro-2H-[1,2']bipyridinyl-4-carboxylic acid ethyl ester;
(A12) 3'-Nitro-6'-(4-[1,2,4]triazol-1-yl-phenoxy)-3,4,5,6-tetrahydro-2H-[1,2']bipyridinyl-4-carboxylic acid ethyl ester;
(A13) 6'-(2-Amino-4-ethanesulfonyl-phenoxy)-3'-nitro-3,4,5,6-tetrahydro-2H-[1,2']bipyridinyl-4-carboxylic acid ethyl ester;
(A14) 3'-Nitro-6'-[4-(4-oxo-cyclohexyl)-phenoxy]-3,4,5,6-tetrahydro-2H-[1,2']bipyridinyl-4-carboxylic acid ethyl ester;
(A15) 6'-(4'-Methoxy-biphenyl-4-yloxy)-3'-nitro-3,4,5,6-tetrahydro-2H-[1,2']bipyridinyl-4-carboxylic acid ethyl

ester;
(A16) 3'-Nitro-6'-(4-[1,2,3]thiadiazol-4-yl-phenoxy)-3,4,5,6-tetrahydro-2H-[1,2']bipyridinyl-4-carboxylic acid ethyl ester;
(A17) 6'-[4-(1,3-Dioxo-1,3-dihydro-isoindol-2-yl)-phenoxy]-3'-nitro-3,4,5,6-tetrahydro-2H-[1,2']bipyridinyl-4-carboxylic acid ethyl ester;
(A18) 6'-[4-(2,5-Dioxo-imidazolidin-4-yl)-phenoxy]-3'-nitro-3,4,5,6-tetrahydro-2H-[1,2']bipyridinyl-4-carboxylic acid ethyl ester;
(A19) 3'-Nitro-6'-[4-(3-oxo-butyl)-phenoxy]-3,4,5,6-tetrahydro-2H-[1,2']bipyridinyl-4-carboxylic acid ethyl ester;
(A20) 3-[4-(3'-Nitro-4-propyl-3,4,5,6-tetrahydro-2H-[1,2']bipyridinyl-6'-yloxy)-phenyl]-3-oxo-propionic acid methyl ester;
(A21) 4-[4-(3'-Nitro-4-propyl-3,4,5,6-tetrahydro-2H-[1,2']bipyridinyl-6'-yloxy)-phenyl]-butan-2-one;
(A22) 4-{4-[3'-Nitro-4-(pyridin-2-ylsulfanyl)-3,4,5,6-tetrahydro-2H-[1,2']bipyridinyl-6'-yloxy]-phenyl}-butan-2-one; and
(A23) 3'-Nitro-4-(pyridin-2-ylsulfanyl)-6'-(4-[1,2,4]triazol-1-yl-phenoxy)-3,4,5,6-tetrahydro-2H-[1,2']bipyridinyl.

**40.** A compound according to claim 1, selected from the following compounds and pharmaceutically acceptable salts, hydrates, and solvates thereof:

(A24) 1-[5-(4-Benzoyl-phenoxy)-2-nitro-phenyl]-piperidine-4-carboxylic acid ethyl ester;
(A25) 1-{5-[4-(2-Methoxycarbonyl-acetyl)-phenoxy]-2-nitro-phenyl}-piperidine-4-carboxylic acid ethyl ester;
(A26) 1-[5-(2-Amino-4-ethanesulfonyl-phenoxy)-2-nitro-phenyl]-piperidine-4-carboxylic acid ethyl ester;
(A27) 1-{2-Nitro-5-[4-(3-oxo-butyl)-phenoxy]-phenyl}-piperidine-4-carboxylic acid ethyl ester;
(A28) 4-{4-[4-Nitro-3-(4-propyl-piperidin-1-yl)-phenoxy]-phenyl}-butan-2-one;
(A29) 1-{4-[4-Nitro-3-(4-propyl-piperidin-1-yl)-phenoxy]-phenyl}-ethanone;
(A30) 3-{4-[4-Nitro-3-(4-propyl-piperidin-1-yl)-phenoxy]-phenyl}-3-oxo-propionic acid methyl ester;
(A31) 5-Ethanesulfonyl-2-[4-nitro-3-(4-propyl-piperidin-1-yl)-phenoxy]-phenylamine;
(A32) {4-[4-Nitro-3-(4-propyl-piperidin-1-yl)-phenoxy]-phenyl}-phenyl-methanone;
(A37) 1-{5-[4-(2-Carboxy-ethyl)-phenoxy]-2-nitro-phenyl}-piperidine-4-carboxylic acid ethyl ester;
(A38) 1-{5-[4-(2-Carboxy-2-oxo-ethyl)-phenoxy]-2-nitro-phenyl}-piperidine-4-carboxylic acid ethyl ester;
(A39) 1-[2-Nitro-5-(4-vinyl-phenoxy)-phenyl]-piperidine-4-carboxylic acid ethyl ester;
(A40) 3-{4-[4-Nitro-3-(4-propyl-piperidin-1-yl)-phenoxy]-phenyl}-propionic acid;
(A41) 3-{4-[4-Nitro-3-(4-propyl-piperidin-1-yl)-phenoxy]-phenyl}-2-oxo-propionic acid;
(A42) 1-[2-Nitro-5-(4-vinyl-phenoxy)-phenyl]-4-propyl-piperidine;
(A43) 1-{4-[4-Nitro-3-(4-propyl-piperidin-1-yl)-phenoxy]-phenyl}-butan-1-one;
(A44) 1-{4-[4-Nitro-3-(4-propyl-piperidin-1-yl)-phenoxy]-phenyl}-pentan-1-one;
(A45) 1-{4-[4-Nitro-3-(4-propyl-piperidin-1-yl)-phenoxy]-phenyl}-hexan-1-one;
(A46) 4-{4-[3-(4-Methoxymethyl-piperidin-1-yl)-4-nitro-phenoxy]-phenyl}-butan-2-one;
(A47) 1-{4-[3-(4-Methoxymethyl-piperidin-1-yl)-4-nitro-phenoxy]-phenyl}-ethanone;
(A48) {4-[3-(4-Methoxymethyl-piperidin-1-yl)-4-nitro-phenoxy]-phenyl}-phenyl-methanone;
(A49) 2-(3-Methyl-[1,2,4]oxadiazol-5-yl)-1-{4-[4-nitro-3-(4-propyl-piperidin-1-yl)-phenoxy]-phenyl}-ethanone;
(A50) 4-(4-{3-[4-(3-Methyl-[1,2,4]oxadiazol-5-yl)-piperidin-1-yl]-4-nitro-phenoxy}-phenyl)-butan-2-one;
(A51) 4-(4-{4-Nitro-3-[4-(pyridin-2-ylsulfanyl)-piperidin-1-yl]-phenoxy}-phenyl)-butan-2-one;
(A52) 2-{1-[2-Nitro-5-(4-[1,2,4]triazol-1-yl-phenoxy)-phenyl]-piperidin-4-ylsulfanyl}-pyridine;
(A53) 2-Methyl-5-{4-[4-nitro-3-(4-propyl-piperidin-1-yl)-phenoxy]-phenyl}-2H-pyrazol-3-ol;
(A54) 2-[4-Nitro-3-(4-propyl-piperidin-1-yl)-phenoxy]-5-trifluoromethylpyridine;
(A55) 5-Bromo-2-[4-nitro-3-(4-propyl-piperidin-1-yl)-phenoxy]-pyridine;
(A56) 1-(4-{4-Nitro-3-[4-(pyridin-2-ylsulfanyl)-piperidin-1-yl]-phenoxy}-phenyl)-ethanone;
(A57) 2-{1-[5-(4-Methanesulfonyl-phenoxy)-2-nitro-phenyl]-piperidin-4-ylsulfanyl}-pyridine;
(A59) 1-{5-[4-(5-Methyl-[1,3,4]oxadiazol-2-yl)-phenoxy]-2-nitro-phenyl}-4-propyl-piperidine; and
(A60) 1-{5-[3-(3-Methyl-[1,2,4]oxadiazol-5-yl)-phenoxy]-2-nitro-phenyl}-4-propyl-piperidine.

**41.** A compound according to claim 1, selected from the following compound and pharmaceutically acceptable salts, hydrates, and solvates thereof:

(A58) 5-Bromo-1-[4-nitro-3-(4-propyl-piperidin-1-yl)-phenyl]-1H-pyridin-2-one.

**42.** A compound according to claim 1, selected from the following compounds and pharmaceutically acceptable salts, hydrates, and solvates thereof:

(B1) 6'-Benzenesulfonylamino-3'-nitro-3,4,5,6-tetrahydro-2H-[1,2']bipyridinyl-4-carboxylic acid ethyl ester;
(B2) 6'-(Benzenesulfonyl-methyl-amino)-3'-nitro-3,4,5,6-tetrahydro-2H-[1,2']bipyridinyl-4-carboxylic acid ethyl ester;
(B3) 6'-(Benzenesulfonyl-butyl-amino)-3'-nitro-3,4,5,6-tetrahydro-2H-[1,2']bipyridinyl-4-carboxylic acid ethyl ester;
(B4) 6'-(5-Ethanesulfonyl-2-hydroxy-phenylamino)-3'-nitro-3,4,5,6-tetrahydro-2H-[1,2']bipyridinyl-4-carboxylic acid ethyl ester;
(B5) 6'-(2-Bromo-4-trifluoromethyl-benzenesulfonylamino)-3'-nitro-3,4,5,6-tetrahydro-2H-[1,2']bipyridinyl-4-carboxylic acid ethyl ester;
(B6) {4-[3'-Nitro-4-(pyridin-2-ylsulfanyl)-3,4,5,6-tetrahydro-2H-[1,2']bipyridinyl-6'-ylamino]-phenyl}-phenyl-methanone; and
(B7) [3'-Nitro-4-(pyridin-2-ylsulfanyl)-3,4,5,6-tetrahydro-2H-[1,2']bipyridinyl-6'-yl]-(4-[1,2,4]triazol-1-yl-phenyl)-amine.

**43.** A compound according to claim 1, selected from the following compounds and pharmaceutically acceptable salts, hydrates, and solvates thereof:

(B8) 1-[5-(4-Benzoyl-phenylamino)-2-nitro-phenyl]-piperidine-4-carboxylic acid ethyl ester; and
(B9) {4-[4-Nitro-3-(4-propyl-piperidin-1-yl)-phenylamino]-phenyl}-phenyl-methanone.

**44.** A pharmaceutical composition comprising at least one compound according to any one of claims 1 to 43 in combination with a pharmaceutically acceptable carrier.

**45.** A method of producing a pharmaceutical composition comprising admixing at least one compound according to any one of claims 1 to 43 and a pharmaceutically acceptable carrier.

**46.** A compound according to any one of claims 1 to 43 for use in a method of treatment of the human or animal body by therapy.

**47.** A compound according to any one of claims 1 to 43 for use in a method of prophylaxis or treatment of a metabolic disorder.

**48.** Use of a compound according to any one of claims 1 to 43 for the production of a medicament for use in prophylaxis or treatment of a metabolic disorder.

**49.** A compound according to any one of claims 1 to 43 for use in a method of treatment or prophylaxis of type II diabetes, inadequate glucose tolerance, insulin resistance, hyperglycemia, hyperlipidemia, hypertriglyceridemia, hypercholesterolemia, dyslipidemia, syndrome X, or metabolic syndrome.

**50.** Use of a compound according to any one of claims 1 to 43 for the production of a medicament for use in prophylaxis or treatment of type II diabetes, inadequate glucose tolerance, insulin resistance, hyperglycemia, hyperlipidemia, hypertriglyceridemia, hypercholesterolemia, dyslipidemia, syndrome X, or metabolic syndrome.

**51.** A compound according to any one of claims 1 to 43 for use in a method of treatment or prophylaxis of type II diabetes.

**52.** Use of a compound according to any one of claims 1 to 43 for the production of a medicament for use in prophylaxis or treatment of type II diabetes.

**53.** A compound according to any one of claims 1 to 43 for use in a method of controlling or decreasing weight gain in an individual.

**54.** Use of a compound according to any one of claims 1 to 43 for the production of a medicament for use in controlling or decreasing weight gain in an individual.

## Patentansprüche

**1.** Verfahren, ausgewählt aus Verbindungen der Formel (Ia) und pharmazeutisch annehmbaren Salzen, Hydraten und

Solvaten davon:

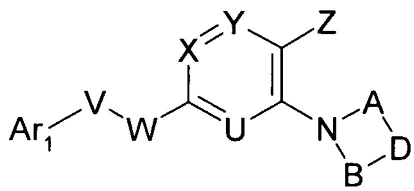

(Ia)

worin:

A und B beide Ethylen sind, das gegebenenfalls mit 1 bis 4 Methylgruppen substituiert ist;
U N oder $CR_1$ ist;
D $CR_2R_3$ ist;
V fehlt;
W $-S(O)_2NR_4-$, $-NR_4-$, -O-, -S-, -S(O)- oder $-S(O)_2-$ ist;
oder W fehlt;
X $CR_5$ ist;
Y N oder $CR_6$ ist;
Z Nitro ist;
$Ar_1$ Aryl oder Heteroaryl ist, das gegebenenfalls mit $R_9$, $R_{10}$, $R_{11}$, $R_{12}$ und $R_{13}$ substituiert ist;
$R_1$ und $R_6$ unabhängig voneinander ausgewählt sind aus H, $C_{1-5}$-Acyloxy, $C_{2-6}$-Alkenyl, $C_{1-4}$-Alkoxy, $C_{1-8}$-Alkyl, $C_{1-4}$-Alkylcarboxamid, $C_{2-6}$-Alkinyl, $C_{1-4}$-Alkylsulfonamid, $C_{1-4}$-Alkylsulfinyl, $C_{1-4}$-Alkylsulfonyl, $C_{1-4}$-Alkylthio, $C_{1-4}$-Alkylureyl, Amino, $C_{1-4}$-Alkylamino, $C_{2-8}$-Dialkylamino, Carboxamid, Cyano, $C_{3-6}$-Cycloalkyl, $C_{2-6}$-Dialkylcarboxamid, $C_{2-6}$-Dialkylsulfonamid, Halogen, $C_{1-4}$-Halogenalkoxy, $C_{1-4}$-Halogenalkyl, $C_{1-4}$-Halogenalkylsulfinyl, $C_{1-4}$-Halogenalkylsulfonyl, $C_{1-4}$-Halogenalkylthio, Hydroxyl und Nitro;
$R_5$ H oder Nitro ist;
$R_2$ ausgewählt ist aus $C_{1-5}$-Acyl, $C_{1-5}$-Acyloxy, $C_{1-4}$-Alkoxy, $C_{1-8}$-Alkyl, $C_{1-4}$-Alkylcarboxamid, $C_{1-4}$-Alkylthiocarboxamid, $C_{1-4}$-Alkylsulfinyl, $C_{1-4}$-Alkylsulfonyl, $C_{1-4}$-Alkylthio, Amino, Carbo-$C_{1-6}$-alkoxy, Carboxamid, Carboxy, Cyano, $C_{3-6}$-Cycloalkyl, $C_{2-6}$-Dialkylcarboxamid, $C_{1-4}$-Halogenalkoxy, $C_{1-4}$-Halogenalkyl, Halogen, Heteroaryl, Hydroxyl und Phenyl; und worin $C_{1-8}$-Alkyl, Heteroaryl und Phenyl gegebenenfalls mit 1 bis 5 Substituenten substituiert sind, die ausgewählt sind aus $C_{1-5}$-Acyl, $C_{1-5}$-Acyloxy, $C_{1-4}$-Alkoxy, $C_{1-8}$-Alkyl, $C_{1-4}$Alkylamino, $C_{1-4}$-Alkylcarboxamid, $C_{1-4}$-Alkylthiocarboxamid, $C_{1-4}$-Alkylsulfonamid, $C_{1-4}$-Alkylsulfinyl, $C_{1-4}$-Alkylsulfonyl, $C_{1-4}$-Alkylthio, $C_{1-4}$-Alkylthioureyl, $C_{1-4}$-Alkylureyl, Amino, Carbo-$C_{1-6}$-alkoxy, Carboxamid, Carboxy, Cyano, $C_{3-6}$-Cycloalkyl, $C_{3-6}$-Cycloalkyl-$C_{1-3}$-heteroalkylen, $C_{2-8}$-Dialkylamino, $C_{2-6}$-Dialkylcarboxamid, $C_{1-4}$-Dialkylthiocarboxamid, $C_{2-6}$-Dialkylsulfonamid, $C_{1-4}$-Alkylthioureyl, $C_{1-4}$-Halogenalkoxy, $C_{1-4}$-Halogenalkyl, $C_{1-4}$-Halogenalkylsulfinyl, $C_{1-4}$-Halogenalkylsulfonyl, $C_{1-4}$-Halogenalkylthio, Halogen, Heterocyclyl, Hydroxyl, Hydroxylamino und Nitro; oder
$R_2$ $-Ar_2-Ar_3$ ist, worin $Ar_2$ und $Ar_3$ unabhängig voneinander Aryl oder Heteroaryl sind, die gegebenenfalls mit 1 bis 5 Substituenten substituiert sind, die ausgewählt sind aus H, $C_{1-5}$-Acyl, $C_{1-5}$-Acyloxy, $C_{1-4}$-Alkoxy, $C_{1-8}$-Alkyl, $C_{1-4}$-Alkylcarboxamid, $C_{1-4}$-Alkylthiocarboxamid, $C_{1-4}$-Alkylsulfinyl, $C_{1-4}$-Alkylsulfonyl, $C_{1-4}$-Alkylthio, Amino, Carbo-$C_{1-6}$-alkoxy, Carboxamid, Carboxy, Cyano, $C_{3-6}$-Cycloalkyl, $C_{2-6}$-Dialkylcarboxamid, $C_{1-4}$-Halogenalkoxy, $C_{1-4}$-Halogenalkyl, Halogen, Hydroxyl und Nitro; oder
$R_2$ eine Gruppe der Formel (B) ist:

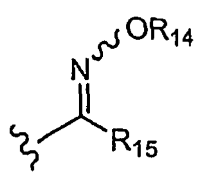

(B)

worin:

$R_{14}$ $C_{1-8}$-Alkyl oder $C_{3-6}$-Cycloalkyl ist; und $R_{15}$ F, Cl, Br oder CN ist; oder
$R_2$ eine Gruppe der Formel (C) ist:

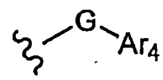

(C)

worin:
G C=O, $CR_{16}R_{17}$, O, S, S(O), $S(O)_2$ ist; worin $R_{16}$ und $R_{17}$ unabhängig voneinander H oder $C_{1-8}$-Alkyl sind; und
$Ar_4$ Phenyl oder Heteroaryl ist, das gegebenenfalls mit 1 bis 5 Substituenten substituiert ist, die ausgewählt sind aus $C_{1-5}$-Acyl, $C_{1-5}$-Acyloxy, $C_{1-4}$-Alkoxy, $C_{1-8}$-Alkyl, $C_{1-4}$-Alkylcarboxamid, $C_{1-4}$-Alkylthiocarboxamid, $C_{1-4}$-Alkylsulfonamid, $C_{1-4}$-Alkylsulfinyl, $C_{1-4}$-Alkylsulfonyl, $C_{1-4}$-Alkylthio, $C_{1-4}$-Alkylthioureyl, $C_{1-4}$-Alkylureyl, Amino, Carbo-$C_{1-6}$-alkoxy, Carboxamid, Carboxy, Cyano, $C_{3-6}$-Cycloalkyl, $C_{2-6}$-Dialkylcarboxamid, $C_{1-4}$-Dialkylthiocarboxamid, $C_{2-6}$-Dialkylsulfonamid, $C_{1-4}$-Alkylthioureyl, $C_{1-4}$-Halogenalkoxy, $C_{1-4}$-Halogenalkyl, $C_{1-4}$-Halogenalkylsulfinyl, $C_{1-4}$-Halogenalkylsulfonyl, $C_{1-4}$-Halogenalkylthio, Halogen, Heteroaryl, Hydroxy, Hydroxylamino und Nitro;

$R_3$ H, $C_{1-8}$-Alkyl, $C_{1-4}$-Alkoxy oder Hydroxyl ist;
$R_4$ H oder $C_{1-8}$-Alkyl ist;
$R_9$ ausgewählt ist aus $C_{1-5}$-Acyl, $C_{1-5}$-Acyloxy, $C_{2-6}$-Alkenyl, $C_{1-4}$-Alkoxy, $C_{1-8}$-Alkyl, $C_{1-4}$-Alkylcarboxamid, $C_{2-6}$-Alkinyl, $C_{1-4}$-Alkylsulfonamid, $C_{1-4}$-Alkylsulfinyl, $C_{1-4}$-Alkylsulfonyl, $C_{1-4}$-Alkylthio, $C_{1-4}$-Alkylureyl, Amino, Arylsulfonyl, Carbo-$C_{1-6}$-alkoxy, Carboxamid, Carboxy, Cyano, $C_{3-6}$-Cycloalkyl, $C_{2-6}$-Dialkylcarboxamid, Halogen, $C_{1-4}$-Halogenalkoxy, $C_{1-4}$-Halogenalkyl, $C_{1-4}$-Halogenalkylsulfinyl, $C_{1-4}$-Halogenalkylsulfonyl, $C_{1-4}$-Halogenalkylthio, Heterocyclyl, Heterocyclylsulfonyl, Heteroaryl, Hydroxyl, Nitro, $C_{4-7}$-Oxocycloalkyl, Phenoxy, Phenyl, Sulfonamid und Sulfonsäure; worin $C_{1-5}$-Acyl, $C_{1-4}$-Alkoxy, $C_{1-8}$-Alkyl, $C_{1-4}$-Alkylsulfonamid, Alkylsulfonyl, Arylsulfonyl, Heteroaryl, Phenoxy und Phenyl gegebenenfalls mit 1 bis 5 Substituenten substituiert sind, die unabhängig voneinander ausgewählt sind aus $C_{1-5}$-Acyl, $C_{1-5}$-Acyloxy, $C_{2-6}$-Alkenyl, $C_{1-4}$-Alkoxy, $C_{1-8}$-Alkyl, $C_{1-4}$-Alkylcarboxamid, $C_{2-6}$-Alkinyl, $C_{1-4}$-Alkylsulfonamid, $C_{1-4}$-Alkylsulfinyl, $C_{1-4}$-Alkylsulfonyl, $C_{1-4}$-Alkylthio, $C_{1-4}$-Alkylureyl, Carbo-$C_{1-6}$-alkoxy, Carboxamid, Carboxy, Cyano, $C_{3-6}$-Cycloalkyl, $C_{2-6}$-Dialkylcarboxamid, Halogen, $C_{1-4}$-Halogenalkoxy, $C_{1-4}$-Halogenalkyl, $C_{1-4}$-Halogenalkylsulfinyl, $C_{1-4}$-Halogenalkylsulfonyl, $C_{1-4}$-Halogenalkylthio, Heteroaryl, Heterocyclyl, Hydroxyl, Nitro und Phenyl; oder
$R_9$ eine Gruppe der Formel (D) ist:

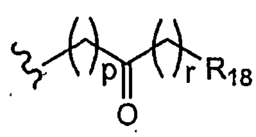

(D)

worin:

"p" und "r" unabhängig voneinander 0, 1, 2 oder 3 sind; und
$R_{18}$ H, $C_{1-5}$-Acyl, $C_{2-6}$-Alkenyl, $C_{1-8}$-Alkyl, $C_{1-4}$-Alkylcarboxamid, $C_{2-6}$-Alkinyl, $C_{1-4}$-Alkylsulfonamid, Carbo-$C_{1-6}$-alkoxy, Carboxamid, Carboxy, Cyano, $C_{3-6}$-Cycloalkyl, $C_{2-6}$-Dialkylcarboxamid, Halogen, Heteroaryl oder Phenyl ist, worin Heteroaryl und Phenyl gegebenenfalls mit 1 bis 5 Substituenten substituiert sind, die unabhängig voneinander ausgewählt sind aus $C_{1-4}$-Alkoxy, $C_{1-8}$-Alkyl, Amino, $C_{1-4}$-Alkylamino, $C_{2-6}$-Alkinyl, $C_{2-8}$-Dialkylamino, Halogen, $C_{1-4}$-Halogenalkoxy, $C_{1-4}$-Halogenalkyl und Hydroxyl; und

$R_{10}$-$R_{13}$ unabhängig voneinander ausgewählt sind aus $C_{1-5}$-Acyl, $C_{1-5}$-Acyloxy, $C_{2-6}$-Alkenyl, $C_{1-4}$-Alkoxy, $C_{1-8}$-Alkyl, $C_{1-4}$-Alkylcarboxamid, $C_{2-6}$-Alkinyl, $C_{1-4}$-Alkylsulfonamid, $C_{1-4}$-Alkylsulfinyl, $C_{1-4}$-Alkylsulfonyl,

$C_{1-4}$-Alkylthio, $C_{1-4}$-Alkylureyl, Amino, Carbo-$C_{1-6}$-alkoxy, Carboxamid, Carboxy, Cyano, $C_{3-6}$-Cycloalkyl, $C_{2-6}$-Dialkylcarboxamid, Halogen, $C_{1-4}$-Halogenalkoxy, $C_{1-4}$-Halogenalkyl, $C_{1-4}$-Halogenalkylsulfinyl, $C_{1-4}$-Halogenalkylsulfonyl, $C_{1-4}$-Halogenalkylthio, Hydroxyl und Nitro; oder
zwei benachbarte $R_{10}$-$R_{11}$-Gruppen eine 5-, 6- oder 7-gliedrige Cycloalkyl-, Cycloalkenyl- oder Heterocyclylgruppe mit $Ar_1$ bilden, worin die 5-, 6- oder 7-gliedrige Gruppe gegebenenfalls mit Halogen substituiert ist.

**2.** Verfahren nach Anspruch 1, worin:

A und B beide Ethylen sind, das gegebenenfalls mit 1 bis 4 Methylgruppen substituiert ist;
U N oder $CR_1$ ist;
D $CR_2R_3$ ist;
V fehlt;
W $-S(O)_2NR_4$, $-NR_4-$ oder -O- ist;
oder W fehlt;
X $CR_5$ ist;
Y $CR_6$ ist;
Z Nitro ist;
$Ar_1$ Aryl oder Heteroaryl ist, das gegebenenfalls mit $R_9$, $R_{10}$, $R_{11}$, $R_{12}$ und $R_{13}$ substituiert ist;
$R_1$ und $R_6$ unabhängig voneinander ausgewählt sind aus H, Halogen und Nitro;
$R_5$ H oder Nitro ist;
$R_2$ ausgewählt ist aus $C_{1-5}$-Acyl, $C_{1-8}$-Alkyl und Heteroaryl; und worin $C_{1-8}$-Alkyl und Heteroaryl gegebenenfalls mit 1 bis 5 Substituenten substituiert sind, die ausgewählt sind aus $C_{1-4}$-Alkoxy, $C_{1-8}$-Alkyl und Halogen;
oder
$R_2$ eine Gruppe der Formel (C) ist, worin G S, S(O) oder $S(O)_2$ ist; und $Ar_4$ Phenyl oder Heteroaryl ist, das gegebenenfalls mit 1 bis 5 Substituenten substituiert ist, die ausgewählt sind aus $C_{1-4}$-Alkoxy, $C_{1-8}$-Alkyl, Cyano, $C_{1-4}$-Halogenalkoxy, $C_{1-4}$-Halogenalkyl und Halogen;
$R_3$ H ist;
$R_4$ H oder $C_{1-8}$-Alkyl ist;
$R_9$ ausgewählt ist aus $C_{1-5}$-Acyl, $C_{2-6}$-Alkenyl, $C_{1-4}$-Alkoxy, $C_{1-8}$-Alkyl, $C_{1-4}$-Alkylsulfonyl, Amino, Arylsulfonyl, Carbo-$C_{1-6}$-alkoxy, Carboxamid, Carboxy, Cyano, $C_{3-6}$-Cycloalkyl, Halogen, $C_{1-4}$-Halogenalkoxy, $C_{1-4}$-Halogenalkyl, Heterocyclyl, Heteroaryl, Hydroxyl, $C_{4-7}$-Oxocycloalkyl, Phenyl und Sulfonsäure; worin $C_{1-5}$-Acyl, $C_{1-8}$-Alkyl, Arylsulfonyl, Heteroaryl und Phenyl gegebenenfalls mit 1 bis 5 Substituenten substituiert sind, die unabhängig voneinander ausgewählt sind aus $C_{1-4}$-Alkoxy, $C_{1-8}$-Alkyl, $C_{1-4}$-Alkylsulfonyl, Cyano, Halogen, $C_{1-4}$-Halogenalkoxy, $C_{1-4}$-Halogenalkyl, Heteroaryl und Hydroxyl;
oder
$R_9$ eine Gruppe der Formel (D) ist, worin "p" und "r" unabhängig voneinander 0, 1, 2 oder 3 sind; und $R_{18}$ H, Carbo-$C_{1-6}$-alkoxy, Carboxy, Heteroaryl oder Phenyl ist; worin Heteroaryl und Phenyl gegebenenfalls mit 1 bis 5 Substituenten substituiert sind, die unabhängig voneinander ausgewählt sind aus $C_{1-4}$-Alkoxy, $C_{1-8}$-Alkyl, Halogen, $C_{1-4}$-Halogenalkoxy und $C_{1-4}$-Halogenalkyl; und
$R_{10}$-$R_{13}$ unabhängig voneinander ausgewählt sind aus $C_{1-4}$-Alkoxy, $C_{1-8}$-Alkyl, Amino, Cyano, Halogen, $C_{1-4}$-Halogenalkoxy, $C_{1-4}$-Halogenalkyl und Hydroxyl.

**3.** Verbindung nach Anspruch 1 oder 2, worin W $-S(O)_2NR_4-$ ist.

**4.** Verbindung nach Anspruch 1 oder 2, worin W $-NR_4-$ ist.

**5.** Verbindung nach Anspruch 3 oder 4, worin $R_4$ H ist.

**6.** Verbindung nach Anspruch 1 oder 2, worin W -O- ist.

**7.** Verbindung nach Anspruch 1 oder 2, worin W fehlt.

**8.** Verbindung nach einem der Ansprüche 1 bis 7, worin A und B beide Ethylen sind.

**9.** Verbindung nach einem der Ansprüche 1 bis 8, worin D $CR_2R_3$ ist, worin $R_2$ ausgewählt ist aus $C_{1-5}$-Acyl, $C_{1-8}$-Alkyl und Heteroaryl; und worin $C_{1-8}$-Alkyl und Heteroaryl gegebenenfalls mit 1 bis 5 Substituenten substituiert sind, die ausgewählt sind aus $C_{1-4}$-Alkoxy, $C_{1-8}$-Alkyl und Halogen.

**10.** Verbindung nach einem der Ansprüche 1 bis 9, worin $R_2$ ausgewählt ist aus $C(O)CH_3$, $C(O)CH_2CH_3$, $C(O)CH_2CH_2CH_3$, $C(O)CH(CH_3)_2$, $C(O)CH_2CH_2CH_2CH_3$, $CH_3$, $CH_2CH_3$, $CH_2CH_2CH_3$, $CH(CH_3)_2$, $CH(CH_3)(CH_2CH_3)$, $CH_2(CH_2)_2CH_3$ und $CH_2(CH_2)_3CH_3$.

**11.** Verbindung nach einem der Ansprüche 1 bis 9, worin $R_2$ ausgewählt ist aus $CO_2CH_3$, $CO_2CH_2CH_3$, $CO_2CH_2CH_2CH_3$, $CO_2CH(CH_3)_2$ und $CO_2CH_2(CH_2)_2CH_3$.

**12.** Verbindung nach einem der Ansprüche 1 bis 9, worin $R_2$ $C_{1-8}$-Alkyl ist, das gegebenenfalls mit 1 bis 5 Substituenten substituiert ist, die ausgewählt sind aus $C_{1-4}$-Alkoxy, $C_{1-8}$-Alkyl und Halogen.

**13.** Verbindung nach einem der Ansprüche 1 bis 9, worin $R_2$ ausgewählt ist aus $CH_2OCH_3$, $CH_2CH_2OCH_3$, $CH_2OCH_2CH_3$, $CH_2OCH_2CH_2CH_3$, $CH_2CH_2OCH_2CH_3$, $CH_2CH_2OCH_2CH_2CH_3$, $CH_2OCH(CH_3)_2$ und $CH_2OCH_2CH(CH_3)_2$.

**14.** Verbindung nach einem der Ansprüche 1 bis 9, worin $R_2$ ein 1,2,4-Oxadiazolyl ist, das gegebenenfalls mit $C_{1-8}$-Alkyl substituiert ist.

**15.** Verbindung nach einem der Ansprüche 1 bis 9, worin $R_2$ 3-Methyl-1,2,4-oxadiazol-5-yl, 3-Ethyl-1,2,4-oxadiazol-5-yl, 3-Propyl-1,2,4-oxadiazol-5-yl, 3-Isopropyl-1,2,4-oxadiazol-5-yl, 3-Butyl-1,2,4-oxadiazol-5-yl oder 3-Isobutyl-1,2,4-oxadiazol-5-yl ist.

**16.** Verbindung nach einem der Ansprüche 1 bis 8, worin D $CR_2R_3$ ist, worin $R_2$ die Gruppe der Formel (C) ist, worin

G S, S(O) oder $S(O)_2$ ist; und
$Ar_4$ Phenyl oder Heteroaryl ist, das gegebenenfalls mit 1 bis 5 Substituenten substituiert ist, die ausgewählt sind aus $C_{1-4}$-Alkoxy, $C_{1-8}$-Alkyl, Cyano, $C_{1-4}$-Halogenalkoxy, $C_{1-4}$-Halogenalkyl und Halogen.

**17.** Verbindung nach einem der Ansprüche 1 bis 8 und 16, worin $Ar_4$ Heteroaryl ist, das gegebenenfalls mit 1 bis 5 Substituenten substituiert ist, die ausgewählt sind aus $C_{1-4}$-Alkoxy, $C_{1-8}$-Alkyl, Cyano, $C_{1-4}$-Halogenalkoxy, $C_{1-4}$-Halogenalkyl und Halogen.

**18.** Verbindung nach einem der Ansprüche 1 bis 8, 16 und 17, worin $Ar_4$ eine Pyridylgruppe ist.

**19.** Verbindung nach einem der Ansprüche 1 bis 8 und 16 bis 18, worin $Ar_4$ 2-Pyridyl ist.

**20.** Verbindung nach einem der Ansprüche 1 bis 8 und 16 bis 19, worin G -S- ist.

**21.** Verbindung nach einem der Ansprüche 1 bis 20, worin $R_3$ H ist.

**22.** Verbindung nach einem der Ansprüche 1 bis 21, worin $R_1$ H ist.

**23.** Verbindung nach einem der Ansprüche 1 bis 22, worin $Ar_1$ Phenyl, Pyridyl oder Pyridinon ist, das gegebenenfalls mit $R_9$ und $R_{10}$ substituiert ist.

**24.** Verbindung nach einem der Ansprüche 1 bis 23, worin $R_9$ ausgewählt ist aus $C_{1-5}$-Acyl, Vinyl, $C_{1-8}$-Alkyl, $C_{1-4}$-Alkylsulfonyl, Amino, Benzolsulfonyl, Carboxamid, Cyclopentyl, Halogen, $C_{1-4}$-Halogenalkyl, 2,5-Dioxoimidazolidinyl, Imidazolyl, Pyrrolyl, Triazol-1-yl, Thiadiazolyl, 1,3-Dioxo-1,3-dihydroisoindolyl, Pyrazolyl, [1,3,4]Oxadiazolyl, [1,2,4]Oxadiazolyl, Hydroxyl, Oxocyclohexyl, Phenyl und Sulfonsäure, und worin $C_{1-5}$-Acyl, $C_{1-8}$-Alkyl, Benzolsulfonyl und Phenyl gegebenenfalls mit 1 bis 5 Substituenten substituiert sind, die unabhängig voneinander ausgewählt sind aus $C_{1-4}$-Alkoxy, $C_{1-8}$-Alkyl, Cyano, Heteroaryl und Hydroxyl.

**25.** Verbindung nach einem der Ansprüche 1 bis 23, worin $R_9$ ausgewählt ist aus Acetyl, 4-Hydroxybenzolsulfonyl, 2-Methoxyethyl, Vinyl, Methyl, Methylsulfonyl, Ethansulfonyl, Amino, 4-Hydroxybenzolsulfonyl, 4-Cyanophenyl, 4-Methoxyphenyl, Carboxamid, Cyclopentyl, Fluor, Chlor, Brom, Trifluormethyl, 2,5-Dioxoimidazolidinyl, Imidazol-1-yl, Pyrrolyl, Triazol-1-yl, Thiadiazol-4-yl, 1,3-Dioxo-1,3-dihydroisoindolyl, Pyrazolyl, 5-Methyl[1,3,4]oxydiazol-2-yl, 3-Methyl[1,2,4]oxadiazol-5-yl, Hydroxyl, 4-Oxocyclohexyl, Phenyl und Sulfonsäure.

**26.** Verbindung nach einem der Ansprüche 1 bis 23, worin $R_9$ die Gruppe der Formel (D) ist, worin:

"p" und "r" unabhängig voneinander 0, 1, 2 oder 3 sind; und
$R_{18}$ H, Carbo-$C_{1-6}$-Alkoxy, Carboxy, Heteroaryl oder Phenyl ist, und worin Heteroaryl und Phenyl gegebenenfalls mit 1 bis 5 Substituenten substituiert sind, die unabhängig voneinander ausgewählt sind aus $C_{1-4}$-Alkoxy, $C_{1-8}$-Alkyl, Halogen, $C_{1-4}$-Halogenalkoxy und $C_{1-4}$-Halogenalkyl.

**27.** Verbindung nach einem der Ansprüche 1 bis 23, worin $R_9$ 2-Methoxycarbonylacetyl, Benzoyl, 3-Oxobutyl, 2-Carboxyethyl, 2-Carboxy-2-oxoethyl, $CH_3(CH_2)_2C(O)$, $CH_3(CH_2)_3C(O)$ oder $CH_3(CH_2)_4C(O)$ ist.

**28.** Verbindung nach einem der Ansprüche 1 bis 27, worin $R_{10}$ ausgewählt ist aus $C_{1-4}$-Alkoxy, $C_{1-8}$-Alkyl, Amino, Cyano, Halogen, $C_{1-4}$-Halogenalkoxy, $C_{1-4}$-Halogenalkyl und Hydroxyl.

**29.** Verbindung nach einem der Ansprüche 1 bis 27, worin $R_{10}$ ausgewählt ist aus Amino, Methoxy, Methyl, Cyano, Fluor, Chlor, Brom, Trifluormethoxy, Trifluormethyl und Hydroxyl.

**30.** Verbindung nach einem der Ansprüche 1 bis 29, worin Y $CR_6$ ist.

**31.** Verbindung nach Anspruch 30, worin $R_6$ H ist.

**32.** Verbindung nach einem der Ansprüche 1 bis 31, worin U N ist.

**33.** Verbindung nach einem der Ansprüche 1 bis 31, worin U $CR_1$ ist.

**34.** Verbindung nach Anspruch 33, worin $R_1$ H ist.

**35.** Verbindung nach Anspruch 1 oder 2, worin:

U N ist und
X und Y beide CH sind.

**36.** Verbindung nach Anspruch 35, worin:

A und B beide $-CH_2CH_2-$ sind;
D $CR_2R_3$ ist, worin $R_2$ ausgewählt ist aus $C(O)CH_3$, $CO_2CH_2CH_3$, $CH_2CH_2CH_3$ und Pyridin-2-ylsulfanyl; und $R_3$ H ist;
V fehlt;
W -O- ist;
Z Nitro ist; und
$Ar_1$ Phenyl ist, das gegebenenfalls mit $R_9$ und $R_{10}$ substituiert ist, worin $R_9$ Acetyl, 2-Methoxyethyl, Ethansulfonyl, 4-Hydroxybenzolsulfonyl, 4-Cyanophenyl, 4-Methoxyphenyl, Carboxamid, Cyclopentyl, 2,5-Dioxoimidazolidinyl, Imidazol-1-yl, Pyrrolyl, Triazol-1-yl, Thiadiazol-4-yl, 1,3-Dioxo-1,3-dihydroisoindolyl, 4-Oxocyclohexyl, Sulfonsäure, 2-Methoxycarbonylacetyl, Benzoyl oder 3-Oxobutyl ist; und $R_{10}$ Amino ist.

**37.** Verbindung nach Anspruch 1 oder 2, worin:

U CH ist,
X CH oder $C-NO_2$ ist und
Y CH ist.

**38.** Verbindung nach Anspruch 37, worin:

A und B beide $-CH_2CH_2-$ sind;
D $CR_2R_3$ ist, worin $R_2$ ausgewählt ist aus $CO_2CH_2CH_3$, $CH_2CH_2CH_3$, Pyridin-2-ylsulfanyl, $CH_2OCH_3$ und 3-Methyl-1,2,4-oxadiazol-5-yl; und $R_3$ H ist;
V fehlt;
W -O- ist;
Z Nitro ist; und
$Ar_1$ Phenyl ist, das gegebenenfalls mit $R_9$ und $R_{10}$ substituiert ist, worin $R_9$ Acetyl, Vinyl, Ethansulfonyl, Triazol-1-yl, 2-(3-Methyl[1,2,4]oxadiazolo-5-yl)acetyl, 5-Hydroxy-1-methyl-1H-pyrazol-3-yl, 5-Trifluormethylpyridin-2-yl, 5-Brompyridin-2-yl, 2-Methoxycarbonylacetyl, Benzoyl, 3-Oxobutyl, 2-Carboxyethyl, 2-Carboxy-2-oxo-ethyl, $CH_3(CH_2)_2C(O)$, $CH_3(CH_2)_3C(O)$ oder $CH_3(CH_2)_4C(O)$ ist; und worin $R_{10}$ Amino ist.

**39.** Verbindung nach Anspruch 1, ausgewählt aus den folgenden Verbindungen und pharmazeutisch annehmbaren Salzen, Hydraten und Solvaten davon:

(A1) 6'-[4-(2-Methoxycarbonylacetyl)phenoxy]-3'-nitro-3,4,5,6-tetrahydro-2H-[1,2']bipyridinyl-4-carbonsäureethylester;
(A2) 1-[4-(4-Acetyl-3'-nitro-3,4,5,6-tetrahydro-2H-[1,2']bipyridinyl-6'-yloxy)-phenyl]ethanon;
(A3) 6'-[4-(4-Hydroxybenzolsulfonyl)phenoxy]-3'-nitro-3,4,5,6-tetrahydro-2H-[1,2']bipyridinyl-4-carbonsäureethylester;
(A4) 6'-(4-Imidazol-1-yl-phenoxy)-3'-nitro-3,4,5,6-tetrahydro-2H-[1,2']bipyridinyl-4-carbonsäureethylester;
(A5) 6'-(4-Benzoylphenoxy)-3'-nitro-3,4,5,6-tetrahydro-2H-[1,2']bipyridinyl-4-carbonsäureethylester;
(A6) 6'-[4-(2-Methoxyethyl)phenoxy]-3'-nitro-3,4,5,6-tetrahydro-2H-[1,2']-bipyridinyl-4-carbonsäureethylester;
(A7) 6'-(4-Cyclopentylphenoxy)-3'-nitro-3,4,5,6-tetrahydro-2H-[1,2']bipyridinyl-4-carbonsäureethylester;
(A8) 6'-(4'-Cyanobiphenyl-4-yloxy)-3'-nitro-3,4,5,6-tetrahydro-2H-[1,2']bipyridinyl-4-carbonsäureethylester;
(A9) 3'-Nitro-6'-(4-sulfophenoxy)-3,4,5,6-tetrahydro-2H-[1,2']bipyridinyl-4-carbonsäureethylester;
(A10) 3'-Nitro-6'-(4-pyrrol-1-yl-phenoxy)-3,4,5,6-tetrahydro-2H-[1,2']bipyridinyl-4-carbonsäureethylester;
(A11) 6'-(4-Carbamoylphenoxy)-3'-nitro-3,4,5,6-tetrahydro-2H-[1,2']bipyridinyl-4-carbonsäureethylester;
(A12) 3'-Nitro-6'-(4-[1,2,4]triazol-1-yl-phenoxy)-3,4,5,6-tetrahydro-2H-[1,2']-bipyridinyl-4-carbonsäureethylester;
(A13) 6'-(2-Amino-4-ethansulfonylphenoxy)-3'-nitro-3,4,5,6-tetrahydro-2H-[1,2']bipyridinyl-4-carbonsäureethylester;
(A14) 3'-Nitro-6'-[4-(4-oxocyclohexyl)phenoxy]-3,4,5,6-tetrahydro-2H-[1,2']-bipyridinyl-4-carbonsäureethylester;
(A15) 6'-(4'-Methoxybiphenyl-4-yloxy)-3'-nitro-3,4,5,6-tetrahydro-2H-[1,2']-bipyridinyl-4-carbonsäureethylester;
(A16) 3'-Nitro-6'-(4-[1,2,3]thiadiazol-4-ylphenoxy)-3,4,5,6-tetrahydro-2H-[1,2']bipyridinyl-4-carbonsäureethylester;
(A17) 6'-[4-(1,3-Dioxo-1,3-dihydroisoindol-2-yl)phenoxy]-3'-nitro-3,4,5,6-tetrahydro-2H-[1,2']bipyridinyl-4-carbonsäureethylester;
(A18) 6'-[4-(2,5-Dioxoimidazolidin-4-yl)phenoxy]-3'-nitro-3,4,5,6-tetrahydro-2H-[1,2']bipyridinyl-4-carbonsäureethylester;
(A19) 3'-Nitro-6'-[4-(3-oxobutyl)phenoxy]-3,4,5,6-tetrahydro-2H-[1,2']bipyridinyl-4-carbonsäureethylester;
(A20) 3-[4-(3'-Nitro-4-propyl-3,4,5,6-tetrahydro-2H-[1,2']bipyridinyl-6'-yloxy)-phenyl]-3-oxopropionsäuremethylester;
(A21) 4-[4-(3'-Nitro-4-propyl-3,4,5,6-tetrahydro-2H-[1,2']bipyridinyl-6'-yloxy)-phenyl]butan-2-on;
(A22) 4-{4-[3'-Nitro-4-(pyridin-2-ylsulfanyl)-3,4,5,6-tetrahydro-2H-[1,2']bipyridinyl-6'-yloxy]phenyl}butan-2-on; und
(A23) 3'-Nitro-4-(pyridin-2-ylsulfanyl)-6'-(4-[1,2,4]triazol-1-ylphenoxy)-3,4,5,6-tetrahydro-2H-[1,2']bipyridinyl.

**40.** Verbindung nach Anspruch 1, ausgewählt aus den folgenden Verbindungen und pharmazeutisch annehmbaren Salzen, Hydraten und Solvaten davon:

(A24) 1-[5-(4-Benzoylphenoxy)-2-nitrophenyl)piperidin-4-carbonsäureethylester;
(A25) 1-{5-[4-(2-Methoxycarbonylacetyl)phenoxy]-2-nitrophenyl}piperidin-4-carbonsäureethylester;
(A26) 1-[5-(2-Amino-4-ethansulfonylphenoxy)-2-nitrophenyl]piperidin-4-carbonsäureethylester;
(A27) 1-{2-Nitro-5-[4-(3-oxobutyl)phenoxy]phenyl}piperidin-4-carbonsäureethylester;
(A28) 4-{4-[4-Nitro-3-(4-propylpiperidin-1-yl)phenoxy]phenyl}butan-2-on;
(A29) 1-{4-[4-Nitro-3-(4-propylpiperidin-1-yl)phenoxy]phenyl}ethanon;
(A30) 3-{4-[4-Nitro-3-(4-propylpiperidin-1-yl)phenoxy]phenyl}-3-oxopropionsäuremethylester;
(A31) 5-Ethansulfonyl-2-[4-nitro-3-(4-propylpiperidin-1-yl)phenoxy]phenylamin;
(A32) {4-[4-Nitro-3-(4-propylpiperidin-1-yl)phenoxy]phenyl}phenylmethanon;
(A37) 1-{5-[4-(2-Carboxyethyl)phenoxy]-2-nitrophenyl}piperidin-4-carbonsäureethylester;
(A38) 1-{5-[4-(2-Carboxy-2-oxoethyl)phenoxy]-2-nitrophenyl}piperidn-4-carbonsäureethylester;
(A39) 1-[2-Nitro-5-(4-vinylphenoxy)phenyl]piperidin-4-carbonsäureethylester;
(A40) 3-{4-[4-Nitro-3-(4-propylpiperidin-1-yl)phenoxy]phenyl}propionsäure;
(A41) 3-{4-[4-Nitro-3-(4-propylpiperidin-1-yl)phenoxy]phenyl}-2-oxopropionsäure;

(A42) 1-[2-Nitro-5-(4-vinylphenoxy)phenyl]-4-propylpiperidin;
(A43) 1-{4-[4-Nitro-3-(4-propylpiperidin-1-yl)phenoxy]phenyl}butan-1-on;
(A44) 1-{4-[4-Nitro-3-(4-propylpiperidin-1-yl)phenoxy]phenyl}pentan-1-on;
(A45) 1-{4-[4-Nitro-3-(4-propylpiperidin-1-yl)phenoxy]phenyl}hexan-1-on;
(A46) 4-{4-[3-(4-Methoxymethylpiperidin-1-yl)-4-nitrophenoxy]phenyl}butan-2-on;
(A47) 1-{4-[3-(4-Methoxymethylpiperidin-1-yl)-4-nitrophenoxy]phenyl}ethanon;
(A48) {4-[3-(4-Methoxymethylpiperidin-1-yl)-4-nitrophenoxy]phenyl}phenylmethanon;
(A49) 2-(3-Methyl[1,2,4]oxadiazol-5-yl)-1-{4-[4-nitro-3-(4-propylpiperidin-1-yl)-phenoxy]phenyl}ethanon;
(A50) 4-(4-{3-[4-(3-Methyl[1,2,4]oxadiazol-5-yl)piperidin-1-yl]-4-nitrophenoxy}phenyl)butan-2-on;
(A51) 4-(4-{4-Nitro-3-[4-(pyridin-2-ylsulfanyl)piperidin-1-yl]phenoxy}phenyl)butan-2-on;
(A52) 2-{1-[2-Nitro-5-(4-[1,2,4]triazol-1-ylphenoxy)phenyl]piperidin-4-ylsulfanyl}pyridin;
(A53) 2-Methyl-5-{4-[4-nitro-3-(4-propylpiperidin-1-yl)phenoxy]phenyl}-2H-pyrazol-3-ol;
(A54) 2-[4-Nitro-3-(4-propylpiperidin-1-yl)phenoxy]-5-trifluormethylpyridin;
(A55) 5-Brom-2-[4-nitro-3-(4-propylpiperidin-1-yl)phenoxy]pyridin;
(A56) 1-(4-{4-Nitro-3-[4-(pyridin-2-ylsulfanyl)piperidin-1-yl]phenoxy}phenyl)-ethanon;
(A57) 2-{1-[5-(4-Methansulfonylphenoxy)-2-nitrophenyl]piperidin-4-ylsulfanyl}pyridin;
(A59) 1-{5-[4-(5-Methyl[1,3,4]oxadiazol-2-yl)phenoxy]-2-nitrophenyl}-4-propylpiperidin; und
(A60) 1-{5-[3-(3-Methyl[1,2,4]oxadiazol-5-yl)phenoxy]-2-nitrophenyl}-4-propylpiperidin.

**41.** Verbindung nach Anspruch 1, ausgewählt aus der folgenden Verbindung und pharmazeutisch annehmbaren Salzen, Hydraten und Solvaten davon:

(A58) 5-Brom-1-[4-nitro-3-(4-propylpiperidin-1-yl)phenyl]-1H-pyridin-2-on.

**42.** Verbindung nach Anspruch 1, ausgewählt aus den folgenden Verbindungen und pharmazeutisch annehmbaren Salzen, Hydraten und Solvaten davon:

(B1) 6'-Benzolsulfonylamino-3'-nitro-3,4,5,6-tetrahydro-2H-[1,2']bipyridinyl-4-carbonsäureethylester;
(B2) 6'-(Benzolsulfonylmethylamino)-3'-nitro-3,4,5,6-tetrahydro-2H-[1,2']-bipyridinyl-4-carbonsäureethylester;
(B3) 6'-(Benzolsulfonylbutylamino)-3'-nitro-3,4,5,6-tetrahydro-2H-[1,2']bipyridinyl-4-carbonsäureethylester;
(B4) 6'-(5-Ethansulfonyl-2-hydroxyphenylamino)-3'-nitro-3,4,5,6-tetrahydro-2H- [1,2']bipyridinyl-4-carbonsäureethylester;
(B5) 6'-(2-Brom-4-trifluoromethylbenzolsulfonylamino)-3'-nitro-3,4,5,6-tetrahydro-2H-[1,2']bipyridinyl-4-carbonsäureethylester;
(B6) {4-[3'-Nitro-4-(pyridin-2-ylsulfanyl)-3,4,5,6-tetrahydro-2H-[1,2']bipyridinyl-6'-ylamino]phenyl}phenylmethanon; und
(B7) [3'-Nitro-4-(pyridin-2-ylsulfanyl)-3,4,5,6-tetrahydro-2H-[1,2']bipyridinyl-6'-yl]- (4-[1,2,4]triazol-1-ylphenyl)amin.

**43.** Verbindung nach Anspruch 1, ausgewählt aus den folgenden Verbindungen und pharmazeutisch annehmbaren Salzen, Hydraten und Solvaten davon:

(B8) 1-[5-(4-Benzoylphenylamino)-2-nitrophenyl]piperidin-4-carbonsäureethylester; und
(B9) {4-[4-Nitro-3-(4-propylpiperidin-1-yl)phenylamino]phenyl}-phenylmethanon.

**44.** Pharmazeutische Zusammensetzung, die zumindest eine Verbindung nach einem der Ansprüche 1 bis 43 in Kombination mit einem pharmazeutisch annehmbaren Träger umfasst.

**45.** Verfahren zur Herstellung einer pharmazeutischen Zusammensetzung, umfassend das Vermischen zumindest einer Verbindung nach einem der Ansprüche 1 bis 43 und eines pharmazeutisch annehmbaren Trägers.

**46.** Verbindung nach einem der Ansprüche 1 bis 43 zur Verwendung in einem Behandlungsverfahren für den menschlichen oder tierischen Körper durch Therapie.

**47.** Verbindung nach einem der Ansprüche 1 bis 43 zur Verwendung in einem Verfahren zur Prophylaxe oder Behandlung einer Stoffwechselstörung.

**48.** Verwendung einer Verbindung nach einem der Ansprüche 1 bis 43 zur Herstellung eines Medikaments zur Verwendung bei der Prophylaxe oder Behandlung einer Stoffwechselstörung.

**49.** Verbindung nach einem der Ansprüche 1 bis 43 zur Verwendung in einem Verfahren zur Behandlung oder Prophylaxe von Typ-II-Diabetes, mangelhafter Glucosetoleranz, Insulinresistenz, Hyperglykämie, Hyperlipidämie, Hypertriglyceridämie, Hypercholesterinämie, Dyslipidämie, Syndrom X oder eines metabolischen Syndroms.

**50.** Verwendung einer Verbindung nach einem der Ansprüche 1 bis 43 zur Herstellung eines Medikaments zur Verwendung bei der Prophylaxe oder Behandlung von Typ-II-Diabetes, mangelhafter Glucosetoleranz, Insulinresistenz, Hyperglykämie, Hyperlipidämie, Hypertriglyceridämie, Hypercholesterinämie, Dyslipidämie, Syndrom X oder eines metabolischen Syndroms.

**51.** Verbindung nach einem der Ansprüche 1 bis 43 zur Verwendung in einem Verfahren zur Behandlung oder Prophylaxe von Typ-II-Diabetes.

**52.** Verwendung einer Verbindung nach einem der Ansprüche 1 bis 43 zur Herstellung eines Medikaments zur Verwendung bei der Prophylaxe oder Behandlung von Typ-II-Diabetes.

**53.** Verbindung nach einem der Ansprüche 1 bis 43 zur Verwendung in einem Verfahren zur Steuerung oder Senkung der Gewichtszunahme bei einem Individuum.

**54.** Verwendung einer Verbindung nach einem der Ansprüche 1 bis 43 zur Herstellung eines Medikaments zur Steuerung oder Senkung der Gewichtszunahme bei einem Individuum.

## Revendications

**1.** Composé sélectionné parmi des composés de Formule (**Ia**) et leurs sels, hydrates, et solvates pharmaceutiquement acceptables:

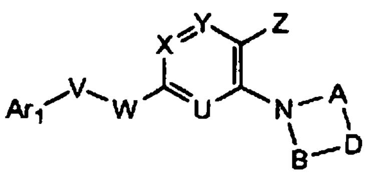

**(Ia)**

où:

A et B sont tous deux de l'éthylène facultativement substitué par 1 à 4 groupes méthyles;
U est N ou $CR_1$;
D est $CR_2R_3$;
V est absent;
W est $-S(O)_2NR_4-$, $-NR_4-$, -O-, -S-, -S(O)-, ou $-S(O)_2-$;
ou W est absent;
X est $CR_5$;
Y est N ou $CR_6$;
Z est nitro;
$Ar_1$ est aryle ou hétéroaryle facultativement substitué par $R_9$, $R_{10}$, $R_{11}$, $R_{12}$ et $R_{13}$;
$R_1$ et $R_6$ sont indépendamment sélectionnés parmi H, acyloxy $C_{1-5}$, alcényle $C_{2-6}$, alcoxy $C_{1-4}$, alkyle $C_{1-8}$, alkylcarboxamide $C_{1-4}$, alkynyle $C_{2-6}$, alkylsulfonamide$C_{1-4}$, alkylsulfinyle $C_{1-4}$, alkylsulfonyle $C_{1-4}$, alkylthio $C_{1-4}$, alkyluréyle $C_{1-4}$, amino, alkylamino $C_{1-4}$, dialkylamino $C_{2-8}$, carboxamide, cyano, cycloalkyle $C_{3-6}$, dialkylcarboxamide $C_{2-6}$, dialkylsulfonamide $C_{2-6}$, halogène, haloalcoxy $C_{1-4}$, haloalkyle $C_{1-4}$, haloalkylsulfinyle $C_{1-4}$, haloalkylsulfonyle $C_{1-4}$, haloalkylthio $C_{1-4}$, hydroxyle et nitro;
$R_5$ est H ou nitro;
$R_2$ est sélectionné parmi acyle $C_{1-5}$, acyloxy $C_{1-5}$, alcoxy $C_{1-4}$, alkyle $C_{1-8}$, alkylcarboxamide $C_{1-4}$, alkylthiocarboxamide $C_{1-4}$, alkylsulfinyle $C_{1-4}$, alkylsulfonyle $C_{1-4}$, alkylthio $C_{1-4}$, amino, carbo-alcoxy $C_{1-6}$, carboxamide,

carboxy, cyano, cycloakyle $C_{3-6}$, dialkylcarboxamide $C_{2-6}$, haloalcoxy $C_{1-4}$, haloalkyle $C_{1-4}$, halogène, hétéroaryle, hydroxyle et phényle; et où alkyle $C_{1-8}$, hétéroaryle et phényle sont facultativement substitués par 1 à 5 substituants sélectionnés parmi acyle $C_{1-5}$, acyloxy $C_{1-5}$, alcoxy $C_{1-4}$, alkyle $C_{1-8}$, alkylamino $C_{1-4}$, alkylcarboxamide $C_{1-4}$, alkylthiocarboxamide $C_{1-4}$, alkylsulfonamide $C_{1-4}$, alkylsulfinyle $C_{1-4}$, alkylsulfonyle $C_{1-4}$, alkylthio $C_{1-4}$, alkylthiouréyle $C_{1-4}$, alkyluréyle $C_{1-4}$, amino, carbo-alcoxy $C_{1-6}$, carboxamide, carboxy, cyano, cycloalkyle $C_{3-6}$, cycloalkyle-$C_{3-6}$-hétéroalkylène $C_{1-3}$, dialkylamino $C_{2-8}$, dialkylcarboxamide $C_{2-6}$, dialkylthiocarboxamide $C_{1-4}$, dialkylsulfonamide $C_{2-6}$, alkylthiouréyle $C_{1-4}$, haloalcoxy $C_{1-4}$, haloalkyle $C_{1-4}$, haloalkylsulfinyle $C_{1-4}$, haloalkylsulfonyle $C_{1-4}$, haloalkylthio $C_{1-4}$, halogène, hétérocyclique, hydroxyle, hydroxylamino et nitro; ou $R_2$ est -$Ar_2$-$Ar_3$ où $Ar_2$ et $Ar_3$ sont indépendamment aryle ou hétéroaryle facultativement substitués par 1 à 5 substituants sélectionnés parmi H, acyle $C_{1-5}$, acyloxy $C_{1-5}$, alcoxy $C_{1-4}$, alkyle $C_{1-8}$, alkylcarboxamide $C_{1-4}$, dialkylthiocarboxamide $C_{1-4}$, alkylsulfinyle $C_{1-4}$, alkylsulfonyle $C_{1-4}$, alkylthio $C_{1-4}$, amino, carbo-alcoxy $C_{1-6}$, carboxamide, carboxy, cyano, cycloalkyle $C_{3-6}$, dialkylcarboxamide $C_{2-6}$, haloalcoxy $C_{1-4}$, haloalkyle $C_{1-4}$, halogène, hydroxyle et nitro; ou

$R_2$ est un groupe de la Formule (B):

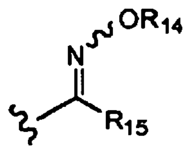

(B)

où:

$R_{14}$ est alkyle $C_{1-8}$ ou cycloalkyle $C_{3-6}$; et $R_{15}$ est F, Cl, Br ou CN; ou

$R_2$ est un groupe de la Formule (**C**):

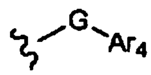

(C)

où:

G est C=O, $CR_{16}R_{17}$, O, S, S(O), $S(O)_2$; où $R_{16}$ et $R_{17}$ sont indépendamment H ou alkyle $C_{1-8}$; et $Ar_4$ est phényle ou hétéroaryle facultativement substitué par 1 à 5 substituants sélectionnés parmi acyle $C_{1-5}$, acyloxy $C_{1-5}$, alcoxy $C_{1-4}$, alkyle $C_{1-8}$, alkylcarboxamide $C_{1-4}$, alkylthiocarboxamide $C_{1-4}$, alkylsulfonamide $C_{1-4}$, alkylsulfinyle $C_{1-4}$, alkylsulfonyle $C_{1-4}$, alkylthio $C_{1-4}$, alkylthiouréyle $C_{1-4}$, alkyluréyle $C_{1-4}$, amino, carbo-alcoxy $C_{1-6}$, carboxamide, carboxy, cyano, cycloalkyle $C_{3-6}$, dialkylcarboxamide $C_{2-6}$, dialkylthiocarboxamide $C_{1-4}$, dialkylsulfonamide $C_{2-6}$, alkylthiouréyle $C_{1-4}$, haloalcoxy $C_{1-4}$, haloalkyle $C_{1-4}$, haloalkylsulfinyle $C_{1-4}$, haloalkylsulfonyle $C_{1-4}$, haloalkylthio $C_{1-4}$, halogène, hétéroaryle, hydroxyle, hydroxylamino et nitro;

$R_3$ est H, alkyle $C_{1-8}$, alcoxy $C_{1-4}$ ou hydroxyle;
$R_4$ est H ou alkyle $C_{1-8}$;
$R_9$ est sélectionné parmi alcyle $C_{1-5}$, acyloxy, $C_{1-5}$, alcényle $C_{1-6}$, alcoxy $C_{1-4}$, alkyle $C_{1-8}$, alkylcarboxamide $C_{1-4}$, alkynyle $C_{2-6}$, alkylsulfonamide $C_{1-4}$, alkylsulfinyle $C_{1-4}$, alkysulfonyle $C_{1-4}$, alkylthio $C_{1-4}$, alkyluréyle $C_{1-4}$, amino, arylsulfonyle, carbo-alcoxy $C_{1-6}$, carboxamide, carboxy, cyano, cycloalkyle $C_{3-6}$, dialkylcarboxamide $C_{2-6}$, halogène, haloalcoxy $C_{1-4}$, haloalkyle $C_{1-4}$, haloalkylsulfinyle $C_{1-4}$, haloalkylsulfonyle $C_{1-4}$, haloalkylthio $C_{1-4}$, hétérocyclique, sulfonylehétérocyclique, hétéroaryle, hydroxyle, nitro, oxo-cycloalkyle $C_{4-7}$, phénoxy, phényle, sulfonamide et acide sulfonique; où acyle $C_{1-5}$, alcoxy $C_{1-4}$, alkyle $C_{1-8}$, alkylsulfonamide $C_{1-4}$, alkylsulfonyle, arylsulfonyle, hétéroaryle, phénoxy et phényle sont facultativement substitués par 1 à 5 substituants indépendamment sélectionnés parmi acyle $C_{1-5}$, acyloxy $C_{1-5}$, alcényle $C_{2-6}$, alcoxy $C_{1-4}$, alkyle $C_{1-8}$, alkylcarboxamide $C_{1-4}$, alkynyle $C_{2-6}$, alkylsulfonamide $C_{1-4}$, alkylsulfinyle $C_{1-4}$, alkylsulfonyle $C_{1-4}$, alkylthio $C_{1-4}$, alkyluréyle $C_{1-4}$, carbo-alcoxy $C_{1-6}$, carboxamide, carboxy, cyano, cycloalkyle $C_{3-6}$, dialkylcarboxamide $C_{2-6}$,

halogène, haloalcoxy $C_{1-4}$, haloalkyle $C_{1-4}$, haloalkylsulfinyle, haloalkylsulfonyle $C_{1-4}$, haloalkylthio $C_{1-4}$, hétéroaryle, hétérocyclique, hydroxyle, nitro et phényle; ou
$R^9$ est un groupe de Formule (D):

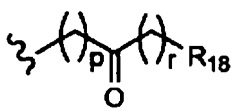

(D)

où:

"p" et "r" sont indépendamment 0, 1, 2 ou 3; et
$R_{18}$ est H, acyle $C_{1-5}$, alcényle $C_{2-6}$, alkyle $C_{1-8}$, alkylcarboxamide $C_{1-4}$, alkynyle $C_{2-6}$, alkylsulfonamide $C_{1-4}$, carbo-alcoxy $C_{1-6}$, carboxamide, carboxy, cyano, cycloalkyle $C_{3-6}$, dialkylcarboxamide $C_{2-6}$, halogène, hétéroaryle ou phényle, où hétéroaryle et phényle sont facultativement substitués par 1 à 5 substituants sélectionnés indépendamment parmi alcoxy $C_{1-4}$, alkyle $C_{1-8}$, amino, alkylamino $C_{1-4}$, alkynyle $C_{2-6}$, dialkylamino $C_{2-8}$, halogène, haloalcoxy $C_{1-4}$, haloalkyle $C_{1-4}$ et hydroxyle; et

$R_{10}$-$R_{13}$ sont indépendamment sélectionnés parmi acyle $C_{1-5}$, acyloxy$C_{1-5}$, alcényle$C_{2-6}$, alcoxy$C_{1-4}$, alkyle$C_{1-8}$, alkylcarboxamide $C_{1-4}$, alkynyle $C_{2-6}$, alkylsulfonamide $C_{1-4}$, alkylsulfinyle $C_{1-4}$, alkylsulfonyle $C_{1-4}$, alkylthio $C_{1-4}$, alkyluréyle $C_{1-4}$, amino, carbo-alcoxy $C_{1-6}$, carboxamide, carboxy, cyano, cycloalkyle $C_{3-6}$, dialkylcarboxamide $C_{2-6}$, halogène, haloalcoxy $C_{1-4}$, haloalkyle $C_{1-4}$ , haloalkylsulfinyle $C_{1-4}$, haloalkylsulfonyle $C_{1-4}$, haloalkylthio $C_{1-4}$, hydroxyle et nitro; ou
deux groupes $R_{10}$-$R_{11}$ adjacents forment un groupe cycloalkyle, cycloalcényle ou hétérocyclique à 5, 6 ou 7 membres avec $Ar_1$ où le groupe à 5, 6 ou 7 membres est facultativement substitué par halogène.

**2.** Composé selon la revendication 1 où:

A et B sont tous deux de l'éthylène facultativement substitué par 1 à 4 groupes méthyles;
U est N ou $CR_1$;
D est $CR_2R_3$;
V est absent;
W est $-S(O)_2NR_4$-, $-NR_4$-, -0-;
ou W est absent;
X est $CR_5$;
Y est $CR_6$;
Z est nitro;
$Ar_1$ est aryle ou hétéroaryle facultativement substitué par $R_9$, $R_{10}$, $R_{11}$, $R_{12}$ et $R_{13}$;
$R_1$ et $R_6$ sont chacun indépendamment sélectionnés parmi H, halogène, et nitro;
$R_5$ est H ou nitro;
$R_2$ est sélectionné parmi acyle $C_{1-5}$, alkyle $C_{1-8}$, et hétéroaryle; et où alkyle $C_{1-8}$, et hétéroaryle sont facultativement substitués par 1 à 5 substituants sélectionnés parmi alcoxy $C_{1-4}$, alkyle $C_{1-8}$, et halogène;
ou
$R_2$ est un groupe de la Formule (C), où G est S, S(O), ou $S(O)_2$; et $Ar_4$ est phényle ou hétéroaryle facultativement substitué par 1 à 5 substituants sélectionnés parmi alcoxy $C_{1-4}$, alkyle $C_{1-8}$, cyano, haloalcoxy $C_{1-4}$, haloalkyle $C_{1-4}$, et halogène;
$R_3$ est H;
$R_4$ est H ou alkyle $C_{1-8}$;
$R_9$ est sélectionné parmi acyle $C_{1-5}$, alcényle $C_{2-6}$, alcoxy $C_{1-4}$, alkyle $C_{1-8}$, alkylsulfonyle $C_{1-4}$, amino, arylsulfonyle, carbo-alcoxy $C_{1-6}$, carboxamide, carboxy, cyano, cycloalkyle $C_{3-6}$, halogène, haloalcoxy $C_{1-4}$, haloalkyle $C_{1-4}$, hétérocyclique, hétéroaryle, hydroxyle, oxocycloalkyle $C_{4-7}$, phényle, et acide sulfonique; où acyle $C_{1-5}$, alkyle $C_{1-8}$, arylsulfonyle, hétéroaryle, et phényle sont facultativement substitués par 1 à 5 substituants indépendamment sélectionnés parmi alcoxy $C_{1-4}$, alkyle $C_{1-8}$, alkylsulfonyle $C_{1-4}$, cyano, halogène, haloalcoxy $C_{1-4}$, haloalkyle $C_{1-4}$, hétéroaryle, et hydroxyle;
ou

$R_9$ est un groupe de la Formule (**D**) où "p" et "r" sont indépendamment 0, 1, 2 ou 3; et $R_{18}$ est H, carbo-alcoxy $C_{1-6}$, carboxy, hétéroaryle ou phényle; où hétéroaryle et phényle sont facultativement substitués par 1 à 5 substituants indépendamment sélectionnés parmi alcoxy $C_{1-4}$, alkyle $C_{1-8}$, halogène, haloalcoxy $C_{1-4}$, et haloalkyle $C_{1-4}$; et
$R_{10}$-$R_{13}$ sont indépendamment sélectionnés parmi alcoxy $C_{1-4}$, alkyle $C_{1-8}$, amino, cyano, halogène, haloalcoxy $C_{1-4}$, haloalkyle $C_{1-4}$ et hydroxyle.

**3.** Composé selon la revendication 1 ou 2, où W est $-S(O)_2NR_4-$.

**4.** Composé selon la revendication 1 ou 2, où W est $-NR_4-$.

**5.** Composé selon la revendication 3 ou 4, où $R_4$ est H.

**6.** Composé selon la revendication 1 ou 2, où W est -O-.

**7.** Composé selon la revendication 1 ou 2, où W est absent.

**8.** Composé selon l'une quelconque des revendications 1 à 7, où A et B sont tous deux de l'éthylène.

**9.** Composé selon l'une quelconque des revendications 1 à 8, où D est $CR_2R_3$, où $R_2$ est sélectionné parmi acyle $C_{1-5}$, alkyle $C_{1-8}$, et hétéroaryle; et où alkyle $C_{1-8}$, et hétéroaryle sont facultativement substitués par 1 à 5 substituants sélectionnés parmi alcoxy $C_{1-4}$, alkyle $C_{1-8}$, et halogène.

**10.** Composé selon l'une quelconque des revendications 1 à 9, où $R_2$ est sélectionné parmi $C(O)CH_3$, $C(O)CH_2CH_3$, $C(O)CH_2CH_2CH_3$, $C(O)CH(CH_3)_2$, $C\ (O)\ CH_2CH_2CH_2CH_3$, $CH_3$, $CH_2CH_3$, $CH_2CH_2CH_3$, $CH(CH_3)_2$, $CH\ (CH_3)(CH_2CH_3)$, $CH_2(CH_2)_2CH_3$, et $CH_2(CH_2)_3CH_3$.

**11.** Composé selon l'une quelconque des revendications 1 à 9, où $R_2$ est sélectionné parmi $CO_2CH_3$, $CO_2CH_2CH_3$, $CO_2CH_2CH_2CH_3$, $CO_2CH(CH_3)_2$ et $CO_2CH_2(CH_2)_2CH_3$.

**12.** Composé selon l'une quelconque des revendications 1 à 9, où $R_2$ est alkyle $C_{1-8}$ facultativement substitué par 1 à 5 substituants sélectionnés parmi alcoxy $C_{1-4}$, alkyle $C_{1-8}$, et halogène.

**13.** Composé selon l'une quelconque des revendications 1 à 9, où $R_2$ est sélectionné parmi $CH_2OCH_3$, $CH_2CH_2OCH_3$, $CH_2OCH_2CH_3$, $CH_2OCH_2CH_2CH_3$, $CH_2CH_2OCH_2CH_3$, $CH_2CH_2OCH_2CH_2CH_3$, $CH_2OCH(CH_3)_2$, et $CH_2OCH_2CH(CH_3)_2$.

**14.** Composé selon l'une quelconque des revendications 1 à 9, où $R_2$ est un 1,2,4-oxadiazolyle facultativement substitué par alkyle $C_{1-8}$.

**15.** Composé selon l'une quelconque des revendications 1 à 9, où $R_2$ est 3-méthyl-1,2,4-oxadiazol-5-yle, 3-éthyl-1,2,4-oxadiazol-5-yle, 3-propyl-1,2,4-oxadiazol-5-yle, 3-isopropyl-1,2,4-oxadiazol-5-yle, 3-butyl-1,2,4-oxadiazol-5-yle, ou 3-isobutyl-1,2,4-oxadiazol-5-yle.

**16.** Composé selon l'une quelconque des revendications 1 à 8, où D est $CR_2R_3$, où $R_2$ est le groupe de Formule (C), où:

G est S, S(O), ou $S(O)_2$; et
$Ar_4$ est phényle ou hétéroaryle facultativement substitué par 1 à 5 substituants sélectionnés parmi alcoxy $C_{1-4}$, alkyle $C_{1-8}$, cyano, haloalcoxy $C_{1-4}$, haloalkyle $C_{1-4}$, et halogène.

**17.** Composé selon l'une quelconque des revendications 1 à 8 et 16, où $Ar_4$ est hétéroaryle facultativement substitué par 1 à 5 substituants sélectionnés parmi alcoxy $C_{1-4}$, alkyle $C_{1-8}$, cyano, haloalcoxy $C_{1-4}$, haloalkyle $C_{1-4}$ et halogène.

**18.** Composé selon l'une quelconque des revendications 1 à 8 et 16 et 17, où $Ar_4$ est un groupe pyridyle.

**19.** Composé selon l'une quelconque des revendications 1 à 8 et 16 à 18, où $Ar_4$ est 2-pyridyle.

**20.** Composé selon l'une quelconque des revendications 1 à 8 et 16 à 19, où G est -S-.

**21.** Composé selon l'une quelconque des revendications 1 à 20, où $R_3$ est H.

**22.** Composé selon l'une quelconque des revendications 1 à 21, où $R_1$ est H.

**23.** Composé selon l'une quelconque des revendications 1 à 22, où $Ar_1$ est phényle, pyridyle, ou pyridinone facultativement substitué par $R_9$ et $R_{10}$.

**24.** Composé selon l'une quelconque des revendications 1 à 23, où $R_9$ est sélectionné parmi acyle $C_{1-5}$, vinyle, alkyle $C_{1-8}$, alkylsulfonyle $C_{1-4}$, amino, benzènesulfonyle, carboxamide, cyclopentyle, halogène, haloalkyle $C_{1-4}$, 2,5-dioxo-imidazolidinyle, imidazolyle, pyrrolyle, triazol-1-yle, thiadiazolyle, 1,3-dioxo-1,3-dihydro-isoindolyle, pyrazolyle, [1,3,4]oxadiazolyle, [1,2,4]oxadiazolyle, hydroxyle, oxo-cyclohexyle, phényle, et acide sulfonique, et où acyle $C_{1-5}$, alkyle $C_{1-8}$, benzènesulfonyle, et phényle sont facultativement substitués par 1 à 5 substituants indépendamment sélectionnés parmi alcoxy$C_{1-4}$, alkyle$C_{1-8}$, cyano, hétéroaryle, et hydroxyle.

**25.** Composé selon l'une quelconque des revendications 1 à 23, où $R_9$ est sélectionné parmi acétyle, 4-hydroxy-benzènesulfonyle, 2-méthoxy-éthyle, vinyle, méthyle, méthylsulfonyle, éthansulfonyle, amino, 4-hydroxybenzènesulfonyle, 4-cyanophényle, 4-méthoxyphényle, carboxamide, cyclopentyle, fluoro, chloro, bromo, trifluorométhyle, 2,5-dioxo-imidazolidinyle, imidazol-1-yle, pyrrolyle, triazol-1-yle, thiadiazol-4-yle, 1,3-dioxo-1,3-dihydro-isoindolyle, pyrazolyle, 5-méthyl-[1,3,4]oxadiazol-2-yle, 3-méthyl-[1,2,4]oxadiazol-5-yle, hydroxyle, 4-oxo-cyclohexyle, phényle, et acide sulfonique.

**26.** Composé selon l'une quelconque des revendications 1 à 23, où $R_9$ est le groupe de Formule (D), où:

"p" et "r" sont indépendamment 0,1,2 ou 3; et
$R_{18}$ est H, carbo-alcoxy $C_{1-6}$, carboxy, hétéroaryle ou phényle, et où l'hétéroaryle et le phényle sont facultativement substitués par 1 à 5 substituants indépendamment sélectionnés parmi alcoxy $C_{1-4}$, alkyle $C_{1-8}$, halogène, haloalcoxy $C_{1-4}$, et haloalkyle $C_{1-4}$.

**27.** Composé selon l'une quelconque des revendications 1 à 23, où $R_9$ est 2-méthoxycarbonyl-acétyle, benzoyle, 3-oxo-butyle, 2-carboxy-éthyle, 2-carboxy-2-oxo-éthyle, $CH_3(CH_2)_2C$ (O), $CH_3(CH_2)_3C(O)$, ou $CH_3(CH_2)_4C(O)$.

**28.** Composé selon l'une quelconque des revendications 1 à 27, où $R_{10}$ est sélectionné parmi alcoxy $C_{1-4}$, alkyle $C_{1-8}$, amino, cyano, halogène, haloalcoxy $C_{1-4}$, haloalkyle $C_{1-4}$, et hydroxyle.

**29.** Composé selon l'une quelconque des revendications 1 à 27, où $R_{10}$ est sélectionné parmi amino, méthoxy, méthyle, cyano, fluoro, chloro, bromo, trifluorométhoxy, trifluorométhyle, et hydroxyle.

**30.** Composé selon l'une quelconque des revendications 1 à 29, où Y est $CR_6$.

**31.** Composé selon la revendication 30, où $R_6$ est H.

**32.** Composé selon l'une quelconque des revendications 1 à 31, où U est N.

**33.** Composé selon l'une quelconque des revendications 1 à 31, où U est $CR_1$.

**34.** Composé selon la revendication 33, où $R_1$ est H.

**35.** Composé selon la revendication 1 ou 2, où:

U est N, et
X et Y sont tous deux CH.

**36.** Composé selon la revendication 35, où:

A et B sont tous deux $-CH_2CH_2-$;
D est $CR_2R_3$, où $R_2$ est sélectionné parmi $C(O)CH_3$, $CO_2CH_2CH_3$, $CH_2CH_2CH_3$, et pyridin-2-ylsulfanyle; et $R_3$ est H;
V est absent,

W est -O-;
Z est nitro; et
$Ar_1$ est phényle facultativement substitué par $R_9$ et $R_{10}$, où $R_9$ est acétyle, 2-méthoxy-éthyle, éthansulfonyle, 4-hydroxy-benzènesulfonyle, 4-cyanophényle, 4-méthoxyphényle, carboxamide, cyclopentyle, 2,5-dioxo-imidazolidinyle, imidazol-1-yle, pyrrolyle, triazol-1-yle, thiadiazol-4-yle, 1,3-dioxo-1,3-dihydro-isoindolyle, 4-oxo-cyclohexyle, acide sulfonique, 2-méthoxycarbonyl-acétyle, benzoyle, ou 3-oxo-butyle; et $R_{10}$ est amino.

**37.** Composé selon la revendication 1 ou 2, où:

U est CH,
X est CH ou C-$NO_2$, et
Y est CH.

**38.** Composé selon la revendication 37, où:

A et B sont tous deux -$CH_2CH_2$-;
D est $CR_2CR_3$, où $R_2$ est sélectionné parmi $CO_2CH_2CH_3$, $CH_2CH_2CH_3$, pyridin-2-ylsulfanyle, $CH_2OCH_3$, et 3-méthyl-1,2,4-oxadidazol-5-yle; et $R_3$ est H;
V est absent,
W est -O-;
Z est nitro; et
$Ar_1$ est phényle facultativement substitué par $R_9$ et $R_{10}$, où $R_9$ est acétyle, vinyle, éthansulfonyle, triazol-1-yle, 2-(3-méthyl-[1,2,4]oxadiazol-5-yl)-acétyle, 5-hydroxy-1-méthyl-1H-pyrazol-3-yle, 5-trifluorométhylpyridin-2-yle, 5-bromo-pyridin-2-yle, 2-méthoxycarbonyl-acétyle, benzoyle, 3-oxo-butyle, 2-carboxy-éthyle,2-carboxy-2-oxo-éthyle, $CH_3(CH_2)_2C(O)$, $CH_3(CH_2)_3C$ (O), ou $CH_3(CH_2)_4C(O)$; et $R_{10}$ est amino.

**39.** Composé selon la revendication 1, sélectionné parmi les composés qui suivent et leurs sels, hydrates et solvates pharmaceutiquement acceptables:

(A1) Ester éthylique de l'acide 6'-[4-(2-méthoxycarbonyl-acétyl)-phénoxy]-3'-nitro-3,4,5,6-tétrahydro-2H-[1,2'] bipyridinyl-4-carboxylique;
(A2) 1-[4-(4-Acétyl-3'-nitro-3,4,5,6-tétrahydro-2H-[1,2']bipyridinyl-6'-yloxy)-phényl]-éthanone;
(A3) Ester éthylique de l'acide 6'-[4-(4-hydroxybenzènesulfonyl)-phénoxy]-3'-nitro-3,4,5,6-tétrahydro-2H-[1,2'] bipyridinyl-4-carboxylique;
(A4) Ester éthylique de l'acide 6'-(4-imidazol-1-yl-phénoxy)-3'-nitro-3,4,5,6-tétrahydro-2H-[1,2']bipyridinyl-4-carboxylique;
(A5) Ester éthylique de l'acide 6'-(4-benzoylphénoxy)-3'-nitro-3,4,5,6-tétrahydro-2H-[1,2']bipyridinyl-4-carboxylique;
(A6) Ester éthylique de l'acide 6'-[4-(2-méthoxyéthyl)-phénoxy]-3'-nitro-3,4,5,6-tétrahydro-2H-[1,2']bipyridinyl-4-carboxylique;
(A7) Ester éthylique de l'acide 6'-(4-cyclopentylphénoxy)-3'-nitro-3,4,5,6-tétrahydro-2H-[1,2']bipyridinyl-4-carboxylique;
(A8) Ester éthylique de l'acide 6'-(4'-cyano-biphényl-4-yloxy)-3'-nitro-3,4,5,6-tétrahydro-2H-[1,2']bipyridinyl-4-carboxylique;
(A9) Ester éthylique de l'acide 3'-nitro-6'-(4-sulfophénoxy)-3,4,5,6-tétrahydro-2H-[1,2']bipyridinyl-4-carboxylique;
(A10) Ester éthylique de l'acide 3'-nitro-6'-(4-pyrrol-1-yl-phénoxy)-3,4,5,6-tétrahydro-2H-[1,2']bipyridinyl-4-carboxylique;
(A11) Ester éthylique de l'acide 6'-(4-carbamoylphénoxy)-3'-nitro-3,4,5,6-tétrahydro-2H-[1,2']bipyridinyl-4-carboxylique;
(A12) Ester éthylique de l'acide 3'-nitro-6'-(4-[1,2,4]triazol-1-yl-phénoxy)-3,4,5,6-tétrahydro-2H-[1,2']bipyridinyl-4-carboxylique;
(A13) Ester éthylique de l'acide 6'-(2-amino-4-éthanesulfonyl-phénoxy)-3'-nitro-3,4,5,6-tétrahydro-2H-[1,2']bipyridinyl-4-carboxylique;
(A14) Ester éthylique de l'acide 3'-nitro-6'-[4-(4-oxo-cyclohexyl)-phénoxy]-3,4,5,6-tétrahydro-2H-[1,2'] bipyridinyl-4-carboxylique;
(A15) Ester éthylique de l'acide 6'-(4'-méthoxybiphényl-4-yloxy)-3'-nitro-3,4,5,6-tétrahydro-2H-[1,2']bipyridinyl-4-carboxylique;

(A16) Ester éthylique de l'acide 3'-nitro-6'-(4-[1,2,3]thiadiazol-4-yl-phénoxy)-3,4,5,6-tétrahydro-2H-[1,2']bipyridinyl-4-carboxylique;
(A17) Ester éthylique de l'acide 6'-[4-(1,3-dioxo-1,3-dihydro-isoindol-2-yl)-phénoxy]-3'-nitro-3,4,5,6-tétrahydro-2H-[1,2']bipyridinyl-4-carboxylique;
(A18) Ester éthylique de l'acide 6'-[4-(2,5-dioxoimidazolidin-4-yl)-phénoxy]-3'-nitro-3,4,5,6-tétrahydro-2H-[1,2'] bipyridinyl-4-carboxylique;
(A19) Ester éthylique de l'acide 3'-nitro-6'-[4-(3-oxo-butyl)-phénoxy]-3,4,5,6-tétrahydro-2H-[1,2']bipyridinyl-4-carboxylique;
(A20) Ester méthylique de l'acide 2-[4-(3'-nitro-4-propyl-3,4,5,6-tétrahydro-2H-[1,2']bipyridinyl-6'-yloxy)-phényl]-3-oxo-propionique;
(A21) 4-[4-(3'-Nitro-4-propyl-3,4,5,6-tétrahydro-2H-[1,2']bipyridinyl-6'-yloxy)-phényl]-butan-2-one;
(A22) 4-{4-[3'-Nitro-4-(pyridin-2-ylsulfanyl)-3,4,5,6-tétrahydro-2H-[1,2']bipyridinyl-6'-yloxy]-phényl}-butan-2-one; et
(A23) 3'-Nitro-4-(pyridin-2-ylsulfanyl)-6'-(4-[1,2,4]triazol-1-yl-phénoxy)-3,4,5,6-tétrahydro-2H-[1,2']bipyridinyle.

**40.** Composé selon la revendication 1, sélectionné parmi les composés qui suivent et leurs sels, hydrates, et solvates pharmaceutiquement acceptables:

(A24) Ester éthylique de l'acide 1-[5-(4-benzoylphénoxy)-2-nitro-phényl]-pipéridine-4-carboxylique;
(A25) Ester éthylique de l'acide 1-{5-[4-(2-méthoxycarbonyl-acétyl)-phénoxy]-2-nitro-phényl}pipéridine-4-carboxylique;
(A26) Ester éthylique de l'acide 1-[5-(2-amino-4-éthanesulfonyl-phénoxy)-2-nitro-phényl]-pipéridine-4-carboxylique;
(A27) Ester éthylique de l'acide 1-{2-nitro-5-[4-(3-oxo-butyl)-phénoxy]-phényl}-pipéridine-4-carboxylique;
(A28) 4-{4-[4-Nitro-3-(4-propyl-pipéridin-1-yl)-phénoxy]-phényl}-butan-2-one;
(A29) 1-{4-[4-Nitro-3-(4-propyl-pipéridin-1-yl)-phénoxy]-phényl}-éthanone;
(A30) Ester méthylique de l'acide 3-{4-[4-Nitro-3-(4-propyl-pipéridin-1-yl)-phénoxy]-phényl}-3-oxo-propionique;
(A31) 5-Ethanesulfonyl-2-[4-nitro-3-(4-propylpipéridin-1-yl)-phénoxy]-phénylamine;
(A32) {4-[4-Nitro-3-(4-propyl-pipéridin-1-yl)-phénoxy]-phényl}-phényl-méthanone;
(A37) Ester éthylique de l'acide 1-{5-[4-(2-carboxyéthyl)-phénoxy]-2-nitro-phényl}-pipéridine-4-carboxylique;
(A38) Ester éthylique de l'acide 1-{5-[4-(2-carboxy-2-oxo-éthyl)-phénoxy]-2-nitro-phényl}-pipéridine-4-carboxylique;
(A39) Ester éthylique de l'acide 1-[2-nitro-5-(4-vinyl-phénoxy)-phényl]-pipéridine-4-carboxylique;
(A40) Acide 3-{4-[4-Nitro-3-(4-propyl-pipéridin-1-yl)-phénoxy]-phényl}-propionique;
(A41) Acide 3-{4-[4-Nitro-3-(4-propyl-pipéridin-1-yl)-phénoxy]-phényl}-2-oxo-propionique;
(A42) 1-[2-Nitro-5-(4-vinyl-phénoxy)-phényl}-4-propylpipéridine;
(A43) 1-{4-[4-Nitro-3-(4-propyl-pipéridin-1-yl)-phénoxy]-phényl}-butan-1-one;
(A44) 1-{4-[4-Nitro-3-(4-propyl-pipéridin-1-yl)-phénoxy]-phényl)-pentan-1-one;
(A45) 1-{4-[4-Nitro-3-(4-propyl-pipéridin-1-yl)-phénoxy]-phényl}-hexan-1-one;
(A46) 4-{4-[3-(4-Méthoxyméthyl-pipéridin-1-yl)-4-nitro-phénoxy]-phényl)-butan-2-one;
(A47) 1-{4-[3-(4-Méthoxyméthyl-pipéridin-1-yl)-4-nitro-phénoxy]-phényl}-éthanone;
(A48) {4-[3-(4-Méthoxyméthyl-pipéridin-1-yl)-4-nitrophénoxy]-phényl}-phényl-méthanone;
(A49) 2-(3-Méthyl-[1,2,4]oxadiazol-5-yl)-1-{4-[4-nitro-3-(4-propyl-pipéridin-1-yl)-phénoxy]-phényl}-éthanone;
(A50) 4-(4-[3-[4-(3-Méthyl-[1,2,4]oxadiazol-5-yl)-pipéridin-1-yl]-4-nitro-phénoxy)-phényl)-butan-2-one;
(A51) 4-(4-{4-Nitro-3-[4-pyridin-2-ylsulfanyl)-pipéridin-1-yl]-phénoxy}-phényl)-butan-2-one;
(A52) 2-{1-[2-Nitro-5-(4-[1,2,4]triazol-1-yl-phénoxy)-phényl]-pipéridin-4-ylsulfanyl}-pyridine;
(A53) 2-Méthyl-5-{4-[4-nitro-3-(4-propyl-pipéridin-1-yl)-phénoxy]-phényl}-2H-pyrazol-3-ol;
(A54) 2-[4-Nitro-3-(4-propyl-pipéridin-1-yl)-phénoxy]-5-trifluorométhyl-pyridine;
(A55) 5-Bromo-2-[4-nitro-3-(4-propyl-pipéridin-1-yl)-phénoxy]-pyridine;
(A56) 1-(4-{4-Nitro-3-[4-(pyridin-2-ylsulfanyl)-pipéridin-1-yl]-phénoxy}-phényl)-éthanone;
(A57) 2-{1-[5-(4-Méthanesulfonyl-phénoxy)-2-nitrophényl]-pipéridin-4-ylsulfanyl}-pyridine;
(A59) 1-{5-[4-(5-Méthyl-[1,3,4]oxadiazol-2-yl)-phénoxy]-2-nitro-phényl}-4-propyl-pipéridine; et
(A60) 1-{5-[3-(3-Méthyl-[1,2,4]oxadiazol-5-yl)-phénoxy]-2-nitro-phényl}-4-propyl-pipéridine.

**41.** Composé selon la revendication 1, sélectionné parmi le composé suivant et ses sels hydrates et solvates pharmaceutiquement acceptables:

(A58) 5-Bromo-1-[4-nitro-3-(4-propyl-pipéridin-1-yl)-phényl]-1H-pyridin-2-one.

**42.** Composé selon la revendication 1, sélectionné parmi les composés suivants et leurs sels, hydrates et solvates pharmaceutiquement acceptables:

(B1) Ester éthylique de l'acide 6'-benzènesulfonylamino-3'-nitro-3,4,5,6-tétrahydro-2H-[1,2']bipyridinyl-4-carboxylique;
(B2) Ester éthylique de l'acide 6'(benzènesulfonylméthyl-amino)-3'-nitro-3,4,5,6-tétrahydro-2H-[1,2']bipyridinyl-4-carboxylique;
(B3) Ester éthylique de l'acide 6'(benzènesulfonylbutyl-amino)-3'-nitro-3,4,5,6-tétrahydro-2H-[1,2']bipyridinyl-4-carboxylique;
(B4) Ester éthylique de l'acide 6'-(5-éthanesulfonyl-2-hydroxy-phénylamino)-3'-nitro-3,4,5,6-tétrahydro-2H-[1,2']bipyridinyl-4-carboxylique;
(B5) Ester éthylique de l'acide 6'-(2-bromo-4-trifluorométhyl-benzènesulfonylamino)-3'-nitro-3,4,5,6-tétrahydro-2H-[1,2']bipyridinyl-4-carboxylique;
(B6){4-[3'-Nitro-4-(pyridin-2-ylsulfanyl)-3,4,5,6-tétrahydro-2H-[1,2']bipyridinyl-6'-ylamino]-phényl}-phényl}-phényl-méthanone; et
(B7) [3'-Nitro-4-(pyridin-2-ylsulfanyl)-3,4,5,6-tétrahydro-2H-[1,2']bipyridinyl-6'-yl]-(4-[1,2,4]triazol-1-yl-phényl)-amine.

**43.** Composé selon la revendication 1, sélectionné parmi les composés qui suivent et leurs sels, hydrates et solvates pharmaceutiquement acceptables:

(B8) Ester éthylique de l'acide 1-[5-(4-benzoylphénylamino)-2-nitro-phényl]-pipéridine-4-carboxylique; et
(B9) {4-[4-Nitro-3-(4-propyl-pipéridin-1-yl)-phénylamino]-phényl}-phényl-méthanone.

**44.** Composition pharmaceutique comprenant au moins un composé selon l'une quelconque des revendications 1 à 43 en combinaison avec un support pharmaceutiquement acceptable.

**45.** Méthode de production d'une composition pharmaceutique comprenant le mélange d'au moins un composé selon l'une quelconque des revendications 1 à 43 et d'un support pharmaceutiquement acceptable.

**46.** Composé selon l'une quelconque des revendications 1 à 43 à utiliser dans une méthode de traitement du corps humain ou animal par thérapie.

**47.** Composé selon l'une quelconque des revendications 1 à 43 à utiliser dans une méthode de prophylaxie ou de traitement d'un trouble métabolique.

**48.** Utilisation d'un composé selon l'une quelconque des revendications 1 à 43 pour la production d'un médicament à utiliser dans la prophylaxie ou le traitement d'un trouble métabolique.

**49.** Composé selon l'une quelconque des revendications 1 à 43 à utiliser dans une méthode de traitement ou de prophylaxie du diabète type II, d'une tolérance inadéquate au glucose, d'une résistance à l'insuline, d'une hyperglycémie, d'une hyperlipidémie, d'une hypertriglycéridémie, d'une hypercholestérolémie, d'une dyslipidémie, du syndrome X, ou d'un syndrome métabolique.

**50.** Utilisation d'un composé selon l'une quelconque des revendications 1 à 43 pour la production d'un médicament à utiliser dans la prophylaxie ou le traitement du diabète type II, d'une tolérance inadéquate au glucose, d'une résistance à l'insuline, d'une hyperglycémie, d'une hyperlipidémie, d'une hypertriglycéridémie, d'une hypercholestérolémie, d'une dyslipidémie, d'un syndrome X, ou d'un syndrome métabolique.

**51.** Composé selon l'une quelconque des revendications 1 à 43 à utiliser dans une méthode de traitement ou de prophylaxie du diabète type II.

**52.** Utilisation d'un composé selon l'une quelconque des revendications 1 à 43 pour la production d'un médicament à utiliser dans la prophylaxie ou le traitement du diabète type II.

**53.** Composé selon l'une quelconque des revendications 1 à 43 à utiliser dans une méthode de contrôle ou de diminution de la prise de poids chez un individu.

**54.** Utilisation d'un composé selon l'une quelconque des revendications 1 à 43 pour la production d'un médicament à utiliser dans le contrôle ou la diminution de la prise de poids chez un individu.

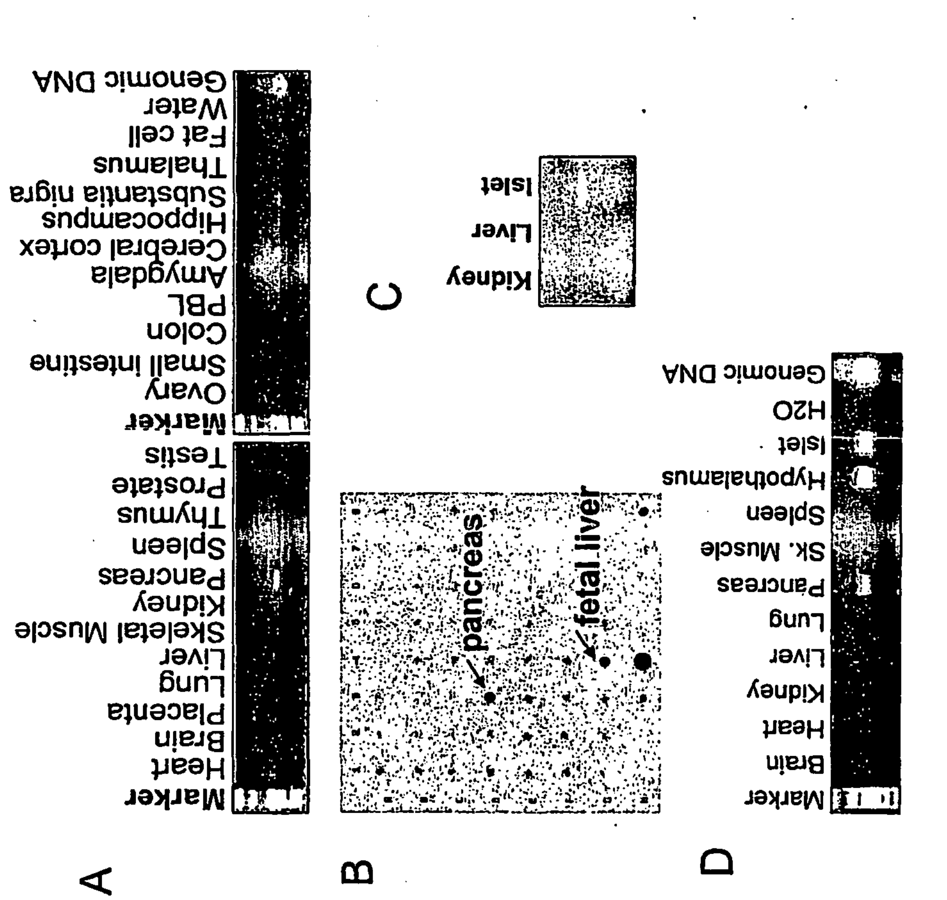
A
Marker
Heart
Brain
Placenta
Lung
Liver
Skeletal Muscle
Kidney
Pancreas
Spleen
Thymus
Prostate
Testis
Marker
Ovary
Small intestine
Colon
PBL
Amygdala
Cerebral cortex
Hippocampus
Substantia nigra
Thalamus
Fat cell
Water
Genomic DNA
B
pancreas
fetal liver
C
Kidney
Liver
Islet
D
Marker
Brain
Heart
Kidney
Liver
Lung
Pancreas
SK. Muscle
Spleen
Hypothalamus
Islet
H2O
Genomic DNA

FIGURE 1

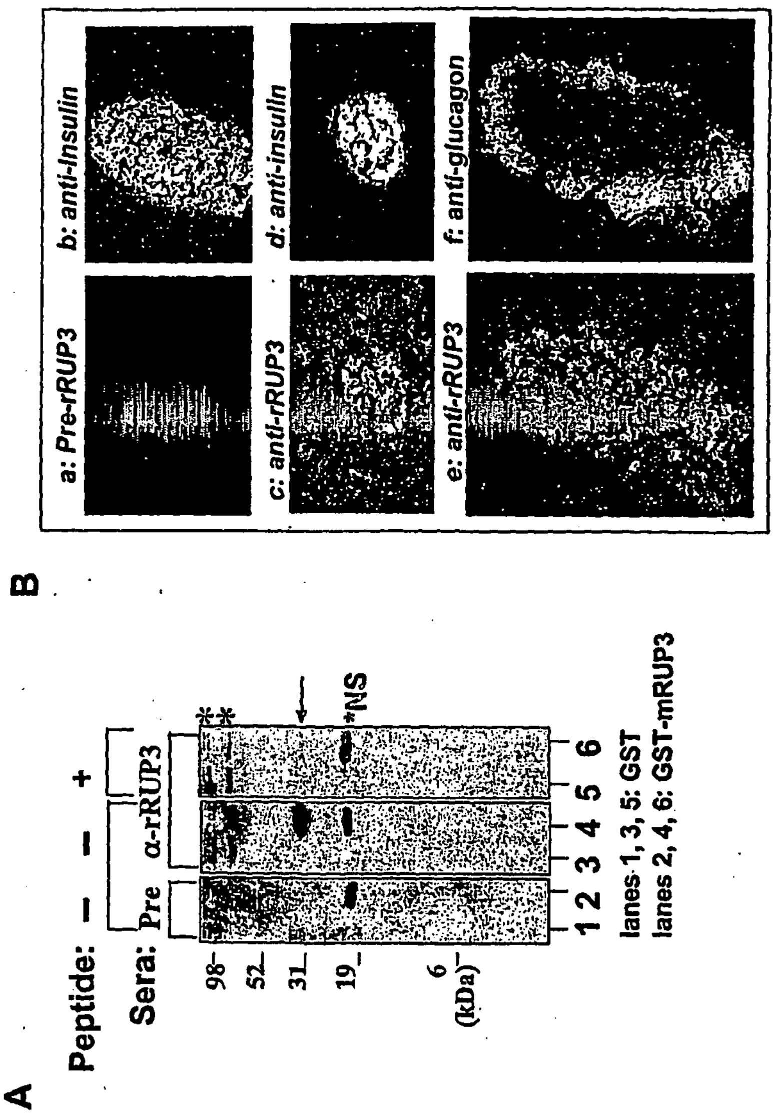
A
Peptide: – – +
Sera: Pre α-rRUP3
98
52
31
19
6
(kDa)
*
*
*NS
1 2 3 4 5 6
lanes 1, 3, 5: GST
lanes 2, 4, 6: GST-mRUP3
B
a: Pre-rRUP3
b: anti-Insulin
c: anti-rRUP3
d: anti-insulin
e: anti-rRUP3
f: anti-glucagon

FIGURE 2

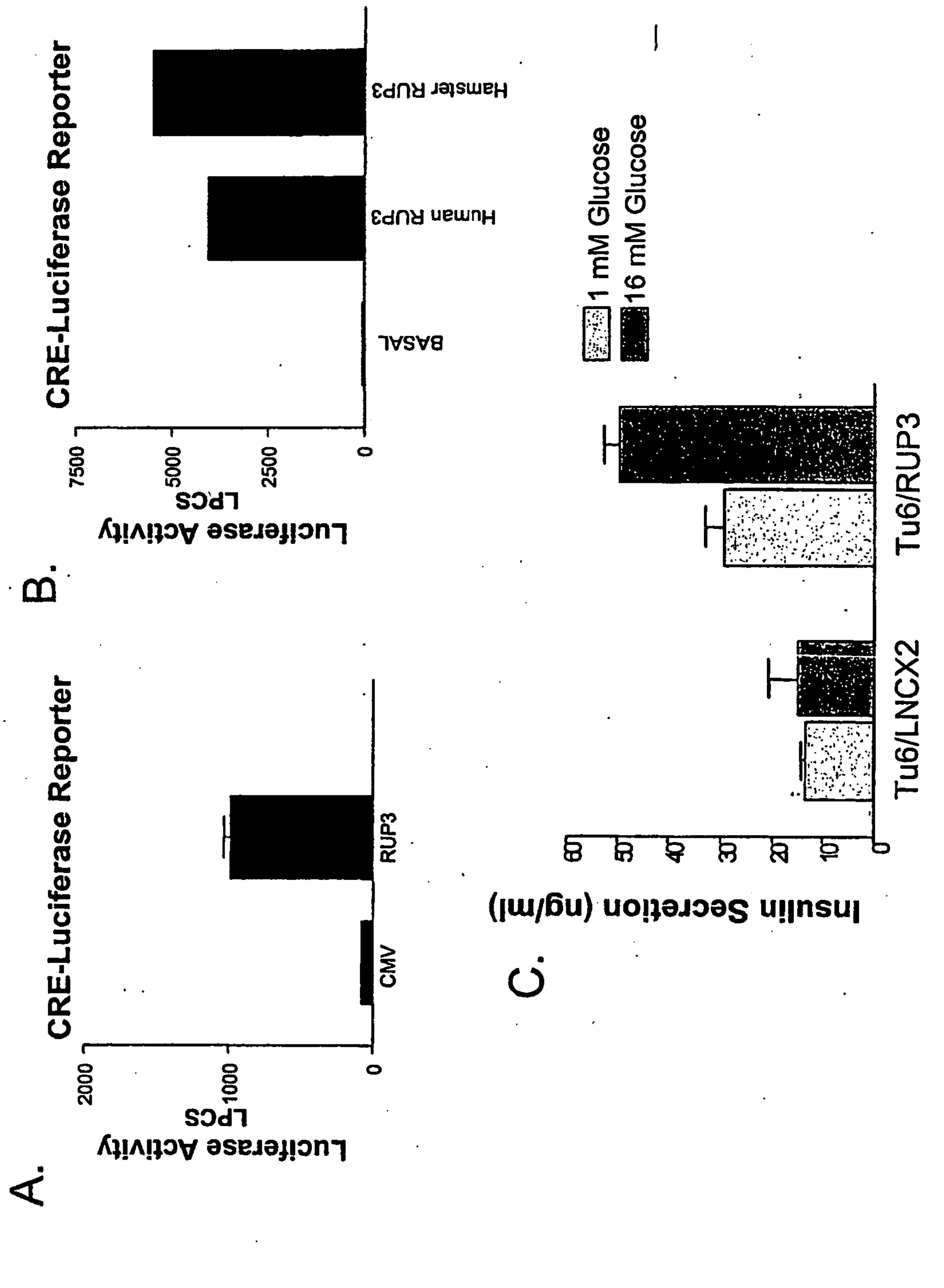
A.
CRE-Luciferase Reporter
Luciferase Activity LPCS
2000
1000
0
CMV
RUP3
B.
CRE-Luciferase Reporter
Luciferase Activity LPCS
7500
5000
2500
0
BASAL
Human RUP3
Hamster RUP3
C.
Insulin Secretion (ng/ml)
60
50
40
30
20
10
0
Tu6/LNCX2
Tu6/RUP3
1 mM Glucose
16 mM Glucose

FIGURE 3

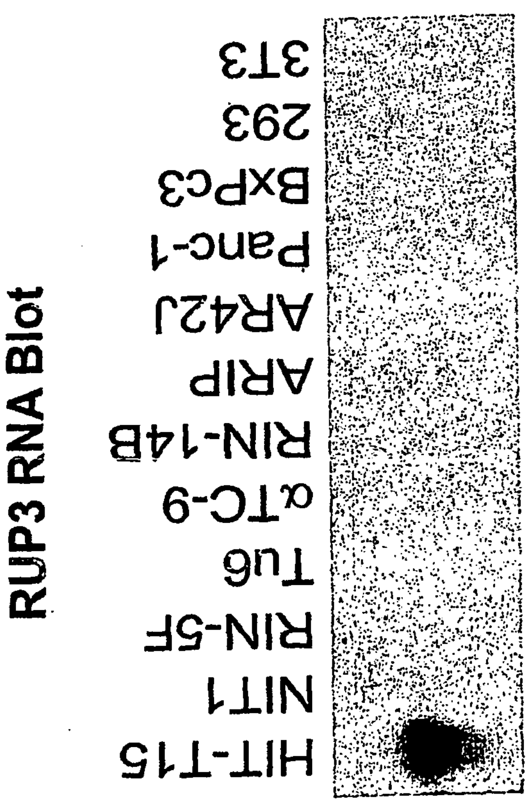
RUP3 RNA Blot
HIT-T15
NIT1
RIN-5F
Tu6
αTC-9
RIN-14B
ARIP
AR42J
Panc-1
BxPc3
293
3T3

FIGURE 4

## Representative Syntheses

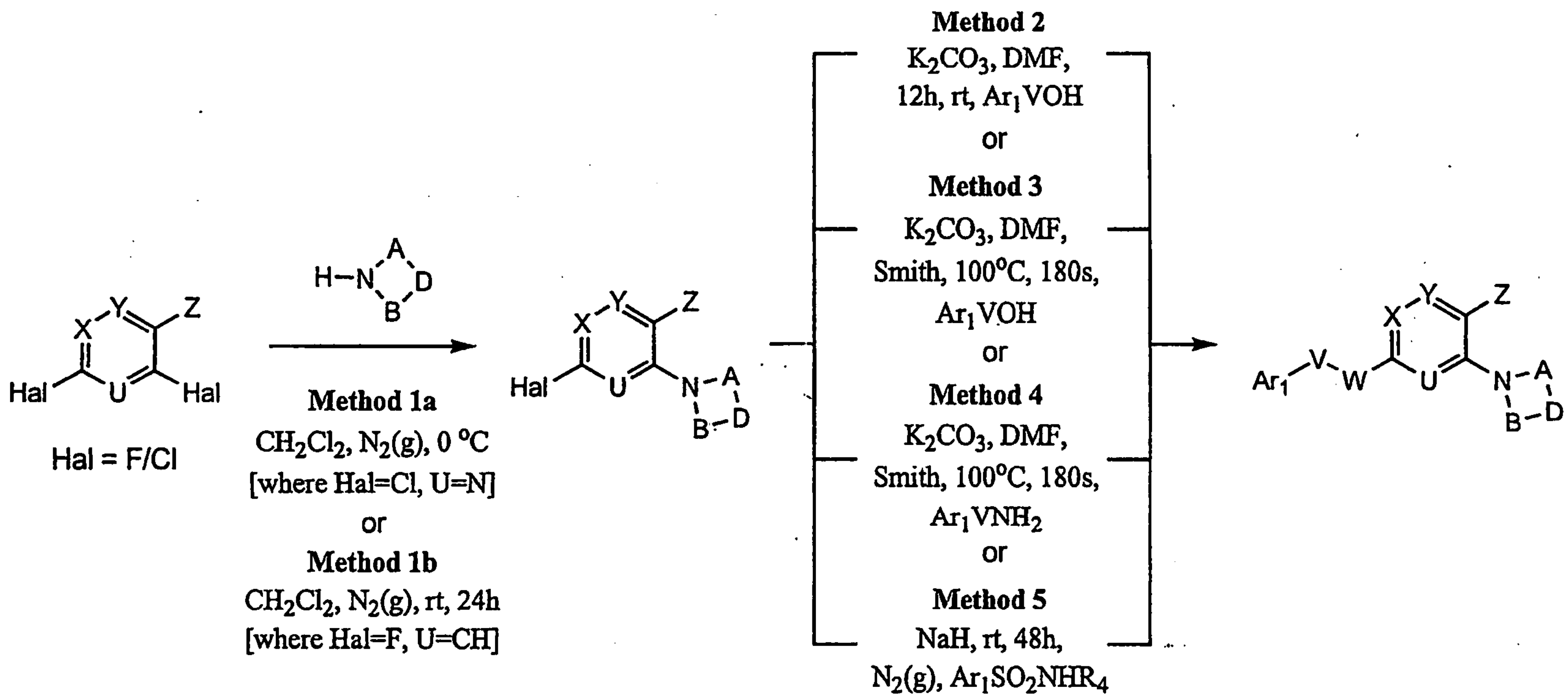


**Figure 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0857483 A
- EP 0604800 A
- US 6239126 B
- WO 0035875 A
- US 60449788 B


### Non-patent literature cited in the description


- **ROTWEIN, R et al.** *N. Engl. J. Med.,* 1983, vol. 308, 65-71
- **RIMOIN, D. L. et al.** Emery and Rimoin's Principles and Practice of Medical Genetics. 1996, vol. 1, 1401-1402
- **LE STUNFF et al.** *Diabetes,* 1989, vol. 43, 696-702
- **PEDERSON, P.** *Diab. Metab. Rev.,* 1989, vol. 5, 505-509
- **BRANCATI, F. L. et al.** *Arch. Intern. Med.,* 1999, vol. 159, 957-963
- **HILL, J. O. et al.** *Science,* 1998, vol. 280, 1371-1374
- **PERRY, I. J. et al.** *BMJ,* 1995, vol. 310, 560-564
- Remington's Pharmaceutical Sciences. Mack Publishing Company, 1985, 1418
- *Journal of Pharmaceutical Science,* 1977, vol. 66, 2
- Remington's Pharmaceutical Sciences. Mack Publishing Co, 1980
- **T. HIGUCHI ; V. STELLA.** Pro-drugs as Novel Delivery Systems. A.C.S. Symposium Series, vol. 14
- Bioreversible Carriers in Drug Design. American Pharmaceutical Association and Pergamon Press, 1987
- **ZHU, D.-G.** *J. Org. Chem.,* 2002, vol. 67, 943-948
- **COLLIER, T. L.** *J. Labelled Compd Radiopharm.,* 1999, vol. 42, S264-S266
- **BAS, M.-D.** *J. Labelled Compd Radiopharm.,* 2001, vol. 44, S280-S282